# EUROPEAN PATENT APPLICATION

(11) **EP 3 943 496 A1**
(43) Date of publication of application: **26.01.2022**
(21) Application number: 20779433.0
(22) Date of filing: 20.03.2020
(51) Int. Cl.: C07D 487/04, A61K 31/519, A61K 31/5377, A61K 31/541, A61P 35/00

(54) **WEE1 INHIBITOR AND PREPARATION AND USE THEREOF**

(30) Priority: 22.03.2019 CN 201910225653; 14.10.2019 CN 201910972247
(71) Applicant: Shouyao Holdings (Beijing) Co., Ltd., Beijing 100176 (CN)
(72) Inventor: ZHU, Yan, Beijing 100195 (CN); YAO, Jinsuo, Beijing 100195 (CN); WANG, Yeliu, Beijing 100195 (CN); YAN, Qin, Beijing 100195 (CN); HE, Weinan, Beijing 100195 (CN); CHEN, Changjun, Beijing 100195 (CN); SHANG, Xianxing, Beijing 100195 (CN); LI, Jijun, Beijing 100195 (CN); SUN, Yinghui, Beijing 100195 (CN); LI, Hongjuan, Beijing 100195 (CN); LU, Chang, Beijing 100195 (CN); ZHANG, Jiuqing, Beijing 100195 (CN); HOU, Deng, Beijing 100195 (CN); ZHANG, Xiaojun, Beijing 100195 (CN); LIU, Qichao, Beijing 100195 (CN)
(74) Representative: Hoffmann Eitle
(86) International application number: PCT/CN2020/080401
(87) International publication number: WO 2020/192581

(57) **Abstract**

The present invention relates to a WEE1 inhibitor, and the preparation and use thereof. The WEE1 inhibitor of the present invention is a compound of Formula I or a compound of Formula 11, or a pharmaceutically acceptable salt, solvate, polymorph or tautomer thereof. The present invention also provides use of the WEE1 inhibitor in the manufacture of a medicament for the treatment of a disease associated with WEE1 activity. The WEE1 inhibitor provided by the present invention can effectively treat the disease associated with WEE1 activity.

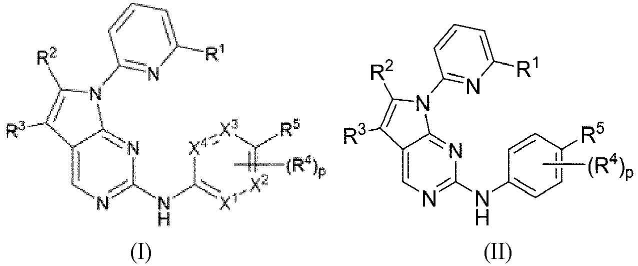

## Description

### Cross Reference

This application claims priority to Chinese Patent Application No. 201910225653.2, filed on March 22, 2019, entitled "WEE1 Inhibitor and Preparation and Use Thereof", and to Chinese Patent Application No. 201910972247.2, filed on October 14, 2019, entitled "WEE1 Inhibitor and Preparation and Use Thereof", the entire disclosures of which are incorporated herein by reference in their entirety.

### Technical Field

The present invention relates to compounds for inhibiting WEE1 kinase activity, and also relates to a preparation method of the compounds and a pharmaceutical composition and use thereof.

### Background Art

WEE1 tyrosine kinase is required for activation of G2 phase checkpoint of the cell cycle. The cell cycle is tightly regulated, and when the DNA of the cell is undamaged, G1-checkpoint, S-checkpoint, and G2-checkpoint promote entry into mitosis to ensure the cell-cycle progression. (Clinical Cancer Research, 2011, 17 (13): 4200-4207.) Cyclin-dependent kinases (CDKs) that are families of 14 serine/threonine kinases play a very important role in cell cycle regulation. The activity of CDKs is regulated by phosphorylation and binding to different cyclin proteins. The transition of cells from G2 phase to mitosis is positively regulated by the phosphorylation of CDK1 (also known as CDC2) and its related cyclin B. CDK1 is kept inactive before cell mitosis, tyrosine 15 of which is phosphorylated by WEE1, and threonine 14 of which is then phosphorylated by myelin transcription factor (MYT1). Thus, as a negative regulator of the cell cycle, WEE1 negatively regulates cells from G2 phase to mitosis by preventing cyclin B and activated CDK1 complexes from entering into the nucleus. Both the expression and activity of WEE1 increase in S phase and G2 phase, and decrease in M phase of hyperphosphorylation. When cells enter G2 phase and no DNA damage occurs, polo-like protein kinase 1 (PLK1) phosphorylates WEE1, which is degraded by the ubiquitin ligase complex. PLK1 also phosphorylates and activates protein phosphatase cell division cycle 25 analogs (CDC25), which activates CDK1 through dephosphorylation. Active CDK1 may bind to cyclin B and promote entry into mitosis. (Molecular & Cellular Biology, 2012, 32 (20): 4226.)

G1-checkpoint, S-checkpoint, and G2-checkpoint delay entry into mitosis in the presence of DNA damage, and ensure genomic integrity by repairing damaged DNA before cell-cycle progression. The P53 tumor suppressor is a key regulator for G1 checkpoint and is present in a mutant form in many malignant tumor cells. *(*Proceedings of the National Academy of Sciences of the United States of America, 2007, 104 (10): 3753-3758.) P53-deficient tumor cells fail to induce cell-cycle arrest at G1 phase in the presence of DNA damage and therefore are more dependent on the G2 checkpoint. In response to DNA damage, the G2 checkpoint inhibits phosphorylation of CDK1 through two parallel and interrelated pathways, thereby delaying entry into mitosis. Depending on the type of DNA damage, ataxia telangiectasia mutated (ATM) or ataxia telangiectasia-related (ATR) protein kinases are activated. (Oncotarget, 2016, 7 (31): 49902-49916.)

ATM is activated by ionizing radiation, radioactive agents, and agents that cause the cleavage of double-stranded DNA. ATM phosphorylates and activates checkpoint kinase 2 (CHK2), which phosphorylates Ser216 of cell division cycle 25C phosphatase (CDC25C). This results in the nuclear export of CDC25C and cytoplasmic segregation, thereby inhibiting its phosphorylation activity. Inhibition of CDC25C activity results in the inhibitory phosphorylation of CDK1/CDK2-cyclin B complex, rendering CDK1 in an inactive form, inhibiting cell entry into mitosis. *(*Molecular Cancer, 2014, 13 (1): 72.)

ATR which is activated by a wide range of genotoxic stimulators leading to single-stranded DNA breakage is the major kinase responsible for phosphorylation and activation of CHK1. CHK1 can be activated by ATM and ATR compared to CHK2 being only activated by ATM. CHK1 simultaneously phosphorylates WEE1 and CDC25C, activates WEE1 kinase activity and inhibits phosphatase activity of CDC25C. WEE1 phosphorylates CDK1-cyclin B, leading to cell cycle arrest in G2, providing time for DNA repair. (Drug News & Perspectives, 2010, 23 (7): 425.)

WEE1 is overexpressed in many malignancies, such as liver cancer, breast cancer, glioblastoma, melanoma, adult and pediatric brain tumors. Some of the tumor cells display abnormal G1 checkpoints, so that the inhibition of WEE1 activity will lead to impaired G2 checkpoint, at the moment, the division of cells with unrepaired damaged DNA continues, resulting in apoptosis. *(*Molecular Cancer Therapeutics, 2013, 12 (12): 2675-2684.) Inhibition of WEE1 activity, whether by pyrimidine derivatives (PD0166285) or small interference RNA knockdown, makes ovarian, colon, cervical, osteosarcoma, glioblastoma, and lung cancer cells more sensitive to DNA damage resulting from radiation and topoisomerase inhibition. Therefore, both single WEE1 inhibitor and combination therapy have broad development space. *(*Cancer Biology & Therapy, 2010, 9 (7): 523-525.)

Small molecule compounds having WEE1 kinase inhibitory activity are described in patents WO2007126122, WO2008133866, WO2013012681, WO2013126656, WO2014167347, WO2015092431, WO2018011569, WO2018011570, WO2018090939, WO2018133829, and WO2018171633. The currently leading compound, AZD 1775, has entered a phase 11 clinical trial showing good cancer therapeutic efficacy.

### Summary of the Invention

In one aspect, the invention provides a compound of Formula 1, or a pharmaceutically acceptable salt, solvate, polymorph or tautomer thereof, 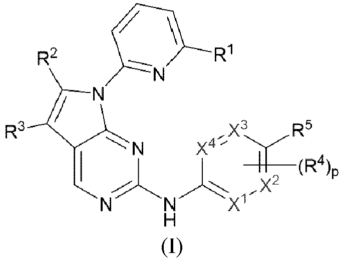 wherein,
X¹, X², X³ and X⁴ are independently selected from N and C-R⁴;
R¹ is selected from C₁₋₆alkyl, -O-C₁₋₆alkyl, 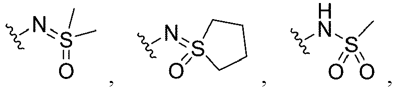 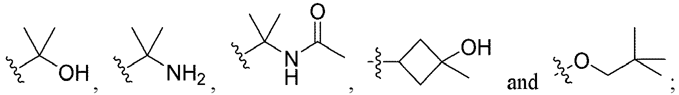
R² is selected from H, C₁₋₆alkyl, C₃₋₈cycloalkyl, C₂₋₆alkenyl, allyl, -(CH₂)ₙ-OH and -(CH₂)ₙ-O-C₁₋₆alkyl;
R³ is selected from H, halogen and C₁₋₆alkyl;
R⁴ is selected from H, halogen, -CF₃, C₁₋₆alkyl and -O-C₁₋₆alkyl;
R⁵ is selected from -(CH₂)ₘ-NR^{a}R^{b}, -(CH₂)-(CO)-N(CH₃)₂ and 3-10 membered heterocycle, said 3-10 membered heterocycle can be optionally substituted with 1-3 R⁶; or
R⁴ and R⁵ joins together to form a 3-8 membered heterocycle, said 3-8 membered heterocycle can be optionally substituted with C₁₋₆alkyl;
R⁶ is selected from C₁₋₆alkyl, C₃₋₈cycloalkyl, -(CO)-NH₂, -CH₂(CO)OH, -CH₂(CO)OCH₃, -CH2CN, -CH2(CO)NHOH, -(CH₂)₂-NR^{c}R^{d}, -NR^{c}R^{d}, -(CH2)n-OH, -(CO)-O-C₁₋₆alkyl, and 3-8 membered heterocycle, said 3-8 membered heterocycle can be optionally substituted with C₁₋₆alkyl or C₃₋₈cycloalkyl;
R^{a} and R^{b} are independently selected from H and C₁₋₆alkyl, said alkyl can be optionally substituted with -NR^{c}R^{d};
R^{c} and R^{d} are independently selected from H and C₁₋₆alkyl;
m is 0, 1, 2,3, or 4;
n is independently 1, 2,3, or 4;
p is 1 or 2.

In some embodiments, R¹ is selected from

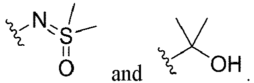

In some embodiments, R² is selected from C₁₋₆alkyl and C₃₋₈cycloalkyl, preferably methyl and cyclopropyl.

In some embodiments, R³ is selected from H and halogen, preferably H and fluoro, more preferably H.

In some embodiments, R⁴ is selected from H and halogen, preferably H and fluoro.

R⁵ is selected from -(CH₂)ₘ-NR^{a}R^{b}, -(CH₂)-(CO)-N(CH₃)₂ and 3-8 membered heterocycle, said 3-8 membered heterocycle can be optionally substituted with 1-3 R⁶; R⁶ is selected from C₁₋₆alkyl, -NR^{c}R^{d}, -(CH₂)ₙ-OH, -(CO)-O-C₁₋₆alkyl, and 3-8 membered heterocycle, said 3-8 membered heterocycle can be optionally substituted with C₁₋₆alkyl.

In some embodiments, R⁵ is selected from 3-8 membered heterocycle, said 3-8 membered heterocycle can be optionally substituted with 1-3 R⁶, R⁶ is selected from C₁₋₆alkyl, -NR^{c}R^{d}, -(CH₂)ₙ-OH, -(CO)-O-C₁₋₆alkyl, and 3-8 membered heterocycle, said 3-8 membered heterocycle can be optionally substituted with C₁₋₆alkyl.

In some embodiments, R⁵ is selected from 3-8 membered heterocycle, said 3-8 membered heterocycle can be optionally substituted with 1-3 R⁶, R⁶ is selected from 3-8 membered heterocycle, and R⁶ can be optionally substituted with C₁₋₆alkyl.

In some embodiments, R^{a} and R^{b} are independently selected from C₁₋₆alkyl, said alkyl can be optionally substituted with -NR^{c}R^{d}, R^{c} and R^{d} are independently selected from H and C₁₋₆alkyl.

In some embodiments, R^{a} and R^{b} are independently selected from C₁₋₆alkyl, said alkyl can be optionally substituted with -NR^{c}R^{d}, R^{c} and R^{d} are independently selected from C₁₋₆alkyl.

In some embodiments, R⁵ is selected from 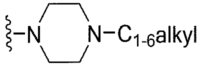 and

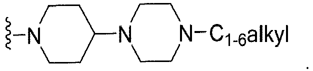

In some embodiments, m is 0, 1, or 2.

In some embodiments, n is independently 1 or 2.

In some embodiments, the compounds of the invention are selected from: 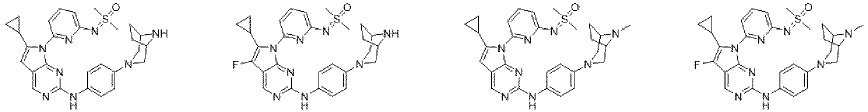 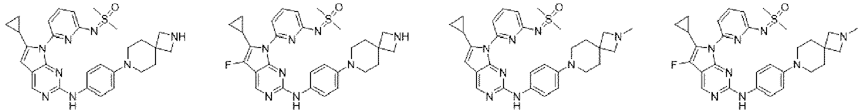 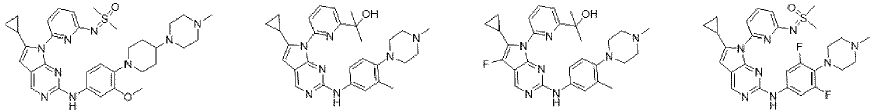 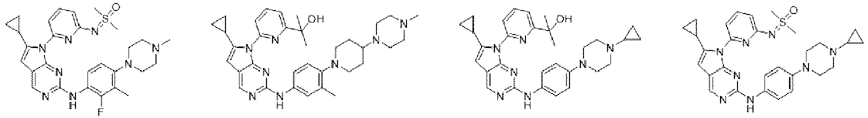 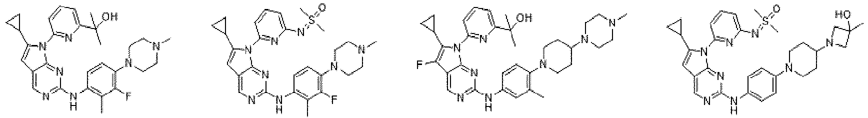 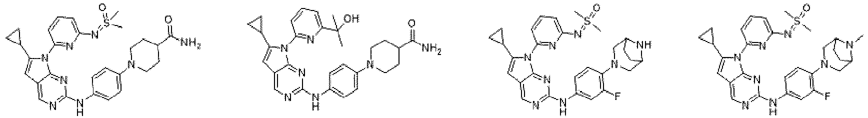 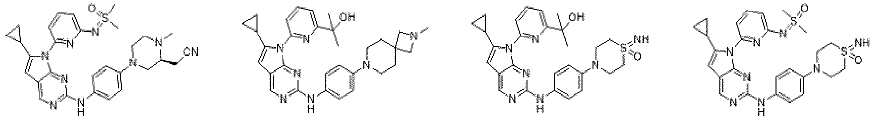 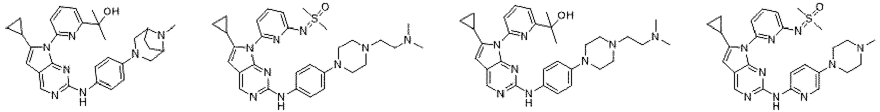 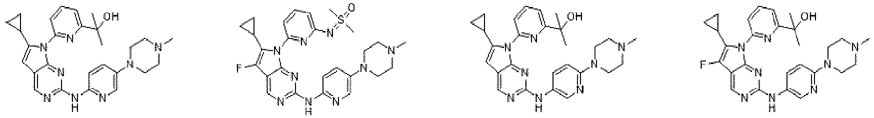 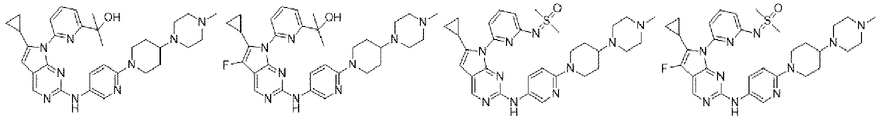 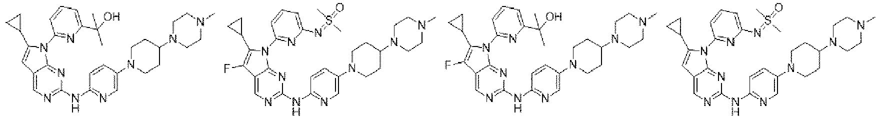 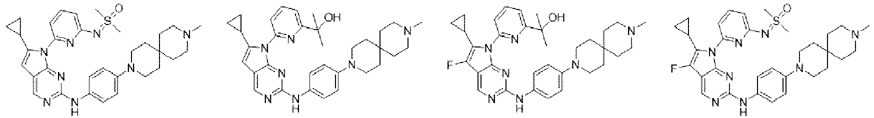 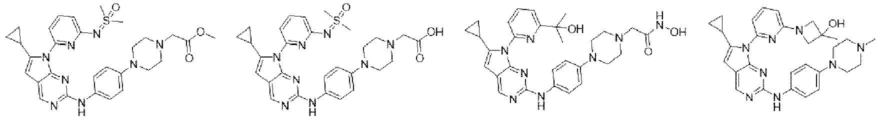 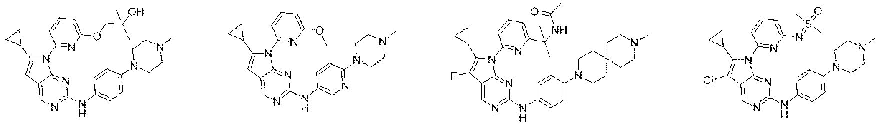 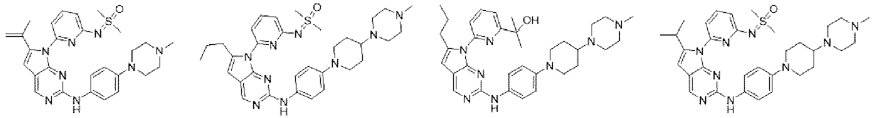 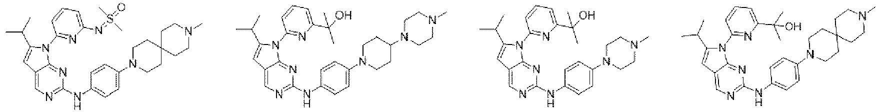 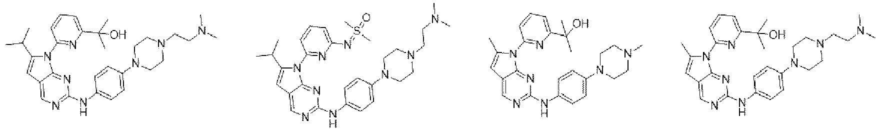 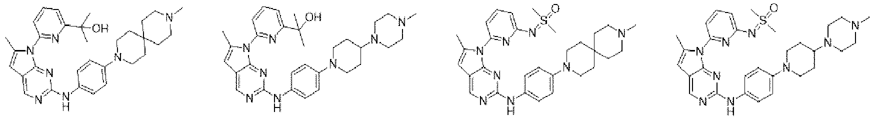 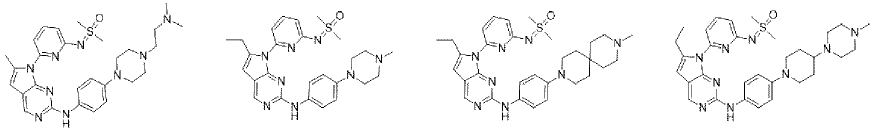 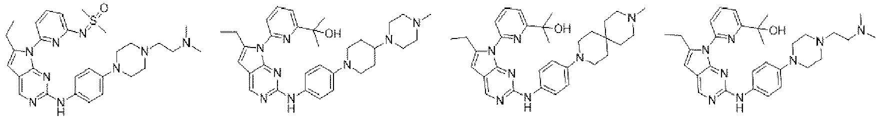 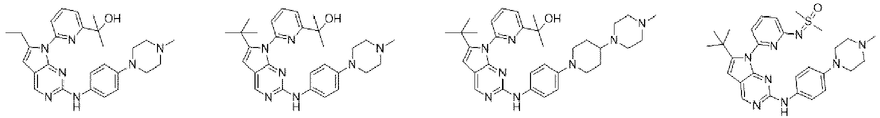 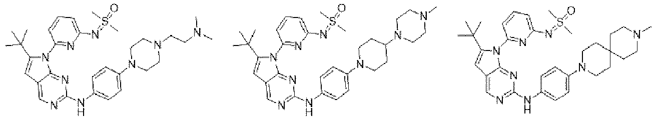 or a pharmaceutically acceptable salt, solvate, polymorph or tautomer thereof.

In another aspect, the invention provides a compound of Formula 11, or a pharmaceutically acceptable salt, solvate, polymorph or tautomer thereof, 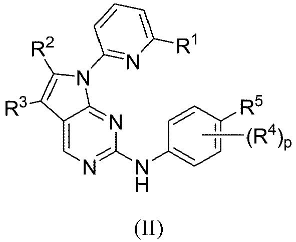 wherein,
R¹ is selected from C₁₋₆alkyl, 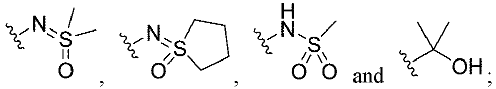
R² is selected from H, C₁₋₆alkyl, C₃₋₈cycloalkyl, allyl, -(CH₂)ₙ-OH and -(CH₂)ₙ-O-C₁₋₆alkyl;
R³ is selected from H, halogen and C₁₋₆alkyl;
R⁴ is selected from H, halogen, CF₃, C₁₋₆alkyl and -O-C₁₋₆alkyl;
R⁵ is selected from -(CH₂)ₘ-NR^{a}R^{b}, -(CH₂)-(CO)-N(CH₃)₂ and 3-8 membered heterocycle, said 3-8 membered heterocycle can be optionally substituted with 1-3 R⁶; or
R⁴ and R⁵ joins together to form a 3-8 membered heterocycle, said 3-8 membered heterocycle can be optionally substituted with C₁₋₆alkyl;
R⁶ is selected from C₁₋₆alkyl, C₃₋₈cycloalkyl, -NR^{c}R^{d}, -(CH₂)ₙ-OH, -(CO)-O-C₁₋₆alkyl, and 3-8 membered heterocycle, said 3-8 membered heterocycle can be optionally substituted with C₁₋₆alkyl or C₃₋₈cycloalkyl;
R^{a} and R^{b} are independently selected from H and C₁₋₆alkyl, said alkyl can be optionally substituted with -NR^{c}R^{d};
R^{c} and R^{d} are independently selected from H and C₁₋₆alkyl;
m is 0, 1, 2,3, or 4;
n is independently 1, 2,3, or 4;
p is 1 or 2.

In some embodiments, R¹ is selected from

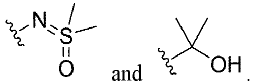

In some embodiments, R² is selected from C₁₋₆alkyl and C₃₋₈cycloalkyl, preferably C₃₋₈cycloalkyl, more preferably cyclopropyl.

In some embodiments, R³ is selected from H and halogen, preferably H and fluoro.

In some embodiments, R⁴ is selected from H and halogen, preferably H and fluoro.

In some embodiments, R⁵ is selected from 3-8 membered heterocycle, said 3-8 membered heterocycle can be optionally substituted with 1-3 R⁶,

R⁶ is selected from C₁₋₆alkyl, -NR^{c}R^{d}, -(CH₂)ₙ-OH, -(CO)-O-C₁₋₆alkyl, and 3-8 membered heterocycle, said 3-8 membered heterocycle can be optionally substituted with C₁₋₆alkyl.

In some embodiments, R⁵ is selected from 3-8 membered heterocycle, said 3-8 membered heterocycle can be optionally substituted with 1-3 R⁶,

R⁶ is selected from 3-8 membered heterocycle, and R⁶ can be optionally substituted with C₁₋₆alkyl.

In some embodiments, R^{a} and R^{b} are independently selected from C₁₋₆alkyl, said alkyl can be optionally substituted with -NR^{c}R^{d}, R^{c} and R^{d} are independently selected from H and C₁₋₆alkyl.

In some embodiments, R^{a} and R^{b} are independently selected from C₁₋₆alkyl, said alkyl can be optionally substituted with -NR^{c}R^{d}, R^{c} and R^{d} are independently selected from C₁₋₆alkyl.

In some embodiments, R⁵ is selected from 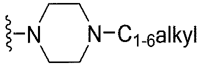 and

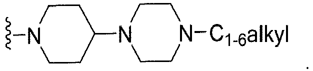

In some embodiments, m is 0, 1, or 2.

In some embodiments, n is independently 1 or 2.

In some embodiments, the compounds of the invention are selected from: 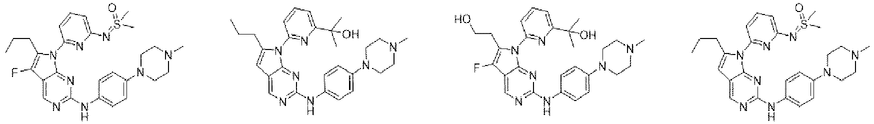 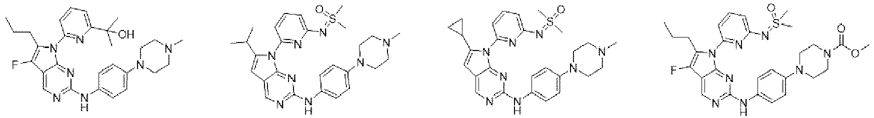 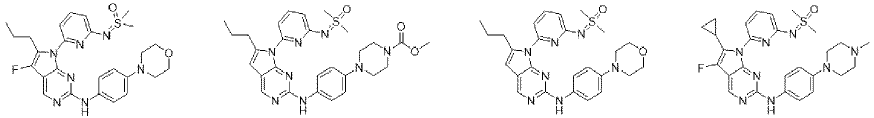 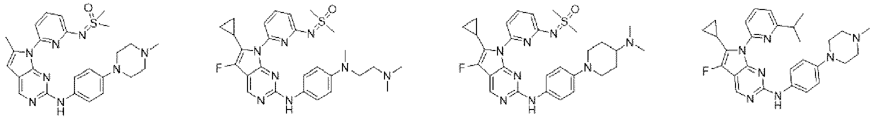 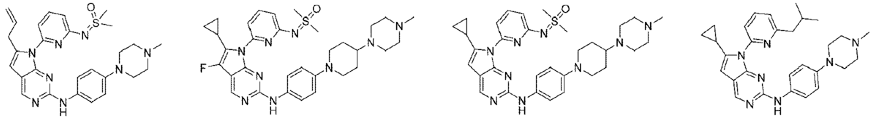 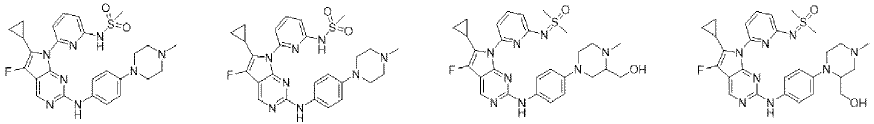 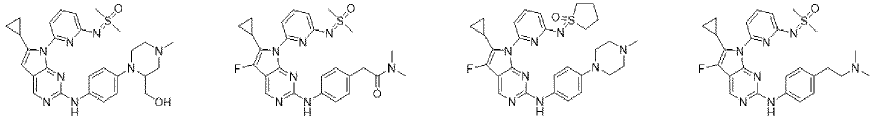 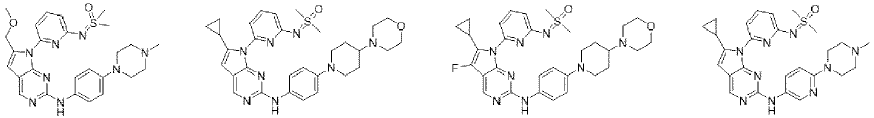 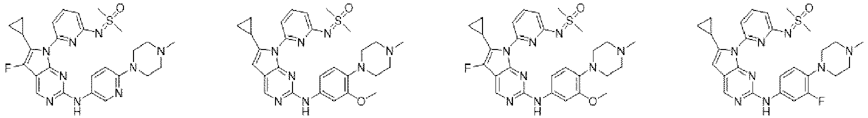 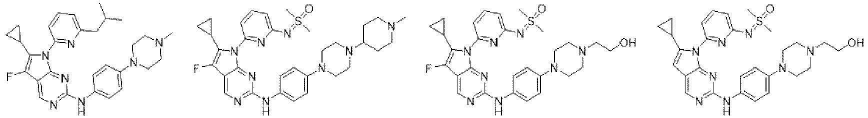 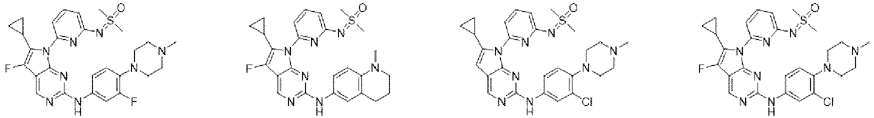 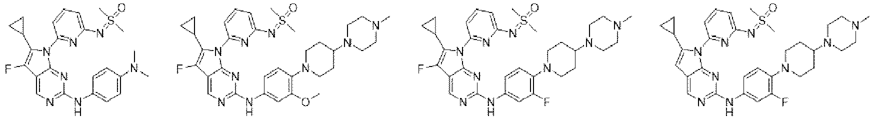 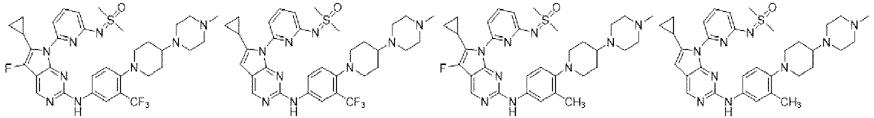 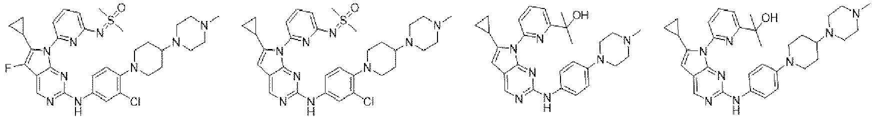 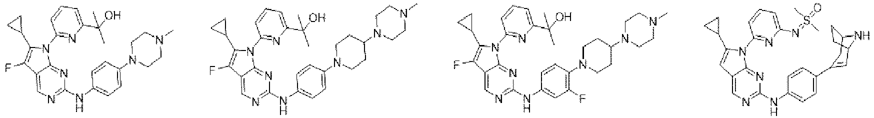 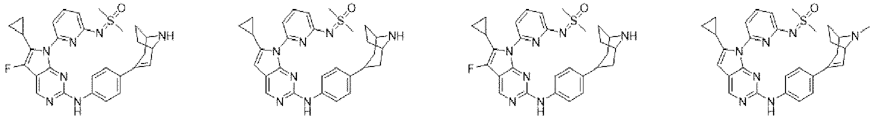 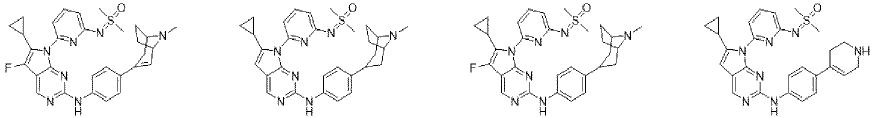 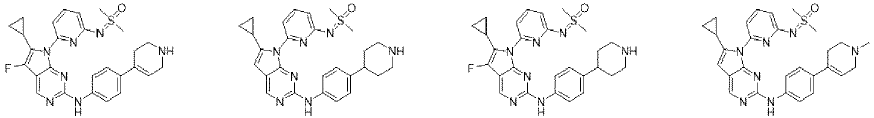 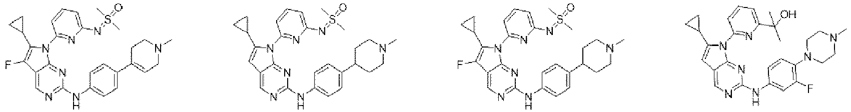 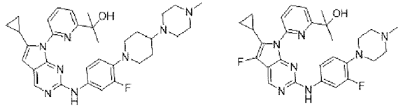 or a pharmaceutically acceptable salt, solvate, polymorph or tautomer thereof.

The compound of Formula 1 and Formula 11 of the present invention can be used to treat diseases associated with WEE1 activity; in some embodiments, the disease associated with WEE1 activity is liver cancer, breast cancer, glioblastoma, melanoma, adult brain tumor, pediatric brain tumor, ovarian cancer, colon cancer, cervical cancer, osteosarcoma, lung cancer, gastric cancer, head and neck cancer, or leukemia.

Another aspect of the present invention also relates to a pharmaceutical composition comprising a compound of Formula I of the present invention, or a pharmaceutically acceptable salt, solvate, polymorph or tautomer thereof, and/or a compound of Formula 11, or a pharmaceutically acceptable salt, solvate, polymorph or tautomer thereof, and a pharmaceutically acceptable carrier.

The WEE1 inhibitor of the present invention may be a compound of Formula I or Formula II, or a pharmaceutically acceptable salt, solvate, polymorph or tautomer thereof, or a pharmaceutical composition as described above.

In another aspect, the present invention provides a method for the treatment of diseases associated with WEE1 activity comprising administering to a subject an effective amount of a compound of Formula I, or a pharmaceutically acceptable salt, solvate, polymorph, or tautomer thereof, and/or a compound of Formula 11, or a pharmaceutically acceptable salt, solvate, polymorph, or tautomer thereof, or a composition as described above; in some embodiments, the disease associated with WEE1 activity is liver cancer, breast cancer, glioblastoma, melanoma, adult brain tumor, pediatric brain tumor, ovarian cancer, colon cancer, cervical cancer, osteosarcoma, lung cancer, gastric cancer, head and neck cancer, or leukemia.

In some embodiments of the present invention, the subject involved in the present invention is a mammal including humans.

In another aspect, the present invention provides use of a compound of Formula I or a pharmaceutically acceptable salt thereof, a compound of Formula 11 or a pharmaceutically acceptable salt, solvate, polymorph, or tautomer thereof in the manufacture of a medicament for the treatment of diseases associated with WEE1 activity; in some embodiments, the disease associated with WEE1 activity is liver cancer, breast cancer, glioblastoma, melanoma, adult brain tumor, pediatric brain tumor, ovarian cancer, colon cancer, cervical cancer, osteosarcoma, lung cancer, gastric cancer, head and neck cancer, or leukemia.

### Detailed Description

Exemplary embodiments utilizing the principles of the present invention are set forth in the following detailed description. The features and advantages of the present invention may be better understood with reference to the following contents of the present invention.

It is to be understood that the scope of the various aspects of the present invention is determined by the claims and that methods and structures within the scope of the claims, as well as equivalents thereof, are intended to be within the scope of the claims.

Unless defined otherwise, all technical and scientific terms used herein have the same meaning as commonly understood by one of the ordinary skills in the art. All patents, patent applications, and published materials cited throughout this article are hereby incorporated by reference in their entirety unless otherwise indicated.

It is to be understood that both the foregoing general description and the following detailed description are exemplary and explanatory and are not restrictive of any inventive subject matter. The use of the singular also includes the plural unless specifically stated otherwise. The use of "or" means "and/or" unless otherwise indicated. Furthermore, the use of the term "include" and other forms, such as "comprise", "have", and "contain", are not restrictive.

### Some chemical terms

The term "option", "optional" or "optionally" means that the event or situation described later may or may not occur, and the description includes the occurrence of the event or situation and the non-occurrence of the event or situation. For example, "optionally substituted alkyl" means "unsubstituted alkyl" or "substituted alkyl". Also, an optionally substituted group may be unsubstituted (for example: -CH₂CH₃), completely substituted (for example: -CF₂CF₃), monosubstituted (for example: -CH₂CH₂F) or any level between mono- and completely substituted (e.g: -CH₂CHF₂, -CF₂CH₃, -CFHCHF₂, etc.). Those skilled in the art can understand that for any group containing one or more substituents, any substitution or substitution pattern that is impossible to exist in space and/or cannot be synthesized will not be introduced.

Unless otherwise indicated, conventional methods within the skill of the art are employed, such as mass spectrometry, nuclear magnetic resonance, high-performance liquid chromatography, infrared and ultraviolet/visible spectroscopy, and pharmacological methods. Unless specifically defined otherwise, the relevant terms and experimental procedures and techniques in analytical chemistry, organic synthetic chemistry, and pharmaceutical and medicinal chemistry herein are known in the art. Standard techniques may be used in chemical synthesis, chemical analysis, pharmaceutical preparation, formulation and delivery, and treatment of patients. For example, reactions and purifications can be carried out using the manufacturer's instructions for the kit, or in a manner well known in the art or under the instructions of the present invention. The techniques and methods described above can generally be implemented according to conventional methods well known in the art, as described in various general and more specific documents cited and discussed in this specification. In the present specification, groups and substituents thereof may be selected by those skilled in the art to provide stable moieties and compounds.

When a substituent is described by a conventional chemical formula written from left to right, the substituent also includes chemically equivalent substituents obtained when the structural formula is written from right to left. For example, -CH₂O- is equivalent to -OCH₂-.

As used herein, the terms "group" and "chemical group" refer to a particular moiety or functional group of a molecule. Chemical groups are often considered to be chemical entities embedded or attached to a molecule.

Some of the chemical groups named herein may use abbreviated notation to indicate the total number of carbon atoms. For example, C₁-C₆ alkyl describes an alkyl group, as defined below, having a total of 1 to 6 carbon atoms. The total number of carbon atoms indicated in the abbreviated notation does not include the carbon atoms on the possible substituents.

The term "halogen", "halo" or "halide" refers to bromine, chlorine, fluorine, or iodine.

As used herein, the terms "aroma", "aromatic ring", "aromatic", "aromaticity", and "aromatic ring" refer to a planar ring portion of one or more rings having a delocalized electron conjugation system containing 4n+2 electrons, where n is an integer. The aromatic ring may be formed from 5, 6, 7, 8, 9, or more atoms. The aromatic compound may be optionally substituted and may be monocyclic or fused polycyclic. The term aromatic compounds include all carbocyclic rings (e.g., benzene rings) and rings containing one or more heteroatoms (e.g., pyridine).

The term "heteroatom" or "hetero" as used herein alone or as part of another ingredient refers to an atom other than carbon and hydrogen. The heteroatoms are independently selected from oxygen, nitrogen, sulfur, phosphorus, silicon, selenium, and stannum, but are not limited to these atoms. In embodiments where two or more heteroatoms are present, the two or more heteroatoms may be the same as each other, or some or all of the two or more heteroatoms may be different from each other.

The terms "fused" or "fused ring" as used herein, alone or in combination, refer to a cyclic structure in which two or more rings share one or more bonds.

The terms "spiro" or "spirocyclic", as used herein, alone or in combination, refer to a cyclic structure in which two or more rings share one or more atoms.

The term "alkyl" as used herein alone or as part of other components (such as monoalkylamino) refers to an optionally substituted linear or optionally substituted branched monovalent saturated hydrocarbon having 1 to 12 carbon atoms, preferably 1 to 8 carbon atoms, more preferably 1 to 6 carbon atoms, attached to the rest of the molecule via a single bond, for example, methyl, ethyl, n-propyl, isopropyl, n-butyl, isobutyl, sec-butyl, tert-butyl, n-pentyl, n-hexyl, n-heptyl, 2-methylhexyl, 3-methylhexyl, n-octyl, n-nonyl, n-decyl and the like.

The term "alkenyl" as used herein alone or in combination, refers to an optionally substituted linear or optionally substituted branched monovalent hydrocarbon radical having one or more C = C double bonds and having from 2 to about 10 carbon atoms, more preferably from 2 to about 6 carbon atoms. The double bond in these groups may be in cis or trans conformation and should be understood to encompass both isomers. Examples include, but are not limited to, ethenyl (CH=CH₂), 1-propenyl (CH₂CH=CH₂), isopropenyl (C(CH3)=CH₂), butenyl, and 1,3-butadienyl.

The term "cycloalkyl" as used herein alone or as part of another component refers to a stable monovalent non-aromatic monocyclic or polycyclic hydrocarbon group containing only carbon and hydrogen atoms, possibly including fused, spiro, or bridged ring systems, containing from 3 to 15 ring-forming carbon atoms, preferably from 3 to 10 ring-forming carbon atoms, more preferably from 3 to 8 ring-forming carbon atoms, which may be saturated or unsaturated, attached to the rest of the molecule via a single bond. Non-limiting examples of "cycloalkyl" include, but are not limited to, cyclopropyl, cyclobutyl, cyclopentyl, cyclohexyl, cycloheptyl, and the like.

The terms "heterocyclyl", "heterocycloalkyl", "heterocycle" as used herein alone or as part of another component, refer to a stable 3-18 membered monovalent non-aromatic ring containing from 2 to 12 carbon atoms and from 1 to 6 heteroatoms selected from nitrogen, oxygen, and sulfur. Unless otherwise specified, a heterocyclyl group may be a monocyclic, bicyclic, tricyclic, or tetracyclic ring system, which may contain fused, spiro, or bridged ring systems, nitrogen, carbon or sulfur on the heterocyclyl may be optionally oxidized, the nitrogen atom may be optionally quaternized, and the heterocyclyl may be partially or completely saturated. The heterocyclyl may be attached to the rest of the molecule via a single bond through a carbon atom or heteroatom on the ring. A heterocyclyl containing fused rings may contain one or more aromatic or heteroaromatic rings as long as the atoms on the non-aromatic ring are attached to the rest of the molecule. For purposes of this application, the heterocyclyl is preferably a stable 4-11 membered monovalent non-aromatic monocyclic or bicyclic ring containing from 1 to 3 heteroatoms selected from nitrogen, oxygen, and sulfur, more preferably a stable 4-8 membered monovalent non-aromatic monocyclic ring containing from 1 to 3 heteroatoms selected from nitrogen, oxygen, and sulfur. Non-limiting examples of heterocyclyl include azepanyl, azetidinyl, decahydroisoquinolinyl, dihydrofuranyl, indolinyl, dioxolanyl, 1,1-dioxo-thiomorpholinyl, imidazolidinyl, imidazolinyl, isothiazolidinyl, isoxazolidinyl, morpholinyl, octahydroindolyl, octahydroisoindolyl, oxazinyl, piperazinyl, piperidinyl, 4-piperidonyl, pyranyl, pyrazolidinyl, pyrrolidinyl, quinolizinyl, quinuclidinyl, tetrahydrofuranyl, tetrahydropyranyl, and the like.

The term "aryl" refers to an all-carbon monocyclic or fused ring having a fully conjugated π electronic system having 6 to 14 carbon atoms, preferably 6 to 12 carbon atoms, and most preferably 6 carbon atoms. Aryl groups may be unsubstituted or substituted with one or more substituents, examples of which include, but are not limited to, alkyl, alkyloxy, aryl, aralkyl, amino, halogen, hydroxy, sulfonyl, sulfinyl, phosphoryl, and heteroalicyclyl groups. Non-limiting examples of unsubstituted aryl groups include, but are not limited to, phenyl, naphthyl, and anthracenyl groups.

The term "heteroaryl" refers to a monocyclic or fused ring of 5 to 12 ring atoms having 5, 6, 7, 8, 9, 10, 11, or 12 ring atoms containing 1, 2, 3, or 4 ring atoms selected from N, O, S, the remaining ring atoms being C, and having a fully conjugated π-electron system. Heteroaryl groups may be unsubstituted or substituted and include, but are not limited to, alkyl, alkyloxy, aryl, aralkyl, amino, halogen, hydroxy, cyano, nitro, carbonyl, and heteroalicyclic groups. Non-limiting examples of unsubstituted heteroaryl groups include, but are not limited to, pyrrolyl, furanyl, thienyl, imidazolyl, oxazolyl, pyrazolyl, pyridinyl, pyrimidinyl, pyrazinyl, quinolinyl, isoquinolinyl, tetrazolyl, and triazinyl.

As used herein, the term "polymorph" or " polymorphism" refers to a compound of the present invention having a variety of lattice morphologies. Some of the compounds of the present invention may have more than one crystal form, and the present invention encompasses all polymorphs or mixtures thereof.

Intermediates of the compounds of the present invention and polymorphs thereof are also within the scope of the present invention.

Unless otherwise specified, the olefinic double bond contained in the compound of the present invention includes E and Z isomers.

It is to be understood that the compounds of the present invention may contain asymmetric centers. These asymmetric centers may independently be in the *R* or S configuration. Some of the compounds of the present invention may also show cis-trans isomerism, which is obvious to those skilled in the art. It is to be understood that the compounds of the present invention include individual geometric and stereoisomers thereof as well as mixtures thereof, including racemic mixtures. These isomers may be separated from their mixtures by practicing or modifying known methods, such as chromatographic techniques and recrystallization techniques, or they may be prepared separately from the appropriate isomers of their intermediates.

As used herein, the term "pharmaceutically acceptable salt" includes both acid and alkali addition salts.

"Pharmaceutically acceptable acid addition salts" refer to those salts formed with inorganic acids such as, but not limited to, hydrochloric acid, hydrobromic acid, sulfuric acid, nitric acid, and phosphoric acid; or with organic acids such as, but not limited to, acetic acid, 2,2-dichloroacetic acid, adipic acid, alginic acid, ascorbic acid, aspartic acid, benzenesulfonic, benzoic acid, capric acid caproic acid, carbonic acid, cinnamic acid, and citric acids, which retain the biological effectiveness and properties of the free alkali of the compound, and are not biologically or otherwise undesirable. "Pharmaceutically acceptable alkali addition salts" refer to those salts that retain the biological effectiveness and properties of the free acids of the compounds and are not biologically or otherwise undesirable. These salts are prepared by reacting free acids with inorganic or organic alkalis. Salts formed by reaction with inorganic alkalis include, but are not limited to, sodium, potassium, lithium, ammonium, calcium, magnesium, iron, zinc, copper, manganese, aluminum salts, and the like. Preferred inorganic salts are ammonium, sodium, potassium, calcium, and manganese salts.

Organic alkalis that form salts include, but are not limited to, primary, secondary, tertiary, and cyclic amines, such as ammonia, isopropylamine, trimethylamine, diethylamine, triethylamine, tripropylamine, ethanolamine, diethanolamine, ethanolamine, dicyclohexylamine, ethylenediamine, purine, piperazine, piperidine, choline, and caffeine. Particularly preferred organic alkalis include isopropylamine, diethylamine, ethanolamine, trimethylamine, dicyclohexylamine, choline, and caffeine.

Crystallization often produces solvates of the compounds of the present invention. The term "solvate" as used herein refers to a complex composed of one or more molecules of the compound of the present invention and one or more solvent molecules.

The solvent may be water, in which case the solvate is a hydrate. It may additionally be an organic solvent. Thus, the compounds of the present invention may exist as hydrates, including monohydrate, dihydrate, hemihydrate, trihydrate, and tetrahydrate, as well as the corresponding solvated forms. The compounds of the present invention may be true solvates, but in other cases, the compounds of the present invention may simply accidentally retain water or a mixture of water with some other solvent. The compounds of the present invention may be reacted in a solvent or precipitated or crystallized in a solvent. Solvates of the compounds of the present invention are also included in the scope of the present invention.

The term "pharmaceutical composition" as used herein refers to a formulation incorporating a compound of the present invention and a medium generally accepted in the art for delivering a biologically active compound to a mammal, such as a human. Such media comprise all pharmaceutically acceptable carriers.

The term "acceptable" as used herein in connection with a formulation, composition, or ingredient means there is no sustained deleterious effect on the overall health of the subject being treated.

The term "pharmaceutically acceptable" as used herein refers to a substance (e.g., a carrier or diluent) that does not affect the biological activity or properties of the compounds of the present invention, and is relatively nontoxic, i.e., the substance can be administered to an individual without causing an undesirable biological response or interaction in an undesirable manner with any of the components contained in the composition.

"Pharmaceutically acceptable carrier" includes, but is not limited to, adjuvants, carriers, excipients, auxiliaries, deodorants, diluents, preservatives, dyes/colorants, flavor enhancers, surfactants and wetting agents, dispersing agents, suspending agents, stabilizers, isotonic agents, solvents, or emulsifying agents, which may be used in humans and domesticated animals, as approved by the relevant government administration.

As used herein, the terms "subject", "patient, "object", or "individual" refers to an individual suffering from a disease, disorder, or condition, etc., including mammals and non-mammals. Examples of mammals include, but are not limited to, any member of the class Mammalia: humans, non-human primates (e.g., chimpanzees and other apes and monkeys); livestock, such as cattle, horses, sheep, goats, pigs; domestic animals such as rabbits, dogs, and cats; laboratory animals, including rodents, such as rats, mice, and guinea pigs, etc. Non-mammals include, but are not limited to, birds, and fish. In an embodiment related to methods and compositions provided herein, the mammal is a human.

The term "treatment" as used herein refers to the treatment of a disease or condition associated with a mammal, particularly a human, including
(i) preventing a mammal, particularly a mammal that has previously been exposed to a disease or condition but has not yet been diagnosed with the disease or condition, from developing the corresponding disease or condition;
(ii) inhibiting the disease or disorder, i.e., controlling its development;
(iii) alleviating the disease or disorder, i.e., causing regression of the disease or disorder; and
(iv) alleviating symptoms caused by the disease or disorder.

The terms "disease" and "condition" as used herein may be used interchangeably and may have different meanings, as some specific diseases or conditions have no known pathogenic factors (so the cause of the disease remains unknown), and therefore they cannot be considered a disease but can only be considered an unwanted condition or syndrome, with more or less specific symptoms having been confirmed by clinical researchers.

As used herein, the terms "effective amount", "therapeutically effective amount", or "pharmaceutically effective amount" refer to an amount of at least one agent or compound that, upon administration, is sufficient to relieve to some extent one or more symptoms of the disease or disorder being treated. The result may be reduction and/or alleviation of signs, symptoms, or causes, or any other desired change in the biological system. For example, an "effective amount" for therapy is the amount of a composition comprising a compound disclosed herein required to provide a clinically significant disease remission effect. Effective amounts suitable for use in any individual case may be determined using techniques such as dose escalation test.

The terms "taking", "administration", "administering", etc., as used herein, refer to a method of delivering a compound or composition to the desired site for biological action. These methods include, but are not limited to, oral routes, duodenal routes, parenteral injection (including intravenous, subcutaneous, intraperitoneal, intramuscular, intraarterial injection or infusion), topical administration, and rectal administration. In preferred embodiments, the compounds and compositions discussed herein are administered orally.

### Preparation of compound of the invention

The following reaction schemes show methods for preparing the compounds of the present application.

It is to be understood that in the following description, combinations of substituents and/or variables of the formula are permissible only if stable compounds are formed.

It will also be appreciated by those skilled in the art that in the schemes described below, functional groups of intermediate compounds may need to be protected by suitable protecting groups. The protecting groups may be added or removed by standard techniques known to those skilled in the art.

### Detailed Description of the Invention

### SYNTHETIC PROTOCOLS

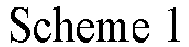

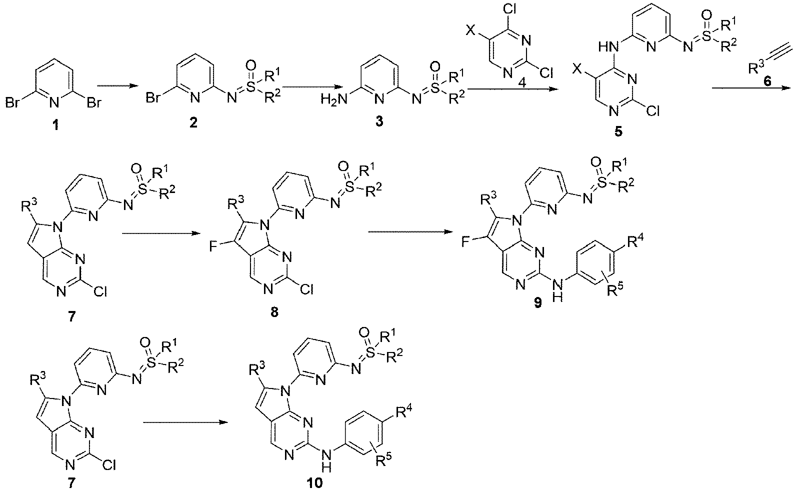

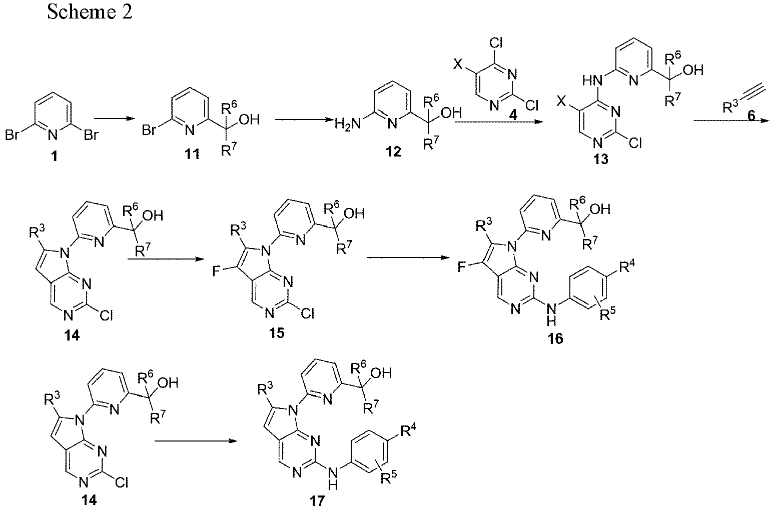

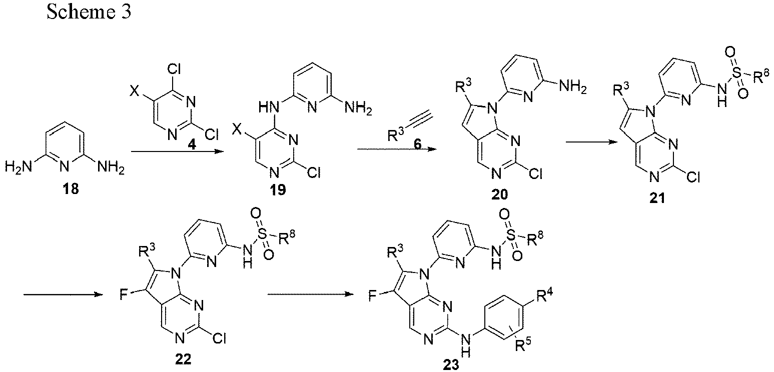

Compounds of the present invention may be prepared according to the routes described in Scheme 1 or Scheme 2 or Scheme 3. Each of the products resulting from the reactions in Scheme 1 or Scheme 2 or Scheme 3 may be obtained by conventional separation techniques including, but not limited to, filtration, distillation, crystallization, chromatographic separation, etc. Starting materials can be synthesized by oneself or purchased from commercial establishments such as, but not limited to, AdricH or Sigma. These materials may be characterized using conventional means, such as physical constants and spectral data. The compounds described herein may be synthesized to give individual isomers or mixtures of isomers.

In the Scheme 1, a raw material 1 was subjected to coupling reaction with sulfimide raw materials in the presence of a suitable palladium catalyst, a ligand and an alkali to obtain an intermediate 2; SN2 reaction was conducted on the intermediate 2 and ammonia water under catalysis of cuprous oxide to obtain an intermediate 3; substitution reaction was conducted on the intermediate 3 and a raw material 4 in the presence of a suitable alkali to generate an intermediate 5; Sonogashira coupling reaction was conducted on the intermediate 5 and alkyne raw material 6 in the presence of a suitable palladium and copper catalyst, a ligand and an alkali, and intramolecular isomerization cyclization reaction was conducted after further heating to produce an intermediate 7; substitution reaction was conducted on the intermediate 7 and a fluorinating reagent to generate an intermediate 8; BucHwald-Hartwig reaction was conducted on the intermediate 8 and an aromatic amine starting material in the presence of a suitable palladium catalyst, a ligand and an alkali to give a target product 9. Alternatively, the non-fluorinated intermediate 7 is subjected to a BucHwald-Hartwig reaction with an aromatic amine starting material in the presence of a suitable palladium catalyst, a ligand, and an alkali to produce a target product 10.

In the Scheme 1, a raw material 1 was subjected to butyl lithium or a suitable alkali treatment and then to reaction with a ketone raw material to generate an intermediate 11; SN2 reaction was conducted on the intermediate 11 and ammonia water under catalysis of cuprous oxide to obtain an intermediate 12; substitution reaction was conducted on the intermediate 12 and a raw material 4 in the presence of a suitable alkali to generate an intermediate 13; Sonogashira coupling reaction was conducted on the intermediate 13 and alkyne raw material 6 in the presence of a suitable palladium and copper catalyst, a ligand and an alkali, and intramolecular isomerization cyclization reaction was conducted after further heating to produce an intermediate 14; substitution reaction was conducted on the intermediate 14 and a fluorinating reagent to generate an intermediate 15; BucHwald-Hartwig reaction was conducted on the intermediate 15 and an aromatic amine starting material in the presence of a suitable palladium catalyst, a ligand and an alkali to give a target product 16. Alternatively, the non-fluorinated intermediate 14 is subjected to a BucHwald-Hartwig reaction with an aromatic amine starting material in the presence of a suitable palladium catalyst, a ligand, and an alkali to produce a target product 17.

In Scheme 3, substitution reaction was conducted on the intermediate 18 and a raw material 4 in the presence of a suitable alkali to generate an intermediate 19; Sonogashira coupling reaction was conducted on the intermediate 19 and alkyne raw material 6 in the presence of a suitable palladium and copper catalyst, a ligand, and an alkali, and intramolecular isomerization cyclization reaction was conducted after further heating to produce an intermediate 20; the intermediate 20 was subjected to reaction with alkyl sulfonyl chloride to obtain an intermediate 21; substitution reaction was conducted on the intermediate 21 and a fluorinating reagent to generate an intermediate 22; BucHwald-Hartwig reaction was conducted on the intermediate 22 and an aromatic amine starting material in the presence of a suitable palladium catalyst, a ligand, and an alkali to give a target product 23.

### Examples

The following non-limiting examples are merely illustrative and do not limit the application in any way.

Unless otherwise stated, temperatures are in degrees Celsius. Reagents were purchased from commercial suppliers such as Sinopharm Chemical Reagent Beijing Co., Ltd., Alfa Aesar, or Beijing J&K Scientific Ltd., and these reagents were used without further purification unless otherwise specified.

Unless otherwise stated, the following reactions were carried out at room temperature, in an anhydrous solvent, under a positive pressure of nitrogen or argon, or using a drying tube; the reaction bottle was provided with a rubber diaphragm to add a substrate and a reagent through a syringe; glassware was baked and/or dried by drying.

Unless otherwise stated, silica gel for column chromatography was supplied from Qingdao Haiyang Chemical Plant in 200-300 mesh size; thin layer chromatography silica gel precast slab (HSGF254) for preparative Thin Layer Chromatography was produced by Yantai Chemical Industry Research Institute; Thermo LCQ Fleet type (ESI) Liquid Chromatograph Mass Spectrometer was used for MS assay; SGW-3 Automtic Polarimeter from Shanghai Shenguang Instrument and Meter Co., Ltd. was used for polarimetry.

Nuclear magnetic data (¹H NMR) were run at 400 MHz using a Varian apparatus. The solvents used for nuclear magnetic data are CDCl₃, CD₃OD, D₂O, DMSO-*d*₆, etc., based on tetramethylsilane (0.00 ppm) or based on the residual solvent (CDCl₃: 7.26 ppm; CD3OD: 3.31 ppm; D2O: 4. 79 ppm; d6-DMSO: 2. 50 ppm). When peak shape diversity is indicated, the following abbreviations denote different peak shapes: s (singlet), d (doublet), t (triplet), q (quartet), m (multiplet), br (broad), dd (doublet of doublets), dt (doublet of triplets). If a coupling constant is given, it is in Hertz (Hz).

**Abbreviation:**

| | |
|---|---|
| DCM | dichloromethane |
| DMAP | 4-N,N -dimethylaminopyridine |
| DMF | N,N-dimethylformamide |
| DMSO | dimethyl sulfoxide |
| EDCI | 1-(3-dimethylaminopropyl)-3 -ethylcarbodiimide hydrochloride |
| EtOAc | ethyl acetate |
| h | hour |
| HPLC | High performance liquid chromatography |
| MeOH | methanol |
| MS | mass spectroscopy |
| Pd₂(dba)₃ | Tris(dibenzylideneacetone)dipalladium |
| Pd(dppf)Cl₂ | 1,1-Bis(diphenylphosphino)ferrocene-palladium(II)dichloride |
| PE | petroleum ether |
| TEA | triethylamine |
| THF | tetrahydrofuran |

### Example 1

### ((6-(5-fluoro-2-((4-(4-methylpiperazin-1-yl)phenyl)amino)-6-propyl-7H-pyrrolo[2,3-d]pyrim idin-7-yl)pyridin-2-yl)imino)dimethyl-λ⁶-sulfanone

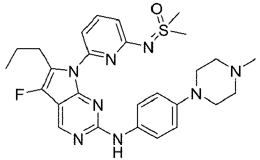

### Step A: ((6-bromopyridin-2-yl)imino)dimethyl-λ⁶-sulfanone

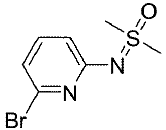

Under nitrogen gas, a suspension of 2,6-di-bromopyridine (6.00 g), S,S-dimethyl sulfoximine (1.63 g), 9,9-Dimethyl-4,5-bis(diphenylphosphino)xanthene (1.50 g), Pd₂(dba)₃ (0.793 g) and caesium carbonate (8.59 g) in 1,4-dioxane (100 mL) was heated and refluxed overnight. The mixture was cooled to room temperature, filtered and washed with dichloromethane, the solvent was removed from the filtrate, and the residue was purified through silica gel column chromatography (100%EtOAc) to obtain the product (4.0 g).

¹H NMR (400 MHz CDCl₃) δ 7.31-7.35 (m, 1H), 6.93 (dd, J = 7.6 Hz, 0.8 Hz, 1H), 6.69 (dd, J = 7.6 Hz, 0.8 Hz, 1H), 3.36 (s, 6H).

### Step B: ((6-aminopyridin-2-yl)imino)dimethyl-λ⁶-sulfanone

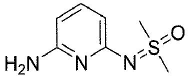

A mixture of ((6-bromopyridin-2-yl)imino)dimethyl-λ⁶-sulfanone (4.0 g), copper (1) oxide (0.230 g), N,N-Dimethyl-1,2-ethanediamine (0.142 g), anhydrous potassium carbonate (4.43 g), ammonia (20 mL) and ethylene glycol (20 mL) was heated to 60 °C and stirred overnight. The mixture was cooled to room temperature, filtered through celite, washed with dichloromethane and partitioned, the aqueous phase was extracted with dichloromethane (100 mL x 4), and the organic phases were combined and evaporated to remove the solvent to obtain the product (1.4 g).

¹H NMR (400 MHz, CDCl₃) δ7.27-7.31 (m, 1H), 6.19 (d, J = 7.6 Hz, 1H), 6.04 (d, J = 7.6 Hz, 1H), 4.26 (brs, 2H), 3.35 (s, 6H).

### Step C: ( (6- ( (5- bromo- 2- chloropyrimidin- 4- yl) amino) pyridin- 1- yl) imino) dimethyl-λ⁶- sulfanone

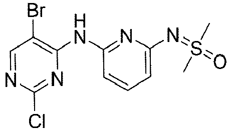

Under nitrogen gas, a mixture of ((6-aminopyridin-2-yl)imino)dimethyl-λ⁶-sulfanone (0.83 g), 5-bromo-2,4-dichloropyrimidine (1.02 g), ethyldiisopropylamine (1.16 g) and isopropanol (10 mL) was heated and refluxed overnight. The mixture was cooled to room temperature, and the solid precipitated was collected by filtration, washed with ethyl acetate, and dried to obtain the product (1.20 g).

¹H NMR (400 MHz, CDCl₃) δ 8.35 (s, 1H), 7.76-7.81 (m, 2H), 7.62 (d, J = 8.0 Hz, 1H), 6.63 (d, J = 8.0 Hz, 1H), 3.36 (s, 6H).

### Step D: ((6-(2-chloro-6-propyl-7H-pyrrolo[2,3-d]pyrimidin-7-yl)pyridin-2-yl)imino) dimethyl-λ⁶-sulfanone

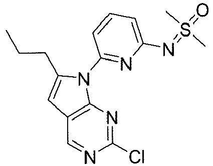

Under nitrogen gas, to a solution of ((6-((5-bromo-2-chloropyrimidin-4-yl)amino) pyridin-1-yl)imino)dimethyl-λ⁶-sulfanone (1.95 g), copper(I) iodide (0.393 g) and Pd(dppf)Cl₂ (0.37 g) in DMF (20 mL) and triethylamine (20 mL) was slowly added 1-pentyne (0.352 g), the mixture was stirred at room temperature overnight, heated to 50 °C and stirred overnight, the reaction solution was filtered through celite and washed with dichloromethane, the solvent was removed from the filtrate, and the residue was purified through silica gel column chromatography (50%EtOAc/PE-100%EtOAc) to obtain the product (0.65 g).

¹H NMR (400 MHz, CDCl₃) δ 8.72 (s, 1H), 8.02 (s, 1H), 6.98 (dd, J = 8.0 Hz, 0.4 Hz, 1H), 6.83 (dd, *J* = 8.8 Hz, 0.4 Hz, 1H), 6.41 (s, 1H), 3.36 (s, 6H), 2.88-2.90 (m, 2H), 1.58-1.64 (m, 2H), 0.95 (t, *J* = 7.2 Hz, 3H).

### Step E: ((6-(2-chloro-5-fluoro-6-propyl-7H-pyrrolo[2,3-d]pyrimidin-7-yl)pyridin-2-yl)imino) dimethyl-λ⁶-sulfanone

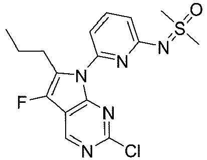

Under nitrogen gas, a solution of ((6-(2-chloro-6-propyl-7*H*-pyrrolo[2,3-*d*]pyrimidin -7-yl) pyridin-2-yl)imino)dimethyl-λ⁶-sulfanone (184 mg) in acetonitrile (3 mL) was cooled to 0 °C, Selectfluor^{®} was added portionwise (90 mg), and the mixture was allowed to warm up to room temperature slowly and stirred overnight. Water was added to quench the reaction, the mixture was extracted with dichloromethane, the organic phase was concentrated, and the residue was purified through preparative TLC (100%EtOAc) to obtain the product (30 mg).

¹H NMR (400 MHz, CDCl₃) δ 8.81 (s, 1H), 7.71-7.75 (m, 1H), 7.01 (d, J = 8.0 Hz, 1H), 6.85 (d, J = 8.0 Hz, 1H), 3.34 (s, 6H), 2.98-3.03(m, 2H), 1.42-1.47 (m, 2H), 0.86 (t, *J* = 7.2 Hz, 3H).

### Step F: ((6-(5-fluoro-2-((4-(4-methylpiperazin-1-yl)phenyl)amino)-6-propyl-7H-pyrrolo[2,3-d]pyrimidin-7-yl)pyridin-2-yl)imino)dimethyl-λ⁶-sulfanone

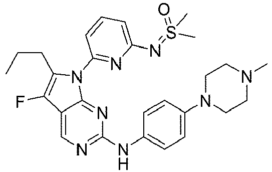

((6-(2-Chloro-5-fluoro-6-propyl-7*H*-pyrrolo[2,3-d]pyrimidin-7-yl)pyridin-2-yl)imino)dimeth yl-λ⁶-sulfanone (30 mg), 4-(4-methylpiperazin-1-yl)aniline (30 mg), Pd₂(dba)₃ (7.5 mg), 2-dicyclohexylphosphino-2',4',6'-trisopropylbiphenyl (11 mg) and caesium carbonate (40 mg) were mixed in a mixed solvent of 1,4-dioxane (5 mL) and DMF (1 mL), the system was sufficiently purged with nitrogen gas, and the tube was heated in a microwave reactor to 101 °C for the mixture to react for 2 hours. The reaction solution was evaporated to remove the solvent, and the residue was purified through preparative TLC (10%MeOH/DCM) to obtain the target product (10 mg).

¹H NMR (400 MHz, CDCl₃) δ 8.64 (s, 1H), 7.72-7.76 (m, 1H), 7.52-7.55 (m, 3H), 7.19(d, J = 8.0 Hz, 1H), 6.80-6.86 (m, 3H), 3.41-3.43 (m, 4H), 3.31 (s, 6H), 3.13 (brs, 4H), 3.01-3.05 (m, 2H), 2.75 (s, 3H), 1.35-1.41(m, 2H), 0.84 (t, *J* = 7.6 Hz, 3H).

### Example 2

### 2-(6-(2-((4-(4-methylpiperazin-1-yl)phenyl)amino)-6-propyl-7H-pyrrolo[2,3-d]pyrimidin-7-y l)pyridin-2-yl)propan-2-ol

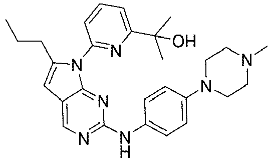

### Step A: 2-(6-bromopyridin-2-yl)propan-2-ol

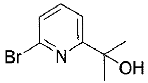

Under nitrogen gas, to a three-neck flask was added a solution of 2.5 M butyl lithium in n-hexane (19.4 mL), the mixture was cooled to -78 °C, to the reaction system was slowly dropwise added a solution of 2,6-di-bromopyridine (11.5 g) in tetrahydrofuran (50 mL), and the mixture was stirred at this temperature for 0.5 hours. Acetone (6.0 mL) was slowly added. The reaction system was slowly heated to 25 °C and stirred at this temperature for 1 hour. To the reaction system was slowly added saturated ammonium chloride solution (100 mL) to quench the reaction and extracted with dichloromethane. The solvent was evaporated to obtain the product (10.5 g).

¹H NMR (400 MHz CDCl₃) δ 7.56 (t, J = 7.6 Hz, 1H), 7.35-7.39 (m, 2H), 3.72-4.42 (brs, 1H), 1.55 (s, 6H).

### Step B: 2-(6-aminopyridin-2-yl)propan-2-ol

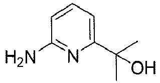

According to the method of step B of example 1, 2-(6-bromopyridin-2-yl)propan-2-ol and ammonia were used as the starting material to obtain the product (4.0 g).

¹H NMR (400 MHz, CDCl₃) δ 7.44 (t, J = 8.0 Hz, 1H), 6.66 (d, J = 8.0 Hz, 1H), 6.37 (d, J = 8.0 Hz, 1H), 5.10 (s, 1H), 4.39-4.53 (brs, 2H), 1.48 (s, 6H).

### Step C: 2-(6-((5-bromo-2-chloropyrimidin-4-yl)amino)pyridin-2-yl)propan-2-ol

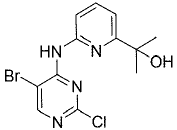

According to the method of step C of example 1, 2-(6-aminopyridin-2-yl)propan-2-ol and 5-bromo-2,4-dichloropyrimidine were used as the starting material to obtain the product (5.41 g).

¹H NMR (400 MHz, CDCl₃) δ 8.40 (s, 1H), 8.29 (d, J = 8.0 Hz, 1H), 7.98 (s, 1H), 7.82 (t, J = 8.0 Hz, 1H), 7.17 (d, *J* = 8.0 Hz, 1H), 4.41 (s, 1H), 1.56 (s, 6H).

### Step D: 2-(6-(2-chloro-6-propyl-7H-pyrrolo[2,3-d]pyrimidin-7-yl)pyridin-2-yl) propan-2-ol

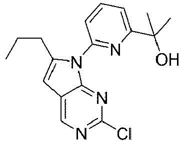

According to the method of step D of example 1, 2-(6-((5-bromo-2-chloropyrimidin-4-yl)amino)pyridin-2-yl)propan-2-ol and 1-pentyne were used as the starting material to obtain the product (50 mg).

¹H NMR (400 MHz, CDCl₃) δ 8.75 (s, 1H), 7.98 (t, J = 8.0 Hz, 1H), 7.61 (d, J = 8.0 Hz, 1H), 7.53 (d, J = 8.0 Hz, 1H), 6.47 (s, 1H), 3.92 (s, 1H), 2.92 (t, J = 7.6 Hz, 2H), 1.50-1.67 (m, 8H), 0.93 (t, J = 7.6 Hz, 3H).

### Step E: 2-(6-(2-((4-(4-methylpiperazin-1-yl)phenyl)amino)-6-propyl-7H-pyrrolo[2,3-d] pyrimidin-7-yl)pyridin-2-yl)propan-2-ol

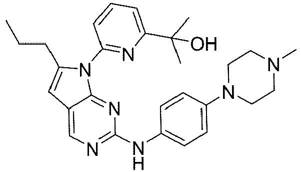

According to the method of step F of example 1, 2-(6-(2-chloro-6-propyl-7*H-*pyrrolo[2,3-d]pyrimidin-7-yl)pyridin-2-yl)propan-2-ol and 4-(4-methylpiperazin-1-yl) aniline were used as the starting material to obtain the product (10 mg).

¹H NMR (400 MHz, CDCl₃) δ 8.60 (s, 1H), 7.94 (t, *J =* 8.0 Hz, 1H), 7.71 (d, *J =* 8.0 Hz, 1H), 7.51 (d, J = 8.8 Hz, 2H), 7.45 (d, J = 8.0 Hz, 1H), 7.18-7.21 (brs, 1H), 6.86 (d, J = 8.8 Hz, 2H), 6.30 (s, 1H), 4.07 (s, 1H), 3.15-3.25 (m, 4H), 2.91 (t, *J* = 7.6 Hz, 2H), 2.51-2.85 (m, 4H), 2.44 (s, 3H), 1.59-1.66 (m, 8H), 0.92 (t, *J* = 9.6 Hz, 3H).

### Example 3

### 2-(6-((5-fluoro-6-(2-hydroxyethyl)-2-(4-(4-methylpiperazin-1-yl)phenyl)amino)-7H-pyrrolo[ 2,3-d]pyrimidin-7-yl)pyridin-2-yl)propan-2-ol

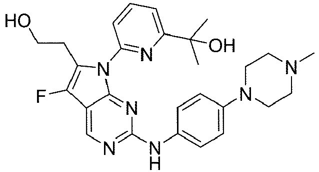

### Step A: 2-(6-(2-chloro-6-(2-hydroxyethyl)-7H-pyrrolo[2,3-d]pyrimidin-7-yl)pyridin- 2-yl) propan-2-ol

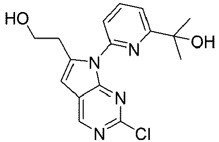

According to the synthetic method of step D of example 1, 3-butyn-1-ol and 2-(6-((5-bromo-2-chloropyrimidin-4-yl)amino)pyridin-2-yl)propan-2-ol were used as the starting material to obtain the product (1.0 g).

¹H NMR (400 MHz CDCl₃) δ 8.79 (s, 1H), 7.99 (t, J = 8.0 Hz, 1H), 7.68 (d, J = 8.0 Hz, 1H), 7.55 (d, J = 8.0 Hz, 1H), 6.61 (s, 1H), 3.95 (t, J = 6.0 Hz, 2H), 3.67-3.79 (brs, 1H), 3.20 (t, J = 6.0 Hz, 2H), 2.53-2.60 (brs, 1H), 1.62 (s, 6H).

### Step B: 2-(6-(2-chloro-5-fluoro-6-(2-hydroxyethyl)-7H-pyrrolo[2,3-d]pyrimidin-7-yl) pyridin- 2-yl)propan-2-ol

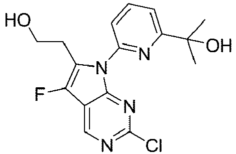

According to the synthetic method of step E of example 1, 2-(6-(2-chloro-6-(2-hydroxyethyl)-7*H*-pyrrolo[2,3-d]pyrimidin-7-yl)pyridin-2-yl)propan-2-ol was used as the starting material to obtain the product (0.2 g).

¹H NMR (400 MHz, CDCl₃) δ 8.88 (s, 1H), 7.99 (t, J = 8.0 Hz, 1H), 7.72 (d, J = 8.0 Hz, 1H), 7.56 (d, J = 8.0 Hz, 1H), 4.10-4.45 (brs, 2H), 3.92 (t, J = 6.4 Hz, 2H), 3.26 (t, J = 6.4 Hz, 2H), 1.62 (s, 6H).

### Step C: 2-(6-((5-fluoro-6-(2-hydroxyethyl)-2-(4-(4-methylpiperazin-1-yl)phenyl) amino)-7H-pyrrolo[2,3-d]pyrimidin-7-yl)pyridin-2-yl)propan-2-ol

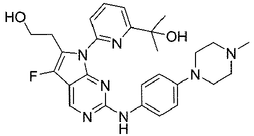

According to the synthetic method of step F of example 1, 2-(6-(2-chloro-5-fluoro- 6-(2-hydroxyethyl)-7*H*-pyrrolo[2,3-d]pyrimidin-7-yl)pyridin-2-yl)propan-2-ol and 4-(4-methyl piperazin- 1- yl) aniline were used as the starting material to obtain the product (5 mg).

¹H NMR (400 MHz CDCl₃) δ 8.66 (s, 1H), 7.95 (t, J = 8.0 Hz, 1H), 7.95 (d, J = 8.0 Hz, 1H), 7.48-7.53 (m, 3H), 7.10 (s, 1H), 6.89 (d, J = 7.6 Hz, 2H), 4.39-4.40(brs, 2H), 3.90 (t, J = 6.4 Hz, 2H), 3.32-3.37 (m, 4H), 3.23 (t, *J* = 6.4 Hz, 2H), 2.88-2.99 (m, 4H), 2.60 (s, 3H), 1.63 (s, 6H).

### Example 4

((6-(2-((4-(4-methylpiperazin-1-yl)phenyl)amino)-6-propyl-7*H*-pyrrolo[2,3-d]pyrimidin-7-yl) pyridin-2-yl)imino)dimethyl-λ⁶-sulfanone

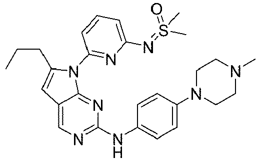

((6-(2-Chloro-6-propyl-7*H*-pyrrolo[2,3-d]pyrimidin-7-yl)pyridin-2-yl)imino)dimethyl-λ⁶-sulf anone (30 mg), 4-(4-methylpiperazin-1-yl)aniline (30 mg), Pd₂(dba)₃ (7.5 mg), 2-dicyclohexylphosphino-2',4',6'-trisopropylbiphenyl (11 mg) and caesium carbonate (40 mg) were mixed in a mixed solvent of 1,4-dioxane (5 mL) and DMF (1 mL), the system was sufficiently purged with nitrogen gas, and the tube was heated in a microwave reactor to 101 °C for the mixture to react for 2 hours. The reaction solution was evaporated to remove the solvent, and the residue was purified through preparative TLC (10%MeOH/DCM) to obtain the target product (5 mg).

¹H NMR (400 MHz, CDCl₃) δ 8.57 (s, 1H), 8.25 (s, 1H), 7.72-7.74 (m, 1H), 7.52-7.55 (m, 2H), 7.16 (d, J = 7.6 Hz, 1H), 6.82-6.86 (m, 3H), 6.25 (s, 1H), 3.30-3.34 (m, 4H), 3.28 (s, 6H), 2.91-2.99 (m, 6H), 2.64 (s, 3H), 1.33-1.39 (m, 2H), 0.86 (t, *J* = 7.6 Hz, 3H).

### Example 5

### 2-(6-(5-fluoro-2-((4-(4-methylpiperazin-1-yl)phenyl)amino)-6-propyl-7H-pyrrolo[2,3-d]pyri midin-7-yl)pyridin-2-yl)propan-2-ol

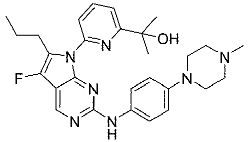

### Step A: 2-(6-(5-fluoro-2-chloro-6-propyl-7H-pyrrolo[2,3-d]pyrimidin-7-yl)pyridin-2-yl) propan-2-ol

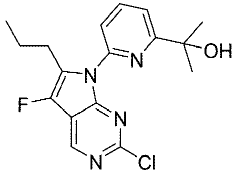

According to the method of step E of example 1, 2-(6-(2-chloro-6-propyl-7*H*-pyrrolo[2,3-d] pyrimidin-7-yl)pyridin-2-yl)propan-2-ol and Selectfluor^{®} were used as the starting material to obtain the product (50 mg).

¹H NMR (400 MHz, CDCl₃) δ 8.84 (s, 1H), 7.98 (t, J = 8.0 Hz, 1H), 7.64 (d, J = 8.0 Hz, 1H), 7.56 (d, J = 8.0 Hz, 1H), 3.65-3.92 (brs, 1H), 2.99 (t, J = 7.6 Hz, 2H), 1.60 (s, 6H), 1.39-1.49 (m, 2H), 0.87 (t, J = 7.6 Hz, 3H).

### Step B: 2-(6-(5-fluoro-2-((4-(4-methylpiperazin-1-yl)phenyl)amino)-6-propyl-7H- pyrrolo [2,3-d] pyrimidin-7-yl)pyridin-2-yl)propan-2-ol

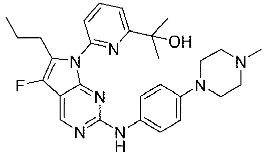

According to the method of step F of example 1, 2-(6-(5-fluoro-2-chloro-6-propyl-7*H-*pyrrolo[2,3-d]pyrimidin-7-yl)pyridin-2-yl)propan-2-ol and 4-(4-methylpiperazin-1-yl) aniline were used as the starting material to obtain the product (10 mg).

¹H NMR (400 MHz, CDCl₃) δ 8.65 (s, 1H), 7.96 (t, J = 8.0 Hz, 1H), 7.75 (t, J = 8.0 Hz, 1H), 7.53 (d, *J* = 8.8 Hz, 2H), 7.47 (d, *J* = 8.0 Hz, 1H), 7.18 (s, 1H), 6.87 (d, *J* = 8.8 Hz, 2H), 3.81-4.16 (brs, 1H), 3.39-3.48 (m, 4H), 3.00-3.12 (m, 4H), 2.98 (d, J = 8.0 Hz, 2H), 2.74 (s, 3H), 1.61 (s, 6H), 1.37-1.46 (m, 2H), 0.86 (t, *J* = 7.6 Hz, 3H).

### Example 6

### ((6-(6-isopropyl-2-((4-(4-methylpiperazin-1-yl)phenyl)amino)-7H-pyrrolo[2,3-d]pyrimidin-7 -yl)pyridin-2-yl)imino)dimethyl-λ⁶-sulfanone

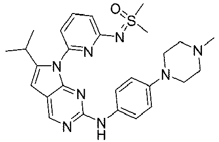

### Step A: ((6-(2-chloro-6-isopropyl-7H-pyrrolo[2,3-d]pyrimidin-7-yl)pyridin-2-yl)imino) dimethyl-λ⁶-sulfanone

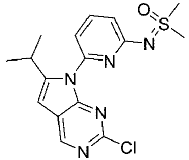

At room temperature, to a tube charged with DMF/TEA (2 ml/2 mL) were added ((6-((5-bromo-2-chloropyrimidin-4-yl)amino)pyridin-2-yl)imino)dimethyl-λ⁶-sulfanone (226mg), copper(I) iodide (12 mg) and Pd(dppf)Cl₂ (42 mg), the system was purged with nitrogen gas, 3-methyl butyne (40 mg) was added, the mixture was stirred at 25 °C for 7 hours, heated to 50 °C and stirred overnight, cooled to room temperature, filtered, and washed with dichloromethane, the solvent was removed from the filtrate, and the residue was purified through silica gel column chromatography (DCM/MeOH=30/1) to obtain the product (70 mg).

¹H NMR (400 MHz, CDCl₃) δ 8.24 (s, 1H), 7.95 (s, 1H), 7.85 (d, J = 8.0 Hz, 1H), 7.61 (t, J = 8.0 Hz, 1H), 6.58 (d, *J* = 8.0 Hz, 1H), 3.37 (s, 6H), 2.86-2.93 (m, 1H), 1.34 (d, J = 6.8 Hz, 6H).

### Step B: ((6-(6-isopropyl-2-((4-(4-methylpiperazin-1-yl)phenyl)amino)-7H-pyrrolo[2,3-d] pyrimidin-7-yl)pyridin-2-yl)imino)dimethyl-λ⁶-sulfanone

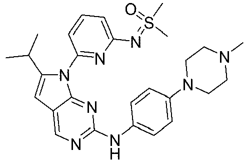

((6-(2-Chloro-6-isopropyl-7*H*-pyrrolo[2,3-d]pyrimidin-7-yl)pyridin-2-yl)imino)dimethyl-λ⁶-s ulfanone (50 mg), 4-(4-methylpiperazin-1-yl)aniline (53 mg), Pd₂(dba)₃ (13 mg), 2-dicyclohexylphosphino-2',4',6'-trisopropylbiphenyl (20 mg) and caesium carbonate (68 mg) were mixed in a mixed solvent of 1,4-dioxane (5 mL) and DMF (1 mL), the system was sufficiently purged with nitrogen gas, and the tube was sealed and heated to 101 °C in a microwave reactor for the mixture to react for 2 hours. The reaction solution was evaporated to remove the solvent, and the residue was purified through preparative TLC (10%MeOH/DCM) to obtain the target product (20 mg).

¹H NMR (400 MHz, CDCl₃) δ 8.55 (s, 1H), 7.75 (t, J = 8.0 Hz, 1H), 7.51 (d, J = 8.8 Hz, 2H), 7.15 (d, *J* = 8.0 Hz, 1H), 6.99 (s, 1H), 6.83-6.88 (m, 3H), 6.27 (s, 1H), 3.63-3.71 (m, 1H), 3.28 (s, 6H), 3.24-3.28 (m, 4H), 2.76-2.86 (m, 4H), 2.51 (s, 3H), 1.16 (d, J = 6.8 Hz, 6H).

### Example 7

### ((6-(6-cyclopropyl-2-((4-(4-methylpiperazin-1-yl)phenyl)amino)-7H-pyrrolo[2,3-d]pyrimidin -7-yl)pyridin-2-yl)imino)dimethyl-λ⁶-sulfanone

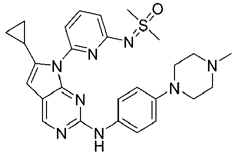

### Step A: ((6-(2-chloro-6-cyclopropyl-7H-pyrrolo[2,3-d]pyrimidin-7-yl)pyridin-2-yl) imino) dimethyl-λ⁶⁶-sulfanone

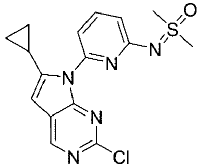

At room temperature, to a tube charged with DMF/TEA (2 ml/2 mL) were added ((6-((5-bromo-2-chloropyrimidin-4-yl)amino)pyridin-2-yl)imino)dimethyl-λ⁶-sulfanone (226 mg), copper(I) iodide (12 mg) and Pd(dppf)Cl₂ (42 mg), the system was purged with nitrogen gas, cyclopropylacetylene (60 mg) was added, the mixture was stirred at 35 °C overnight, cooled to room temperature, filtered, and washed with dichloromethane, the solvent was removed from the filtrate, and the residue was purified through silica gel column chromatography (DCM/MeOH=30/1) to obtain the product (50 mg).

¹H NMR (400 MHz CDCl₃) δ 8.69 (s, 1H), 7.72 (t, J = 8.0 Hz, 1H), 7.06 (d, J = 7.6 Hz, 1H), 6.85 (d, J = 8.0 Hz, 1H), 6.18 (s, 1H), 3.43 (s, 6H), 2.12-2.18 (m, 1H), 0.99-1.05 (m, 2H), 0.78-0.83 (m, 2H).

### Step B: ((6-(6-cyclopropyl-2-((4-(4-methylpiperazin-1-yl)phenyl)amino)-7H-pyrrolo [2,3-d] pyrimidin-7-yl)pyridin-2-yl)imino)dimethyl-λ⁶-sulfanone

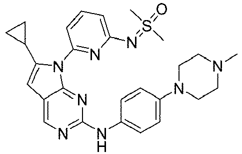

((6-(2-Chloro-6-cyclopropyl-7*H*-pyrrolo[2,3-d]pyrimidin-7-yl)pyridin-2-yl)imino)dimethyl-λ ⁶-sulfanone (40 mg), 4-(4-methylpiperazin-1-yl)aniline (40 mg), Pd₂(dba)₃ (9 mg), 2-dicyclohexyl phosphino-2',4',6'-triisopropylbiphenyl (14 mg) and caesium carbonate (52 mg) were mixed in a mixed solvent of 1,4-dioxane (5 mL) and DMF (1 mL), the system was sufficiently purged with nitrogen gas, and the tube was heated in a microwave reactor to 101 °C for the mixture to react for 2 hours. The reaction solution was evaporated to remove the solvent, and the residue was purified through preparative TLC (10%MeOH/DCM) to obtain the target product (10 mg).

¹H NMR (400 MHz, CDCl₃) δ 8.50 (s, 1H), 7.76 (t, *J =* 8.0 Hz, 1H), 7.54 (d, *J =* 8.8 Hz, 2H), 7.20 (d, J = 8.0 Hz, 1H), 7.23-7.28(m, 1H), 6.83-6.87 (m, 3H), 6.08 (s, 1H), 3.46-3.53 (m, 4H), 3.31 (s, 6H), 3.15-3.29 (m, 4H), 2.80 (s, 3H), 2.31-2.39 (m, 1H), 0.86-0.94 (m, 2H), 0.66-0.71 (m, 2H).

### Example 8

### methyl 4-(4-((7-(6-((dimethyl(oxo)-λ⁶-sulfanylidene)amino)pyridin-2-yl)-5-fluoro-6- propyl-7H-pyrrolo[2,3-d]pyrimidin-2-yl)amino)phenyl)piperazine-1-carboxylate

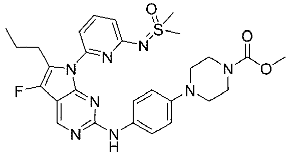

### Step A: 1-(4-nitrophenyl)piperazine

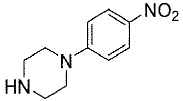

Under nitrogen gas, a solution of piperazine (4.6 g), 4-fluoronitrobenzene (5.0 g) and triethylamine (7.2 g) in acetonitrile (50 mL) was heated and refluxed overnight. The mixture was cooled to room temperature and partitioned between water and dichloromethane, the solvent was removed from the organic phase, and the residue was purified through silica gel column chromatography (100%EtOAc) to obtain the product (5.0 g).

¹H NMR (400 MHz CDCl₃) δ 8.13 (d, J = 9.6 Hz, 2H), 6.83 (d, J = 9.6 Hz, 2H), 3.38-3.41 (m, 4H), 3.02-3.04 (m, 4H).

### Step B: methyl 4-(4-nitrophenyl)piperazine-1-carboxylate

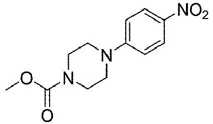

A solution of 1-(4-nitrophenyl)piperazine (5.0 g) and ethyldiisopropylamine (4.58 g) in tetrahydrofuran (100 mL) was cooled to 0 °C, with stirring, to the system was dropwise added methyl carbonochloridate (3.17 g), the mixture was let warm up to room temperature and stirred for 1 hour, water was added to quench the reaction, the mixture was extracted with dichloromethane, the organic phase was evaporated to remove the solvent, and the crude product was purified through silica gel column chromatography (50%EtOAc/PE) to obtain the product (4.0 g).

¹H NMR (400 MHz, CDCl₃) δ 8.14 (d, J = 9.6 Hz, 2H), 6.83 (d, *J* = 9.6 Hz, 2H), 3.75 (s, 3H), 3.63-3.69 (m, 4H), 3.41-3.46 (m, 4H).

### Step C: methyl 4-(4-aminophenyl)piperazine-1-carboxylate

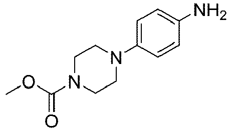

To a flask were sequentially added methyl 4-(4-nitrophenyl)piperazine-1-carboxylate (300 mg), 10% Pd/C (10 mg) and methanol (10 mL), the system was sufficiently purged with hydrogen gas, and the mixture was stirred at room temperature overnight. The reaction solution was filtered through celite, and the filtrate was concentrated and purified through silica gel column chromatography (100%EtOAc) to obtain the product (100 mg).

¹H NMR (400 MHz, CDCl₃) δ 6.81 (d, J = 8.8 Hz, 2H), 6.66 (d, J = 8.8 Hz, 2H), 3.72 (s, 3H), 3.58-3.65 (m, 4H), 2.94-3.02 (m, 4H).

### Step D: methyl 4-(4-((7-(6-((dimethyl(oxo)-λ⁶-sulfanylidene)amino)pyridin-2-yl)-5- fluoro-6-propyl-7H-pyrrolo[2,3-d]pyrimidin-2-yl)amino)phenyl)piperazine-1-carboxylate

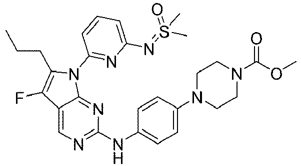

According to the method of step F of example 1, ((6-(2-chloro-5-fluoro-6-propyl-7*H*-pyrrolo[2,3-d]pyrimidin-7-yl)pyridin-2-yl)imino)dimethyl-λ⁶-sulfanone and methyl 4-(4-aminophenyl)piperazine-1-carboxylate were used as the starting material to obtain the product (12 mg).

¹H NMR (400 MHz, CDCl₃) δ 8.61 (s, 1H), 7.72 (t, J = 8.0 Hz, 1H), 7.52 (d, J = 8.8 Hz, 2H), 7.20 (d, J = 8.0 Hz, 1H), 7.18-7.20(brs, 1H), 6.86 (d, J = 8.8 Hz, 2H), 6.80 (d, J = 8.0 Hz, 1H), 3.73 (s, 3H), 3.59-3.67 (m, 4H), 3.29 (s, 6H), 3.01-3.09 (m, 6H), 1.33-1.42 (m, 2H), 0.84 (t, J = 7.2 Hz, 3H).

### Example 9

### ((6-(5-fluoro-2-((4-morpholinophenyl)amino)-6-propyl-7H-pyrrolo[2,3-d]pyrimidin-7-yl)pyri din-2-yl)imino)dimethyl-λ⁶-sulfanone

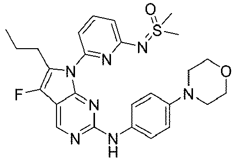

### Step A: N-(4-nitrophenyl)morpholine

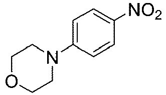

According to the method of step A of example 8, morpholine and 1-Fluoro-4-nitrobenzene were used as the starting material to obtain the product (28 g).

¹H NMR (400 MHz, CDCl₃) δ 8.15 (d, J = 9.2 Hz, 2H), 6.84 (d, J = 9.2 Hz, 2H), 3.85-3.89 (m, 4H), 3.36-3.39 (m, 4H).

### Step B: 4-morpholinoaniline

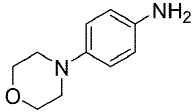

According to the method of step C of example 8, N-(4-nitrophenyl)morpholine was used as the starting material to obtain the product (10 g).

¹H NMR (400 MHz, CDCl₃) δ 6.80 (d, J = 8.8 Hz, 2H), 6.67 (d, J = 8.8 Hz, 2H), 3.83-3.87(m, 4H), 2.99-3.05(m, 4H).

### Step C: ((6-(5-fluoro-2-((4-morpholinophenyl)amino)-6-propyl-7H-pyrrolo[2,3-d] pyrimidin-7- yl) pyridin-2-yl)imino)dimethyl-λ⁶-sulfanone

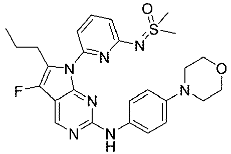

According to the method of step F of example 1, ((6-(2-chloro-5-fluoro-6-propyl- 7H-pyrrolo[2,3-d]pyrimidin-7-yl)pyridin-2-yl)imino)dimethyl-λ⁶-sulfanone and 4-morpholinoaniline were used as the starting material to obtain the product (8 mg).

¹H NMR (400 MHz, CDCl₃) δ 8.61 (s, 1H), 7.72 (t, J = 8.0 Hz, 1H), 7.52 (d, J = 9.2 Hz, 2H), 7.20 (d, J = 8.0 Hz, 1H), 7.18-7.20 (brs, 1H), 6.85 (d, J = 9.2 Hz, 2H), 6.80 (d, *J* = 8.0 Hz, 1H), 3.83-3.91 (m, 4H), 3.29 (s, 6H), 3.07-3.13 (m, 4H), 3.03 (t, J = 7.6 Hz, 2H), 1.32-1.42 (m, 2H), 0.84 (t, J = 7.6 Hz, 3H).

### Example 10

### methyl 4-(4-((7-(6-((dimethyl(oxo)_λ⁶-sulfanylidene)amino)pyridin-2-yl)-6-propyl-7H-pyrrolo [2,3-d]pyrimidin-2-yl)amino)phenyl)piperazine-1-carboxylate

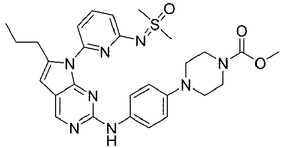

((6-(2-Chloro-6-propyl-7*H*-pyrrolo[2,3-d]pyrimidin-7-yl)pyridin-2-yl)imino)dimethyl-λ⁶-sulf anone (10 mg), methyl 4-(4-aminophenyl)piperazine-1-carboxylate (13 mg), Pd₂(dba)₃ (2.5 mg), 2-dicyclohexylphosphino-2',4',6'-trisopropylbiphenyl (4 mg) and caesium carbonate (13 mg) were mixed in a mixed solvent of 1,4-dioxane (5 mL) and DMF (1 mL), Under nitrogen protection, the mixture was heated to 100 °C and stirred overnight. The reaction solution was evaporated to remove the solvent, and the residue was purified through preparative TLC (10%MeOH/DCM) to obtain the target product (3 mg).

¹H NMR (400 MHz, CDCl₃) δ 8.53 (s, 1H), 7.74 (t, J = 8.0 Hz, 1H), 7.53 (d, J = 9.2 Hz, 2H), 7.18 (d, J = 8.0 Hz, 1H), 6.86 (d, J = 8.8 Hz, 2H), 6.81 (d, J = 8.0 Hz, 1H), 6.25 (s, 1H), 3.74 (s, 3H), 3.61-3.67 (m, 4H), 3.30 (s, 6H), 3.02-3.08 (m, 4H), 2.95 (t, *J =* 7.2 Hz, 2H), 1.51-1.59 (m, 2H), 0.93 (t, *J* = 7.2 Hz, 3H).

### Example 11

### ((6-(2-((4-(morpholine-1-yl)phenyl)amino)-6-propyl-7H-pyrrolo[2,3-d]pyrimidin-7-yl)pyridi n-2-yl)imino)dimethyl-λ⁶-sulfanone

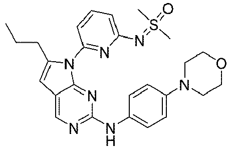

((6-(2-Chloro-6-propyl-7*H*-pyrrolo[2,3-d]pyrimidin-7-yl)pyridin-2-yl)imino)dimethyl-λ⁶-sulf anone (10 mg), 4-(morpholine-1-yl)aniline (10 mg), Pd₂(dba)₃ (2.5 mg), 2-dicyclohexyl phosphino-2',4',6'-triisopropylbiphenyl (4 mg) and caesium carbonate (13 mg) were mixed in a mixed solvent of 1,4-dioxane (5 mL) and DMF (1 mL). Under nitrogen protection, the mixture was heated to 100 °C and stirred overnight. The reaction solution was evaporated to remove the solvent, and the residue was purified through preparative TLC (10%MeOH/DCM) to obtain the target product (3 mg).

¹H NMR (400 MHz, CDCl₃) δ 8.50 (s, 1H), 7.74 (t, J = 8.0 Hz, 1H), 7.53 (d, J = 8.4 Hz, 2H), 7.20-7.25 (m, 1H), 7.13 (d, J = 8.0 Hz, 1H), 6.80-6.85 (m, 3H), 6.27 (s, 1H), 3.83-3.89 (m, 4H), 3.27 (s, 6H), 3.06-3.12 (m, 4H), 2.92 (t, J = 7.6 Hz, 2H), 1.49-1.58 (m, 2H), 0.93 (t, J = 7.6 Hz, 3H).

### Example 12

### ((6-(6-cyclopropyl-5-fluoro-2-((4-(4-methylpiperazin-1-yl)phenyl)amino)-7H-pyrrolo[2,3-d] pyrimidin-7-yl)pyridin-2-yl)imino)dimethyl-λ⁶-sulfanone

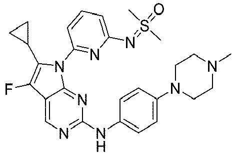

### Step A: ((6-(2-chloro-6-cyclopropyl-5-fluoro-7H-pyrrolo[2,3-d]pyrimidin-7-yl)pyridin-2-yl) imino)dimethyl-λ⁶-sulfanone

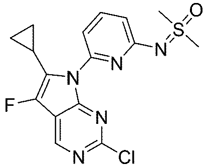

At room temperature, ((6-(2-chloro-6-cyclopropyl-7*H*-pyrrolo[2,3-d]pyrimidin- 7-yl)pyridin-2-yl)imino)dimethyl-λ⁶-sulfanone (280 mg) was added to acetonitrile (10 mL, with ice-water bath and nitrogen gas protection, Selectfluor^{®} (248 mg) was added, the mixture was stirred for 1 hour, water was added to quench the reaction, the mixture was extracted with ethyl acetate, the extract was dried over anhydrous sodium sulfate, the solvent was removed, and the residue was purified through silica gel column chromatography (DCM/EA=1/1) to obtain the product (100 mg).

¹H NMR (400 MHz, CDCl₃) δ 8.78 (s, 1H), 7.72 (t, J = 8.0 Hz, 1H), 7.05 (d, J = 7.6 Hz, 1H), 6.85 (d, *J* = 8.0 Hz, 1H), 3.40 (s, 6H), 2.05-2.11 (m, 1H), 0.88-0.99 (m, 4H).

### Step B: ((6-(6-cyclopropyl-5-fluoro-2-((4-(4-methylpiperazin-1-yl)phenyl)amino)-7H-pyrrolo[2,3-d]pyrimidin-7-yl)pyridin-2-yl)imino)dimethyl-λ⁶-sulfanone

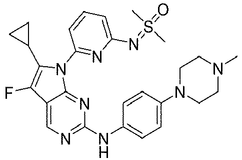

((6-(2-Chloro-6-cyclopropyl-5-fluoro-7*H*-pyrrolo[2,3-d]pyrimidin-7-yl)pyridin-2-yl)imino)di methyl-λ⁶-sulfanone (50 mg), 4-(4-methylpiperazin-1-yl)aniline (50 mg), Pd₂(dba)₃ (12 mg), 2-dicyclohexylphosphino-2',4',6'-trisopropylbiphenyl (19 mg) and caesium carbonate (65 mg) were mixed in a mixed solvent of 1,4-dioxane (5 mL) and DMF (1 mL), the system was sufficiently purged with nitrogen gas, and the tube was heated in a microwave reactor to 101 °C for the mixture to react for 2 hours. The reaction solution was evaporated to remove the solvent, and the residue was purified through preparative TLC (10%MeOH/DCM) to obtain the target product (8 mg).

¹H NMR (400 MHz, CDCl₃) δ 8.58 (s, 1H), 7.73 (t, *J =* 8.0 Hz, 1H), 7.52 (d, *J =* 8.8 Hz, 2H), 7.21 (d, J = 7.6 Hz, 1H), 7.03 (s, 1H), 6.87 (d, J = 8.8 Hz, 2H), 6.81 (d, J = 8.0 Hz, 1H), 3.36-3.43 (m, 4H), 3.30 (s, 6H), 2.96-3.09 (m, 4H), 2.66 (s, 3H), 2.18-2.27 (m, 1H), 0.82-0.91 (m, 2H), 0.72-0.78 (m, 2H).

### Example 13

### ((6-(6-methyl-2-((4-(4-methylpiperazin-1-yl)phenyl)amino)-7H-pyrrolo[2,3-d]pyrimidin-7-yl) pyridin-2-yl)imino)dimethyl-λ⁶-sulfanone

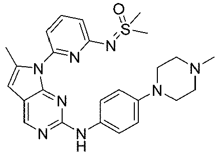

### Step A: ((6-(2-chloro-6-hydroxymethyl-7H-pyrrolo[2,3-d]pyrimidin-7-yl)pyridin-2-yl) imino) dimethyl-λ⁶-sulfanone

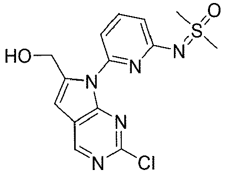

Under nitrogen gas, to a solution of ((6-((5-bromo-2-chloropyrimidin-4-yl)amino) pyridin-1-yl)imino)dimethyl-λ⁶-sulfanone (0.756 g), copper(I) iodide (0.191g) and Pd(dppf)Cl₂ (0.143g) in DMF (10 mL) and triethylamine (10 mL) was slowly added prop-2-yn-1-ol (0.135 g), the mixture was heated to 50 °C and stirred overnight, the reaction solution was filtered through celite and washed with dichloromethane, the solvent was removed from the filtrate, and the residue was purified through silica gel column chromatography (50%EtOAc/PE-100%EtOAc) to obtain the product (0.25g).

¹H NMR (400 MHz, CDCl₃) δ 8.84 (s, 1H), 7.79 (t, *J =* 8.0 Hz, 1H), 7.29 (d, *J =* 8.0 Hz, 1H), 6.85 (d, J=8.0Hz, 1H), 6.70 (s, 1H), 4.69 (d, J = 6.8 Hz, 2H), 4.57 (t, J = 6.8 Hz, 1H), 3.30 (s, 6H).

### Step B: ((6-(2-chloro-6-methyl-7H-pyrrolo[2,3-d]pyrimidin-7-yl)pyridin-2-yl)imino) dimethyl-λ⁶-sulfanone

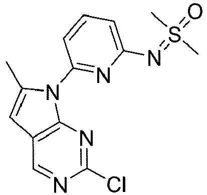

Under nitrogen gas, to a solution of ((6-(2-chloro-6-hydroxymethyl-7*H*-pyrrolo[2,3-*d*] pyrimidin-7-yl)pyiridin-2-yl)imino)dimethyl-λ⁶-sulfanone (112 mg) in dichloromethane (3 mL) were added trifluoroacetic acid (1.5 mL) and triethylsilane (10 mL), and the mixture was heated to 70 °C and stirred overnight. After the temperature was cooled to room temperature, an aqueous solution of saturated sodium bicarbonate was added, the mixture was extracted with dichloromethane, and the organic phase was concentrated and purified through preparative TLC (100%EtOAc) to obtain the product (30 mg).

¹H NMR (400 MHz, CDCl₃) δ 8.72 (s, 1H), 7.75 (t, J = 8.0 Hz, 1H), 6.99 (d, J = 8.0 Hz, 1H), 6.85 (d, *J* = 8.0 Hz, 1H), 6.40 (s, 1H), 3.39 (s, 6H), 2.54 (s, 3H).

### Step C: ((6-(6-methyl-2-((4-(4-methylpiperazin-1-yl)phenyl)amino)-7H-pyrrolo[2,3-d] pyrimidin-7-yl)pyridin-2-yl)imino)dimethyl-λ⁶-sulfanone

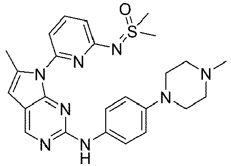

((6-(2-Chloro-6-methyl-7*H*-pyrrolo[2,3-d]pyrimidin-7-yl)pyridin-2-yl)imino)dimethyl-λ⁶-sulf anone (33 mg), 4-(4-methylpiperazin-1-yl)aniline (20 mg), Pd₂(dba)₃ (10 mg), 2-dicyclohexylphosphino-2',4',6'-trisopropylbiphenyl (15 mg) and caesium carbonate (49 mg) were mixed in a mixed solvent of 1,4-dioxane (5 mL) and DMF (1 mL), the system was sufficiently purged with nitrogen gas, the tube was sealed and heated in a microwave reactor to 95 °C for the mixture to react for 4 hours. The reaction solution was evaporated to remove the solvent, and the residue was purified through preparative TLC (10%MeOH/DCM) to obtain the target product (10 mg).

¹H NMR (400 MHz, CD₃OD) δ 8.42 (s, 1H), 7.73 (t, J = 8.0 Hz, 1H), 7.50-7.55 (m, 8H), 7.47 (d, J = 8.4 Hz, 2H), 7.08 (d, J = 8.4 Hz, 1H), 6.82 (d, J = 8.0 Hz, 2H), 6.72 (d, J = 8.0 Hz, 1H), 6.21 (s, 1H), 3.32 (s, 6H), 3.25-3.11(m, 8H), 2.72 (s, 3H), 2.37 (s, 3H).

### Example 14

### ((6-(5-fluoro-2-((4-((2-(dimethylamino)ethyl)(methyl)amino)phenyl)amino)-6-cyclopropyl-7 H-pyrrolo[2,3-dlpyrimidin-7-yl)pyridin-2-yl)imino)dimethyl-λ⁶-sulfanone

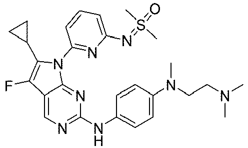

### Step A: N¹,N¹,N²-trimethyl-N²-(4-nitrophenyl)-ethane-1,2-diamine

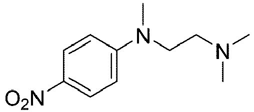

Under nitrogen gas, a suspension of N¹,N¹,N²-trimethyl-ethane-1,2-diamine (1.0 g), 4-fluoronitrobenzene (1.4 g) and potassium carbonate (4.2 g) in dimethyl sulfoxide (20 mL) was heated to 100 °C and stirred overnight. The mixture was cooled to room temperature and partitioned between water and dichloromethane, the solvent was removed from the organic phase, and the residue was purified through silica gel column chromatography (1%MeOH/DCM) to obtain the product (1.7 g).

¹H NMR (400 MHz, CDCl₃) δ 8.11 (d, J = 9.2 Hz, 2H), 6.61 (d, J = 9.2 Hz, 2H), 3.55 (t, J = 7.2 Hz, 2H), 3.10 (s, 3H), 2.50 *(t, J=* 7.2 Hz, 2H), 2.30 (s, 6H).

### Step B: N¹-(2-(dimethylamino)ethyl)-N¹-methylbenzene-1,4-diamine

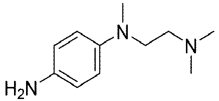

To a flask were sequentially added N¹,N¹,N²-trimethyl-N²-(4-nitrophenyl)-ethane-1,2-diamine (1.7 g), 10% Pd/C (500 mg) and methanol (15 mL), the system was sufficiently purged with hydrogen gas, and the mixture was stirred at room temperature overnight. The reaction solution was filtered through celite, and the filtrate was concentrated and purified through silica gel column chromatography (10%MeOH/DCM) to obtain the product (1.4 g).

¹H NMR (400 MHz, CDCl₃) δ 6.61-6.70 (m, 4H), 3.33 (t, J = 7.6 Hz, 2H), 2.96-3.28 (brs, 2H), 2.84 (s, 3H), 2.46 (t, J = 7.6 Hz, 2H), 2.29 (s, 6H).

### Step C: ((6-(5-fluoro-2-((4-((2-(dimethylamino)ethyl)(methyl)amino)phenyl)amino)-6-cyclopropyl-7H-pyrrolo[2,3-d]pyrimidin-7-yl)pyridin-2-yl)imino)dimethyl-λ⁶-sulfanone

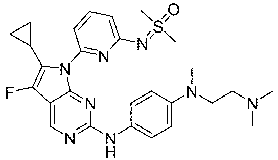

((6-(2-Chloro-5-fluoro-6-cyclopropyl-7*H*-pyrrolo[2,3-d]pyrimidin-7-yl)pyridin-2-yl)imino)di methyl-λ⁶-sulfanone (25 mg), N¹-(2-(dimethylamino)ethyl)-N¹-methylbenzene-1,4-diamine (30 mg), Pd₂(dba)₃ (6 mg), 2-dicyclohexylphosphino-2',4',6'-triisopropylbiphenyl (8 mg) and caesium carbonate (43 mg) were mixed in 1,4-dioxane (5 mL). Under nitrogen protection, the mixture was heated to 100 °C and stirred overnight. The reaction solution was evaporated to remove the solvent, and the residue was purified through preparative TLC (10%MeOH/DCM) to obtain the target product (4 mg).

¹H NMR (400 MHz, CDCl₃) δ 8.57 (s, 1H), 7.76 (t, *J =* 8.0 Hz, 1H), 7.48 (d, *J =* 8.8 Hz, 2H), 7.19 (d, J = 8.0 Hz, 1H), 6.97 (s, 1H), 6.80 (d, J = 8.0 Hz, 1H), 6.76 (d, J = 8.8 Hz, 2H), 3.85 (t, *J* = 6.8 Hz, 2H), 3.28 (s, 6H), 3.11 (t, *J* = 6.8 Hz, 2H), 2.96 (s, 3H), 2.83 (s, 6H), 2.15-2.22 (m, 1H), 0.83-0.90 (m, 2H), 0.73-0.77 (m, 2H).

### Example 15

### ((6-(5-fluoro-2-((4-(4-(dimethylamino)piperidin-1-yl)phenyl)amino)-6-cyclopropyl-7H-pyrro lo[2,3-d]pyrimidin-7-yl)pyridin-2-yl)imino)dimethyl-λ⁶-sulfanone

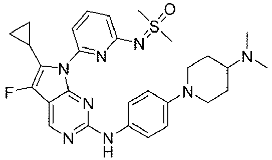

### Step A: N,N-dimethyl-1-(4-nitrophenyl)piperidin-4-amine

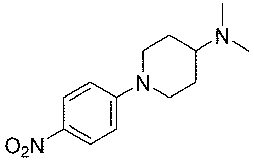

Under nitrogen gas, a suspension of N,N-dimethylpiperidin-4-amine hydrochloride (1.0 g), 4-fluoronitrobenzene (706 mg) and potassium carbonate (2.8 g) in dimethyl sulfoxide (20 mL) was heated to 100 °C and stirred overnight. The mixture was cooled to room temperature and partitioned between water and dichloromethane, the solvent was removed from the organic phase, and the residue was purified through silica gel column chromatography (1%MeOH/DCM) to obtain the product (1.2 g).

¹H NMR (400 MHz, CDCl₃) δ 8.11 (d, J = 9.6 Hz, 2H), 6.81 (d, J = 9.6 Hz, 2H), 3.85-4.01 (m, 2H), 2.94-3.03 (m, 2H), 2.36-2.43 (m, 1H), 2.31 (s, 6H),, 1.91-1.98 (m, 2H), 1.52-1.64 (m, 2H).

### Step B: 1-(4-aminophenyl)-N,N-dimethylpiperidin-4-amine

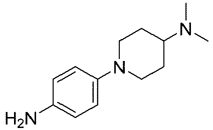

To a flask were sequentially added N,N-dimethyl-1-(4-nitrophenyl)piperidin-4-amine (1.2 g), 10% Pd/C (360 mg) and methanol (10 mL). After being sufficiently purged with hydrogen gas, the mixture was stirred at room temperature overnight. The reaction solution was filtered through celite, and the filtrate was concentrated and purified through silica gel column chromatography (10%MeOH/DCM) to obtain the product (900 mg).

¹H NMR (400 MHz, CDCl₃) δ 6.82 (d, J = 8.8 Hz, 2H), 6.64 (d, J = 8.8 Hz, 2H), 3.48-3.56 (m, 2H), 3.43 (s, 2H), 2.58-2.64 (m, 2H), 2.33-2.42 (m, 7H), 1.93-2.01 (m, 2H), 1.64-1.77 (m, 2H).

### Step C: ((6-(5-fluoro-2-((4-(4-(dimethylamino)piperidin-1-yl)phenyl)amino)-6- cyclopropyl-7H-pyrrolo[2,3-d]pyrimidin-7-yl)pyridin-2-yl)imino)dimethyl-λ⁶-sulfanone

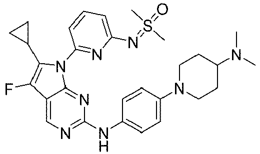

((6-(2-Chloro-5-fluoro-6-cyclopropyl-7*H*-pyrrolo[2,3-d]pyrimidin-7-yl)pyridin-2-yl)imino)di methyl-λ⁶-sulfanone (30 mg), 1-(4-aminophenyl)-N,N-dimethylpiperidin-4-amine (35 mg), Pd₂(dba)₃ (8 mg), 2-dicyclohexylphosphino-2',4',6'-triisopropylbiphenyl (12 mg) and caesium carbonate (40 mg) were mixed in 1,4-dioxane (5 mL). Under nitrogen protection, the mixture was heated to 100 °C and stirred overnight. The reaction solution was evaporated to remove the solvent, and the residue was purified through preparative TLC (10%MeOH/DCM) to obtain the target product (6 mg).

¹H NMR (400 MHz, CDCl₃) δ 12.15-12.20 (brs, 1H), 8.61 (s, 1H), 7.73 (t, J = 8.0 Hz, 1H), 7.51 (d, J = 8.0 Hz, 2H), 7.19 (d, J = 8.0 Hz, 1H), 6.80-6.85 (m, 3H), 3.64-3.73 (m, 2H), 3.30 (s, 6H), 3.17-3.28 (m, 1H), 2.81 (s, 6H), 2.74 (t, J = 12.0 Hz, 2H), 2.18-2.30 (m, 3H), 1.86-1.99 (m, 2H), 0.82-0.91 (m, 2H), 0.72-0.78 (m, 2H).

### Example 16

### 6-cyclopropyl-5-fluoro-7-(6-isopropylpyridin-2-yl)-N-4-(4-methylpiperazin-1-yl)phenyl)-7H -pyrrolo [2,3 -d]pyrimidin-2-amine

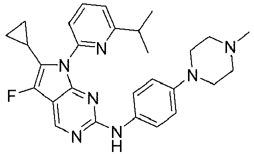

### Step A: 5-bromo-2-chloro-N-(6-isopropylpyridin-2-yl)pyrimidin-4-amine

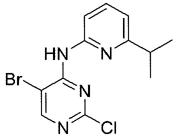

According to the method of step C of example CT-4500, 2-isopropyl-6-aminopyridine and 5-bromo-2,4-dichloropyrimidine were used as the starting material to obtain the product (1.9 g).

¹H NMR (400 MHz CDCl₃) δ 8.36 (s, 1H), 8.17 (d, *J* = 8.0 Hz, 1H), 7.97 (s, 1H), 7.71 (t, J = 8.0 Hz, 1H), 6.97 (d, J = 8.0 Hz, 1H), 2.93-3.01 (m, 1H), 1.29 (t, J = 6.8 Hz, 6H).

### Step B: 2-chloro-6-cyclopropyl-7-(6-isopropylpyridin-2-yl)-7H-pyrrolo[2,3-d] pyrimidine

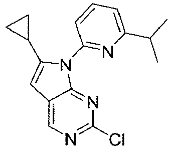

According to the method of step D of example CT-4500, 5-bromo-2-chloro-N-(6-isopropylpyridin-2-yl)pyrimidin-4-amine and cyclopropylacetylene were used as the starting material to obtain the product (250 mg).

¹H NMR (400 MHz, CDCl₃) δ 8.71 (s, 1H), 7.86 (t, J = 8.0 Hz, 1H), 7.50 (d, J = 8.0 Hz, 1H), 7.25 (d, *J =* 8.0 Hz, 1H), 6.26 (s, 1H), 3.07-3.15 (m, 1H), 2.25-2.33(m, 1H), 1.31 (d, *J =* 7.2 Hz, 6H), 0.86-0.92 (m, 2H), 0.73-0.77 (m, 2H).

### Step C: 2-chloro-6-cyclopropyl 5-fluoro-7-(6-isopropylpyridin-2-yl)-7H-pyrrolo [2,3-d] pyrimidine

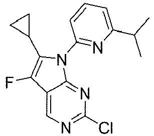

According to the method of step E of example CT-4500, 2-chloro-6-cyclopropyl-7-(6-isopropylpyridin-2-yl)-7*H*-pyrrolo[2,3-*d*]pyrimidine and Selectfluor^{®} were used as the starting material to obtain the product (40 mg).

¹H NMR (400 MHz, CDCl₃) δ 8.80 (s, 1H), 7.85 (t, J = 8.0 Hz, 1H), 7.51 (d, J = 8.0 Hz, 1H), 7.24 (d, *J* = 8.0 Hz, 1H), 3.05-3.14 (m, 1H), 2.13-2.21 (m, 1H), 1.30 (d, *J* = 7.2 Hz, 6H), 0.81-0.87 (m, 4H).

### Step D: 6-cyclopropyl-5-fluoro-7-(6-isopropylpyridin-2-yl)-N-4-(4-methylpiperazin-1-yl) phenyl)-7H-pyrrolo[2,3-d]pyrimidin-2-amine

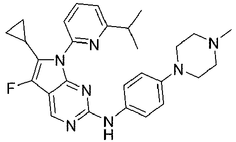

According to the method of step F of example 1, 2-chloro-6-cyclopropyl-5-fluoro-7-(6-isopropylpyridin-2-yl)-7*H*-pyrrolo[2,3-*d*]pyrimidine and 4-(4-methylpiperazin-1-yl) aniline were used as the starting material to obtain the product (8 mg).

¹H NMR (400 MHz, CDCl₃) δ 8.59 (s, 1H), 7.84 (t, J = 8.0 Hz, 1H), 7.61 (d, J = 8.0 Hz, 1H), 7.55 (d, *J* = 8.8 Hz, 2H), 7.24 (s, 1H), 7.20 (d, *J* = 8.0 Hz, 1H), 6.87 (d, *J* = 8.0 Hz, 2H), 3.44-3.11 (m, 4H), 3.06-3.28 (m, 5H), 2.78(s, 3H), 2.15-2.24 (m, 1H), 1.32 (d, *J* = 6.8 Hz, 6H), 0.68-0.82 (m, 4H).

### Example 17

### (6-(6-allyl-2-((4-(4-methylpiperazin-1-yl)phenyl)amino)-7H-pyrrolo[2,3-d]pyrimidin-7-yl)py ridin-2-yl)imino)dimethyl-λ⁶-sulfanone

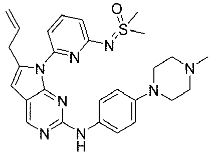

### Step A: ((6-(2-chloro-6-(2-hyroxyethyl)-7H-pyrrolo[2,3-d]pyrimidin-7-yl)pyridin-2-yl) imino) dimethyl-λ⁶-sulfanone

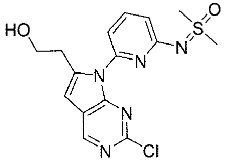

Under nitrogen gas, to a solution of ((6-((5-bromo-2-chloropyrimidin-4-yl) amino) pyridin-1-yl) imino)dimethyl-λ⁶-sulfanone (0.756 g), copper(I) iodide (0.191 g) and Pd(dppf)Cl₂ (0.143 g) in DMF (10 mL) and triethylamine (10 mL) was slowly added 3-butyne-1-ol (0.140g), the mixture was stirred at room temperature overnight, heated to 50 °C and stirred overnight, the reaction solution was filtered through celite and washed with dichloromethane, the solvent was removed from the filtrate, and the residue was purified through silica gel column chromatography (50%EtOAc/PE-100%EtOAc) to obtain the product (0.37g).

¹H NMR (400 MHz, CDCl₃) δ 8.74 (s, 1H), 7.75 (t, J = 8.0Hz, 1H), 7.11 (d, J = 8.0Hz, 1H), 6.85 (d, *J*=8.0Hz, 1H), 6.53 (s, 1H), 3.84 (t, J = 6.8 Hz, 2H), 3.36 (s, 6H), 3.23 (t, J= 6.8 Hz, 2H), 3.10 (s, 1H).

### Step B: ((6-(2-chloro-6-(2-oxoethyl)-7H-pyrrolo[2,3-d]pyrimidin-7-yl)pyridin-2-yl) imino) dimcthyl-λ⁶-sulfanone

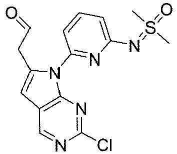

Under nitrogen gas, ((6-(2-chloro-6-(2-hyroxyethyl)-7*H*-pyrrolo[2,3-d]pyrimidin-7-yl) pyridin-2-yl)imino)dimethyl-λ⁶-sulfanone (120 mg) was dissolved in dichloromethane (10 mL), and the mixture was placed in an ice bath. To the solution was added Dess-Martin Periodinane (200 mg), and the mixture was stirred for 15 minutes, let warm up slowly to room temperature and stirred for 1 hour. After the reaction was completed, the mixture was washed with an aqueous solution of saturated sodium bicarbonate and saturated sodium thiosulfate and extracted with dichloromethane, and the organic phase was concentrated and purified through preparative TLC (50%EtOAc/PE) to obtain the product (60 mg).

¹H NMR (400 MHz, CDCl₃) δ 9.70 (s, 1H), 8.81 (s, 1H), 7.75 (t, *J =* 8.0 Hz, 1H), 7.24 (d, J = 8.0 Hz, 1H), 6.79 (d, *J* = 8.0 Hz, 1H), 6.58 (s, 1H), 4.35 (s, 2H), 3.36 (s, 6H).

### Step C: ((6-(2-chloro-6-allyl-7H-pyrrolo[2,3-d]pyrimidin-7-yl)pyridin-2-yl)imino) dimethyl-λ⁶-sulfanonc

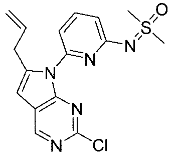

Under nitrogen gas, to a 50 mL dry three-neck flask was added methyltriphenylphosphonium bromide (100 mg), then tetrahydrofuran (3 mL) was added, the reaction was cooled to -20 °C, a solution of 2 mol//L LDA in tetrahydrofuran (0.14 mL) was added using a syringe, the mixture was stirred for 10 minutes, let warm up to 0 °C, and stirred for 1 hour, and then a solution of ((6-(2-chloro-6-allyl-7*H*-pyrrolo[2,3-*d*]pyrimidin-7-yl)pyridin-2-yl)imino)dimethyl-λ⁶-sulfanone (76 mg) in dichloromethane (2 mL) was slowly added, and the mixture was stirred for 15 minutes, let warm up to room temperature slowly and stirred for 2 hours. The mixture was quenched with a solution of saturated ammonium chloride and extracted with dichloromethane, and the organic phase was concentrated and purified through preparative TLC (50%EtOAc/PE) to obtain the product (20 mg).

¹H NMR (400 MHz, CDCl₃) δ8.75 (s, 1H), 7.72 (t, J = 8.0 Hz, 1H), 7.01 (d, J = 8.0 Hz, 1H), 6.84 (d, J = 8.0 Hz, 1H), 6.44 (s, 1H), 5.93-5.81 (m, 1H), 5.11-5.06 (m, 2H), 3.73 (d, J = 6.8 Hz, 2H), 3.37 (s, 6H).

### Step D: (6-(6-allyl-2-((4-(4-methylpiperazin-1-yl)phenyl)amino)-7H-pyrrolo[2,3-d] pyrimidin- 7-yl) pyridin-2-yl)imino)dimethyl-λ⁶-sulfanone

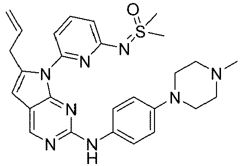

((6-(2-Chloro-6-allyl-7*H*-pyrrolo[2,3-d]pyrimidin-7-yl)pyridin-2-yl)imino)dimethyl-λ⁶-sulfan one (36 mg), 4-(4-methylpiperazin-1-yl)aniline (20 mg), Pd₂(dba)₃ (10 mg), 2-dicyclohexylphosphino-2',4',6'-trisopropylbiphenyl (15 mg) and caesium carbonate (49 mg) were mixed in 1,4-dioxane (5 mL) and DMF (1mL), the system was sufficiently purged with nitrogen gas, and the tube was sealed and heated in a microwave reactor to 85 °C for the mixture to react for 5 hours. The reaction solution was evaporated to remove the solvent, and the residue was purified through preparative TLC (10%MeOH/DCM) to obtain the target product (7 mg).

¹H NMR (400 MHz, CDCl₃) δ8.42 (s, 1H), 7.69 (t, J = 8.0 Hz, 1H), 7.46 (d, J = 8.8 Hz, 2H), 7.07 (d, J = 8.0 Hz, 1H), 6.81 (d, J = 8.8 Hz, 2H), 6.70 (d, J = 8.0 Hz, 1H), 6.24 (s, 1H), 5.78-5.62 (m, 1H), 5.15 (d, J = 12.0 Hz, 1H), 4.90-4.83 (m, 1H), 3.65 (d, J = 6.8 Hz, 2H), 3.24 (s, 6H), 3.18-3.11 (m, 4H), 2.98-2.91 (m, 4H), 2.57 (s, 3H).

### Example 18

### ((6-(6-cyclopropyl-5-fluoro-2-((4-(4-(4-methylpiperazin-1-yl)piperidin-1-yl)phenyl)amino)-7 H-pyrrolo[2,3-d]pyrimidin-7-yl)pyridin-2-yl)imino)dimethyl-λ⁶-sulfanone

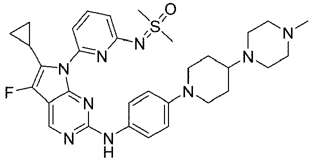

### Step A: 1-methyl-4-(1-(4-nitrophenyl)piperidin-4-yl)piperazine

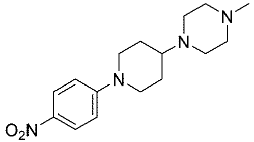

According to the method of step A of example 8, 1-fluoro-4-nitrobenzene and 1-methyl-4-(piperidin-4-yl)-piperazine were used as the starting material to obtain the product (0.5 g).

¹H NMR (400 MHz, CDCl₃) δ 8.11 (d, J = 9.6 Hz, 2H), 6.81 (d, J = 9.6 Hz, 2H), 3.94-4.03 (m, 2H), 2.94-3.02 (m, 2H), 2.56-2.68 (m, 4H), 2.39-2.55 (m, 5H), 2.29 (s, 3H), 1.93-2.01 (m, 2H), 1.57-1.66 (m, 2H).

### Step B: 1-methyl-4-(1-(4-aminophenyl)piperidin-4-yl)piperazine

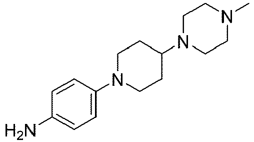

According to the method of step C of example 8, 1-methyl-4-(1-(4-nitrophenyl) piperidin-4-yl) piperazine was used as the starting material to obtain the product (0.4 g).

¹H NMR (400 MHz, CDCl₃) δ 6.81 (d, J = 8.8 Hz, 2H), 6.64 (d, J = 8.8 Hz, 2H), 3.94-3.56 (m, 2H), 3.31-3.48 (brs, 2H), 2.41-2.70 (m, 10H), 2.30-2.39 (m, 1H), 2.30 (s, 3H), 1.87-1.96 (m, 2H), 1.65-1.75 (m, 2H).

### Step C: ((6-(6-cyclopropyl-5-fluoro-2-((4-(4-(4-methylpiperazin-1-yl)piperidin-1-yl) phenyl) amino)-7H-pyrrolo[2,3-d]pyrimidin-7-yl)pyridin-2-yl)imino)dimethyl-λ⁶-sulfanone

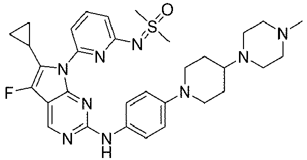

((6-(2-Chloro-6-cyclopropyl-7*H*-pyrrolo[2,3-d]pyrimidin-7-yl)pyridin-2-yl)imino)dimethyl-λ ⁶-sulfanone (50 mg), 4-(4-(4-methylpiperazin-1-yl)piperidin-1-yl)aniline (18 mg), Pd₂(dba)₃ (12 mg), Dave-phos (16 mg) and sodium tert-butoxide (18 mg) were mixed in toluene (3 mL), the system was sufficiently purged with nitrogen gas, and the tube was sealed and heated to 101 °C for the mixture to react for 5 hours. The reaction solution was evaporated to remove the solvent, and the residue was purified through preparative TLC (10%MeOH/DCM) to obtain the target product (10 mg).

¹H NMR (400 MHz, CDCl₃) δ 8.57 (s, 1H), 7.72 (t, *J* = 8.0 Hz, 1H), 7.48 (d, *J* = 8.8 Hz, 2H), 7.21 (d, *J* = 7.6 Hz, 1H), 6.99 (s, 1H), 6.86 (d, *J* = 8.8 Hz, 2H), 6.79 (d, *J* = 8.0 Hz, 1H), 3.59-3.68 (m, 2H), 3.30 (s, 6H), 2.72-3.02 (m, 8H), 2.68 (t, *J* = 12.4 Hz, 2H), 2.48-2.59 (m, 4H), 2.17-2.26 (m, 1H), 1.95-2.05 (m, 2H), 1.67-1.82 (m, 2H), 0.82-0.91 (m, 2H), 0.71-0.77(m, 2H).

### Example 19

### ((6-(6-cyclopropyl-2-((4-(4-(4-methylpiperazin-1-yl)piperidin-1-yl)phenyl)amino)-7H-pyrrol o[2,3-d]pyrimidin-7-yl)pyridin-2-yl)imino)dimethyl-λ⁶-sulfanone

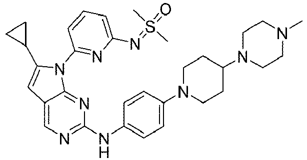

((6-(2-Chloro-6-cyclopropyl-7*H*-pyrrolo[2,3-d]pyrimidin-7-yl)pyridin-2-yl)imino)dimethyl-λ ⁶-sulfanone (144 mg), 4-(4-(4-methylpiperazin-1-yl)piperidin-1-yl)aniline (55 mg), Pd₂(dba)₃ (37 mg), Dave-phos (47 mg) and sodium tert-butoxide (58 mg) were mixed in toluene (5 mL), the system was sufficiently purged with nitrogen gas, and the tube was sealed and heated to 101 °C for the mixture to react for 5 hours. The reaction solution was evaporated to remove the solvent, and the residue was purified through preparative TLC (10%MeOH/DCM) to obtain the target product (100 mg).

¹H NMR (400 MHz, CDCl₃) δ 8.50 (s, 1H), 7.73 (t, *J =* 8.0 Hz, 1H), 7.48 (d, *J =* 8.8 Hz, 2H), 7.21 (d, *J* = 8.0 Hz, 1H), 6.89 (s, 1H), 6.86 (d, *J* = 8.8 Hz, 2H), 6.81 (d, *J* = 8.0 Hz, 1H), 6.05 (s, 1H), 3.62 (d, *J* = 11.6 Hz, 2H), 3.30 (s, 6H), 2.55-2.89 (m, 10H), 2.41-2.53 (m, 1H), 2.41 (s, 3H), 2.29-2.38 (m, 1H), 1.97 (d, *J* = 12.0 Hz, 2H), 1.65-1.78 (m, 2H), 0.87-0.92 (m, 2H), 0.65-0.69 (m, 2H).

### Example 20

### 6-cyclopropyl-7-(6-isobutylpyridin-2-yl)-N-(4-(4-methylpiperazin-1-yl)phenyl)-7H-pyrrolo[2, 3 -d]pyrimidin-2-amine

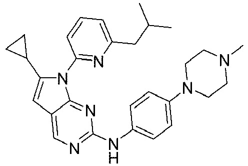

### Step A: 2-(2,5-dimethyl-1H-pyrrol-1-yl)-6-methylpyridine

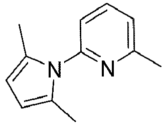

A mixture of 2-amino-6-methylpyridine(10.0 g), 2,5-hexanedione (10.8 mL), acetic acid (1.0 mL) and toluene (100 mL) was heated and refluxed, and the water generated was removed by Dean-Stark Trap. The mixture was refluxed with stirring overnight. The mixture was cooled to room temperature, diluted with dichloromethane, and washed sequentially with an aqueous solution of saturated NaHCO₃ and brine, and the solvent was removed from the organic phase to obtain the product (12.0 g).

¹H NMR (400 MHz CDCl₃) δ 7.70 (t, *J* = 8.0 Hz, 1H), 7.15 (d, *J* = 8.0 Hz, 1H), 7.01 (d, *J* = 8.0 Hz, 1H), 5.88 (s, 2H), 2.59 (s, 3H), 2.12 (s, 6H).

### Step B: 2-(2,5-dimethyl-1H-pyrrol-1-yl)-6-isobutylpyridine

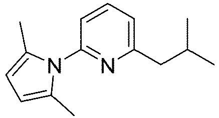

A solution of 2-(2,5-dimethyl-1*H*-pyrrol-1-yl)-6-methylpyridine (1.86 g) in anhydrous diethyl ether (10 mL) was cooled to -70 °C, a solution of 2 mol/L LDA in tetrahydrofuran (7.5 mL) was dropwise added with stirring, after the addition was completed, the mixture was stirred at this temperature for 30 minutes, and then 2-bromopropane (1.85 g) was added. The reaction solution was let gradually warm up to room temperature and stirred at room temperature for 2 hours, and an aqueous solution of saturated ammonium chloride was used to quench the reaction. The reaction solution was extracted with diethyl ether, and the organic phase was washed with brine, concentrated, and purified through silica gel column chromatography (50%EtOAc/PE) to obtain the product (1.5 g).

¹H NMR (400 MHz, CDCl₃) δ7.70 (t, *J* = 7.6 Hz, 1H), 7.10 (d, *J* = 7.6 Hz, 1H), 7.02 (d, *J* = 8.0 Hz, 1H)5.88 (s, 2H), 2.69 (d*, J =* 7.2 Hz, 2H), 2.11-2.20 (m, 1H), 2.12 (s, 6H), 0.93 (d, *J* = 6.8 Hz, 6H).

### Step C: 2-amino-6-isobutylpyridine

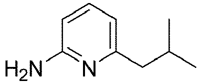

A mixture of 2-(2,5-dimethyl-1*H*-pyrrol-1-yl)-6-isobutylpyridine (1.5 g), hydroxylamine hydrochloride (2.27 g), methanol (10 mL) and water (5 mL) was heated to 100 °C and stirred overnight. The mixture was evaporated to dryness to remove the solvent, the residue was dissolved in dichloromethane, the solution thus obtained was washed with an aqueous solution of saturated NaHCO₃ and brine and evaporated to dryness, and the residue was purified through silica gel column chromatography (5%-20%MeOH/DCM) to obtain the product (0.6 g).

¹H NMR (400 MHz, CDCl₃) δ 7.70 (t, *J* = 7.6 Hz, 1H), 6.46 (d, *J* = 7.6 Hz, 1H), 6.31 (d, *J* = 8.0 Hz, 1H), 4.42 (s, 2H), 2.46 (d, *J* = 7.6 Hz, 2H), 2.01-2.08 (m, 1H), 0.92 (d, *J* = 6.8 Hz, 6H).

### Step D: 5-bromo-2-chloro-N-(6-isobutylpyridin-2-yl)pyrimidin-4-amine

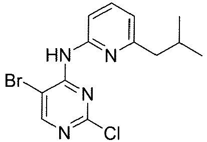

According to the method of step C of example 1, 2-amino-6-isobutylpyridine and 5-bromo-2,4-dichloropyrimidine were used as the starting material to obtain the product (0.66 g).

¹H NMR (400 MHz, CDCl₃) δ 8.36 (s, 1H), 8.18 (d, *J* = 8.4 Hz, 1H), 7.96 (s, 1H), 7.68 (t, *J* = 7.6 Hz, 1H), 6.90 (d, *J* = 7.6 Hz, 1H), 2.58 (d, *J* = 7.2 Hz, 2H), 2.05-2.15 (m, 2H), 0.94 (t, *J* = 6.4 Hz, 6H).

### Step E: 2-chloro-6-cyclopropyl-7-(6-isobutylpyridin-2-yl)-7H-pyrrolo[2,3-d]pyrimidine

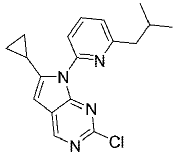

At room temperature, to a tube charged with DMF/TEA (5 ml/5 mL) were added 5-bromo-2-chloro-*N*-(6-isobutylpyridin-2-yl)pyrimidin-4-amine(760 mg), copper(I) iodide (125 mg), and Pd(dppf)Cl₂ (157 mg), the system was purged with nitrogen gas, cyclopropylacetylene (145 mg) was added, the mixture was stirred at 30 °C overnight, cooled to room temperature, filtered, and washed with dichloromethane, the solvent was removed from the filtrate, and the residue was purified through silica gel column chromatography (DCM/MeOH=30/1) to obtain the product (230 mg).

¹H NMR (400 MHz CDCl₃) δ 8.69 (s, 1H), 7.85 (t, *J* = 8.0 Hz, 1H), 7.47 (d, *J* = 8.0 Hz, 1H), 7.22 (d, *J =* 7.6 Hz, 1H), 6.25 (s, 1H), 2.71 (d, *J* = 7.6 Hz, 2H), 2.09-2.24 (m, 2H), 0.95 (d, *J* = 6.8 Hz, 6H), 0.85-0.90 (m, 2H), 0.73-0.77(m, 2H).

### Step F: 6-cyclopropyl-7-(6-isobutylpyridin-2-yl)-N-(4-(4-methylpiperazin-1-yl)phenyl)-7H-pyrrolo [2,3-d]pyrimidin-2-amine

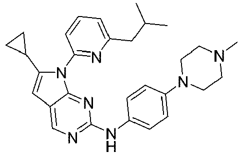

2-Chloro-6-cyclopropyl-7-(6-isobutylpyridin-2-yl)-7*H*-pyrrolo[2,3-*d*|pyrimidine (34 mg), 4-(4-methylpiperazin-1-yl)aniline (19 mg), Pd₂(dba)₃ (9 mg), 2-dicyclohexylphosphino-2',4',6'-triisopropylbiphenyl (14 mg) and caesium carbonate (49 mg) were mixed in a mixed solvent of 1,4-dioxane (5 mL) and DMF (1 mL), the system was sufficiently purged with nitrogen gas, and the tube was heated in a microwave reactor to 101 °C for the mixture to react for 2 hours. The reaction solution was evaporated to remove the solvent, and the residue was purified through preparative TLC (10%MeOH/DCM) to obtain the target product (10 mg).

¹H NMR (400 MHz, CDCl₃) δ 8.51 (s, 1H), 7.81 (t, *J* = 7.6 Hz, 1H), 7.58 (d, *J* = 8.0 Hz, 1H), 7.50 (d, *J* = 7.6 Hz, 2H), 7.16 (d, *J* = 7.6 Hz, 1H), 6.93 (s, 1H), 6.86 (d, *J* = 8.8 Hz, 2H), 6.12 (s, 1H), 3.13-3.19 (m, 4H), 2.72 (d, *J* = 7.6 Hz, 2H), 2.58-2.66 (m, 4H), 2.38 (s, 3H), 2.23-2.29 (m, 1H), 2.14-2.21 (m, 1H), 0.95 (d, *J* = 6.8 Hz, 6H), 0.76-0.81 (m, 2H), 0.65-0.69 (m, 2H).

### Example 21

### 6-cyclopropyl-5-fluoro-7-(6-isobutylpyridin-2-yl)-N-(4-(4-methylpiperazin-1-yl)phenyl)-7H-pyrrolo [2,3 -d]pyrimidin-2-amine

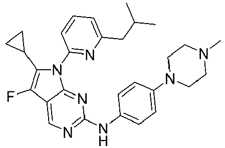

### Step A: 2-chloro-6-cyclopropyl-5 -fluoro-7-(6-isobutylpyridin-2-yl)-7H-pyrrolo[2,3-d] pyrimidine

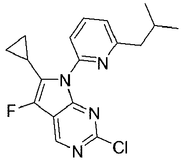

At room temperature, 2-chloro-6-cyclopropyl-7-(6-isobutylpyridin-2-yl)-7*H*-pyrrolo [2,3-*d*|pyrimidine(135 mg) was added to acetonitrile (10 mL), the mixture was cooled in an ice-water bath to 0 °C, Selectfluor^{®} (146 mg) was added under nitrogen gas, the mixture was stirred for 1 hour, water was added, the mixture was extracted with ethyl acetate and dried over anhydrous sodium sulfate, the solvent was removed, and the residue was purified through silica gel column chromatography (DCM/EA=1/1) to obtain the product (50 mg).

¹H NMR (400 MHz, CDCl₃) δ 8.79 (s, 1H), 7.84 (t, *J* = 8.0 Hz, 1H), 7.47 (d, *J* = 8.0 Hz, 1H), 7.21 (d, *J* = 8.0 Hz, 1H), 2.70 (d, *J* = 7.2 Hz, 2H), 2.04-2.18 (m, 2H), 0.95 (d, *J* = 6.8 Hz, 6H), 0.81-0.86 (m, 4H).

### Step B: 6-cyclopropyl-5-fluoro-7-(6-isobutylpyridin-2-yl)-N-(4-(4-methylpiperazin-1-yl) phenyl)-7H-pyrrolo[2,3-d]pyrimidin-2-amine

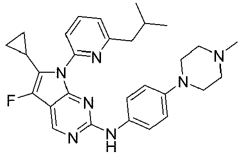

2-Chloro-6-cyclopropyl-5-fluoro-7-(6-isobutylpyridin-2-yl)-7*H*-pyrrolo[2,3-*d*]pyrimidine (42 mg), 4-(4-methylpiperazin-1-yl)aniline (35 mg), Pd₂(dba)₃ (9 mg), 2-dicyclohexyl phosphino-2',4',6'-triisopropylbiphenyl (19 mg) and caesium carbonate (58 mg) were mixed in a mixed solvent of 1,4-dioxane (5 mL) and DMF (1 mL), the system was sufficiently purged with nitrogen gas, and the tube was heated in a microwave reactor to 101 °C for the mixture to react for 2 hours. The reaction solution was evaporated to remove the solvent, and the residue was purified through preparative TLC (10%MeOH/DCM) to obtain the target product (15 mg).

¹H NMR (400 MHz, CDCl₃) δ 8.58 (s, 1H), 7.81 (t, *J* = 8.0 Hz, 1H), 7.57 (d, *J* = 8.0 Hz, 1H), 7.50 (d, *J* = 9.2 Hz, 2H), 7.15 (d, *J* = 7.6 Hz, 1H), 7.01 (s, 1H), 6.86 *(d, J =* 9.2 Hz, 2H), 3.21-3.28 (m, 4H), 2.75-2.86 (m, 4H), 2.70 (d, *J* = 7.2 Hz, 2H), 2.51 (s, 3H), 2.07-2.22 (m, 2H), 0.95 (d, *J* = 6.8 Hz, 6H), 0.69-0.81 (m, 4H).

### Example 22

### ((6-(5-fluoro-2-((4-(4-(1-methylpiperidin-4-yl)piperazin-1-yl)phenyl)amino)-6-cyclopropyl-7 H-pyrrolo[2,3-d]pyrimidin-7-yl)pyridin-2-yl)imino)dimethyl-λ⁶-sulfanone

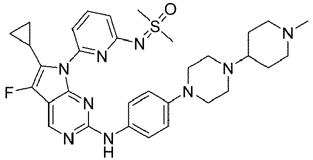

### Step A: 1-(4-nitrophenyl)piperazine

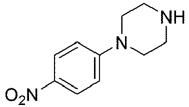

Under nitrogen gas, a solution of anhydrous piperazine (900 mg), 4-fluoronitrobenzene (1.0 g), and potassium carbonate (3.8 g) in dimethyl sulfoxide (20 mL) was heated to 100 °C and stirred overnight. The mixture was cooled to room temperature and partitioned between water and dichloromethane, the solvent was removed from the organic phase, and the residue was purified through silica gel column chromatography (1%MeOH/DCM) to obtain the product (1.1 g).

¹H NMR (400 MHz, CDCl₃) δ 8.13 (d, *J* = 9.2 Hz, 2H), 6.82 (d, *J* = 9.2 Hz, 2H), 3.36-3.42 (m, 4H), 2.99-3.06 (m, 4H).

### Step B: 1-(1-methylpiperidin-4-yl)-4-(4-nitrophenyl)piperazine

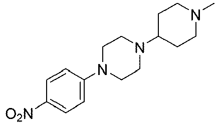

1-(4-Nitrophenyl)piperazine (1.1 g) and 1-methyl-4-piperidone (934 mg) were dissolved in dichloromethane (20 mL), to the mixture was dropwise added glacial acetic acid (1 mL), the mixture was stirred at room temperature for 30 minutes, sodium triacetoxyborohydride (2.3 g) was added, and the mixture was stirred at room temperature overnight. A solution of 1 N sodium hydroxide was used to adjust the pH to basic, the mixture was partitioned between water and dichloromethane, the solvent was removed from the organic phase, and the residue was purified through silica gel column chromatography (1%MeOH/DCM) to obtain the product (1.0 g).

¹H NMR (400 MHz, CDCl₃) δ 8.12 (d, *J* = 9.2 Hz, 2H), 6.81 (d, *J* = 9.2 Hz, 2H), 3.41-3.45 (m, 4H), 2.89-2.96 (m, 2H), 2.68-2.73 (m, 4H), 2.28 (s, 3H), 1.92-2.04 (m, 3H), 1.78-1.85 (m, 2H), 1.57-1.68 (m, 2H).

### Step C: 1-(1-methylpiperidin-4-yl)-4-(4-aminophenyl)piperazine

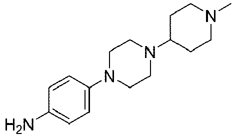

To a flask were sequentially added 1-(1-methylpiperidin-4-yl)-4-(4-nitrophenyl)piperazine (1 g), 10% Pd/C (300 mg) and methanol (20 mL), the system was sufficiently purged with hydrogen gas, and the mixture was stirred at room temperature overnight. The reaction solution was filtered through celite, and the filtrate was concentrated and purified through silica gel column chromatography (10%MeOH/DCM) to obtain the product (800 mg).

¹H NMR (400 MHz, CDCl₃) δ 6.81 (d, *J* = 8.8 Hz, 2H), 6.64 (d, *J* = 8.8 Hz, 2H), 3.32-3.49 (brs, 2H), 3.04-3.08 (m, 4H), 2.89-2.96 (m, 2H), 2.69-2.75 (m, 4H), 2.23-2.32 (m, 4H), 1.94 (td, *J* = 12.0 Hz, 2.4Hz, 2H), 1.80-1.87 (m, 2H), 1.57-1.67 (m, 2H).

### Step D: ((6-(5-fluoro-2-((4-(4-(1-methylpiperidin-4-yl)piperazin-1-yl)phenyl)amino)-6-cyclopropyl-7H-pyrrolo[2,3-d]pyrimidin-7-yl)pyridin-2-yl)imino)dimethyl-λ⁶-sulfanone

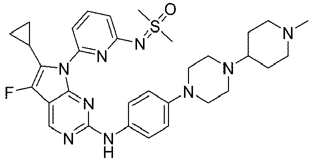

((6-(2-Chloro-5-fluoro-6-cyclopropyl-7*H*-pyrrolo[2,3-d]pyrimidin-7-yl)pyridin-2-yl)imino)di methyl-λ⁶-sulfanone (38 mg), 1-(1-methylpiperidin-4-yl)-4-(4-aminophenyl)piperazine (28 mg), Pd₂(dba)₃ (10 mg), Dave-phos (12 mg) and sodium tert-butoxide (15 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (4 mg).

¹H NMR (400 MHz, CD₃OD) δ 8.55 (s, 1H), 7.80 (t, *J* = 8.0 Hz, 1H), 7.56 (d, *J* = 9.2 Hz, 2H), 7.21 (d, *J* = 8.0 Hz, 1H), 6.92 (d, *J* = 9.2 Hz, 2H), 6.79 (d, *J* = 8.0 Hz, 1H), 3.57-3.68 (m, 2H), 3.34 (s, 6H), 3.01-3.29 (m, 8H), 2.88 (s, 3H), 2.29-2.43 (m, 4H), 2.16-2.24 (m, 1H), 1.90-2.07 (m, 4H), 0.79-0.85 (m, 2H), 0.68-0.74 (m, 2H).

### Example 23

### ((6-(5-fluoro-2-((4-(4-(2-hyroxyethyl)piperazin-1-yl)phenyl)amino)-6-cyclopropyl-7H-pyrrol o[2,3-d]pyrimidin-7-yl)pyridin-2-yl)imino)dimethyl-λ⁶-sulfanone

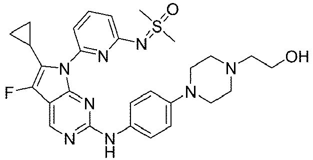

### Step A: tert-butyl 4-(2-hyroxyethyl)piperazine-1-carboxylate

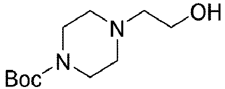

A solution of tert-butyl piperazine-1-carboxylate (1.0 g), 2-bromoethanol (675 mg), and triethylamine (3 mL) in 1,4-dioxane (10 mL) was heated to 50 °C and heated for 3 hours. The mixture was cooled to room temperature and partitioned between water and dichloromethane, the solvent was removed from the organic phase, and the residue was purified through silica gel column chromatography (100% DCM) to obtain the product (1.0 g).

¹H NMR (400 MHz, CDCl₃) δ 3.64 (t, *J* = 5.2 Hz, 2H), 3.42 (s, 1H), 2.83-2.89 (m, 4H), 2.56 (t, *J* = 5.2 Hz, 2H), 2.44-2.49 (m, 4H), 1.46 (s, 9H).

### Step B: 2-(piperazin-1-yl)ethan-1-ol hydrochloride

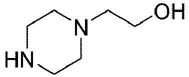

Tert-butyl 4-(2-hyroxyethyl)piperazine-1-carboxylate (1.0 g) was dissolved in methanol (10 mL), to the mixture was dropwise added 12 N hydrochloric acid solution (5 mL), the mixture was stirred at room temperature for 2 hours, and the solvent was removed to obtain the product (1.2 g).

¹H NMR (400 MHz, DMSO_{-d6}) δ 9.58 (s, 3H), 3.79 (t, *J* = 5.2 Hz, 2H), 3.41-3.64 (m, 8H), 3.27 (t, *J* = 5.2 Hz, 2H).

### Step C: 2-(4-(4-nitrophenyl)piperazin-1-yl)ethan-1-ol

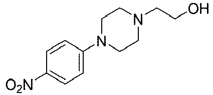

Under nitrogen gas, a solution of 2-(piperazin-1-yl)ethan-1-ol hydrochloride (1.2 g), 4-fluoronitrobenzene (1.3 g) and potassium carbonate (5.5 g) in dimethyl sulfoxide (20 mL) was heated to 100 °C and stirred overnight. The mixture was cooled to room temperature and partitioned between water and dichloromethane, the solvent was removed from the organic phase, and the residue was purified through silica gel column chromatography (1%MeOH/DCM) to obtain the product (500 mg).

¹H NMR (400 MHz, CDCl₃) δ 8.13 (d, *J* = 9.6 Hz, 2H), 6.83 (d, *J* = 9.6 Hz, 2H), 3.69 (t, *J* = 5.2 Hz, 2H), 3.43-3.47 (m, 4H), 2.65-2.71 (m, 4H), 2.63 (t, *J* = 5.2 Hz, 2H).

### Step D: 2-(4-(4-aminophenyl)piperazin-1-yl)ethan-1-ol

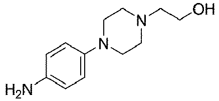

To a flask were sequentially added 2-(4-(4-nitrophenyl)piperazin-1-yl)ethan-1-ol (500 mg), 10% Pd/C (150 mg) and methanol (10 mL), the system was sufficiently purged with hydrogen gas, and the mixture was stirred at room temperature overnight. The reaction solution was filtered through celite, and the filtrate was concentrated and purified through silica gel column chromatography (10%MeOH/DCM) to obtain the product (420 mg).

¹H NMR (400 MHz, CDCl₃) δ 6.81 (d, *J* = 8.8 Hz, 2H), 6.65 (d, *J* = 8.8 Hz, 2H), 3.65 (t, *J* = 5.2 Hz, 2H), 3.26-3.58 (brs, 2H), 3.04-3.09 (m, 4H), 2.65-2.69 (m, 4H), 2.61 (t, *J* = 5.2 Hz, 2H).

### Step E: ((6-(5-fluoro-2-((4-(4-(2-hyroxyethyl)piperazin-1-yl)phenyl)amino)-6-cyclopropyl-7H-pyrrolo[2,3-d]pyrimidin-7-yl)pyridin-2-yl)imino)dimethyl-λ⁶-sulfanone

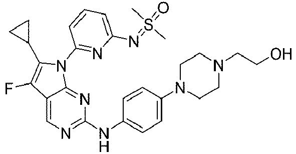

((6-(2-Chloro-5-fluoro-6-cyclopropyl-7*H*-pyrrolo[2,3-d]pyrimidin-7-yl)pyridin-2-yl)imino)di methyl-λ⁶-sulfanone (30 mg), 2-(4-(4-aminophenyl)piperazin-1-yl)ethan-1-ol (35 mg), Pd₂(dba)₃ (8 mg), 2-dicyclohexylphosphino-2',4',6'-tiiisopropylbiphenyl (12 mg) and caesium carbonate (78 mg) were mixed in 1,4-dioxane (5 mL). Under nitrogen protection, the mixture was heated to 100 °C and stirred overnight. The reaction solution was evaporated to remove the solvent, and the residue was purified through preparative TLC (10%MeOH/DCM) to obtain the target product (3 mg).

¹H NMR (400 MHz, CDCl₃) δ 8.58 (s, 1H), 7.73 (t, *J* = 8.0 Hz, 1H), 7.52 (d, *J* = 8.8 Hz, 2H), 7.21 (d, *J* = 8.0 Hz, 1H), 7.01 (s, 1H), 6.87 (d, *J* = 8.8 Hz, 2H), 6.81 (d, *J* = 8.0 Hz, 1H), 3.81-3.87 (m, 4H), 3.38-3.40(m, 2H), 3.28-3.36 (m, 6H), 2.93-3.04 (m, 4H), 2.83-2.91 (m, 3H), 2.18-2.27 (m, 1H), 0.83-0.91 (m, 2H), 0.72-0.77 (m, 2H).

### Example 24

### ((6-(2-((4-(4-(2-hyroxyethyl)piperazin-1-yl)phenyl)amino)-6-cyclopropyl-7H-pyrrolo[2,3-d] pyrimidin-7-yl)pyridin-2-yl)imino)dimethyl-λ⁶-sulfanone

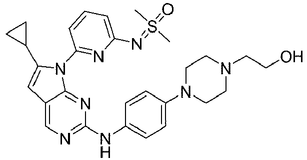

((6-(2-Chloro-6-cyclopropyl-7*H*-pyrrolo[2,3-d]pyrimidin-7-yl)pyridin-2-yl)imino)dimethyl-λ ⁶-sulfanone (100 mg), 2-(4-(4-aminophenyl)piperazin-1-yl)ethan-1-ol (62 mg), Pd₂(dba)₃ (26 mg), Dave-phos (33 mg) and sodium tert-butoxide (41 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (30 mg).

¹H NMR (400 MHz, CDCl₃) δ 8.51 (s, 1H), 7.74 (t, *J* = 8.0 Hz, 1H), 7.51 (d, *J* = 9.2 Hz, 2H), 7.21 (d, *J* = 8.0 Hz, 1H), 6.95 (s, 1H), 6.86 (d, *J* = 8.8 Hz, 2H), 6.82 (d, *J* = 8.0 Hz, 1H), 6.06 (s, 1H), 3.75 (t, *J* = 5.2 Hz, 2H), 3.30 (s, 6H), 3.19-3.26 (m, 4H), 2.81-2.89 (m, 4H), 2.74 (t, *J* = 5.2 Hz, 2H), 2.31-2.38 (m, 1H), 0.87-0.92 (m, 2H), 0.65-0.69 (m, 2H).

### Example 25

### (N-(6-(5-fluoro-2-((4-(4-methylpiperazin-1-yl)phenyl)amino)-6-cyclopropyl-7H-pyrrolo[2,3-d]pyrimidin-7-yl)pyridin-2-yl))methanesulfonamide

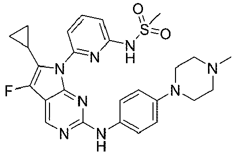

### Step A: N-((5-bromo-2-chloropyrimidin-4-yl)amino)pyridine-2,6-diamine

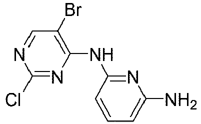

Under nitrogen gas, a mixture of 2,6-diaminopyridine (1.09 g), 5-bromo-2,4-dichloro pyrimidine (2.28 g), ethyldiisopropylamine (2.58 g) and isopropanol (10 mL) was heated to reflux and stirred overnight. The mixture was cooled to room temperature, and the solid precipitated was collected by filtration, washed with small amount of ethyl acetate, and dried to obtain the product (2.32 g).

¹H NMR (400 MHz, CDCl₃) δ 8.55 (s, 1H), 8.19 (s, 1H), 7.48 (d, *J* = 8.0 Hz, 1H), 7.19 (d, *J* = 8.0 Hz, 1H), 6.27 (d, *J* = 8.0 Hz, 1H), 6.09 (s, 2H).

### Step B: 6-(2-chloro-6-cyclopropyl-7H-pyrrolo[2,3-d]pyrimidin-7-yl)pyridin-2-amine

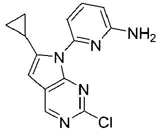

Under nitrogen gas, to a solution of N-((5-bromo-2-chloropyrimidin-4-yl)amino) pyridine-2,6-diamine (1.5 g), copper(I) iodide (0.475g) and Pd(dppf)Cl₂ (0.35g) in DMF (20 mL) and triethylamine (20 mL) was slowly added cyclopropylacetylene (0.33g), the reaction solution was heated to 40 °C, stirred overnight, filtered through celite and washed with dichloromethane, the solvent was removed from the filtrate, and the residue was purified through silica gel column chromatography (50%EtOAc/PE-100%EtOAc) to obtain the product (0.45g).

¹H NMR (400 MHz, CDCl₃) δ 8.69 (s, 1H), 7.66(t, *J =* 8.0 Hz, 1H), 6.91(d, *J =* 8.0 Hz, 1H), 6.60 (d, *J* = 8.8 Hz, 1H), 6.18(s, 1H), 4.63(s, 2H), 2.12-2.03(m, 1H), 0.96-0.90 (m, 2H), 0.81-0.75 (m, 2H).

### Step C: (N-(6-(2-chloro-6-cyclopropyl-7H-pyrrolo[2,3-d]pyrimidin-7-yl)pyridin-2-yl)) methanesulfonamide

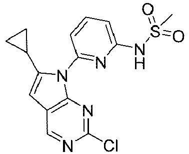

Under nitrogen gas, 6-(2-chloro-6-cyclopropyl-7*H*-pyrrolo[2,3-d]pyrimidin-7-yl) pyridin-2-amine (84 mg) was dissolved in 5 mL dichloromethane, the reaction was cooled to 0 °C, then triethylamine (20 mg) was added, the mixture was stirred and reacted for 10 minutes, then a solution of methanesulfonyl chloride (18 mg) in dichloromethane (1 mL) was slowly dropwise added to the reaction solution, the reaction solution was stirred for 30 minutes, let warm up to room temperature, stirred for 2 hours, filtered through celite, washed with dichloromethane and saturated sodium bicarbonate, the filtrate was extracted with dichloromethane, and the organic phases were combined and rotavaped to dryness to obtain the product, (80 mg), which was directly used in the next step without purification.

¹H NMR (400 MHz, CDCl₃) δ 8.73 (s, 1H), 7.92(t, *J =* 8.0 Hz, 2H), 7.42 (d, *J =* 8.0Hz, 1H), 7.10 (d, *J* = 8.8Hz, 1H), 6.24 (s, 1H), 3.35 (s, 3H), 2.29-2.27 (m, 1H), 0.98-0.96(m, 2H), 0.78-0.74(m, 2H).

### Step D: (N-(6-(2-chloro-5-fluoro-6-cyclopropyl-7H-pyrrolo[2,3-d]pyrimidin-7-yl)pyridin-2-yl))methanesulfonamide

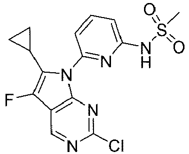

Under nitrogen gas, a slution of (N-(6-(2-chloro-6-cyclopropyl-7*H*-pyrrolo[2,3-d]pyrimidin-7-yl)pyridin-2-yl))methanesulfonamide (108 mg) in acetonitrile (3 mL) was cooled to 0 °C, Selectfluor^{®} (80 mg) was added portionwise, and the mixture was let warm up to room temperature slowly and stirred overnight. Water was added to quench the reaction, the mixture was extracted with dichloromethane, and the organic phase was concentrated and purified through preparative TLC (100%EtOAc) to obtain the product (30 mg).

¹H NMR (400 MHz, CDCl₃) δ 8.82 (s, 1H), 7.93(t, *J =* 8.0 Hz, 1H), 7.65 (s, 1H), 7.43 (d, *J =* 8.0 Hz, 1H), 7.10 (d, *J* = 8.0 Hz, 1H), 3.35 (s, 3H), 2.20-2.12 (m, 1H), 0.98-0.90(m, 2H), 0.89-0.81(m, 2H).

### Step E: (N-(6-(5-fluoro-2-((4-(4-methylpiperazin-1-yl)phenyl)amino)-6-cyclopropyl-7H-pyrrolo[2,3-d]pyrimidin-7-yl)pyridin-2-yl))methanesulfonamide

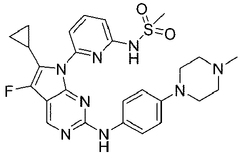

(N-(6-(2-chloro-5-fluoro-6-cyclopropyl-7*H*-pyrrolo[2,3-d]pyrimidin-7-yl)pyridin-2-yl))meth anesulfonamide (38 mg), 4-(4-methylpiperazin-1-yl)aniline (30 mg), Pd₂(dba)₃ (7.5 mg), 2-dicyclohexylphosphino-2',4',6'-tiiisopropylbiphenyl (11 mg) and caesium carbonate (40 mg) were mixed in a mixed solvent of 1,4-dioxane (5 mL) and DMF (1 mL), the system was sufficiently purged with nitrogen gas, and the tube was sealed and heated in a microwave reactor to 95 °C to react for 4 hours. The reaction solution was evaporated to remove the solvent, and the residue was purified through preparative TLC (10%MeOH/DCM) to obtain the target product (11 mg).

¹H NMR (400 MHz, CDCl₃) δ 8.56 (s, 1H), 7.97 (t, *J* = 7.6 Hz, 1H), 7.59 (d, *J* = 9.2 Hz, 2H), 7.49 (d, *J* = 7.2 Hz, 1H), 7.02 (d, *J =* 8.0 Hz, 1H), 6.94 (d, *J* = 8.8 Hz, 2H), 3.46-3.32 (m, 9H), 3.27 (s, 3H), 2.94 (s, 3H), 2.36-2.25 (m, 1H), 0.84-0.78 (m, 2H), 0.69-0.64 (m, 2H).

### Example 26

### (N-(6-(5-fluoro-2-((4-(4-methylpiperazin-1-yl)phenyl)amino)-6-cyclopropyl-7H-pyrrolo[2,3-d]pyrimidin-7-yl)pyridin-2-yl))ethanesulfonamide

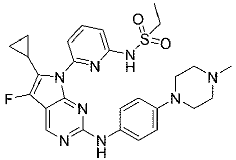

### Step A: (N-(6-(2-chloro-5-fluoro-6-cyclopropyl-7H-pyrrolo[2,3-d]pyrimidin-7-yl)pyridin-2-yl))ethanesulfonamide

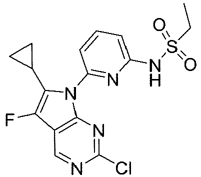

Under nitrogen gas, 6-(2-chloro-6-cyclopropyl-7*H*-pyrrolo[2,3-d]pyrimidin-7-yl) pyridin-2-amine (87mg) was dissolved in 5 mL dichloromethane, the reaction was cooled to 0 °C, then triethylamine (20 mg) was added, the mixture was stirred and reacted for 10 minutes, then a solution of ethanesulfonyl chloride (18 mg) in dichloromethane (1 mL) was dropwise added to the reaction solution slowly , the reaction solution was stirred for 30 minutes, then was let warm up to room temperature, stirred for 5 hours, filtered through celite, washed with dichloromethane and an aqueous solution of saturated sodium bicarbonate, the filtrate was extracted with dichloromethane, and the organic phases were combined, rotavaped to dryness to obtain the product (75 mg), which was used directly in the next step without purification. Under nitrogen gas, a solution of (N-(6-(2-chloro-6-cyclopropyl-7*H*-pyrrolo[2,3-d]pyrimidin-7-yl)pyridin-2-yl))ethanesulfonamide (11 mg) in acetonitrile (2 mL) was cooled to 0 °C, Selectfluor^{®} (80 mg) was portionwise added, and the mixture was let warm up to room temperature slowly and stirred overnight. Water was added to quench the reaction, the mixture was extracted with dichloromethane, and the organic phase was concentrated and purified through preparative TLC (100%EtOAc) to obtain the product (32 mg).

¹H NMR (400 MHz, CDCl₃) δ 8.82 (s, 1H), 7.91 (t, *J* = 8.0 Hz, 1H), 7.89 (s, 1H), 7.41 (d, *J* = 8.0 Hz, 1H), 7.13 (d, *J* = 8.0 Hz, 1H), 3.50 (d, *J* = 8.0 Hz, 2H), 2.20-2.12 (m, 1H), 1.42 (t, *J* = 7.6 Hz, 3H), 0.97-0.91 (m, 2H), 0.86-0.81 (m, 2H).

### Step B: (N-(6-(5-fluoro-2-((4-(4-methylpiperazin-1-yl)phenyl)amino)-6-cyclopropyl-7H-pyrrolo[2,3-d]pyrimidin-7-yl)pyridin-2-yl))ethanesulfonamide

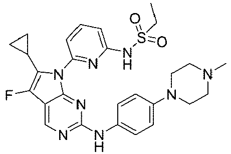

(N-(6-(2-chloro-5-fluoro-6-cyclopropyl-7*H*-pyrrolo[2,3-d]pyrimidin-7-yl)pyridin-2-yl))ethan esulfonamide (40 mg), 4-(4-methylpiperazin-1-yl)aniline (30 mg), Pd₂(dba)₃ (7.5 mg), 2-dicyclohexylphosphino-2',4',6'-tiiisopropylbiphenyl (11 mg) and caesium carbonate (40 mg) were mixed in a mixed solvent of 1,4-dioxane (5 mL) and DMF (1 mL), the system was sufficiently purged with nitrogen gas, and the tube was sealed and heated in a microwave reactor to 95 °C to react for 4 hours. The reaction solution was evaporated to remove the solvent, and the residue was purified through preparative TLC (10%MeOH/DCM) to obtain the target product (11 mg).

¹H NMR (400 MHz, CDCl₃) δ 8.57 (s, 1H), 7.96(t, *J* = 7.6 Hz, 1H), 7.59 (d, *J* = 9.2 Hz, 2H), 7.49 (d, *J* = 7.2 Hz, 1H), 7.04 (d, *J =* 8.0 Hz, 1H), 6.94 (d, *J* = 8.8 Hz, 2H), 3.47-3.25 (m, 9H), 3.20 (d, *J* = 7.6 Hz, 2H), 2.93 (s, 3H), 2.36-2.24 (m, 1H), 1.31(t, *J* = 7.6 Hz, 3H), 0.84-0.78(m, 2H), 0.70-0.64(m, 2H).

### Example 27

### ((6-(6-cyclopropyl-5-fluoro-2-((4-(3-hydroxymethyl-4-methylpiperazin-1-yl)phenyl)amino)-7H-pyrrolo[2,3-d]pyrimidin-7-yl)pyridin-2-yl)imino)dimethyl-λ⁶-sulfanone

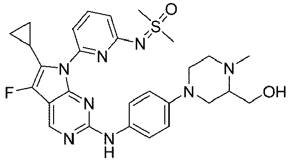

### Step A: tert-butyl 3-(hydroxymethyl)-4-methylpiperazine-1-carboxylate

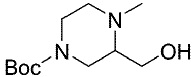

Tert-butyl 3-(hydroxymethyl)piperazine-1-carboxylate (540 mg), aqueous solution of 40% formaldehyde (5 mL) and acetic acid (0.5 mL) were added to dichloromethane (20 mL), the mixture was stirred for 30 minutes, sodium triacetoxyborohydride (2.1 g) was added, and the mixture was stirred at room temperature overnight. An aqueous solution of 1 N sodium hydroxide was used to adjust the pH to 10, the mixture was extracted with dichloromethane, dried over anhydrous sodium sulfate, and the solvent was removed to obtain the product (540 mg).

¹H NMR (400 MHz CDCl₃) δ 3.78-3.94 (m, 3H), 3.49 (dd, *J* = 11.6 Hz, 1.6 Hz, 1H), 2.89-3.09 (m, 2H), 2.78 (d, *J* = 11.6 Hz, 1H), 2.34 (s, 3H), 2.23-2.32 (m, 2H), 2.07-2.16 (m, 1H), 1.46 (s, 9H).

### Step B: (1-methylpiperazine-2-yl)methanol

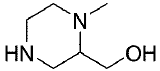

Tert-butyl 3-(hydroxymethyl)-4-methylpiperazine-1-carboxylate (500 mg) was added to 4 mol/L HCl methanol solution (5 mL), and the mixture was stirred at room temperature overnight and evaporated to remove the solvent to obtain the product as a hydrochloride salt (400 mg).

¹H NMR (400 MHz, DMSO_{-*d*6}) δ 10.56-11.02 (brs, 1H), 9.95-10.06 (brs, 2H), 3.12-3.89 (m, 9H), 2.88 (s, 3H).

### Step C: (1-methyl-4-(4-nitrophenyl)piperazine-2-yl)methanol

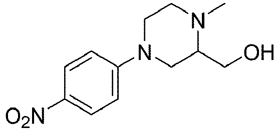

Under room temperature, 4-fluoronitrobenzene (352 mg), (1-methylpiperazine-2-yl) methanol (327 mg) and potassium carbonate (690 mg) were added to DMF (10 mL), and the mixture was stirred at 90 °C overnight. Water was added, the mixture was extracted with ethyl acetate, and the organic phase was washed with water, dried over anhydrous sodium sulfate, concentrated, and purified through column chromatography (DCM/MeOH=20/1) to obtain the product (250 mg).

¹H NMR (400 MHz, CDCl₃) δ 8.12 (d, *J* = 9.2 Hz, 2H), 6.83 (t, *J* = 9.2 Hz, 2H), 3.92 (dd, *J* = 11.6 Hz, 4.8 Hz, 1H), 3.75-3.81 (m, 2H), 3.62 (dd, *J* = 11.6 Hz, 2.0 Hz, 1H), 3.10-3.22 (m, 2H), 2.96-3.02 (m, 1H), 2.51 (td, *J* = 11.6 Hz, 3.2 Hz, 1H), 2.41-2.48 (brs, 1H), 2.41 (s, 3H), 2.31-2.37 (m, 1H).

### Step D: (4-(4-aminophenyl)-1-methylpiperazine-2-yl)methanol

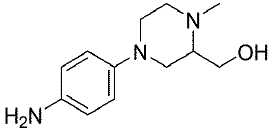

(1-Methyl-4-(4-nitrophenyl)piperazine-2-yl)methanol (100 mg) and Pd/C (10 mg) were added to methanol (5 mL), the reaction solution was stirred under a hydrogen atmosphere overnight, filtered through celite and washed with dichloromethane, and the solvent was removed from the filtrate to obtain the product (60 mg).

¹H NMR (400 MHz, CDCl₃) δ 6.83 (d, *J* = 8.8 Hz, 2H), 6.65 (d, *J* = 8.8 Hz, 2H), 3.89-3.96 (m, 1H), 3.51-3.57 (m, 1H), 3.44 (s, 2H), 3.26-3.33 (m, 2H), 2.93-2.98 (m, 1H), 2.87 (dd, *J* = 11.6 Hz, 9.6 Hz, 1H), 2.78 (td, *J* = 11.2 Hz, 2.8 Hz, 1H), 2.57 (td, *J* = 11.2 Hz, 3.2 Hz, 1H), 2.45 (dd, *J* = 7.6 Hz, 2.8 Hz, 1H), 2.39 (s, 3H), 2.33-2.38 (m, 1H).

### Step E: ((6-(6-cyclopropyl-5-fluoro-2-((4-(3-hydroxymethyl-4-methylpiperazin-1-yl)phenyl) amino)-7H-pyrrolo[2,3-d]pyrimidin-7-yl)pyridin-2-yl)imino)dimethyl-λ⁶-sulfanone

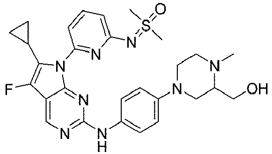

((6-(2-Chloro-5-fluoro-6-cyclopropyl-7*H*-pyrrolo[2,3-d]pyrimidin-7-yl)pyridin-2-yl)imino)di methyl-λ⁶-sulfanone (38 mg), 4-(4-aminophenyl)-1-methylpiperazine-2-yl)methanol (22 mg), Pd₂(dba)₃ (9 mg), Dave-phos (16 mg) and sodium tert-butoxide (18 mg) were mixed in toluene (3 mL), the system was sufficiently purged with nitrogen gas, and the tube was sealed and heated to 101 °C for the mixture to react for 5 hours. The reaction solution was evaporated to remove the solvent, and the residue was purified through preparative TLC (10%MeOH/DCM) to obtain the target product (12 mg).

¹H NMR (400 MHz, CDCl₃) δ 8.46 (s, 1H), 7.71 (t, *J =* 8.0 Hz, 1H), 7.49 (d, *J =* 8.4 Hz, 2H), 7.11 (d, *J* = 7.6 Hz, 1H), 6.83 (d, *J* = 8.8 Hz, 2H), 6.70 (d, *J* = 8.0 Hz, 1H), 3.96 (dd, *J* = 12.0 Hz, 3.6 Hz, 1H), 3.67 (dd, *J* = 12.4 Hz, 2.0 Hz, 1H), 3.52-3.62 (m, 2H), 3.43 (d, *J* = 12.4 Hz, 1H), 3.24-3.34 (m, 2H), 3.25 (s, 6H), 2.89-3.02 (m, 3H), 2.89 (s, 3H), 2.09-2.13 (m, 1H), 0.70-0.75 (m, 2H), 0.59-0.63 (m, 2H).

### Example 28

### ((6-(6-cyclopropyl-5-fluoro-2-((4-(2-hydroxymethyl-4-methylpiperazin-1-yl)phenyl)amino)-7H-pyrrolo[2,3-d]pyrimidin-7-yl)pyridin-2-yl)imino)dimethyl-λ⁶-sulfanone

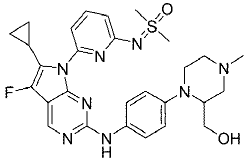

### Step A: tert-butyl 3-(hydroxymethyl)-4-(4-nitrophenyl)piperazine-1-carboxylate

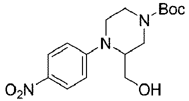

Under room temperature, 4-fluoronitrobenzene (528 mg), tert-butyl 3-(hydroxymethyl) piperazine-1-carboxylate (327 mg) and dipotassium hydrogenphosphate (783 mg) were added to DMSO (5 mL), and the mixture was heated to 90 °C and stirred overnight. The mixture was cooled to room temperature, water was added, the mixture was extracted with ethyl acetate, and the organic phase was washed with water, dried over anhydrous sodium sulfate, concentrated, and purified through column chromatography (DCM/MeOH=20/1) to obtain the product (150 mg).

¹H NMR (400 MHz CDCl₃) δ 8.13 (d, *J* = 9.2 Hz, 2H), 6.83 (d, *J* = 9.2 Hz, 2H), 4.21-4.47 (m, 1H), 4.01-4.18 (m, 2H), 3.48-3.76 (m, 3H), 3.08-3.29 (m, 3H), 2.77-2.96 (brs, 1H), 1.50 (s, 9H).

### Step B: (1-(4-nitrophenyl)piperazine-2-yl)methanol

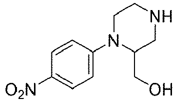

Tert-butyl 3-(hydroxymethyl)-4-(4-nitrophenyl)piperazine-1-carboxylate (200 mg) was added to 3 mol/L HCl methanol solution (3 mL), and the mixture was stirred at room temperature overnight and evaporated to remove the solvent to obtain the product as a hydrochloride salt (130 mg).

### Step C: (4-methyl-1-(4-nitrophenyl)piperazine-2-yl)methanol

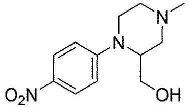

(1-(4-Nitrophenyl)piperazine-2-yl)methanol (170 mg), aqueous solution of 40% formaldehyde (3 mL) and acetic acid (0.5 mL) were added to dichloromethane (20 mL), the mixture was stirred for 30 minutes, sodium triacetoxyborohydride (600 mg) was added, and the mixture was stirred at room temperature overnight. An aqueous solution of 1 N sodium hydroxide was used to adjust the pH to 10, the mixture was extracted with dichloromethane, the extract was dried over anhydrous sodium sulfate, and the solvent was removed to obtain the product (150 mg).

¹H NMR (400 MHz, CDCl₃) δ 8.13 (d, *J* = 9.6 Hz, 2H), 6.81 (d, *J* = 9.6 Hz, 2H), 4.03-4.09 (m, 1H), 3.98 (dd, *J* = 10.8 Hz, 3.2 Hz, 1H), 3.91 (dd, *J* = 11.2 Hz, 3.6 Hz, 1H), 3.55-3.68 (m, 2H), 3.24 (d, *J* = 11.6 Hz, 1H), 3.04 (d, *J* = 11.6 Hz, 1H), 2.51 (dd, *J* = 11.6 Hz, 2.4 Hz, 1H), 2.38 (s, 3H), 2.31 (td, *J* = 11.6 Hz, 4.4 Hz, 1H).

### Step D: (1-(4-aminophenyl)-4-methylpiperazine-2-yl)methanol

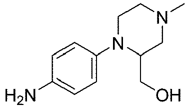

(4-Methyl-1-(4-nitrophenyl)piperazine-2-yl)methanol (200 mg) and Pd/C (20 mg) were added to methanol (5 mL), the reaction solution was stirred under a hydrogen atmosphere overnight, filtered through celite and washed with dichloromethane, and the solvent was removed from the filtrate to obtain the product (130 mg).

¹H NMR (400 MHz, CDCl₃) δ 6.86 (d, *J* = 8.8 Hz, 2H), 6.65 (d, *J* = 8.8 Hz, 2H), 3.75 (dd, *J* = 11.2 Hz, 3.6 Hz, 1H), 3.62 (dd, *J* = 11.2 Hz, 3.2 Hz, 1H), 3.55-3.68 (m, 2H), 3.24 (d, *J* = 11.6 Hz, 1H), 3.04 (d, *J* = 11.6 Hz, 1H), 3.46-3.54 (m, 1H), 3.34-3.41 (m, 1H), 3.10-3.15 (m, 1H), 2.84 (dd, *J* = 11.2 Hz, 4.4 Hz, 1H), 2.68-2.78 (m, 1H), 2.46-2.53 (m, 1H), 2.35 (s, 3H).

### Step E: ((6-(6-cyclopropyl-5-fluoro-2-((4-(2-hydroxymethyl-4-methylpiperazin-1-yl)phenyl) amino)-7H-pyrrolo[2,3-d]pyrimidin-7-yl)pyridin-2-yl)imino)dimethyl-λ⁶-sulfanone

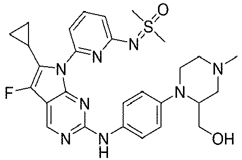

((6-(2-Chloro-5-fluoro-6-cyclopropyl-7*H*-pyrrolo[2,3-d]pyrimidin-7-yl)pyridin-2-yl)imino)di methyl-λ⁶-sulfanone (38 mg), 4-(4-aminophenyl)-1-methylpiperazine-2-yl)methanol (22 mg), Pd₂(dba)₃ (9 mg), Dave-phos (18 mg) and sodium tert-butoxide (18 mg) were mixed in toluene (3 mL), the system was sufficiently purged with nitrogen gas, and the tube was sealed and heated to 101 °C for the mixture to react for 5 hours. The reaction solution was evaporated to remove the solvent, and the residue was purified through preparative TLC (10%MeOH/DCM) to obtain the target product (5 mg).

¹H NMR (400 MHz, CDCl₃) δ 8.58 (s, 1H), 7.72 (t, *J* = 8.0 Hz, 1H), 7.52 (d, *J* = 8.8 Hz, 2H), 7.20 (d, *J* = 7.6 Hz, 1H), 7.07 (s, 1H), 6.91 (d, *J* = 8.8 Hz, 2H), 6.80 (d, *J* = 8.0 Hz, 1H), 3.85-3.94 (m, 1H), 3.76-3.82 (m, 1H), 3.60-3.67 (m, 1H), 3.42-3.51 (m, 1H), 3.33-3.41 (m, 1H), 3.230 (s, 6H), 3.22-3.28 (m, 1H), 3.02-3.08 (m, 1H), 2.84-2.96 (m, 1H), 2.65-2.78 (m, 1H), 2.58 (s, 3H), 2.17-2.6 (m, 1H), 0.82-0.89 (m, 2H), 0.72-0.78 (m, 2H).

### Example 29

### ((6-(6-cyclopropyl-2-((4-(2-hydroxymethyl-4-methylpiperazin-1-yl)phenyl)amino)-7H-pyrrol o[2,3-d]pyrimidin-7-yl)pyridin-2-yl)imino)dimethyl-λ⁶-sulfanone

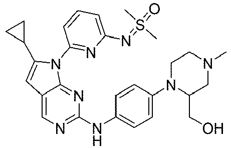

((6-(2-Chloro-6-cyclopropyl-7*H*-pyrrolo[2,3-d]pyrimidin-7-yl)pyridin-2-yl)imino)dimethyl-λ ⁶-sulfanone (144 mg), (1-(4-aminophenyl)-4-methylpiperazine-2-yl)methanol (46 mg), Pd₂(dba)₃ (37 mg), Dave-phos (47 mg) and sodium tert-butoxide (58 mg) were mixed in toluene (5 mL), the system was sufficiently purged with nitrogen gas, and the tube was sealed and heated to 101 °C for the mixture to react for 5 hours. The reaction solution was evaporated to remove the solvent, and the residue was purified through preparative TLC (10%MeOH/DCM) to obtain the target product (50 mg).

¹H NMR (400 MHz, CDCl₃) δ 8.51 (s, 1H), 7.73 (t, *J* = 8.0 Hz, 1H), 7.51 (d, *J* = 8.8 Hz, 2H), 7.20 (d, J = 8.0 Hz, 1H), 7.01 (s, 1H), 6.89 (d, *J* = 8.8 Hz, 2H), 6.82 (d, *J* = 8.0 Hz, 1H), 6.06 (s, 1H), 3.86 (dd, *J* = 10.8 Hz, 4.0 Hz, 1H), 3.69-3.76 (m, 1H), 3.63-3.68 (m, 1H), 3.41-3.47 (m, 1H), 3.30 (s, 6H), 3.24-3.32 (m, 1H), 3.11 (d, *J* = 8.0 Hz, 1H), 2.89-2.97 (m, 1H), 2.77 (d, *J* = 6.8 Hz, 1H), 2.52-2.64 (m, 1H), 2.46 (s, 3H), 2.28-2.37 (m, 1H), 0.84-0.93 (m, 2H), 0.65-0.71 (m, 2H).

### Example 30

### ((6-(5-fluoro-2-((4-(N,N-dimethylacetamido)phenyl)amino)-6-cyclopropyl-7H-pyrrolo[2,3-d] pyrimidin-7-yl)pyridin-2-yl)imino)dimethyl-λ⁶-sulfanone

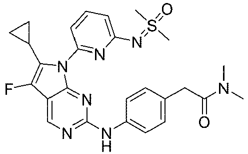

### Step A: N,N-dimethyl-2-(4-nitrophenyl)acetamide

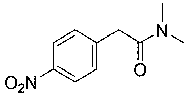

To a flask were sequentially added 2-(4-nitrophenyl)acetic acid (1.0 g), dimethylamine (248 mg), EDCI (2.1 g), DMAP (1.3 g), triethylamine (1.1 g) and dichloromethane (20 mL), and the mixture was stirred at room temperature for 4 hours. The mixture was partitioned between water and dichloromethane, the solvent was removed from the organic phase, and the residue was purified through silica gel column chromatography (1%MeOH/DCM) to obtain the product (800 mg).

¹H NMR (400 MHz, CDCl₃) δ 8.19 (d, *J* = 8.8 Hz, 2H), 7.43 (d, *J* = 8.8 Hz, 2H), 3.81 (s, 2H), 3.06 (s, 3H), 2.99 (s, 3H).

### Step B: N,N-dimethyl-2-(4-aminophenyl)acetamide

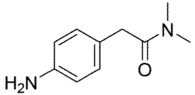

To a flask were sequentially added N,N-dimethyl-2-(4-nitrophenyl)acetamide (200 mg), 10% Pd/C (60 mg) and methanol (10 mL), the system was sufficiently purged with hydrogen gas, and the mixture was stirred at room temperature overnight. The reaction solution was filtered through celite, and the filtrate was concentrated and purified through silica gel column chromatography (10%MeOH/DCM) to obtain the product (180 mg).

¹H NMR (400 MHz, CDCl₃) δ 7.04 (d, *J* = 8.4 Hz, 2H), 6.64 (d, *J* = 8.4 Hz, 2H), 3.60 (s, 4H), 2.98 (s, 3H), 2.95 (s, 3H).

### Step C: ((6-(5-fluoro-2-((4-(N,N-dimethylacetamido)phenyl)amino)-6-cyclopropyl-7H-pyrrolo[2,3-d]pyrimidin-7-yl)pyridin-2-yl)imino)dimethyl-λ⁶-sulfanone

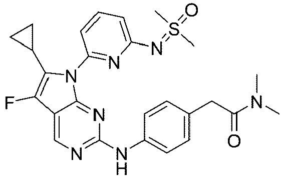

((6-(2-Chloro-5-fluoro-6-cyclopropyl-7*H*-pyrrolo[2,3-d]pyrimidin-7-yl)pyridin-2-yl)imino)di methyl-λ⁶-sulfanone (30 mg), N,N-dimethyl-2-(4-aminophenyl)acetamide (15 mg), Pd₂(dba)₃ (8 mg), Dave-phos (10 mg) and sodium tert-butoxide (12 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (5 mg).

¹H NMR (400 MHz, CDCl₃) δ 8.60 (s, 1H), 7.73 (t, *J* = 8.0 Hz, 1H), 7.55 (d, *J* = 8.4 Hz, 2H), 7.22 (s, 1H), 7.19 (d, *J* = 8.0 Hz, 1H), 7.14 (d, *J* = 8.4 Hz, 2H), 6.80 (d, *J* = 8.0 Hz, 1H), 3.66 (s, 2H), 3.29 (s, 6H), 3.01 (s, 3H), 2.96 (s, 3H), 2.16-2.24 (m, 1H), 0.82-0.91 (m, 2H), 0.73-0.79 (m, 2H).

### Example 31

### 1-((6-(6-cyclopropyl-5-fluoro-2-((4-(4-methylpiperazin-1-yl)phenyl)amino)-7H-pyrrolo[2,3-d]pyrimidin-7-yl)pyridin-2-yl)imino)tetrahydro-1H-1λ⁶-thiophene 1-oxide

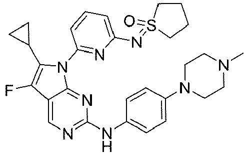

### Step A: 1-iminotetrahydro-1H-1λ⁶-thiophene 1-oxide

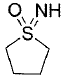

To a flask were added tetramethylene sulfoxide (1.0 g), lodobenzene diacetate (9.3 g), ammoniumcarbamat (3.0 g) and methanol (20 mL), the mixture was stirred at room temperature in an open vessel for 1 hour and evaporated to dryness to remove the solvent, and the crude product was purified through silica gel column chromatography (10% MeOH/DCM) to obtain the product (1.0 g).

¹H NMR (400 MHz CDCl₃) δ 4.56 (s, 1H), 3.09-3.23 (m, 4H), 2.22-2.31 (m, 4H).

### Step B: 1-((6-bromopyridin-2-yl)imino)tetrahydro-1H-1λ⁶-thiophene 1-oxide

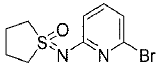

According to the method of step A of example 1, 1-iminotetrahydro-1*H*-1λ⁶-thiophene 1-oxide and 2,6-di-bromopyridine were used as the starting material to obtain the product (1.4 g).

¹H NMR (400 MHz, CDCl₃) δ7.34 (t, *J* = 8.0 Hz, 1H), 6.94 (d, *J* = 8.0 Hz, 1H), 6.73(d, *J* = 8.0 Hz, 1H), 3.61-3.71(m, 2H), 3.27-3.36(m, 2H), 2.24-2.41(m, 4H).

### Step C: 1-((6-aminopyridin-2-yl)imino)tetrahydro-1H-1λ⁶-thiophene 1-oxide

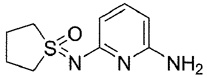

According to the method of step B of example 1, 1-((6-bromopyridin-2-yl) imino)tetrahydro-1*H*-1λ⁶-thiophene 1-oxide and ammonia were used as the starting material to obtain the product (0.44 g).

¹H NMR (400 MHz, CDCl₃) δ7.29 (t, *J* = 8.0 Hz, 1H), 6.20 (d, *J* = 8.0Hz, 1H), 6.02 (d, *J* = 8.0 Hz, 1H), 4.19-4.36 (brs, 2H), 3.58-3.67(m, 2H), 3.28-3.37(m, 2H), 2.14-2.38(m, 4H).

### Step D: 1-((6-((5-bromo-2-chloropyrimidin-4-yl)amino)pyridin-2-yl)imino)tetrahydro-1H-1λ⁶-thiophene 1-oxide

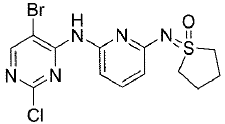

According to the method of step C of example 1, 1-((6-aminopyridin-2-yl)imino) tetrahydro-1*H*-1λ⁶-thiophene 1-oxide and 5-bromo-2,4-dichloropyrimidine were used as the starting material to obtain the product (0.31 g).

¹H NMR (400 MHz, CDCl₃) δ 8.34 (s, 1H), 7.83 (d, *J* = 8.0 Hz, 1H), 7.77 (s, 1H), 7.63 (t, *J* = 8.0 Hz, 1H), 6.64 (d, *J* = 8.0 Hz, 1H), 3.61-3.69 (m, 2H), 3.34-3.43 (m, 2H), 2.20-2.43 (m, 4H).

### Step E: 1-((6-(2-chloro-6-cyclopropyl-7H-pyrrolo[2,3-d]pyrimidin-7-yl)pyridin-2-yl)imino) tetrahydro-1H-1λ⁶-thiophene 1-oxide

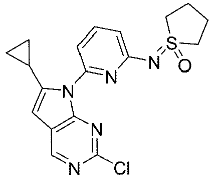

According to the method of step D of example 1, 1-((6-((5-bromo-2-chloropyrimidin-4-yl) amino)pyridin-2-yl)imino)tetrahydro-1*H*-1λ⁶-thiophene 1-oxide and cyclopropylacetylene were used as the starting material to obtain the product (0.31 g).

¹H NMR (400 MHz, CDCl₃) δ 8.68 (s, 1H), 7.73(t, *J =* 8.0 Hz, 1H), 7.07(d, *J =* 8.0 Hz, 1H), 6.89(d, *J* = 8.0 Hz, 1H), 6.18(s, 1H), 3.78-3.87 (m, 2H), 3.21-3.31(m, 2H), 2.14-2.43(m, 5H), 0.98-1.04(m, 2H), 0.76-0.82(m, 2H).

### Step F: 1-((6-(2-chloro-6-cyclopropyl-5-fluoro-7H-pyrrolo[2,3-d]pyrimidin-7-yl)pyridin-2-yl) imino)tetrahydro-1H-1λ⁶-thiophene 1-oxide

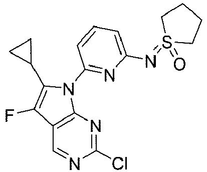

According to the method of step E of example 1, 1-((6-(2-chloro-6-cyclopropyl-7*H*-pyrrolo[2,3-d]pyrimidin-7-yl)pyridin-2-yl)imino)tetrahydro-1*H* -λ⁶-thiophene 1-oxide and Selectfluor^{®} were used as the starting material to obtain the product (30 mg)

¹H NMR (400 MHz, CDCl₃) δ 8.78 (s, 1H), 7.72 (t, *J* = 8.0 Hz, 1H), 7.06 (d, *J* = 8.0 Hz, 1H), 6.88 (d, *J* = 8.0 Hz, 1H), 3.71-3.81 (m, 2H), 3.22-3.31 (m, 2H), 2.08-2.44 (m, 5H), 0.87-0.98 (m, 4H).

### Step G: 1-((6-(6-cyclopropyl-5 -fluoro-2-((4-(4-methylpiperazin-1-yl)phenyl)amino)-7H-pyrrolo [2,3-d]pyrimidin-7-yl)pyridin-2-yl)imino)tetrahydro-1H-1λ⁶-thiophene 1-oxide

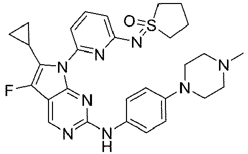

According to the method of step F of example 1, 1-((6-(2-chloro-6-cyclopropyl-5-fluoro-7*H*-pyrrolo[2,3-d]pyrimidin-7-yl)pyridin-2-yl)imino)tetrahydro-1*H*-1λ⁶-thiophene 1-oxide and 4-(4-methylpiperazin-1-yl)aniline were used as the starting material to obtain the product (5 mg).

¹H NMR (400 MHz, CDCl₃) δ 8.58 (s, 1H), 7.74(t, *J =* 8.0 Hz, 1H), 7.54(d, *J =* 8.0 Hz, 2H), 7.22(d, *J* = 8.0 Hz, 1H), 7.08(s, 1H), 6.83-6.88 (m, 3H), 3.60-3.68 (m, 2H), 3.41-3.48 (m, 4H), 3.21-3.28(m, 2H), 3.01-3.19(m, 4H), 2.73(s, 3H), 2.21-2.32(m, 3H), 2.10-2.18(m, 2H), 0.83-0.88(m, 2H), 0.72-0.76(m, 2H).

### Example 32

### ((6-(5-fluoro-2-((4-(2-(dimethylamino)ethyl)phenyl)amino)-6-cyclopropyl-7H-pyrrolo[2,3-d] pyrimidin-7-yl)pyridin-2-yl)imino)dimethyl-λ⁶-sulfanone

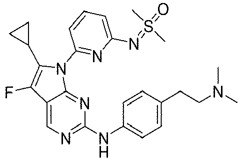

### Step A: N,N-dimethyl-2-(4-nitrophenyl)ethan-1-amine

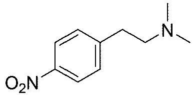

To a flask were sequentially added N,N-dimethyl-2-(4-nitrophenyl)acetamide(350 mg), boron trifluoride diethyl etherate (2 mL), sodium borohydride (128 mg) and tetrahydrofuran (15 mL), and the mixture was heated to 80 °C and stirred for 2 hours. The mixture was cooled to room temperature and partitioned between water and dichloromethane, the solvent was removed from the organic phase, and the residue was purified through silica gel column chromatography (2%MeOH/DCM) to obtain the product (100 mg).

¹H NMR (400 MHz, CDCl₃) δ 8.19 (d, *J* = 8.8 Hz, 2H), 7.39 (d, *J* = 8.8 Hz, 2H), 3.20-3.25 (m, 2H), 2.95-2.99 (m, 2H), 2.69 (s, 6H).

### Step B: 4-(2-(dimethylamino)ethyl)aniline

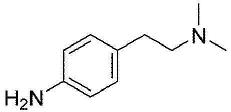

To a flask were sequentially added N,N-dimethyl-2-(4-nitrophenyl)ethan-1-amine (100 mg), 10% Pd/C (20 mg) and methanol (5 mL), the system was sufficiently purged with hydrogen gas, and the mixture was stirred at room temperature overnight. The reaction solution was filtered through celite, and the filtrate was concentrated and purified through silica gel column chromatography (10%MeOH/DCM) to obtain the product (30 mg).

¹H NMR (400 MHz, CDCl₃) δ 6.99 (d, *J* = 8.4 Hz, 2H), 6.62 (d, *J* = 8.4 Hz, 2H), 3.75-3.80 (brs, 2H), 2.73-2.78 (m, 2H), 2.59-2.63 (m, 2H), 2.39 (s, 6H).

### Step C: ((6-(5-fluoro-2-((4-(2-(dimethylamino)ethyl)phenyl)amino)-6-cyclopropyl-7H-pyrrolo [2,3-d]pyrimidin-7-yl)pyridin-2-yl)imino)dimethyl-λ⁶-sulfanone

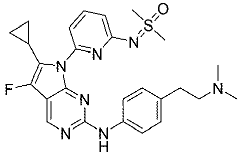

((6-(2-Chloro-5-fluoro-6-cyclopropyl-7*H*-pyrrolo[2,3-d]pyrimidin-7-yl)pyridin-2-yl)imino)di methyl-λ⁶-sulfanone (38 mg), 4-(2-(dimethylamino)ethyl)aniline (30 mg), Pd₂(dba)₃ (10 mg), Dave-phos (12 mg) and sodium tert-butoxide (15 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (7 mg).

¹H NMR (400 MHz, CDCl₃) δ 8.61 (s, 1H), 7.76 (t, *J* = 8.0 Hz, 1H), 7.59 (d, *J* = 8.8 Hz, 2H), 7.19-7.22 (m, 2H), 7.12 (d, *J* = 8.8 Hz, 2H), 6.83 (d, *J* = 8.0 Hz, 1H), 3.31 (s, 6H), 3.16 (s, 4H), 2.83 (s, 6H), 2.21-2.27 (m, 1H), 0.84-0.89 (m, 2H), 0.73-0.78 (m, 2H).

### Example 33

((6-(6-methoxymethyl-2-((4-(4-methylpiperazin-1-yl)phenyl)amino)-7*H*-pyrrolo[2,3-d]pyrim idin-7-yl)pyridin-2-yl)imino)dimethyl-λ⁶-sulfanone

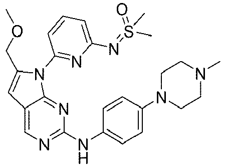

At 0 °C, ((6-(2-Chloro-6-hydroxymethyl-7*H*-pyrrolo[2,3-d]pyrimidin-7-yl) pyridin-2-yl) imino) dimethyl-λ⁶-sulfanone (50 mg) was dissolved in dry tetrahydrofuran (4 mL), then sodium hydride (60%, 6 mg) was added, the mixture was stirred for 30 minutes, iodomethane (40 mg) was added, the mixture was stirred for 4 hours, the reaction was quenched with water, the mixture was extracted with ethyl acetate, and the organic phase was rotavaped to obtain ((6-(2-chloro-6-methoxymethyl-7*H*-pyrrolo[2,3 -d]pyrimidin-7-yl) pyridin-2-yl)imino)dimethyl-λ⁶-sulfanone (36 mg); then ((6-(2-Chloro-6-methoxymethyl-7*H*-pyrrolo[2,3-d]pyrimidin-7-yl) pyridin-2-yl) imino) dimethyl-λ⁶-sulfanone (36 mg), 4-(4-methylpiperazin-1-yl)aniline (20 mg), Pd₂(dba)₃ (10 mg), 2-dicyclohexylphosphino-2',4',6'-tiiisopropylbiphenyl (15 mg) and caesium carbonate (49 mg) were mixed in a mixed solvent of 1,4-dioxane (5 mL) and DMF (1 mL), the system was sufficiently purged with nitrogen gas, the tube was sealed and heated in a microwave reactor to 95 °C for the mixture to react for 5 hours. The reaction solution was evaporated to remove the solvent, and the residue was purified through preparative TLC (10%MeOH/DCM) to obtain the target product (17 mg).

¹H NMR (400 MHz, CD₃OD) δ 8.58 (s, 1H), 7.80 (t, *J* = 8.0 Hz, 1H), 7.56 (d, *J* = 8.4 Hz, 2H), 7.28 (d, *J*=8.4 Hz, 1H), 6.91(d, *J* = 8.0 Hz, 2H), 6.79 (d, *J* = 8.0 Hz, 1H), 6.57 (s, 1H), 4.83 (s, 2H), 3.33 (s, 6H), 3.25(s, 3H), 3.19-3.14(m, 4H), 2.78-2.72 (m, 4H), 2.45 (s, 3H).

### Example 34

### ((6-(2-((4-(4-(morpholine-4-yl)piperidin-1-yl)phenyl)amino)-6-cyclopropyl-7H-pyrrolo[2,3-d] pyrimidin-7-yl)pyridin-2-yl)imino)dimethyl-λ⁶-sulfanone

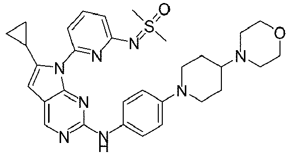

### Step A: 4-(1-(4-nitrophenyl)piperidin-4-yl)morpholine

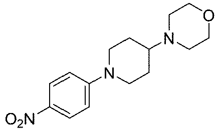

Under nitrogen gas, a solution of 4-(piperidin-4-yl)morpholine (600 mg), 4-fluoro nitrobenzene (1.0 g) and potassium carbonate (1.9 g) in dimethyl sulfoxide (20 mL) was heated to 100 °C and stirred overnight. The mixture was cooled to room temperature and partitioned between water and dichloromethane, the solvent was removed from the organic phase, and the residue was purified through silica gel column chromatography (1%MeOH/DCM) to obtain the product (1.1 g).

¹H NMR (400 MHz, CDCl₃) δ 8.11 (d, *J* = 9.2 Hz, 2H), 6.81 (d, *J* = 9.2 Hz, 2H), 3.95-4.02 (m, 2H), 3.71-3.76 (m, 4H), 2.95-3.04 (m, 2H), 2.54-2.59 (m, 4H), 2.40-2.50 (m, 1H), 1.93-2.02 (m, 2H), 1.53-1.64 (m, 2H).

### Step B: 4-(1-(4-aminophenyl)piperidin-4-yl)morpholine

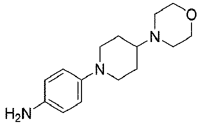

To a flask were sequentially added 4-(1-(4-nitrophenyl)piperidin-4-yl)morpholine (1.1 g), 10% Pd/C (300 mg) and methanol (20 mL), the system was sufficiently purged with hydrogen gas, and the mixture was stirred at room temperature overnight. The reaction solution was filtered through celite, and the filtrate was concentrated and purified through silica gel column chromatography (10%MeOH/DCM) to obtain the product (950 mg).

¹H NMR (400 MHz, CDCl₃) δ 6.82 (d, *J* = 8.8 Hz, 2H), 6.64 (d, *J* = 8.8 Hz, 2H), 3.69-3.78 (m, 4H), 3.48-3.56 (m, 2H), 3.31-3.48 (brs, 2H), 2.55-2.64 (m, 6H), 2.23-2.32 (m, 1H), 1.88-1.98 (m, 2H), 1.61-1.75 (m, 2H).

### Step C: ((6-(2-((4-(4-(morpholine-4-yl)piperidin-1-yl) phenyl) amino) -6-cyclopropyl- 7H-pyrrolo[2,3-d]pyrimidin-7-yl)pyridin-2-yl)imino)dimethyl-λ⁶-sulfanone

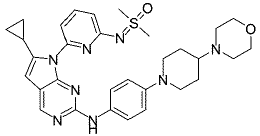

((6-(2-Chloro-6-cyclopropyl-7*H*-pyrrolo[2,3-d]pyrimidin-7-yl)pyridin-2-yl)imino)dimethyl-λ ⁶-sulfanone (100 mg), 4-(1-(4-aminophenyl)piperidin-4-yl)morpholine (73 mg), Pd₂(dba)₃ (26 mg), Dave-phos (33 mg) and sodium tert-butoxide (41 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (40 mg).

¹H NMR (400 MHz, CDCl₃) δ 8.50 (s, 1H), 7.74 (t, *J* = 7.6 Hz, 1H), 7.48 (d, *J* = 8.8 Hz, 2H), 7.21 (d, *J* = 8.0 Hz, 1H), 6.95 (s, 1H), 6.86 (d, *J* = 8.8 Hz, 2H), 6.82 (d, *J* = 8.0 Hz, 1H), 6.06 (s, 1H), 3.76-3.96 (m, 4H), 3.59-3.68 (m, 2H), 3.29 (s, 6H), 2.63-2.84 (m, 6H), 2.40-2.59 (m, 1H), 2.29-2.38 (m, 1H), 1.97-2.08 (m, 2H), 1.68-1.84 (m, 2H),, 0.87-0.92 (m, 2H), 0.65-0.69 (m, 2H).

### Example 35

### ((6-(5-fluoro-2-((4-(4-(morpholine-4-yl)piperidin-1-yl)phenyl)amino)-6-cyclopropyl-7H-pyrr olo[2,3-d]pyrimidin-7-yl)pyridin-2-yl)imino)dimethyl-λ⁶-sulfanone

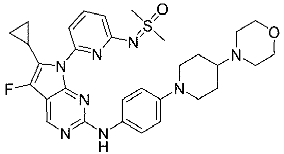

((6-(2-Chloro-5-fluoro-6-cyclopropyl-7*H*-pyrrolo[2,3-d]pyrimidin-7-yl)pyridin-2-yl)imino)di methyl-λ⁶-sulfanone (50 mg), 4-(1-(4-aminophenyl)piperidin-4-yl)morpholine (17 mg), Pd₂(dba)₃ (12 mg), Dave-phos (16 mg) and sodium tert-butoxide (20 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (16 mg).

¹H NMR (400 MHz, CDCl₃) δ 8.57 (s, 1H), 7.72 (t, *J* = 8.0 Hz, 1H), 7.48 (d, *J* = 8.8 Hz, 2H), 7.21 (d, *J* = 8.0 Hz, 1H), 6.99 (s, 1H), 6.87 (d, *J* = 8.8 Hz, 2H), 6.80 (d, *J* = 8.0 Hz, 1H), 4.31-4.47 (m, 2H), 3.76-3.95 (m, 4H), 3.62-3.68 (m, 3H), 3.30 (s, 6H), 2.64-2.81 (m, 3H), 2.41-2.53 (m, 1H), 2.18-2.26 (m, 1H), 1.97-2.11 (m, 2H), 1.67-1.84 (m, 2H), 0.83-0.88 (m, 2H),, 0.72-0.76 (m, 2H).

### Example 36

### ((6-(6-cyclopropyl-2-((6-(4-methylpiperazin-1-yl)pyridin-3-yl)amino)-7H-pyrrolo[2,3-d]pyri midin-7-yl)pyridin-2-yl)imino)dimethyl-λ⁶-sulfanone

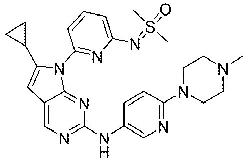

### Step A: 1-methyl-4-(5-nitropyridin-2-yl)piperazine

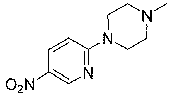

Under nitrogen gas, a suspension of 2-chloro-5-nitropyridine (3.00 g), N-methylpiperazine (1.89 g) and potassium carbonate (2.61 g) in DMF (50 mL) was heated at 100 °C overnight. The mixture was cooled to room temperature, filtered, and washed with dichloromethane, the solvent was removed from the filtrate, and the residue was purified through silica gel column chromatography (100%EtOAc) to obtain the product (2.0 g).

¹H NMR (400 MHz, CDCl₃) δ 9.04 (d, *J* = 2.4 Hz, 1H), 8.20(dd, *J =* 5.6 Hz, 2.8Hz, 1H), 6.57(d, *J* = 9.6 Hz, 1H), 3.76-3.83(m, 4H), 2.48-2.55(m, 4H), 2.36(s, 3H).

### Step B: 1-methyl-4-(5-aminopyridin-2-yl)piperazine

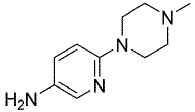

According to the method of step C of example 8, 1-methyl-4-(5-nitropyridin-2-yl)piperazine was used as the starting material to undergo catalytic hydrogenation to obtain the product (310 mg).

¹H NMR (400 MHz, CDCl₃) δ 7.80 (d, *J* = 2.8 Hz, 1H), 6.99 (dd, *J* = 4.8 Hz, 2.8Hz, 1H), 6.58 (d, *J* = 8.8 Hz, 1H), 3.37-3.43 (m, 4H), 3.23-3.36 (brs, 2H), 2.51-2.58 (m, 4H), 2.34 (s, 3H).

### Step C: ((6-(6-cyclopropyl-2-((6-(4-methylpiperazin-1-yl) pyridin-3-yl) amino) -7H- pyrrolo [2,3-d] pyrimidin-7-yl)pyridin-2-yl)imino)dimethyl-λ⁶-sulfanone

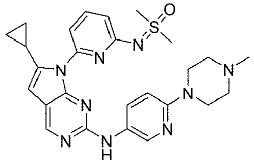

According to the method of step F of example 1, 1-methyl-4-(5-aminopyridin-2-yl) piperazine and ((6-(2-chloro-6-cyclopropyl-7*H*-pyrrolo[2,3-d] pyrimidin-7-yl) pyridin-2-yl) imino) dimethyl- λ⁶-sulfanone were used as the starting material to obtain the product (50 mg).

¹H NMR (400 MHz, CDCl₃) δ 8.50(s, 1H), 8.27 (d, *J* = 2.4 Hz, 1H), 7.99 (dd, *J =* 5.2 Hz, 2.8 Hz, 1H), 7.72 (t, *J* = 8.0 Hz, 1H), 7.19 (s, 1H), 7.16 (d, *J* = 8.0 Hz, 1H), 6.81 (d, *J* = 8.0 Hz, 1H), 6.62 (d, *J* = 9.2 Hz, 1H), 6.06 (s, 1H), 3.64-3.72(m, 4H), 3.31 (s, 6H), 2.78-2.86 (m, 4H), 2.54(s, 3H), 2.27-2.36 (m, 1H), 0.86-0.93 (m, 2H), 0.64-0.70 (m, 2H).

### Example 37

### ((6-(6-cyclopropyl-5-fluoro-2-((6-(4-methylpiperazin-1-yl)pyridin-3-yl)amino)-7H-pyrrolo[2, 3-d]pyrimidin-7-yl)pyridin-2-yl)imino)dimethyl-λ⁶-sulfanone

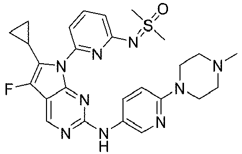

According to the method of step F of example 1, 1-methyl-4-(5-aminopyridin-2-yl) piperazine and ((6-(2-chloro-5-fluoro-6-cyclopropyl-7*H*-pyrrolo[2,3-d] pyrimidin-7-yl) pyridin-2-yl) imino)dimethyl-λ⁶-sulfanone were used as the starting material to obtain the product (31 mg).

¹H NMR (400 MHz, CDCl₃) δ 8.57(s, 1H), 8.28 (d, *J* = 2.4 Hz, 1H), 7.97 (dd, *J* = 5.2Hz, 2.8 Hz, 1H), 7.71 (t, *J* = 8.0 Hz, 1H), 7.25 (s, 1H), 7.15 (d, *J* = 8.0 Hz, 1H), 6.79 (d, *J* = 8.0 Hz, 1H), 6.62 (d, *J* = 9.2 Hz, 1H), 3.67-3.75 (m, 4H), 3.31 (s, 6H), 2.83-2.92 (m, 4H), 2.57 (s, 3H), 2.16-2.23 (m, 1H), 0.82-0.88 (m, 2H), 0.71-0.77 (m, 2H).

### Example 38

### ((6-(2-(((3-methoxy-4-(4-methylpiperazin-1-yl)phenyl))amino)-6-cyclopropyl-7H-pyrrolo[2, 3-d]pyrimidin-7-yl)pyridin-2-yl)imino)dimethyl-λ⁶-sulfanone

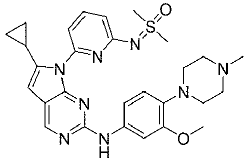

### Step A: 1-(2-methoxy-4-nitrophenyl)-4-methylpiperazine

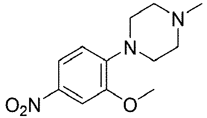

Under nitrogen gas, a solution of N-methylpiperazine (585 mg), 1-fluoro-2- methoxy- 4-nitrobenzene (1.0 g) and potassium carbonate (3.2 g) in dimethyl sulfoxide (20 mL) was heated to 100 °C and stirred overnight. The mixture was cooled to room temperature and partitioned between water and dichloromethane, the solvent was removed from the organic phase, and the residue was purified through silica gel column chromatography (1%MeOH/DCM) to obtain the product (1.3 g).

¹H NMR (400 MHz, CDCl₃) δ 7.86 (dd, *J =* 8.8 Hz, 2.4 Hz, 1H), 7.71 (d, *J* = 2.4 Hz, 1H), 6.89 (d, *J* = 8.8 Hz, 1H), 3.95 (s, 3H), 3.23-3.29 (m, 4H), 2.58-2.64 (m, 4H), 2.37 (s, 3H).

### Step B: 3-methoxy-4-(4-methylpiperazin-1-yl)aniline

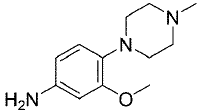

To a flask were sequentially added 1-(2-methoxy-4-nitrophenyl)-4- methylpiperazine (200 mg), 10% Pd/C (50 mg) and methanol (10 mL), the system was sufficiently purged with hydrogen gas, and the mixture was stirred at room temperature overnight. The reaction solution was filtered through celite, and the filtrate was concentrated to obtain the product, which was used directly in the next step.

### Step C: ((6-(2-(((3-methoxy-4-(4-methylpiperazin-1-yl) phenyl)) amino) -6- cyclopropyl-7H- pyrrolo[2,3-d]pyrimidin-7-yl)pyridin-2-yl)imino)dimethyl-λ⁶-sulfanone

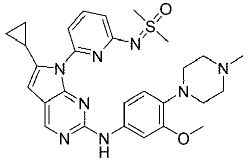

((6-(2-Chloro-6-cyclopropyl-7*H*-pyrrolo[2,3-d]pyrimidin-7-yl)pyridin-2-yl)imino)dimethyl-λ ⁶-sulfanone (100 mg), 3-methoxy-4-(4-methylpiperazin- 1-yl) aniline (31 mg), Pd₂(dba)₃ (26 mg), Dave-phos (33 mg) and sodium tert-butoxide (41 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (48 mg).

¹H NMR (400 MHz, CDCl₃) δ 8.54 (s, 1H), 7.71 (t, *J* = 8.0 Hz, 1H), 7.59 (d, *J* = 2.0 Hz, 1H), 7.13 (d, *J* = 8.0 Hz, 1H), 6.82-6.88 (m, 3H), 6.07 (s, 1H), 3.60 (s, 3H), 3.34-3.43 (m, 4H), 3.28 (s, 6H), 3.01-3.22 (m, 4H), 2.79 (s, 3H), 2.14-2.23 (m, 1H), 0.86-0.92 (m, 2H), 0.67-0.71 (m, 2H).

### Example 39

### ((6-(5-fluoro-2-(((3-methoxy-4-(4-methylpiperazin-1-yl)phenyl))amino)-6-cyclopropyl-7H-p yrrolo[2,3-d]pyrimidin-7-yl)pyridin-2-yl)imino)dimethyl-λ⁶-sulfanone

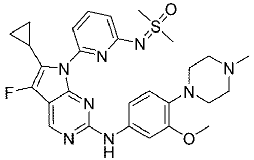

((6-(2-Chloro-5-fluoro-6-cyclopropyl-7*H*-pyrrolo[2,3-d]pyrimidin-7-yl)pyridin-2-yl)imino)di methyl-λ⁶-sulfanone (76 mg), 3-methoxy-4- (4-methylpiperazin- 1-yl) aniline (22 mg), Pd₂(dba)₃ (19 mg), Dave-phos (24 mg) and sodium tert-butoxide (29 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (20 mg).

¹H NMR (400 MHz, CDCl₃) δ 8.60 (s, 1H), 7.69 (t, *J* = 8.0 Hz, 1H), 7.51 (d, *J* = 2.0 Hz, 1H), 7.23 (s, 1H), 7.13 (d, *J* = 8.0 Hz, 1H), 6.90 (dd, *J* = 8.8 Hz, 2.0Hz, 1H), 6.84 (d, *J* = 8.8 Hz, 1H), 6.80 (d, *J* = 8.0 Hz, 1H), 3.62 (s, 3H), 3.21-3.38 (m, 10H), 2.98-3.18 (m, 4H), 2.67 (s, 3H), 2.02-2.11 (m, 1H), 0.82-0.89 (m, 2H), 0.72-0.78 (m, 2H).

### Example 40

### ((6-(2-(((3-fluoro-4-(4-methylpiperazin-1-yl)phenyl))amino)-6-cyclopropyl-7H-pyrrolo[2,3-d] pyrimidin-7-yl)pyridin-2-yl)imino)dimethyl-λ⁶-sulfanone

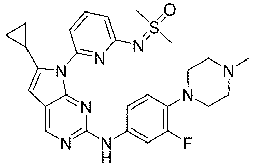

### Step A: 1-(2-fluoro-4-nitrophenyl)-4-methylpiperazine

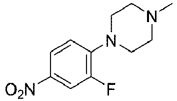

Under nitrogen gas, a solution of N-methylpiperazine (630 mg), 1,2-difluoro-4-nitrobenzene (1.0 g) and potassium carbonate (3.5 g) in dimethyl sulfoxide (20 mL) was heated to 100 °C and stirred overnight. The mixture was cooled to room temperature and partitioned between water and dichloromethane, the solvent was removed from the organic phase, and the residue was purified through silica gel column chromatography (1%MeOH/DCM) to obtain the product (1.2 g).

¹H NMR (400 MHz, CDCl₃) δ 7.97-8.01 (m, 1H), 7.91 (dd, *J* = 13.2 Hz, 2.8 Hz, 1H), 6.92 (t, *J=* 8.8 Hz, 1H), 3.31-3.36 (m, 4H), 2.57-2.63 (m, 4H), 2.37 (s, 3H).

### Step B: 3-fluoro-4-(4-methylpiperazin-1-yl)aniline

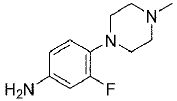

To a flask were sequentially added 1-(2-fluoro-4-nitrophenyl) -4-methylpiperazine (200 mg), 10% Pd/C (50 mg) and methanol (10 mL), the system was sufficiently purged with hydrogen gas, and the mixture was stirred at room temperature overnight. The reaction solution was filtered through celite, and the filtrate was concentrated to obtain the product, which was used directly in the next step.

### Step C: ((6-(2-(((3-fluoro-4-(4-methylpiperazin-1-yl) phenyl)) amino) -6- cyclopropyl- 7H-pyrrolo [2,3-d]pyrimidin-7-yl)pyridin-2-yl)imino)dimethyl-λ⁶-sulfanone

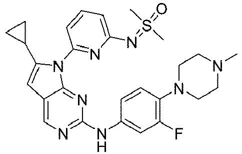

((6-(2-Chloro-6-cyclopropyl-7*H*-pyrrolo[2,3-d]pyrimidin-7-yl)pyridin-2-yl)imino)dimethyl-λ ⁶-sulfanone (100 mg), 3-fluoro-4-(4-methylpiperazin-1-yl)aniline (30 mg), Pd₂(dba)₃ (26 mg), Dave-phos (33 mg) and sodium tert-butoxide (41 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (50 mg).

¹H NMR (400 MHz, CDCl₃) δ 8.52 (s, 1H), 7.75-7.84 (m, 2H), 7.24-7.27 (m, 1H), 7.01 (dd, *J* = 8.4 Hz, 2.4Hz, 1H), 6.82-6.89 (m, 2H), 6.07 (s, 1H), 3.31 (s, 6H), 3.03-3.11 (m, 4H), 2.59-2.67 (m, 4H), 2.38-2.46 (m, 1H), 2.37 (s, 3H), 0.87-0.94 (m, 2H), 0.64-0.70 (m, 2H).

### Example 41

### ((6-(5-fluoro-2-(((3-fluoro-4-(4-methylpiperazin-1-yl)phenyl))amino)-6-cyclopropyl-7H-pyrr olo[2,3-d]pyrimidin-7-yl)pyridin-2-yl)imino)dimethyl-λ⁶-sulfanone

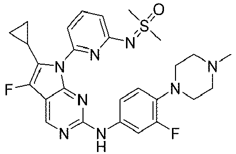

((6-(2-Chloro-5-fluoro-6-cyclopropyl-7*H*-pyrrolo[2,3-d]pyrimidin-7-yl)pyridin-2-yl)imino)di methyl-λ⁶-sulfanone (76 mg), 3-fluoro-4-(4-methylpiperazin-1-yl)aniline (21 mg), Pd₂(dba)₃ (19 mg), Dave-phos (24 mg) and sodium tert-butoxide (29 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (40 mg).

¹H NMR (400 MHz, CDCl₃) δ 8.59 (s, 1H), 7.82 (dd, *J =* 15.4 Hz, 2.4Hz, 1H), 7.76 (t, *J =* 8.0 Hz, 1H), 7.25-7.27 (m, 1H), 7.10 (s, 1H), 7.01 (dd, *J =* 8.4 Hz, 1.6Hz, 1H), 6.88 (t, *J =* 8.8 Hz, 1H), 6.81 (d, *J* = 8.0 Hz, 1H), 3.32 (s, 6H), 3.06-3.12 (m, 4H), 2.61-2.69 (m, 4H), 2.39 (s, 3H), 2.24-2.53 (m, 1H), 0.84-0.91 (m, 2H), 0.72-0.78 (m, 2H).

### Example 42

### ((6-(5-fluoro-2-((1-methyl-1,2,3,4-tetrahydroquinolin-6-yl)amino)-6-cyclopropyl-7H-pyrrolo [2,3-d]pyrimidin-7-yl)pyridin-2-yl)imino)dimethyl-λ⁶-sulfanone

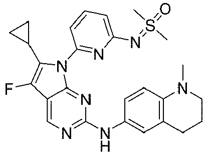

### Step A: N-(quinolin-6-yl)acetamide

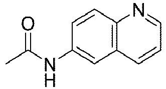

Quinolin-6-amine (2.0 g) was dissolved in dichloromethane (20 mL), to the mixture was dropwise added acetic anhydride (2 mL), and the mixture was stirred at room temperature for 2 hours. The mixture was washed with an aqueous solution of saturated sodium bicarbonate and partitioned between water and dichloromethane, the organic phase was evaporated to remove the solvent to obtain the crude product, which was used directly in the next step.

### Step B: N-(1,2,3,4-tetrahydroquinolin-6-yl)acetamide

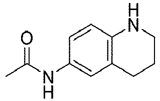

N-(quinolin-6-yl)acetamide (2.0 g), nickel chloride hexahydrate (2.6 g) and sodium borohydride (1.7 g) were mixed in methanol (20 mL), and the mixture was stirred at room temperature for 1 hour. The solvent was evaporated, the residue was dissolved in dichloromethane and filtered through celite, and the filtrate was washed with water and evaporated to obtain the crude product, which was used directly in the next step.

### Step C: N-(1-methyl-1,2,3,4-tetrahydroquinolin-6-yl)acetamide

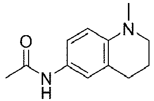

N-(1,2,3,4-tetrahydroquinolin-6-yl)acetamide (2.0 g), iodomethane (2.7 g) and potassium carbonate (2.6 g) were mixed in N,N-dimethylformamide (30 mL), and the mixture was stirred at room temperature for 4 hours. The mixture was partitioned between water and dichloromethane, and the organic phase was evaporated to obtain the crude product, which was used directly in the next step..

### Step D: 1-methyl-1,2,3,4-tetrahydroquinolin-6-amine

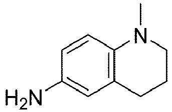

N-(1-methyl-1,2,3,4-tetrahydroquinolin-6-yl)acetamide (1.0 g) was dissolved in 1,4-dioxane (10 mL), to the solution was dropwise added a solution of 12 N hydrochloric acid (5 mL), the mixture was stirred at room temperature for 2 hours, and the solvent was removed to obtain the product (230 mg).

¹H NMR (400 MHz, DMSO) δ 9.58-10.01 (brs, 3H), 8.38-8.68 (brs, 1H), 6.98 (d, *J* = 8.8 Hz, 1H), 6.87 (s, 1H), 6.63 (d, *J* = 8.8 Hz, 1H), 3.21 (t, *J* = 5.6 Hz, 2H), 2.84 (s, 3H), 2.70 (t, *J* = 6.0 Hz, 2H), 1.84-1.92 (m, 2H).

### Step E: ((6-(5-fluoro-2-((1-methyl-1,2,3,4-tetrahydroquinolin-6-yl) amino) -6- cyclopropyl-7H- pyrrolo[2,3-d]pyrimidin-7-yl)pyridin-2-yl)imino)dimethyl-λ⁶-sulfanone

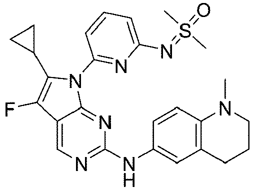

((6-(2-Chloro-5-fluoro-6-cyclopropyl-7*H*-pyrrolo[2,3-d]pyrimidin-7-yl)pyridin-2-yl)imino)di methyl-λ⁶-sulfanone (38 mg), 1-methyl-1,2,3,4-tetrahydroquinolin-6-amine(7 mg), Pd₂(dba)₃ (10 mg), Dave-phos (12 mg) and sodium tert-butoxide (15 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (3 mg).

¹H NMR (400 MHz, CDCl₃) δ 8.56 (s, 1H), 7.70 (t, *J =* 8.0 Hz, 1H), 7.34 (d, *J =* 1.6 Hz, 1H), 7.23 (d, *J =* 8.0 Hz, 1H), 7.13 (dd, *J* = 9.2 Hz, 2.4 Hz, 1H), 6.82-6.99 (brs, 1H), 6.78 (d, *J* = 8.0 Hz, 1H), 6.54 (d, J = 9.2 Hz, 1H), 3.29 (s, 6H), 3.16 (t, J = 5.6 Hz, 2H), 2.85 (s, 3H), 2.72 (t, J = 6.4 Hz, 2H), 2.19-2.27 (m, 1H), 1.94-2.03 (m, 2H), 0.81-0.89 (m, 2H), 0.70-0.76 (m, 2H).

### Example 43

### ((6-(2-(((3-chloro-4-(4-methylpiperazin-1-yl)phenyl))amino)-6-cyclopropyl-7H-pyrrolo[2,3-d]pyrimidin-7-yl)pyridin-2-yl)imino)dimethyl-λ⁶-sulfanone

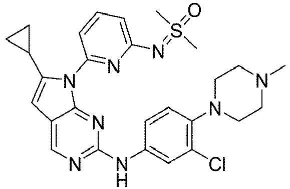

### Step A: 1-(2-chloro-4-nitrophenyl)-4-methylpiperazine

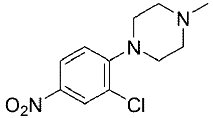

Under nitrogen gas, a mixture of N-methylpiperazine (568 mg), 2-chloro-1- fluoro- 4-nitrobenzene (1.0 g) and potassium carbonate (3.2 g) in dimethyl sulfoxide (20 mL) was heated to 100 °C and stirred overnight. The mixture was cooled to room temperature and partitioned between water and dichloromethane, the solvent was removed from the organic phase, and the residue was purified through silica gel column chromatography (1%MeOH/DCM) to obtain the product (1.3 g).

¹H NMR (400 MHz, CDCl₃) δ 8.25 (d, J = 2.8 Hz, 1H), 8.09 (dd, J = 9.2 Hz, 2.8 Hz, 1H), 7.05 (d, *J* = 9.2 Hz, 1H), 3.22-3.29 (m, 4H), 2.59-2.66 (m, 4H), 2.38 (s, 3H).

### Step B: 3-chloro-4-(4-methylpiperazin-1-yl)aniline

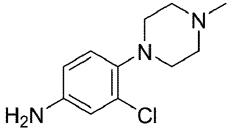

To a flask were sequentially added 1-(2-chloro-4-nitrophenyl)-4-methylpiperazine (200 mg), zinc dust (254 mg), glacial acetic acid (2 mL) and ethanol (10 mL), and the mixture was stirred at room temperature for 1 hour. An aqueous solution of 1 N sodium hydroxide was used to adjust the pH to 10, the mixture was partitioned between water and dichloromethane, the solvent was removed from the organic phase, and the residue was purified through silica gel column chromatography (1%MeOH/DCM) to obtain the product (160 mg).

¹H NMR (400 MHz, CDCl₃) δ 6.90 (d, J = 8.4 Hz, 1H), 6.74 (d, *J =* 2.8 Hz, 1H), 6.55 (dd, *J* = 8.4 Hz, 2.8Hz, 1H), 3.54-3.82 (brs, 2H), 2.97-3.04 (m, 4H), 2.63-2.75 (m, 4H), 2.40 (s, 3H).

### Step C: ((6-(2-(((3-chloro-4-(4-methylpiperazin-1-yl) phenyl)) amino) -6- cyclopropyl- 7H-pyrrolo [2,3-d]pyrimidin-7-yl)pyridin-2-yl)imino)dimethyl-λ⁶-sulfanone

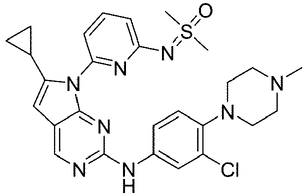

((6-(2-Chloro-6-cyclopropyl-7*H*-pyrrolo[2,3-d]pyrimidin-7-yl)pyridin-2-yl)imino)dimethyl-λ ⁶-sulfanone (50 mg), 4-(1-(4-aminophenyl)piperidin-4-yl)morpholine (32 mg), Pd₂(dba)₃ (13 mg), Dave-phos (17 mg) and sodium tert-butoxide (21 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (25 mg).

¹H NMR (400 MHz, CDCl₃) δ 8.53 (s, 1H), 8.08 (d, *J* = 2.4 Hz, 1H), 7.81 (t, *J* = 8.0 Hz, 1H), 7.19-7.26 (m, 1H), 6.98-7.02 (m, 2H), 6.84 (d, *J* = 7.6 Hz, 1H), 6.09 (s, 1H), 3.33 (s, 6H), 3.12-3.35 (m, 4H), 2.51-2.88 (m, 7H), 2.41-2.49 (m, 1H), 0.82-0.87 (m, 2H), 0.65-0.70 (m, 2H).

### Example 44

### ((6-(5-fluoro-2-(((3-chloro-4-(4-methylpiperazin-1-yl)phenyl))amino)-6-cyclopropyl-7H-pyrr olo[2,3-d]pyrimidin-7-yl)pyridin-2-yl)imino)dimethyl-λ⁶-sulfanone

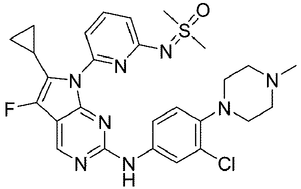

((6-(2-Chloro-5-fluoro-6-cyclopropyl-7*H*-pyrrolo[2,3-d]pyrimidin-7-yl)pyridin-2-yl)imino)di methyl-λ⁶-sulfanone (76 mg), 3-chloro-4-(4-methylpiperazin-1-yl)aniline (23 mg), Pd₂(dba)₃ (19 mg), Dave-phos (24 mg) and sodium tert-butoxide (29 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (25 mg).

¹H NMR (400 MHz, CDCl₃) δ 8.60 (s, 1H), 8.09 (d, *J* = 2.8 Hz, 1H), 7.80 (t, *J* = 8.0 Hz, 1H), 7.27 (d, *J* = 8.0 Hz, 1H), 7.17 (dd, *J =* 8.8 Hz, 2.4Hz, 1H), 7.11 (s, 1H), 6.98 (d, *J* = 8.8 Hz, 1H), 6.80 (d, *J* = 8.0 Hz, 1H), 3.32 (s, 6H), 3.08-3.17 (m, 4H), 2.71-2.86 (m, 4H), 2.48 (s, 3H), 2.26-2.36 (m, 1H), 0.84-0.91 (m, 2H), 0.72-0.78 (m, 2H).

### Example 45

### ((6-(5-fluoro-2-((4-(dimethylamino)phenyl)amino)-6-cyclopropyl-7H-pyrrolo[2,3-d]pyrimidi n-7-yl)pyridin-2-yl)imino)dimethyl-λ⁶-sulfanone

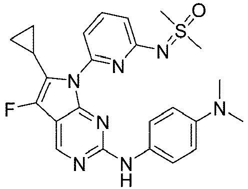

### Step A: N,N-dimethyl-4-nitroaniline

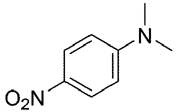

Under nitrogen gas, a solution of dimethylamine (315 mg), 4-fluoronitrobenzene (1.0 g) and potassium carbonate (4.0 g) in dimethyl sulfoxide (15 mL) was heated to 100 °C and stirred overnight. The mixture was cooled to room temperature and partitioned between water and dichloromethane, the solvent was removed from the organic phase, and the residue was purified through silica gel column chromatography (1%MeOH/DCM) to obtain the product (1.1 g).

¹H NMR (400 MHz, CDCl₃) δ 8.13 (d, *J* = 9.6 Hz, 2H), 6.61 (d, *J* = 9.6 Hz, 2H), 3.12 (s, 6H).

### Step B: N¹, N¹-dimethylbenzene-1,4-diamine

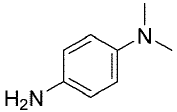

To a flask were sequentially added N,N-dimethyl-4-nitroaniline (1.1 g), 10% Pd/C (330 mg) and methanol (15 mL), the system was sufficiently purged with hydrogen gas, and the mixture was stirred at room temperature overnight. The reaction solution was filtered through celite, and the filtrate was concentrated and purified through silica gel column chromatography (10%MeOH/DCM) to obtain the product (900 mg).

¹H NMR (400 MHz, CDCl₃) δ 6.65-6.71 (m, 4H), 3.22-3.43 (brs, 2H), 2.83 (s, 6H).

### Step C: ((6-(5-fluoro-2-((4-(dimethylamino) phenyl) amino) -6- cyclopropyl -7H- pyrrolo [2,3-d]pyrimidin-7-yl)pyridin-2-yl)imino)dimethyl-λ⁶-sulfanone

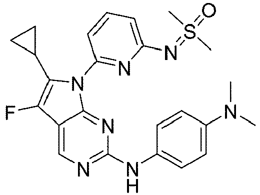

((6-(2-Chloro-5-fluoro-6-cyclopropyl-7*H*-pyrrolo[2,3-d]pyrimidin-7-yl)pyridin-2-yl)imino)di methyl-λ⁶-sulfanone (30 mg), N¹,N¹-dimethylbenzene-1,4-diamine (11 mg), Pd₂(dba)₃ (8 mg), Dave-phos (10 mg) and sodium tert-butoxide (12 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (3 mg).

¹H NMR (400 MHz, CDCl₃) δ 8.55 (s, 1H), 7.72 (t, *J* = 8.0 Hz, 1H),, 7.41-7.51 (m, 2H), 7.22 (d, *J* = 8.0 Hz, 1H), 6.94-7.06 (brs, 1H), 6.79 (d, *J* = 8.0 Hz, 1H), 6.65-6.78 (m, 2H), 3.30 (s, 6H), 2.91 (s, 6H), 2.18-2.26 (m, 1H), 0.82-0.89 (m, 2H), 0.71-0.77 (m, 2H).

### Example 46

### ((6-(5-fluoro-2-((3-methoxy-4-(4-(4-methylpiperazin-1-yl)piperidin-1-yl)phenyl)amino)-6-cy clopropyl-7H-pyrrolo[2,3-d]pyrimidin-7-yl)pyridin-2-yl)imino)dimethyl-λ⁶-sulfanone

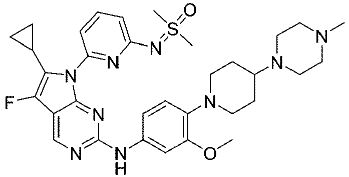

### Step A: 1-(1-(2-methoxy-4-nitrophenyl)piperidin-4-yl)-4-methylpiperazine

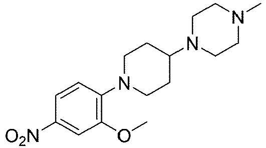

Under nitrogen gas, a solution of 1-methyl-4-(piperidin-4-yl)piperazine hydrochloride (4.2 g), 1-fluoro-2-methoxy-4-nitrobenzene (3.0 g) and potassium carbonate (9.7 g) in dimethyl sulfoxide (50 mL) was heated to 100 °C and stirred overnight. The mixture was cooled to room temperature and partitioned between water and dichloromethane, the solvent was removed from the organic phase, and the residue was purified through silica gel column chromatography (1%MeOH/DCM) to obtain the product (3.6 g).

¹H NMR (400 MHz, CDCl₃) δ 7.84 (dd, *J =* 8.8 Hz, 2.4Hz, 1H), 7.69 (d, *J* = 2.4 Hz, 1H), 6.88 (d, *J* = 8.8 Hz, 1H), 3.94 (s, 3H), 3.73-3.79 (m, 2H), 2.57-2.77 (m, 6H), 2.39-2.56 (m, 5H), 2.30 (s, 3H), 1.89-1.97 (m, 2H), 1.69-1.79 (m, 2H).

### Step B: 3-methoxy-4-(4-(4-methylpiperazin-1-yl)piperidin-1-yl)aniline

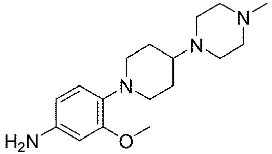

To a flask were sequentially added 1-(1-(2-methoxy-4-nitrophenyl) piperidin-4-yl) -4-methylpiperazine (1.0 g), 10% Pd/C (300 mg) and methanol (10 mL), the system was sufficiently purged with hydrogen gas, and the mixture was stirred at room temperature overnight. The reaction solution was filtered through celite, and the filtrate was concentrated and purified through silica gel column chromatography (10%MeOH/DCM) to obtain the product (900 mg).

¹H NMR (400 MHz, CDCl₃) δ 7.85 (dd, *J =* 8.8 Hz, 2.8Hz, 1H), 7.69 (d, *J* = 2.4 Hz, 1H), 6.88 (d, *J* = 8.8 Hz, 1H), 3.94 (s, 3H), 3.72-3.79 (m, 2H), 2.59-2.78 (m, 6H), 2.39-2.54 (m, 5H), 2.30 (s, 3H), 1.89-1.98 (m, 2H), 1.68-1.81 (m, 2H), 1.59 (brs, 2H).

### Step C: ((6-(5-fluoro-2-((3-methoxy-4-(4-(4-methylpiperazin-1-yl) piperidin-1-yl) phenyl) amino)-6-cyclopropyl-7H-pyrrolo[2,3-d]pyrimidin-7-yl)pyridin-2-yl)imino)dimethyl-λ⁶-sulfanone

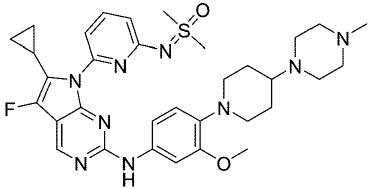

((6-(2-Chloro-5-fluoro-6-cyclopropyl-7*H*-pyrrolo[2,3-d]pyrimidin-7-yl)pyridin-2-yl)imino)di methyl-λ⁶-sulfanone (76 mg), 3-methoxy-4-(4-(4-methylpiperazin-1-yl)piperidin-1-yl)aniline (31 mg), Pd₂(dba)₃ (19 mg), Dave-phos (24 mg) and sodium tert-butoxide (29 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (30 mg).

¹H NMR (400 MHz, CDCl₃) δ 8.59 (s, 1H), 7.68 (t, *J* = 8.0 Hz, 1H), 7.52 (d, *J* = 1.6 Hz, 1H), 7.11-7.14 (m, 2H), 6.78-6.85 (m, 3H), 3.60 (s, 3H), 3.44-3.52 (m, 2H), 3.27 (s, 6H), 2.87-3.17 (m, 8H), 2.49-2.64 (m, 6H), 1.97-2.12 (m, 3H), 1.79-1.94 (m, 2H), 0.80-0.88 (m, 2H), 0.73-0.79 (m, 2H).

### Example 47

### ((6-(5-fluoro-2-((3-fluoro-4-(4-(4-methylpiperazin-1-yl)piperidin-1-yl)phenyl)amino)-6-cycl opropyl-7H-pyrrolo[2,3-d]pyrimidin-7-yl)pyridin-2-yl)imino)dimethyl-λ⁶-sulfanone

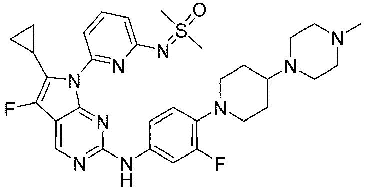

### Step A: 1-(1-(2-fluoro-4-nitrophenyl)piperidin-4-yl)-4-methylpiperazine

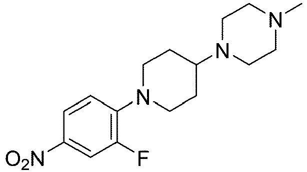

Under nitrogen gas, a solution of 1-methyl-4-(piperidin-4-yl)piperazine hydrochloride (1.5 g), 1,2-difluoro-4-nitrobenzene (1.0 g) and potassium carbonate (3.5 g) in dimethyl sulfoxide (20 mL) was heated to 100 °C and stirred overnight. The mixture was cooled to room temperature and partitioned between water and dichloromethane, the solvent was removed from the organic phase, and the residue was purified through silica gel column chromatography (1%MeOH/DCM) to obtain the product (1.5 g).

¹H NMR (400 MHz, CDCl₃) δ 7.94-7.98 (m, 1H), 7.89 (dd, *J* = 13.2 Hz, 2.8 Hz, 1H), 6.90 (t, *J* = 8.8 Hz, 1H), 3.73-3.82 (m, 2H), 2.85-2.94 (m, 2H), 2.31-2.72 (m, 9H), 2.30 (s, 3H), 1.93-2.01 (m, 2H), 1.65-1.76 (m, 2H).

### Step B: 3-fluoro-4-(4-(4-methylpiperazin-1-yl)piperidin-1-yl)aniline

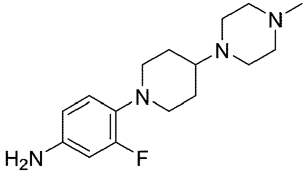

To a flask were sequentially added 1-(1-(2-fluoro-4-nitrophenyl) piperidin- 4-yl) -4-methylpiperazine (300 mg), 10% Pd/C (100 mg) and methanol (10 mL), the system was sufficiently purged with hydrogen gas, and the mixture was stirred at room temperature overnight. The reaction solution was filtered through celite, and the filtrate was concentrated and purified through silica gel column chromatography (10%MeOH/DCM) to obtain the product (270 mg).

¹H NMR (400 MHz, CDCl₃) δ 6.80 (t, *J* = 9.2 Hz, 1H), 6.37-6.44 (m, 2H), 3.52 (s, 2H), 3.29-3.37 (m, 2H), 2.32-2.72 (m, 11H), 2.30 (s, 3H), 1.84-1.94 (m, 2H),, 1.69-1.82 (m, 2H).

### Step C: ((6-(5-fluoro-2-((3-fluoro-4-(4-(4-methylpiperazin-1-yl) piperidin-1- yl) phenyl) amino)-6-cyclopropyl-7H-pyrrolo[2,3-d]pyrimidin-7-yl)pyridin-2-yl)imino)dimethyl-λ⁶-sulfanone

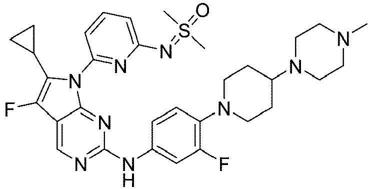

((6-(2-Chloro-5-fluoro-6-cyclopropyl-7*H*-pyrrolo[2,3-d]pyrimidin-7-yl)pyridin-2-yl)imino)di methyl-λ⁶-sulfanone (76 mg), 3-fluoro-4-(4-(4-methylpiperazin-1-yl)piperidin-1-yl)aniline (30 mg), Pd₂(dba)₃ (19 mg), Dave-phos (24 mg) and sodium tert-butoxide (29 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (23 mg).

¹H NMR (400 MHz, CDCl₃) δ 8.59 (s, 1H), 7.81 (dd, *J =* 15.2 Hz, 2.4 Hz, 1H), 7.76 (t, *J =* 8.0 Hz, 1H), 7.25 (d, *J =* 8.0 Hz, 1H), 7.20 (s, 1H), 6.98 (dd, *J =* 8.4 Hz, 2.0 Hz, 1H), 6.86 (t, *J* = 9.2 Hz, 1H), 6.81 (d, *J* = 8.0 Hz, 1H), 3.39-3.46 (m, 2H), 3.32 (s, 6H), 2.61-2.89 (m, 10H), 2.45-2.58 (m, 1H), 2.45 (s, 3H), 2.24-2.33 (m, 1H), 1.92-2.02 (m, 2H), 1.72-1.85 (m, 2H), 0.84-0.91 (m, 2H), 0.72-0.78 (m, 2H).

### Example 48

### ((6-(2-((3-fluoro-4-(4-(4-methylpiperazin-1-yl)piperidin-1-yl)phenyl)amino)-6-cyclopropyl-7 H-pyrrolo[2,3-d]pyrimidin-7-yl)pyridin-2-yl)imino)dimethyl-λ⁶-sulfanone

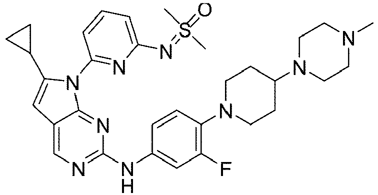

((6-(2-Chloro-6-cyclopropyl-7*H*-pyrrolo[2,3-d]pyrimidin-7-yl)pyridin-2-yl)imino)dimethyl-λ ⁶-sulfanone (72 mg), 3-fluoro-4-(4-(4-methylpiperazin-1-yl)piperidin-1-yl)aniline (30 mg), Pd₂(dba)₃ (19 mg), Dave-phos (24 mg) and sodium tert-butoxide (29 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (20 mg).

¹H NMR (400 MHz, CDCl₃) δ 8.52 (s, 1H), 7.75-7.83 (m, 2H), 7.24-7.26 (m, 2H), 6.98 (dd, *J* = 8.8 Hz, 2.4 Hz, 1H), 6.82-6.87 (m, 2H), 6.07 (s, 1H), 3.38-3.45 (m, 2 H), 3.32 (s, 6H), 2.58-2.86 (m, 10H), 2.40-2.56 (m, 2H), 2.40 (s, 3H), 1.91-1.98 (m, 2H), 1.71-1.84 (m, 2H), 0.87-0.94 (m, 2H), 0.64-0.69 (m, 2H).

### Example 49

### ((6-(5-fluoro-2-((3-trifluoromethyl-4-(4-(4-methylpiperazin-1-yl)piperidin-1-yl)phenyl)amin o)-6-cyclopropyl-7H-pyrrolo[2,3-d]pyrimidin-7-yl)pyridin-2-yl)imino)dimethyl-λ⁶-sulfanone

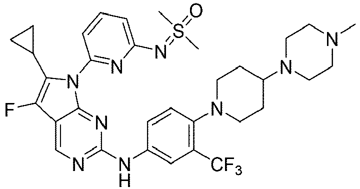

### Step A: 1-(1-(2-trifluoromethyl-4-nitrophenyl)piperidin-4-yl)-4-methylpiperazine

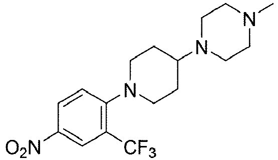

Under nitrogen gas, a suspension of 1-methyl-4-(piperidin-4-yl)piperazine hydrochloride (1.2 g), 1-fluoro-2-trifluoromethyl-4-nitrobenzene (1.0 g) and potassium carbonate (2.7 g) in dimethyl sulfoxide (20 mL) was heated to 100 °C and stirred overnight. The mixture was cooled to room temperature and partitioned between water and dichloromethane, the solvent was removed from the organic phase, and the residue was purified through silica gel column chromatography (1%MeOH/DCM) to obtain the product (1.3 g).

¹H NMR (400 MHz, CDCl₃) δ 8.50 (d, *J* = 2.4 Hz, 1H), 8.29 (dd, *J* = 9.2 Hz, 2.8 Hz, 1H), 7.23 (d, *J* = 9.2 Hz, 1H), 3.41-3.49 (m, 2H), 2.89 (t, *J* = 12.4 Hz, 2H), 2.33-2.74 (m, 9H), 2.30 (s, 3H), 1.92-2.01 (m, 2H), 1.67-1.79 (m, 2H).

### Step B: 3-trifluoromethyl-4-(4-(4-methylpiperazin-1-yl)piperidin-1-yl)aniline

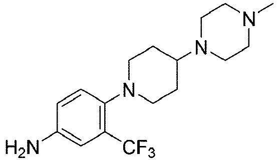

To a flask were sequentially added 1-(1-(2-trifluoromethyl-4-nitrophenyl) piperidin-4- yl) -4-methylpiperazine (200 mg), 10% Pd/C (60 mg) and methanol (10 mL), the system was sufficiently purged with hydrogen gas, and the mixture was stirred at room temperature overnight. The reaction solution was filtered through celite, and the filtrate was concentrated and purified through silica gel column chromatography (10%MeOH/DCM) to obtain the product (150 mg).

¹H NMR (400 MHz, CDCl₃) δ 7.15 (d, *J* = 8.4 Hz, 1H), 6.89 (d, *J* = 2.8 Hz, 1H), 6.77 (dd, *J* = 8.4 Hz, 2.8 Hz, 1H), 3.72 (s, 2H), 2.96-3.03 (m, 2H), 2.52-2.84 (m, 10H), 2.36-2.46 (m, 1H), 2.30 (s, 3H), 1.85-1.94 (m, 2H), 1.63-1.76 (m, 2H).

### Step C: ((6-(5-fluoro-2-((3-trifluoromethyl-4-(4-(4-methylpiperazin-1-yl) piperidin- 1- yl) phenyl)amino)-6-cyclopropyl-7H-pyrrolo[2,3-d]pyrimidin-7-yl)pyridin-2-yl)imino)dimethyl-λ⁶-su lfanone

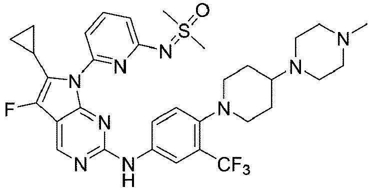

((6-(2-Chloro-5-fluoro-6-cyclopropyl-7*H*-pyrrolo[2,3-d]pyrimidin-7-yl)pyridin-2-yl)imino)di methyl-λ⁶-sulfanone (76 mg), 3-trifluoromethyl- 4-(4-(4-methylpiperazin- 1- yl) piperidin- 1- yl) aniline (35 mg), Pd₂(dba)₃ (19 mg), Dave-phos (24 mg) and sodium tert-butoxide (29 mg) were mixed in toluene (5 mL), the system was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (35 mg).

¹H NMR (400 MHz, CDCl₃) δ 8.61 (s, 1H), 8.13 (d, *J* = 2.0 Hz, 1H), 7.74 (t, *J* = 8.0 Hz, 1H), 7.48 (dd, *J* = 8.8 Hz, 2.4 Hz, 1H), 7.28 (s, 1H), 7.20-7.24 (m, 2H), 6.81 (d, *J* = 8.0 Hz, 1H), 3.30 (s, 6H), 3.02-3.09 (m, 2H), 2.67-2.97 (m, 10H), 2.48-2.59 (m, 1H), 2.47 (s, 3H), 2.23-2.32 (m, 1H), 1.92-2.02 (m, 2H), 1.67-1.80 (m, 2H), 0.83-0.89 (m, 2H), 0.71-0.76 (m, 2H).

### Example 50

### ((6-(2-((3-trifluoromethyl-4-(4-(4-methylpiperazin-1-yl)piperidin-1-yl)phenyl)amino)-6-cycl opropyl-7H-pyrrolo[2,3-d]pyrimidin-7-yl)pyridin-2-yl)imino)dimethyl-λ⁶-sulfanone

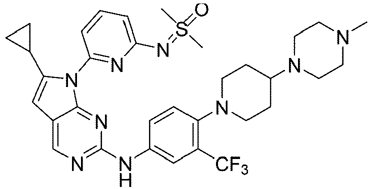

((6-(2-Chloro-6-cyclopropyl-7*H*-pyrrolo[2,3-d]pyrimidin-7-yl)pyridin-2-yl)imino)dimethyl-λ ⁶-sulfanone (72 mg), 3-trifluoromethyl-4-(4-(4-methylpiperazin- 1-yl) piperidin-1- yl)aniline (35 mg), Pd₂(dba)₃ (19 mg), Dave-phos (24 mg) and sodium tert-butoxide (29 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (30 mg).

¹H NMR (400 MHz, CDCl₃) δ 8.55 (s, 1H), 8.13 (d, *J* = 2.4 Hz, 1H), 7.75 (t, *J* = 8.0 Hz, 1H), 7.69 (s, 1H), 7.51 (dd, *J* = 8.4 Hz, 2.4 Hz, 1H), 7.19-7.22 (m, 2H), 6.83 (d, *J* = 8.4 Hz, 1H), 6.09 (s, 1H), 3.30 (s, 6H), 3.01-3.07 (m, 2H), 2.75-2.98 (m, 8H), 2.70 (t, *J* = 10.8 Hz, 2H), 2.49-2.62 (m, 1H), 2.49 (s, 3H), 2.35-2.43 (m, 1H), 1.91-1.99 (m, 2H), 1.65-1.78 (m, 2H), 0.86-0.93 (m, 2H), 0.64-0.69 (m, 2H).

### Example 51

### ((6-(5-fluoro-2-((3-methyl-4-(4-(4-methylpiperazin-1-yl)piperidin-1-yl)phenyl)amino)-6-cycl opropyl-7H-pyrrolo[2,3-d]pyrimidin-7-yl)pyridin-2-yl)imino)dimethyl-λ⁶-sulfanone

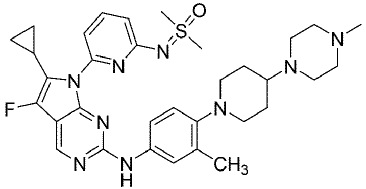

### Step A: 1-(1-(2-methyl-4-nitrophenyl)piperidin-4-yl)-4-methylpiperazine

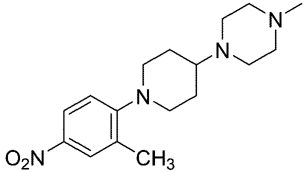

Under nitrogen gas, a solution of 1-methyl-4-(piperidin-4-yl)piperazine hydrochloride (1.6 g), 1-fluoro-2-methyl-4-nitrobenzene (1.0 g) and potassium carbonate (3.6 g) in dimethyl sulfoxide (20 mL) was heated to 100 °C and stirred overnight. The mixture was cooled to room temperature and partitioned between water and dichloromethane, and the solvent was removed from the organic phase to obtain the product, which was used directly in the next step.

### Step B: 3-methyl-4-(4-(4-methylpiperazin-1-yl)piperidin-1-yl)aniline

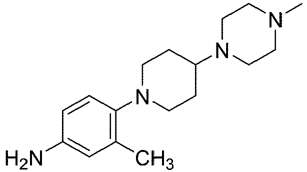

To a flask were sequentially added 1-(1-(2-methyl-4- nitrophenyl) piperidin- 4-yl) -4- methyl piperazine (200 mg), 10% Pd/C (60 mg) and methanol (10 mL), the system was sufficiently purged with hydrogen gas, and the mixture was stirred at room temperature overnight. The reaction solution was filtered through celite, and the filtrate was concentrated and purified through silica gel column chromatography (10%MeOH/DCM) to obtain the product (80 mg).

¹H NMR (400 MHz, CDCl₃) δ 6.84 (d, *J* = 8.4 Hz, 1H), 6.55 (d, *J* = 2.8 Hz, 1H), 6.49 (dd, *J* = 8.0 Hz, 2.8 Hz, 1H), 3.38-3.52 (brs, 2H), 3.03-3.09 (m, 2H), 2.54-2.87 (m, 10H), 2.34-2.48 (m, 1H), 2.38 (s, 3H), 2.21 (s, 3H), 1.91-1.99 (m, 2H), 1.64-1.77 (m, 2H).

### Step C: ((6-(5-fluoro-2-((3-methyl-4-(4-(4-methylpiperazin-1-yl) piperidin- 1-yl) phenyl) amino)-6-cyclopropyl-7H-pyrrolo[2,3-d]pyrimidin-7-yl)pyridin-2-yl)imino)dimethyl-λ⁶-sulfanone

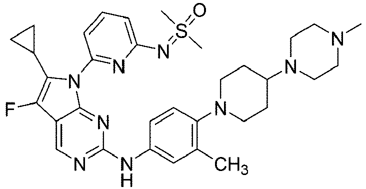

((6-(2-Chloro-5-fluoro-6-cyclopropyl-7*H*-pyrrolo[2,3-d]pyrimidin-7-yl)pyridin-2-yl)imino)di methyl-λ⁶-sulfanone (76 mg), 3-methyl-4-(4-(4-methylpiperazin-1-yl)piperidin-1-yl)aniline (29 mg), Pd₂(dba)₃ (19 mg), Dave-phos (24 mg) and sodium tert-butoxide (29 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (25 mg).

¹H NMR (400 MHz, CDCl₃) δ 8.58 (s, 1H), 7.74 (t, *J* = 8.0 Hz, 1H), 7.56 (d, *J* = 2.4 Hz, 1H), 7.24-7.29 (m, 2H), 7.08-7.15 (brs, 1H), 6.91 (d, *J* = 8.4 Hz, 1H), 6.81 (d, *J* = 8.0 Hz, 1H), 3.30 (s, 6H), 3.09-3.17 (m, 2 H), 2.67-2.99 (m, 9H), 2.63 (t, *J* = 11.2 Hz, 2H), 2.47 (s, 3H), 2.22-2.29 (m, 4H), 1.92-2.03 (m, 2H), 1.66-1.81 (m, 2H), 0.83-0.89 (m, 2H), 0.72-0.77 (m, 2H).

### Example 52

### ((6-(2-((3-methyl-4-(4-(4-methylpiperazin-1-yl)piperidin-1-yl)phenyl)amino)-6-cyclopropyl-7H-pyrrolo[2,3-d]pyrimidin-7-yl)pyridin-2-yl)imino)dimethyl-λ⁶-sulfanone

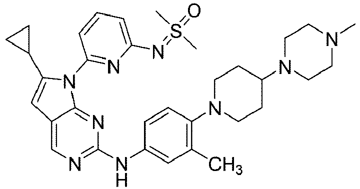

((6-(2-Chloro-6-cyclopropyl-7*H*-pyrrolo[2,3-d]pyrimidin-7-yl)pyridin-2-yl)imino)dimethyl-λ ⁶-sulfanone (72 mg), 3-methyl-4-(4-(4-methylpiperazin-1-yl) piperidin-1- yl)aniline (29 mg), Pd₂(dba)₃ (19 mg), Dave-phos (24 mg) and sodium tert-butoxide (29 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (25 mg).

¹H NMR (400 MHz, CDCl₃) δ 8.51 (s, 1H), 7.75 (t, *J =* 8.0 Hz, 1H), 7.57 (d, *J* = 2.4 Hz, 1H), 7.26-7.29 (m, 1H), 7.24 (d, *J* = 8.0 Hz, 1H), 7.10 (s, 1H), 6.89 (d, *J* = 8.4 Hz, 1H), 6.83 (d, *J* = 8.0 Hz, 1H), 6.06 (s, 1H), 3.30 (s, 6H), 2.90-3.16 (m, 10H), 2.54-2.66 (m, 6H), 2.34-2.42 (m, 1H), 2.23 (s, 3H), 1.98-2.08 (m, 2H), 1.70-1.84 (m, 2H), 0.87-0.93 (m, 2H), 0.64-0.69 (m, 2H).

### Example 53

### ((6-(5-fluoro-2-((3-chloro-4-(4-(4-methylpiperazin-1-yl)piperidin-1-yl)phenyl)amino)-6-cycl opropyl-7H-pyrrolo[2,3-d]pyrimidin-7-yl)pyridin-2-yl)imino)dimethyl-λ⁶-sulfanone

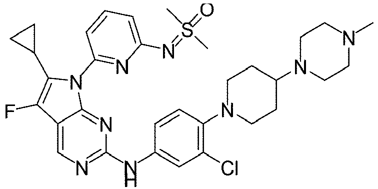

### Step A: 1-(1-(2-chloro-4-nitrophenyl)piperidin-4-yl)-4-methylpiperazine

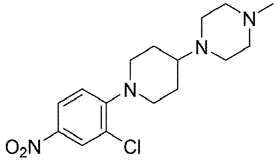

Under nitrogen gas, a solution of 1-methyl-4-(piperidin-4-yl)piperazine hydrochloride (1.4 g), 2-chloro-1-fluoro-4-nitrobenzene (1.0 g) and potassium carbonate (3.1 g) in dimethyl sulfoxide (20 mL) was heated to 100 °C and stirred overnight. The mixture was cooled to room temperature and partitioned between water and dichloromethane, the solvent was removed from the organic phase, and the residue was purified through silica gel column chromatography (1%MeOH/DCM) to obtain the product (1.6 g).

¹H NMR (400 MHz, CDCl₃) δ 8.23 (d, *J* = 2.4 Hz, 1H), 8.07 (dd, *J* = 9.2 Hz, 2.4 Hz, 1H), 7.02 (d, *J* = 8.8 Hz, 1H), 3.61-3.69 (m, 2H), 2.75-2.84 (m, 2H), 2.58-2.74 (m, 4H), 2.36-2.57 (m, 5H), 2.30 (s, 3H), 1.94-2.02 (m, 2H), 1.70-1.82 (m, 2H).

### Step B: 3-chloro-4-(4-(4-methylpiperazin-1-yl)piperidin-1-yl)aniline

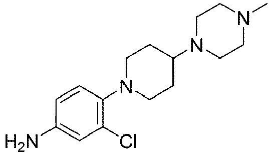

To a flask were sequentially added 1-(1-(2-chloro-4-nitrophenyl) piperidin-4-yl) -4- methyl piperazine (340 mg), zinc dust (325 mg), glacial acetic acid (2 mL) and ethanol (10 mL), and the mixture was stirred at room temperature for 1 hour. An aqueous solution of 1 N sodium hydroxide was used to adjust the pH to 12, the mixture was partitioned between water and dichloromethane, and the solvent was removed from the organic phase to obtain the product, which was used directly in the next step.

### Step C: ((6-(5-fluoro-2- ((3- chloro- 4- (4- (4- methylpiperazin-1-yl) piperidin- 1- yl) phenyl) amino)-6-cyclopropyl-7H-pyrrolo[2,3-d]pyrimidin-7-yl)pyridin-2-yl)imino)dimethyl-λ⁶-sulfanone

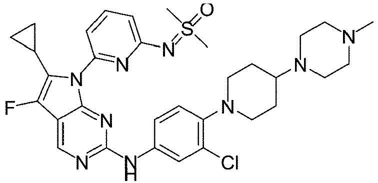

### ((6-(2-Chloro-5-fluoro-6-cyclopropyl-7H-pyrrolo[2,3-d]pyrimidin-7-yl)pyridin-2-yl)imino)di methyl-λ⁶-sulfanone (76 mg), 3-chloro-4-(4-(4-methylpiperazin-1-yl) piperidin-1- yl) aniline (31 mg), Pd₂(dba)₃ (19 mg), Dave-phos (24 mg) and sodium tert-butoxide (29 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (30 mg).

¹H NMR (400 MHz, CDCl₃) δ 8.58 (s, 1H), 8.10 (d, *J* = 2.4 Hz, 1H), 7.80 (t, *J* = 8.0 Hz, 1H), 7.28 (d, *J* = 8.0 Hz, 1H), 7.14 (dd, *J* = 8.8 Hz, 2.4 Hz, 1H), 6.93 (d, *J* = 8.8 Hz, 1H), 6.81 (d, *J* = 8.0 Hz, 1H), 3.34-3.41 (m, 2H), 3.32 (s, 6H), 2.65-2.96 (m, 9H), 2.62 (t, *J* = 11.2 Hz, 2H), 2.46 (s, 3H), 2.25-2.34 (m, 1H), 1.92-2.04 (m, 2H), 1.72-1.87 (m, 2H), 0.83-0.91 (m, 2H), 0.71-0.77 (m, 2H).

### Example 54

### ((6-(2-((3-chloro-4-(4-(4-methylpiperazin-1-yl)piperidin-1-yl)phenyl)amino)-6-cyclopropyl-7 H-pyrrolo[2,3-d]pyrimidin-7-yl)pyridin-2-yl)imino)dimethyl-λ⁶-sulfanone

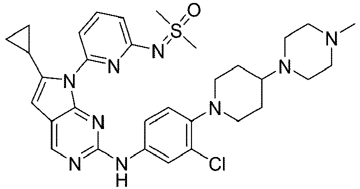

### ((6-(2-Chloro-6-cyclopropyl-7H-pyrrolo[2,3-d]pyrimidin-7-yl)pyridin-2-yl)imino)dimethyl-λ ⁶-sulfanone (72 mg), 3-chloro-4-(4-(4-methylpiperazin-1-yl) piperidin-1-yl) aniline (31 mg), Pd₂(dba)₃ (19 mg), Dave-phos (24 mg) and sodium tert-butoxide (29 mg) were mixed in toluene (5 mL), the system was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (30 mg).

¹H NMR (400 MHz, CDCl₃) δ 8.50 (s, 1H), 8.10 (d, *J* = 2.4 Hz, 1H), 7.82 (t, *J =* 8.0 Hz, 1H), 7.44 (s, 1H), 7.27 (d, *J =* 7.6 Hz, 1H), 7.16 (dd, *J =* 8.8 Hz, 2.8 Hz, 1H), 6.92 (d, *J* = 8.8 Hz, 1H), 6.83 (d, *J* = 8.0 Hz, 1H), 6.08 (s, 1H), 3.35-3.41 (m, 2 H), 3.32 (s, 6H), 2.65-2.91 (m, 8H), 2.61 (t, *J* = 10.8 Hz, 2H), 2.44-2.56 (m, 1H), 2.44 (s, 3H), 2.37-2.45 (m, 1H), 1.93-2.01 (m, 2H), 1.72-1.86 (m, 2H), 0.87-0.96 (m, 2H), 0.64-0.69 (m, 2H).

### Example 55

### 2-(6-(2-((4-(4-methylpiperazin-1-yl)phenyl)amino)-6-cyclopropyl-7H-pyrrolo [2,3-d]pyrimidi n-7-yl)pyridin-2-yl)propan-2-ol

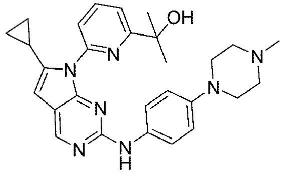

### Step A: 2-(6-((5-iodo-2-chloropyrimidin-4-yl)amino)pyridin-2-yl)propan-2-ol

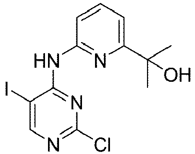

Under nitrogen gas, a mixture of 2-(6-aminopyridin-2-yl)propan-2-ol (2.9 g), 5- iodo- 2,4-dichloro pyrimidine (5.25 g), ethyldiisopropylamine (2.94 g) and isopropanol (30 mL) was heated to reflux and stirred overnight. The mixture was cooled to room temperature, and the solid precipitated was collected by filtration, washed with small amount of ethyl acetate, and dried to obtain the product (2.0 g).

¹H NMR (400 MHz, CDCl₃) δ 8.53 (s, 1H), 8.27 (d, *J =* 8.0 Hz, 1H), 7.91 (s, 1H), 7.82 (t, *J =* 8.0 Hz, 1H), 7.17 (d, *J* = 8.0 Hz, 1H), 4.51 (s, 1H), 1.56 (s, 6H).

### Step B: 2-(6-(2-chloro-6-cyclopropyl-7H- pyrrolo [2,3-d] pyrimidin- 7-yl) pyridin- 2- yl) propan-2-ol

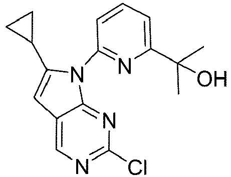

According to the method of step D of example 1, 2-(6-((5- iodo- 2- chloropyrimidin- 4- yl) amino) pyridin-2-yl)propan-2-ol and cyclopropylacetylene were used as the starting material to obtain the product (102 mg).

¹H NMR (400 MHz, CDCl₃) δ 8.73 (s, 1H), 8.00 (t, *J* = 8.0 Hz, 1H), 7.67 (d, *J* = 8.0 Hz, 1H), 7.49 (d, *J* = 8.0 Hz, 1H), 6.29 (s, 1H), 4.31 (s, 1H), 2.05-2.14 (m, 1H), 1.58 (s, 6H), 0.94-0.97 (m, 2H), 0.80-0.83 (m, 2H).

### Step C: 2-(6-(2-((4- (4- methylpiperazin- 1- yl) phenyl) amino)- 6- cyclopropyl- 7H- pyrrolo [2,3-d]pyrimidin-7-yl)pyridin-2-yl)propan-2-ol

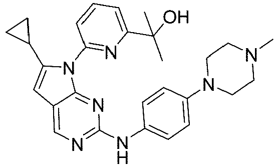

According to the method of step F of example 1, 2-(6-(2- chloro- 6- cyclopropyl- 7*H*-pyrrolo [2,3-d]pyrimidin-7-yl)pyridin-2-yl)propan-2-ol and 4-(4-methylpiperazin-1-yl)aniline were used as the starting material to obtain the product (30 mg).

¹H NMR (400 MHz, CDCl₃) δ 8.55 (s, 1H), 7.96(t, *J =* 8.0 Hz, 1H), 7.79 (d, *J =* 8.0 Hz, 1H), 7.53 (d, *J* = 8.8 Hz, 2H), 7.43 (d, *J* = 8.0 Hz, 1H), 6.87 (d, *J* = 8.8 Hz, 2H), 6.16 (s, 1H), 4.60 (brs, 1H), 3.20-3.31 (m, 4H), 2.79-2.91 (m, 4H), 2.54 (s, 3H), 2.15-2.17 (m, 1H), 1.60 (s, 6H), 0.86-0.91 (m, 2H), 0.71-0.75 (m, 2H).

### Example 56

### 2-(6-(2-((4-(4-(4-methylpiperazin-1-yl)piperidin-1-yl)phenyl)amino)-6-cyclopropyl-7H-pyrro lo[2,3-d]pyrimidin-7-yl)pyridin-2-yl)propan-2-ol

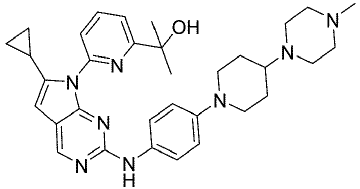

According to the method of step F of example 1, 2-(6-(2-chloro-6-cyclopropyl- 7*H*- pyrrolo [2,3-d]pyrimidin-7-yl)pyridin-2-yl)propan-2-ol and 1-methyl- 4- (1- (4- aminophenyl) piperidin-4- yl)piperazine were used as the starting material to obtain the product (15 mg).

¹H NMR (400 MHz, CDCl₃) δ 8.54 (s, 1H), 7.95 (t, *J* = 8.0 Hz, 1H), 7.79 (d, *J* = 8.0 Hz, 1H), 7.51 (d, *J* = 8.8 Hz, 2H), 7.43 (d, *J* = 8.0 Hz, 1H), 7.04(brs, 1H), 6.87 (d, *J* = 8.8 Hz, 2H), 6.16 (s, 1H), 4.60 (brs, 1H), 3.62-3.65 (m, 2H), 2.79-3.00 (m, 8H), 2.65-2.71 (m, 3H), 2.55 (s, 3H), 2.16-2.18 (m, 1H), 1.95-2.01 (m, 2H), 1.70-1.80 (m, 2H), 1.60 (s, 6H), 0.86-0.90 (m, 2H), 0.72-0.75 (m, 2H).

### Example 57

### 2-(6-(2-((4-(4-methylpiperazin-1-yl)phenyl)amino)-5-fluoro-6-cyclopropyl-7H-pyrrolo[2,3-d] pyrimidin-7-yl)pyridin-2-yl)propan-2-ol

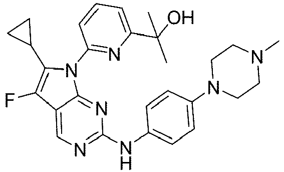

### Step A: 2-(6-(2-chloro-5-fluoro-6-cyclopropyl- 7H- pyrrolo [2,3-d] pyrimidin- 7- yl) pyridin-2- yl)propan-2-ol

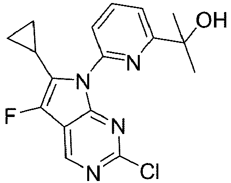

According to the method of step E of example 1, 2-(6-(2- chloro- 6- cyclopropyl- 7H- pyrrolo [2,3-d]pyrimidin-7-yl)pyridin-2-yl)propan-2-ol and Selectfluor^{®} were used as the starting material to obtain the product (112 mg).

¹H NMR (400 MHz, CDCl₃) δ 8.82 (s, 1H), 7.98 (t, *J* = 8.0 Hz, 1H), 7.64 (dd, *J* = 8.0 Hz, 0.8Hz, 1H), 7.51 (dd, *J* = 8.0 Hz, 0.8Hz, 1H), 4.10-4.15 (m, 1H), 2.05 (s, 1H), 1.59 (s, 6H), 0.84-0.91 (m, 4H).

### Step B: 2-(6-(2- ((4- (4-methylpiperazin-1-yl) phenyl) amino)-5- fluoro- 6- cyclopropyl- 7H-pyrrolo [2,3-d]pyrimidin-7-yl)pyridin-2-yl)propan-2-ol

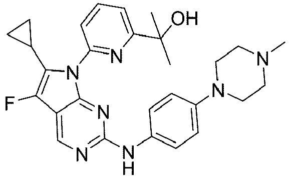

According to the method of step F of example 1, 2-(6- (2-chloro- 5-fluoro- 6-cyclopropyl-*7H*- pyrrolo[2,3-d]pyrimidin-7-yl)pyridin-2-yl)propan-2-ol and 4-(4-methylpiperazin-1-yl) aniline were used as the starting material to obtain the product (25 mg).

¹H NMR (400 MHz, CDCl₃) δ 8.61 (s, 1H), 7.94 (t, *J =* 8.0 Hz, 1H), 7.76 (d, *J =* 8.0 Hz, 1H), 7.53 (d, *J* = 9.2 Hz, 2H), 7.43 (d, *J* = 8.0 Hz, 1H), 7.09 (s, 1H), 6.88 (d, *J* = 8.8 Hz, 2H), 4.180-4.25 (brs, 1H), 3.33-3.40 (m, 4H), 2.90-3.10 (m, 4H), 2.65 (s, 3H), 2.05-2.07 (m, 1H), 1.60 (s, 6H), 0.82-0.85 (m, 2H), 0.75-0.77 (m, 2H).

### Example 58

### 2-(6-(2-((4-(4-(4-methylpiperazin-1-yl)piperidin-1-yl)phenyl)amino)-5-fluoro-6-cyclopropyl-7H-pyrrolo[2,3-d]pyrimidin-7-yl)pyridin-2-yl)propan-2-ol

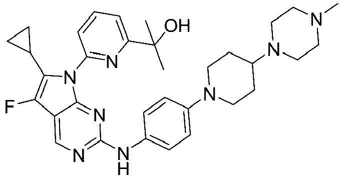

According to the method of step F of example 1, 2-(6-(2-chloro-5-fluoro-6- cyclopropyl- 7H-pyrrolo[2,3-d] pyrimidin-7-yl) pyridin-2-yl) propan-2-ol and 1-methyl-4- (1- (4- aminophenyl) piperidin- 4- yl)piperazine were used as the starting material to obtain the product (50 mg).

¹H NMR (400 MHz, CDCl₃) δ 8.60 (s, 1H), 7.93 (t, J = 8.0 Hz, 1H), 7.78 (d, J = 8.0 Hz, 1H), 7.49 (d, *J* = 8.8 Hz, 2H), 7.42 (d, *J* = 8.0 Hz, 1H), 7.10 (brs, 1H), 6.88 (d, *J* = 8.8 Hz, 2H), 6.16 (s, 1H), 4.60 (brs, 1H), 3.63-3.66 (m, 2H), 2.79-2.95 (m, 8H), 2.65-2.71 (m, 2H), 2.51 (s, 3H), 2.06-2.10 (m, 1H), 1.95-2.01 (m, 2H), 1.72-1.76 (m, 2H), 1.60 (s, 6H), 0.82-0.84 (m, 2H), 0.74-0.75 (m, 2H).

### Example 59

### 2-(6-(2-((4-(4-(4-methylpiperazin-1-yl)piperidin-1-yl)phenyl)amino)-5-fluoro-6-cyclopropyl-7H-pyrrolo[2,3-d]pyrimidin-7-yl)pyridin-2-yl)propan-2-ol

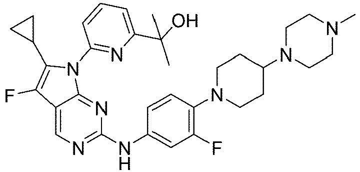

According to the method of step F of example 1, 2-(6-(2-chloro-5- fluoro-6- cyclopropyl-7*H*-pyrrolo[2,3-d] pyrimidin-7-yl) pyridin-2-yl) propan-2-ol and 1-methyl- 4- (1- (3- fluoro- 4-aminophenyl) piperidin-4-yl) piperazine were used as the starting material to obtain the product (20 mg).

¹H NMR (400 MHz, CDCl₃) δ 8.63 (s, 1H), 8.00 (t, *J* = 8.0 Hz, 1H), 7.78-7.85 (m, 2H), 7.45 (d, *J* = 8.0 Hz, 1H), 6.96-6.99 (m, 1H), 7.31 (s, 1H), 6.83-6.88 (m, 1H), 4.40 (brs, 1H), 3.43-3.49 (m, 2H), 2.79-3.20 (m, 8H), 2.60-2.79 (m, 6H), 1.75-2.05 (m, 5H), 1.60 (s, 6H), 0.83-0.88 (m, 2H), 0.77-0.79 (m, 2H).

### Example 60

### ((6-(2-((4-((1S,5R)-8-azabicyclo[3.2.1]oct-2-ene-3-yl)phenyl)amino)-6-cyclopropyl-7H-pyrr olo[2,3-d]pyrimidin-7-yl)pyridin-2-yl)imino)dimethyl-λ⁶-sulfanone

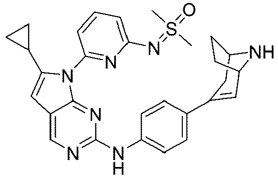

### Step A: 4-(4,4,5,5-tetramethyl-1,3,2-dioxaborolan-2-yl)aniline

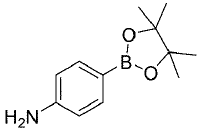

Under nitrogen gas, a suspension of 4-bromo aniline (5.00 g), bis(pinacolato)diboron (14.80 g), tetrakis(triphenylphosphine)palladium (3.30 g) and potassium acetate (5.80 g) in 1,4-dioxane (100 mL) was heated to 100 °C overnight. The mixture was cooled to room temperature, filtered, and washed with dichloromethane, the solvent was removed from the filtrate, and the residue was purified through silica gel column chromatography (1%MeOH/DCM) to obtain the product (4.0 g).

¹H NMR (400 MHz, CDCl₃) δ 7.62 (d, *J* = 7.6 Hz, 2H), 6.66 (d, *J* = 7.6 Hz, 2H), 3.83 (s, 2H), 1.32 (s, 12H).

### Step B: tert-butyl (1S,5R)- 3- ( ( (trifluoromethyl) sulfonyl) oxy) -8- azabicyclo [3.2.1] oct-2- ene- 8-carboxylate

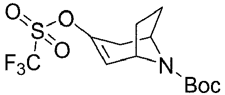

Under nitrogen gas, a suspension of Potassium bis(trimethylsilyl)amide (4.90 g) in tetrahydrofuran (50 mL) was cooled to -60 °C, to the mixture was added tert-butyl (1R,5S)-3-oxo-8-azabicyclo [3.2.1]octan-8-carboxylate (5.00 g), after the mixture was reacted for 2 hours, 1,1,1-trifluoro-*N*-phenyl-*N*-((trifluoromethyl)sulfonyl)methanesulfonamide (4.90 g) was added, and the mixture was reacted for 5 hours, let warm up to room temperature and stirred for 2 hours. Water was added to quench the reaction, the mixture was extracted with dichloromethane, the solvent was removed from the organic phase, and the residue was purified through silica gel column chromatography (2%MeOH/DCM) to obtain the product (7.5 g).

¹H NMR (400 MHz, CDCl₃) δ 6.07-6.12 (m, 1H), 4.31-4.61 (m, 2H), 2.93-3.17 (m, 1H), 2.18-2.32 (m, 1H), 1.95-2.16 (m, 3H), 1.66-1.81 (m, 1H), 1.46 (s, 9H).

### Step C: tert-butyl (1S,5R)-3-(4-aminophenyl)-8-azabicyclo[3.2.1]oct-2-ene-8-carboxylate

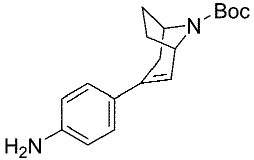

Under nitrogen gas, a mixture of 4-(4,4,5,5-tetramethyl- 1,3,2- dioxaborolan- 2-yl) aniline (4.00 g), tert-butyl (1S,5R)- 3- (((trifluoromethyl) sulfonyl) oxy) -8- azabicyclo [3.2.1] oct- 2- ene-8- carboxylate (5.40 g), Pd(dppf)Cl₂ (1.10 g) and caesium carbonate (9.80 g) in dimethyl sulfoxide (60 mL) and water (15 mL) was heated to 100 °C and stirred overnight. The mixture was cooled to room temperature and filtered, the filtrate was extracted with dichloromethane, the solvent was removed from the organic phase, and the residue was purified through silica gel column chromatography (5%MeOH/DCM) to obtain the product (3.5 g).

¹H NMR (400 MHz, CDCl₃) δ 7.18 (d, *J* = 8.8 Hz, 2H), 6.63 (d, *J* = 8.8 Hz, 2H), 6.23-6.33 (m, 1H), 4.32-4.58 (m, 2H), 3.70 (s, 2H), 2.91-3.19 (m, 1H), 2.11-2.26 (m, 2H), 1.88-2.02 (m, 2H), 1.60-1.74 (m, 1H), 1.44 (s, 9H).

### Step D: tert-butyl (1S,5R)- 3- (4- ((6- cyclopropyl- 7- (6- ((dimethyl(oxo)- λ⁶- sulfanylidene) amino) pyridin-2- yl) -7H- pyrrolo[2,3-d] pyrimidin-2-yl) amino) phenyl)- 8- azabicyclo [3.2.1] oct- 2-ene-8-carboxylate

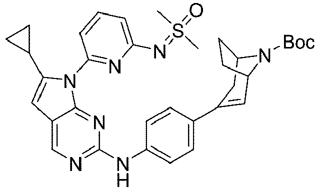

((6-(2-Chloro-6-cyclopropyl-7*H*-pyrrolo[2,3-d]pyrimidin-7-yl)pyridin-2-yl)imino)dimethyl-λ ⁶-sulfanone (73 mg), tert-butyl (1S,5R)-3- (4-aminophenyl)- 8-azabicyclo [3.2.1]oct- 2-ene-8-carboxylate (30 mg), Pd₂(dba)₃ (19 mg), Dave-phos (24 mg) and sodium tert-butoxide (29 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (5%MeOH/DCM) to obtain the target product (40 mg).

¹H NMR (400 MHz, CDCl₃) δ 8.54 (s, 1H), 7.76 (t, *J =* 8.0 Hz, 1H), 7.57 (d, *J =* 8.4 Hz, 2H), 7.21-7.32 (m, 4H), 6.85 (d, *J* = 8.0 Hz, 1H), 6.31-6.41 (m, 1H), 6.08 (s, 1H), 4.36-4.58 (m, 2H), 3.31 (s, 6H), 2.92-3.21 (m, 1H), 2.32-2.42 (m, 1H), 2.13-2.28 (m, 2H), 1.90-2.03 (m, 2H), 1.62-1.77 (m, 1H), 1.45 (s, 9H), 0.86-0.97 (m, 2H), 0.65-0.72 (m, 2H).

### Step E: ((6- (2- ((4- ((1S,5R) -8- azabicyclo [3.2.1] oct- 2- ene- 3- yl) phenyl) amino) -6-cyclopropyl- 7H-pyrrolo[2,3-d]pyrimidin-7-yl)pyridin-2-yl)imino)dimethyl-λ⁶-sulfanone

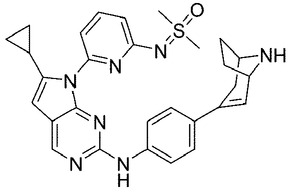

Acetyl chloride (2 mL) was dropwise added to methanol (4 mL) slowly, to the mixture was added tert-butyl (1S,5R)-3-(4- ((6- cyclopropyl- 7- (6- ((dimethyl(oxo)- λ⁶- sulfanylidene) amino) pyridin- 2- yl) -7H- pyrrolo [2,3-d] pyrimidin- 2- yl) amino) phenyl) -8- azabicyclo [3.2.1] oct- 2-ene- 8-carboxylate (40 mg), and the mixture was stirred at room temperature for 1 hour. The solvent was evaporated, and the residue was purified through preparative TLC (10%MeOH/DCM) to obtain the target product (30 mg).

¹H NMR (400 MHz, CD₃OD) δ 8.71 (s, 1H), 8.24 (t, *J* = 7.6 Hz, 1H), 7.42-7.55 (m, 6H), 6.55 (s, 1H), 6.44 (d, *J* = 5.6 Hz, 1H), 4.31-4.42 (m, 2H), 3.59 (s, 6H), 3.14-3.23 (m, 2H), 2.68 (d, *J* = 18.0 Hz, 1H), 2.17-2.45 (m, 3H), 1.93-2.08 (m, 2H), 1.01-1.07 (m, 2H), 0.87-0.93 (m, 2H).

### Example 61

### ((6-(2-((4-((1S,5R)-8-azabicyclo[3.2.1]oct-2-ene-3-yl)phenyl)amino)-6-cyclopropyl-5-fluoro -7H-pyrrolo[2,3-d]pyrimidin-7-yl)pyridin-2-yl)imino)dimethyl-λ⁶-sulfanone

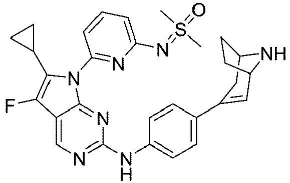

### Step A: tert-butyl (1S,5R)-3-(4- ((6-cyclopropyl- 7- (6- ((dimethyl(oxo)- λ⁶- sulfanylidene) amino) pyridin- 2- yl) -5- fluoro- 7H- pyrrolo[2,3-d] pyrimidin- 2- yl) amino) phenyl)- 8-azabicyclo [3.2.1]oct-2-ene-8-carboxylate

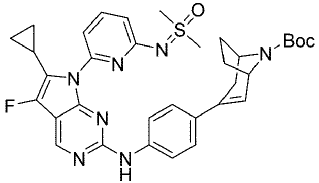

((6-(2-Chloro-5-fluoro-6-cyclopropyl-7*H*-pyrrolo[2,3-d]pyrimidin-7-yl)pyridin-2-yl)imino)di methyl-λ⁶-sulfanone (76 mg), tert-butyl (1S,5R)- 3- (4-aminophenyl)- 8- azabicyclo [3.2.1]oct- 2-ene- 8-carboxylate (30 mg), Pd₂(dba)₃ (19 mg), Dave-phos (24 mg) and sodium tert-butoxide (29 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (5%MeOH/DCM) to obtain the target product (40 mg).

¹H NMR (400 MHz, CDCl₃) δ 8.60 (s, 1H), 7.75 (t, *J =* 8.0 Hz, 1H), 7.57 (d, *J =* 8.4 Hz, 2H), 7.25-7.27 (m, 3H), 7.23 (d, *J* = 8.0 Hz, 1H), 6.82 (d, *J* = 8.0 Hz, 1H), 6.31-6.43 (m, 1H), 4.36-4.61 (m, 2H), 3.31 (s, 6H), 2.91-3.21 (m, 1H), 2.11-2.31 (m, 3H), 1.89-2.06 (m, 2H), 1.62-1.76 (m, 1H), 1.45 (s, 9H), 0.83-0.92 (m, 2H), 0.72-0.79 (m, 2H).

### Step B: ((6- (2- ( (4- ( (1S,5R) - 8- azabicyclo [3.2.1] oct- 2- ene- 3- yl) phenyl) amino) -6-cyclopropyl- 5-fluoro-7H-pyrrolo[2,3-d]pyrimidin-7-yl)pyridin-2-yl)imino)dimethyl-λ⁶-sulfanone

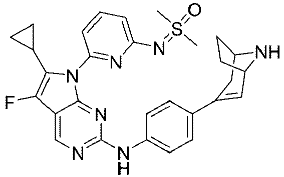

Acetyl chloride (2 mL) was dropwise added to methanol (4 mL) slowly, to the mixture was added tert-butyl (1S,5R)-3-(4- ((6-cyclopropyl- 7-(6- ((dimethyl(oxo)- λ⁶- sulfanylidene) amino) pyridin- 2- yl)- 5- fluoro- 7*H*- pyrrolo [2,3-d] pyrimidin- 2- yl) amino) phenyl)- 8- azabicyclo [3.2.1] oct- 2-ene-8-carboxylate (40 mg), and the mixture was stirred at room temperature for 1 hour. The solvent was evaporated, and the residue was purified through preparative TLC (10%MeOH/DCM) to obtain the target product (25 mg).

¹H NMR (400 MHz, CD₃OD) δ 9.68 (s, 1H), 8.86 (s, 1H), 8.15 (t, *J* = 8.0 Hz, 1H), 7.47-7.55 (m, 4H), 7.34-7.41 (m, 2H), 6.44 (d, *J* = 4.0 Hz, 1H), 4.31-4.43 (m, 2H), 3.55 (s, 6H), 3.13-3.23 (m, 2H), 2.68 (d, *J* = 17.6 Hz, 1H), 2.17-2.45 (m, 3H), 1.93-2.07 (m, 2H), 0.95-1.03 (m, 2H), 0.85-0.93 (m, 2H).

### Example 62

### ((6-(2-((4-((1R,5S)-8-azabicyclo[3.2.1]octan-3-yl)phenyl)amino)-6-cyclopropyl-7H-pyrrolo[ 2,3-d]pyrimidin-7-yl)pyridin-2-yl)imino)dimethyl-λ⁶-sulfanone

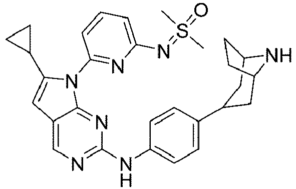

### Step A: tert-butyl (1R,5S)-3-(4-aminophenyl)-8-azabicyclo[3.2.1]octan-8-carboxylate

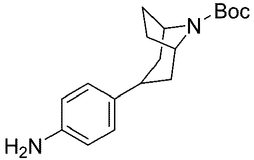

To a flask were sequentially added tert-butyl (1S,5R)- 3- (4- aminophenyl)- 8- azabicyclo [3.2.1]oct- 2-ene-8-carboxylate (300 mg), 10% Pd/C (50 mg) and methanol (10 mL), and after being sufficiently purged with hydrogen gas, the mixture was stirred at room temperature for 2 hours. The reaction solution was filtered through celite, and the filtrate was concentrated and purified through silica gel column chromatography (10%MeOH/DCM) to obtain the product (280 mg).

¹H NMR (400 MHz, CDCl₃) δ 6.96-7.02 (m, 2H), 6.63 (d, *J* = 8.4 Hz, 2H), 4.16-4.37 (m, 2H), 3.57 (s, 2H), 2.91-3.02 (m, 0.5H), 2.48-2.58 (m, 0.5H), 1.96-2.06 (m, 2H), 1.71-1.81 (m, 2H), 1.62-1.69 (m, 2H), 1.54-1.62 (m, 2H), 1.50 (s, 9H).

### Step B: tert-butyl (1R,5S)- 3- (4- ((6-cyclopropyl- 7-(6- ((dimethyl(oxo)-λ⁶- sulfanylidene) amino)pyridin- 2-yl)-7H-pyrrolo [2,3-d]pyrimidin-2- yl)amino) phenyl) -8-azabicyclo [3.2.1] octan- 8- carboxylate

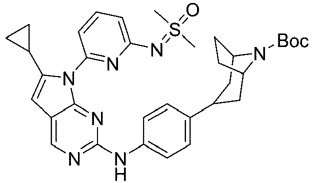

((6-(2-Chloro-6-cyclopropyl-7*H*-pyrrolo[2,3-d]pyrimidin-7-yl)pyridin-2-yl)imino)dimethyl-λ ⁶-sulfanone (50 mg), tert-butyl (1R,5S)-3- (4-aminophenyl) -8-azabicyclo [3.2.1]octan- 8-carboxylate (21 mg), Pd₂(dba)₃ (13 mg), Dave-phos (17 mg) and sodium tert-butoxide (20 mg) were mixed in toluene (5 mL) and purged sufficiently with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (5%MeOH/DCM) to obtain the target product (30 mg).

¹H NMR (400 MHz, CDCl₃) δ 8.52 (s, 1H), 7.72-7.77 (m, 1H), 7.52-7.56 (m, 2H), 7.36 (s, 1H), 7.21-7.23 (m, 1H), 7.05-7.12 (m, 2H), 6.81-6.85 (m, 1H), 6.07 (s, 1H), 4.18-4.39 (m, 2H), 3.30 (s, 6H), 2.97-3.08 (m, 0.5H), 2.56-2.66 (m, 0.5H), 2.32-2.53 (m, 2H), 1.58-2.06 (m, 7H), 1.51 (s, 9H), 0.86-0.94 (m, 2H), 0.65-0.71 (m, 2H).

### Step C: ((6-(2-((4-((1R,5S)-8-azabicyclo[3.2.1] octan- 3- yl) phenyl) amino)- 6- cyclopropyl-7H- pyrrolo [2,3-d]pyrimidin-7-yl)pyridin-2-yl)imino)dimethyl-λ⁶-sulfanone

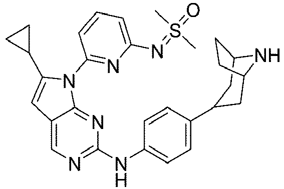

Acetyl chloride (2 mL) was dropwise added to methanol (4 mL) slowly, to the mixture was added tert-butyl (1R,5S)-3-(4- ((6- cyclopropyl- 7-(6- ((dimethyl(oxo)- λ⁶- sulfanylidene) amino) pyridin-2-yl)- 7H- pyrrolo [2,3-d] pyrimidin- 2- yl) amino) phenyl)- 8- azabicyclo [3.2.1] octan- 8-carboxylate (35 mg), and the mixture was stirred at room temperature for 1 hour. The solvent was evaporated, and the residue was purified through preparative TLC (10%MeOH/DCM) to obtain the target product (25 mg).

¹H NMR (400 MHz, CD₃OD) δ 8.44 (s, 1H), 7.71-7.76 (m, 1H), 7.48-7.54 (m, 2H), 7.18 (d, J = 8.8 Hz, 1H), 7.08-7.11 (m, 1H), 7.04 (d, J = 8.8 Hz, 1H), 6.72-6.75 (m, 1H), 6.07-6.09 (m, 1H), 3.92-4.03 (m, 2H), 3.24 (s, 6H), 3.20-3.23(m, 1H), 2.95-3.06 (m, 1H), 2.29-2.37 (m, 1H), 2.17-2.25 (m, 2H), 2.03-2.09 (m, 2H), 1.72-1.97 (m, 4H), 0.72-0.81 (m, 2H), 0.56-0.61 (m, 2H).

### Example 63

### ((6-(2-((4-((1R,5S)-8-azabicyclo[3.2.1]octan-3-yl)phenyl)amino)-6-cyclopropyl-5-fluoro-7H-pyrrolo[2,3-d]pyrimidin-7-yl)pyridin-2-yl)imino)dimethyl-λ⁶-sulfanone

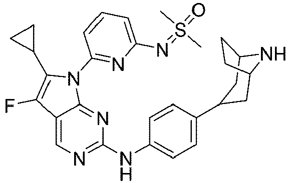

### Step A: tert-butyl (1R,5S)-3- (4- ( (6- cyclopropyl- 7- (6- ( (dimethyl (oxo)- λ⁶- sulfanylidene) amino) pyridin- 2- yl)- 5- fluoro- 7H- pyrrolo [2,3-d] pyrimidin- 2- yl) amino) phenyl)- 8-azabicyclo [3.2.1]octan-8-carboxylate

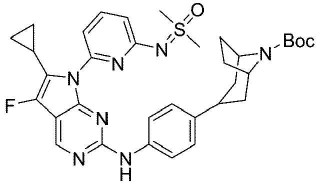

((6-(2-Chloro-5-fluoro-6-cyclopropyl-7*H*-pyrrolo[2,3-d]pyrimidin-7-yl)pyridin-2-yl)imino)di methyl-λ⁶-sulfanone (53 mg), tert-butyl (1R,5S)-3- (4-aminophenyl)- 8-azabicyclo [3.2.1]octan- 8-carboxylate (21 mg), Pd₂(dba)₃ (13 mg), Dave-phos (17 mg) and sodium tert-butoxide (20 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (5%MeOH/DCM) to obtain the target product (30 mg).

¹H NMR (400 MHz, CDCl₃) δ 8.59 (s, 1H), 7.71-7.76 (m, 1H), 7.52-7.56 (m, 2H), 7.22-7.25 (m, 1H), 7.17 (s, 1H), 7.07-7.13 (m, 2H), 6.79-6.83 (m, 1H), 4.19-4.39 (m, 2H), 3.30 (s, 6H), 2.97-3.09 (m, 0.5H), 2.56-2.67 (m, 0.5H), 2.34-2.54 (m, 1H), 2.20-2.29 (m, 1H), 1.88-2.08 (m, 3H), 1.58-1.72 (m, 4H), 1.51 (s, 9H), 0.83-0.91 (m, 2H), 0.74-0.78 (m, 2H).

### Step B: ( ( 6-(2- ( (4- ( (1R,5S) - 8- azabicyclo [3.2.1] octan- 3- yl) phenyl) amino)- 6-cyclopropyl- 5- fluoro-7H-pyrrolo[2,3-d]pyrimidin-7-yl)pyridin-2-yl)imino)dimethyl-λ⁶-sulfanone

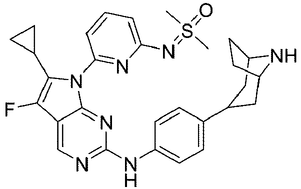

Acetyl chloride (2 mL) was dropwise added to methanol (4 mL) slowly, to the mixture was added tert-butyl (1R,5S)- 3- (4- ( ( 6- cyclopropyl- 7- (6- ((dimethyl (oxo)- λ⁶- sulfanylidene) amino) pyridin- 2- yl)- 5- fluoro- 7*H*- pyrrolo [2,3-d] pyrimidin- 2- yl) amino) phenyl)- 8-azabicyclo [3.2.1]octan-8-carboxylate (30 mg), and the mixture was stirred at room temperature for 1 hour. The solvent was evaporated, and the residue was purified through preparative TLC (10%MeOH/DCM) to obtain the target product (10 mg).

¹H NMR (400 MHz, CD₃OD) δ 8.59 (s, 1H), 7.78-7.83 (m, 1H), 7.60-7.65 (m, 2H), 7.29 (d, *J* = 8.4 Hz, 1H), 7.21-7.23 (m, 1H), 7.15 (d, *J* = 8.8 Hz, 1H), 6.79-6.82 (m, 1H), 4.01-4.13 (m, 2H), 3.33 (s, 6H), 3.06-3.18 (m, 1H), 2.39-2.49 (m, 1H), 2.27-2.36 (m, 1H), 2.11-2.25 (m, 3H), 1.81-2.08 (m, 5H), 0.79-0.85 (m, 2H), 0.67-0.74 (m, 2H).

### Example 64

### ((6-(6-cyclopropyl-2-((4-((1S,5R)-8-methyl-8-azabicyclo[3.2.1]oct-2-ene-3-yl)phenyl)amino) -7H-pyrrolo[2,3-d]pyrimidin-7-yl)pyridin-2-yl)imino)dimethyl-λ⁶-sulfanone

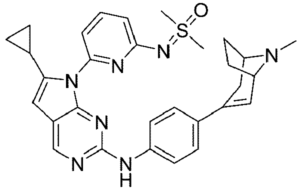

((6-(2-((4-((1S,5R)-8-azabicyclo[3.2.1]oct-2-ene-3-yl)phenyl)amino)-6-cyclopropyl-7*H*-pyrr olo[2,3-d]pyrimidin-7-yl)pyridin-2-yl)imino)dimethyl-λ⁶-sulfanone (30 mg), aqueous solution of 40% formaldehyde (0.1 mL) and glacial acetic acid (0.1 mL) were mixed in dichloromethane (5 mL), the mixture was stirred at room temperature for 20 minutes, then sodium triacetoxyborohydride (64 mg) was added, and the mixture was stirred for 3 hours. An aqueous solution of saturated sodium bicarbonate was used to adjust the pH to 7.0, the mixture was partitioned between water and dichloromethane, the solvent was removed from the organic phase, and the residue was purified through preparative TLC (10%MeOH/DCM) to obtain the product (6 mg).

¹H NMR (400 MHz, CDCl₃) δ 8.52 (s, 1H), 7.76 (t, *J* = 8.0 Hz, 1H), 7.62 (d, *J* = 8.8 Hz, 2H), 7.50 (s, 1H), 7.28 (d, *J* = 8.8 Hz, 2H), 7.23 (d, *J* = 7.6 Hz, 1H), 6.85 (d, *J* = 7.6 Hz, 1H), 6.17 (d, *J* = 5.6 Hz, 1H), 6.09 (s, 1H), 3.71-3.81 (m, 2H), 3.31 (s, 6H), 2.92-2.99 (m, 1H), 2.61 (s, 3H), 2.26-2.45 (m, 4H), 2.01-2.06 (m, 1H), 1.71-1.79 (m, 1H), 0.89-0.95 (m, 2H), 0.65-0.71 (m, 2H).

### Example 65

### ((6-(6-cyclopropyl-5-fluoro-2-((4-((lS,5R)-8-methyl-8-azabicyclo[3.2.1]oct-2-ene-3-yl)phen yl)amino)-7H-pyrrolo[2,3-d]pyrimidin-7-yl)pyridin-2-yl)imino)dimethyl-λ⁶-sulfanone

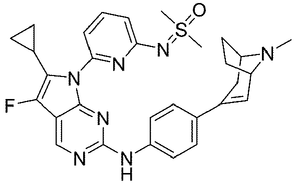

((6-(2-((4-((1S,5R)-8-azabicyclo[3.2.1]oct-2-ene-3-yl)phenyl)amino)-6-cyclopropyl-5-fluoro -7*H*-pyrrolo[2,3-d]pyrimidin-7-yl)pyridin-2-yl)imino)dimethyl-λ⁶-sulfanone (25 mg), aqueous solution of 40% formaldehyde (0.1 mL) and glacial acetic acid (0.1 mL) were mixed in dichloromethane (5 mL), the mixture was stirred at room temperature for 20 minutes, then sodium triacetoxyborohydride (49 mg) was added, and the mixture was stirred for 3 hours. An aqueous solution of saturated sodium bicarbonate was used to adjust the pH to 7.0, the mixture was partitioned between water and dichloromethane, the solvent was removed from the organic phase, and the residue was purified through preparative TLC (10%MeOH/DCM) to obtain the product (10 mg).

¹H NMR (400 MHz, CDCl₃) δ 8.62 (s, 1H), 7.75 (t, *J* = 8.0 Hz, 1H), 7.63 (d, *J* = 8.8 Hz, 2H), 7.29 (d, *J* = 8.8 Hz, 2H), 7.27 (s, 1H), 7.22 (d, *J* = 7.6 Hz, 1H), 6.83 (d, *J* = 8.0 Hz, 1H), 6.18 (d, *J* = 6.0 Hz, 1H), 3.81-3.91 (m, 2H), 3.32 (s, 6H), 2.98-3.07 (m, 1H), 2.71 (s, 3H), 2.47-2.58 (m, 2H), 2.40 (d, *J* = 18.4 Hz, 1H), 2.20-2.28 (m, 1H), 2.09-2.15 (m, 1H), 1.80-1.84 (m, 1H), 0.85-0.90 (m, 2H), 0.74-0.78 (m, 2H).

### Example 66

### ((6-(6-cyclopropyl-2-((4-((1R,5S)-8-methyl-8-azabicyclo[3.2.1]octan-3-yl)phenyl)amino)-7H -pyrrolo[2,3-d]pyrimidin-7-yl)pyridin-2-yl)imino)dimethyl-λ⁶-sulfanone

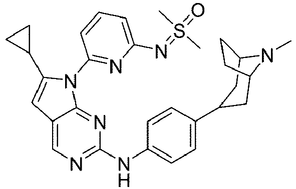

((6-(2-((4-((1R,5S)-8-azabicyclo[3.2.1]octan-3-yl)phenyl)amino)-6-cyclopropyl-7*H*-pyrrolo[ 2,3-d]pyrimidin-7-yl)pyridin-2-yl)imino)dimethyl-λ⁶-sulfanone (25 mg), aqueous solution of 40% formaldehyde (0.1 mL) and glacial acetic acid (0.1 mL) were mixed in dichloromethane (5 mL), the mixture was stirred at room temperature for 20 minutes, then sodium triacetoxyborohydride (50 mg) was added, and the mixture was stirred for 3 hours. An aqueous solution of saturated sodium bicarbonate was used to adjust the pH to 7.0, the mixture was partitioned between water and dichloromethane, the solvent was removed from the organic phase, and the residue was purified through preparative TLC (10%MeOH/DCM) to obtain the product (5 mg).

¹H NMR (400 MHz, CDCl₃) δ 8.55 (s, 1H), 7.75-7.79 (m, 1H), 7.67-7.81 (brs, 1H), 7.62 (d, *J* = 8.4 Hz, 1H), 7.57 (d, *J* = 8.4 Hz, 1H), 7.25-7.28 (m, 2H), 7.16-7.23 (m, 1H), 6.84-6.87 (m, 1H), 6.08 (s, 1H), 3.87-3.92 (m, 1H), 3.78-3.82 (m, 1H), 3.32 (s, 3H), 3.30 (s, 3H), 2.87-3.11 (m, 2H), 2.75-2.82 (m, 3H), 2.48 (d, *J* = 14.8 Hz, 1H), 2.26-2.41 (m, 2H), 2.09-2.14 (m, 1H), 1.97-2.05 (m, 1H), 1.82-1.87 (m, 3H), 0.88-0.94 (m, 2H), 0.66-0.72 (m, 2H).

### Example 67

### ((6-(6-cyclopropyl-5-fluoro-2-((4-((1R,5S)-8-methyl-8-azabicyclo[3.2.1]octan-3-yl)phenyl)a mino)-7H-pyrrolo[2,3-d]pyrimidin-7-yl)pyridin-2-yl)imino)dimethyl-λ⁶-sulfanone

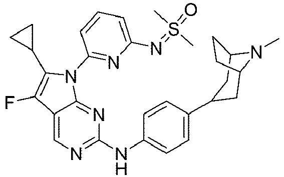

((6-(2-((4-((1R,5S)-8-azabicyclo[3.2.1]octan-3-yl)phenyl)amino)-6-cyclopropyl-5-fluoro-7*H-*pyrrolo[2,3-d]pyrimidin-7-yl)pyridin-2-yl)imino)dimethyl-λ⁶-sulfanone (10 mg), aqueous solution of 40% formaldehyde (0.05 mL) and glacial acetic acid (0.1 mL) were mixed in dichloromethane (5 mL), the mixture was stirred at room temperature for 20 minutes, then sodium triacetoxyborohydride (19 mg) was added, and the mixture was stirred for 3 hours. An aqueous solution of saturated sodium bicarbonate was used to adjust the pH to 7.0, the mixture was partitioned between water and dichloromethane, the solvent was removed from the organic phase, and the residue was purified through preparative TLC (10%MeOH/DCM) to obtain the product (5 mg).

¹H NMR (400 MHz, CDCl₃) δ 8.64 (s, 1H), 7.74-7.79 (m, 1H), 7.62 (d, *J* = 8.8 Hz, 1H), 7.58 (d, *J* = 8.8 Hz, 1H), 7.25-7.33 (m, 3H), 7.17-7.23 (m, 1H), 6.82-6.84 (m, 1H), 3.87-3.92 (m, 1H), 3.78-3.84 (m, 1H), 3.31 (s, 3H), 3.30 (s, 3H), 2.86-3.17 (m, 2H), 2.83 (s, 1.5H), 2.76 (s, 1.5H), 2.46 (d, *J* = 15.2 Hz, 1H), 2.28-2.38 (m, 1H), 2.17-2.28 (m, 1H), 2.09-2.16 (m, 1H), 1.96-2.07 (m, 1H), 1.76-1.92 (m, 3H), 0.83-0.91 (m, 2H), 0.74-0.78 (m, 2H).

### Example 68

### ((6-(6-cyclopropyl-2-((4-(1,2,3,6-tetrahydropyridin-4-yl)phenyl)amino)-7H-pyrrolo[2,3-d]py rimidin-7-yl)pyridin-2-yl)imino)dimethyl-λ⁶-sulfanone

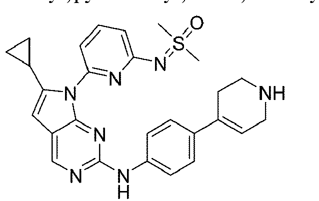

### Step A: tert-butyl 4-(4-aminophenyl)-3,6-dihydropyridin-1(2H)-carboxylate

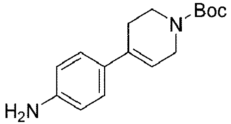

4-Bromo aniline (1.7 g), tert-butyl 4- (4,4,5,5- tetramethyl- 1,3,2- dioxaborolan- 2- yl)- 3,6-dihydropyridin- 1(2H)-carboxylate (3.7 g), potassium phosphate (4.3 g) and Pd(dppf)Cl₂ (720 mg) were mixed in a mixed solvent of 1,2-dimethoxyethane (50 mL) and water (10 mL). Under nitrogen protection, the mixture was heated to 85 °C and stirred overnight. The mixture was cooled to room temperature and partitioned between water and dichloromethane, the solvent was removed from the organic phase, and the residue was purified through silica gel column chromatography (1%MeOH/DCM) to obtain the product (1.1 g).

¹H NMR (400 MHz, CDCl₃) δ 7.19 (d, *J* = 8.8 Hz, 2H), 6.65 (d, *J* = 8.8 Hz, 2H), 5.86-5.94 (m, 1H), 4.02-4.06 (m, 2H), 3.68 (s, 2H), 3.61 (t, *J* = 5.6 Hz, 2H), 2.44-2.51 (m, 2H), 1.49 (s, 9H).

### Step B: tert-butyl 4-(4-((6- cyclopropyl- 7- (6- ((dimethyl (oxo)- λ⁶- sulfanylidene) amino) pyridin- 2- yl)- 7H- pyrrolo [2,3-d] pyrimidin- 2- yl) amino) phenyl)- 3,6- dihydropyridin- 1(2H)-carboxylate

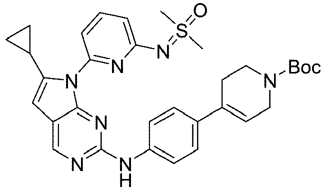

((6-(2-Chloro-6-cyclopropyl-7*H*-pyrrolo[2,3-d]pyrimidin-7-yl)pyridin-2-yl)imino)dimethyl-λ ⁶-sulfanone (50 mg), tert-butyl 4- (4- aminophenyl)- 3,6-dihydropyridin- 1(2*H*)- carboxylate (19 mg), Pd₂(dba)₃ (13 mg), Dave-phos (17 mg) and sodium tert-butoxide (20 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (20 mg).

¹H NMR (400 MHz, CDCl₃) δ 8.54 (s, 1H), 7.75 (t, *J* = 8.0 Hz, 1H), 7.59 (d, *J* = 8.4 Hz, 2H), 7.30 (s, 1H), 7.27 (d, *J =* 8.4 Hz, 2H), 7.22 (d, *J =* 7.6 Hz, 1H), 6.83 (d, *J* = 8.0 Hz, 1H), 6.08 (s, 1H), 5.93-6.01 (m, 1H), 4.04-4.09 (m, 2H), 3.63 (t, *J* = 6.0 Hz, 2H), 3.30 (s, 6H), 2.47-2.54 (m, 2H), 2.32-2.40 (m, 1H), 1.49 (s, 9H), 0.88-0.94 (m, 2H).

### Step C: ( (6- (6- cyclopropyl- 2- ( (4- (1,2,3,6- tetrahydropyridin- 4- yl) phenyl) amino)- 7H-pyrrolo [2,3-d]pyrimidin-7-yl)pyridin-2-yl)imino)dimethyl-λ⁶-sulfanone

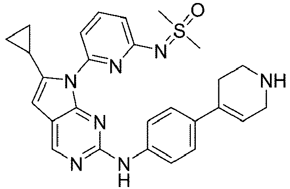

Acetyl chloride (2 mL) was dropwise added to methanol (4 mL) slowly, to the mixture was added tert-butyl 4- (4- ((6- cyclopropyl- 7- (6- ((dimethyl (oxo)- λ⁶- sulfanylidene) amino) pyridin- 2- yl)- *7H-* pyrrolo [2,3-d] pyrimidin- 2- yl) amino) phenyl)- 3,6- dihydropyridin- 1(2*H*)-carboxylate (20 mg), and the mixture was stirred at room temperature for 1 hour. The solvent was evaporated, and the residue was purified through preparative TLC (10%MeOH/DCM) to obtain the target product (10 mg).

¹H NMR (400 MHz, CD₃OD) δ 8.52 (s, 1H), 7.83 (t, *J* = 8.0 Hz, 1H), 7.67 (d, *J* = 8.4 Hz, 2H), 7.32 (d, *J* = 8.4 Hz, 2H), 7.20 (d, *J* = 8.0 Hz, 1H), 6.83 (d, *J* = 8.4 Hz, 1H), 6.18 (s, 1H), 6.04-6.08 (m, 1H), 3.80-3.84 (m, 2H), 3.44 (t, *J* = 6.0 Hz, 2H), 3.35 (s, 6H), 3.19-3.25 (brs, 1H), 2.75-2.81 (m, 2H), 2.26-2.35 (m, 1H), 0.84-0.89 (m, 2H), 0.66-0.70 (m, 2H).

### Example 69

### ((6-(6-cyclopropyl-5-fluoro-2-((4-(1,2,3,6-tetrahydropyridin-4-yl)phenyl)amino)-7H-pyrrolo[ 2,3-d]pyrimidin-7-yl)pyridin-2-yl)imino)dimethyl-λ⁶-sulfanone

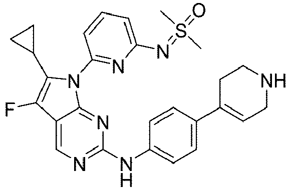

### Step A: tert-butyl 4- (4- ( (6- cyclopropyl- 7- (6- ((dimethyl (oxo)- λ⁶- sulfanylidene) amino) pyridin- 2- yl)- 5- fluoro- 7H- pyrrolo [2,3-d] pyrimidin- 2- yl) amino) phenyl)- 3,6-dihydropyridin- 1(2H)-carboxylate

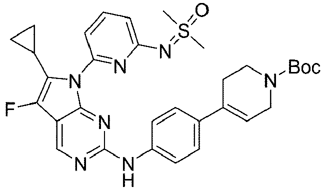

((6-(2-Chloro-5-fluoro-6-cyclopropyl-7*H*-pyrrolo[2,3-d]pyrimidin-7-yl)pyridin-2-yl)imino)di methyl- λ⁶- sulfanone (53 mg), tert-butyl 4- (4-aminophenyl)- 3,6- dihydropyridin- 1(2*H*)-carboxylate (19 mg), Pd₂(dba)₃ (13 mg), Dave-phos (17 mg) and sodium tert-butoxide (20 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (18 mg).

¹H NMR (400 MHz, CDCl₃) δ 8.61 (s, 1H), 7.74 (t, *J* = 8.0 Hz, 1H), 7.59 (d, *J* = 8.8 Hz, 2H), 7.28 (d, *J* = 8.4 Hz, 2H), 7.23 (d, *J* = 8.0 Hz, 1H), 7.22 (s, 1H), 6.81 (d, *J* = 8.0 Hz, 1H), 5.93-6.03 (m, 1H), 4.04-4.11 (m, 2H), 3.63 (t, *J* = 5.6 Hz, 2H), 3.30 (s, 6H), 2.47-2.55 (m, 2H), 2.21-2.28 (m, 1H), 1.49 (s, 9H), 0.84-0.89 (m, 2H), 0.74-0.78 (m, 2H).

### Step B: ((6- (6- cyclopropyl- 5- fluoro- 2- ( (4- (1,2,3,6- tetrahydropyridin- 4- yl) phenyl) amino)- 7H- pyrrolo[2,3-d]pyrimidin-7-yl)pyridin-2-yl)imino)dimethyl-λ⁶-sulfanone

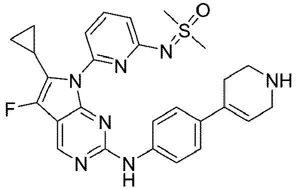

Acetyl chloride (2 mL) was dropwise added to methanol (4 mL) slowly, to the mixture was added tert-butyl 4- (4- ((6- cyclopropyl- 7- (6- ( (dimethyl (oxo)- λ⁶- sulfanylidene) amino) pyridin- 2- yl)- 5- fluoro- 7*H*- pyrrolo [2,3-d] pyrimidin- 2- yl) amino) phenyl)- 3,6-dihydropyridin- 1(2*H*)- carboxylate (18 mg), and the mixture was stirred at room temperature for 1 hour. The solvent was evaporated, and the residue was purified through preparative TLC (10%MeOH/DCM) to obtain the target product (8 mg).

¹H NMR (400 MHz, CD₃OD) δ 8.52 (s, 1H), 7.83 (t, *J* = 8.0 Hz, 1H), 7.67 (d, *J* = 8.4 Hz, 2H), 7.32 (d, *J* = 8.8 Hz, 2H), 7.20 (d, *J* = 8.0 Hz, 1H), 6.83 (d, *J* = 8.4 Hz, 1H), 6.04-6.07 (m, 1H), 3.81-3.84 (m, 2H), 3.44 (t, *J* = 6.0 Hz, 2H), 3.35 (s, 6H), 3.19-3.25 (brs, 1H), 2.75-2.80 (m, 2H), 2.27-2.35 (m, 1H), 0.84-0.89 (m, 2H), 0.66-0.71 (m, 2H).

### Example 70

### ((6-(6-cyclopropyl-2-((4-(piperidin-4-yl)phenyl)amino)-7H-pyrrolo[2,3-d]pyrimidin-7-yl)pyr idin-2-yl)imino)dimethyl-λ⁶-sulfanone

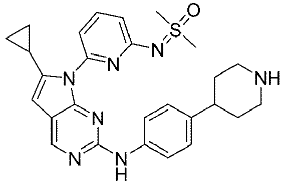

### Step A: tert-butyl 4-(4-aminophenyl)piperidin-1-carboxylate

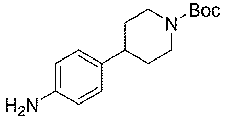

To a flask were sequentially added tert-butyl 4-(4-aminophenyl)- 3,6- dihydropyridin- 1(2*H*)-carboxylate (300 mg), 10% Pd/C (50 mg) and methanol (10 mL), the system was sufficiently purged with hydrogen gas, and the mixture was stirred at room temperature for 2 hours. The reaction solution was filtered through celite, and the filtrate was concentrated and purified through silica gel column chromatography (10%MeOH/DCM) to obtain the product (270 mg).

¹H NMR (400 MHz, CDCl₃) δ 6.99 (d, *J* = 8.4 Hz, 2H), 6.64 (d, *J* = 8.4 Hz, 2H), 4.13-4.29 (m, 2H), 3.58 (s, 2H), 2.77 (t, *J* = 13.2 Hz, 2H), 2.48-2.57 (m, 1H), 1.78 (d, *J* = 12.4 Hz, 2H), 1.49-1.62 (m, 2H), 1.48 (s, 9H).

### Step B: tert-butyl 4- (4- ( (6- cyclopropyl- 7- (6- ((dimethyl (oxo)- λ⁶- sulfanylidene) amino) pyridin- 2- yl)-7H-pyrrolo[2,3-d]pyrimidin-2-yl)amino)phenyl)-piperidin-1-carboxylate

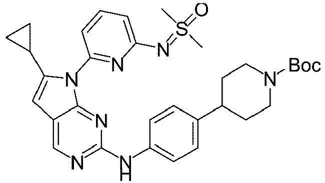

((6-(2-Chloro-6-cyclopropyl-7*H*-pyrrolo[2,3-d]pyrimidin-7-yl)pyridin-2-yl)imino)dimethyl-λ ⁶-sulfanone (50 mg), tert-butyl 4-(4-aminophenyl) piperidin- 1-carboxylate (20 mg), Pd₂(dba)₃ (13 mg), Dave-phos (17 mg) and sodium tert-butoxide (20 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (15 mg).

¹H NMR (400 MHz, CDCl₃) δ 8.54 (s, 1H), 7.75 (t, *J* = 8.0 Hz, 1H), 7.56 (d, *J* = 8.4 Hz, 2H), 7.24-7.31 (brs, 1H), 7.22 (d, *J* = 7.6 Hz, 1H), 7.08 (d, *J* = 8.4 Hz, 2H), 6.83 (d, *J* = 8.0 Hz, 1H), 6.07 (s, 1H), 4.16-4.31 (m, 2H), 3.30 (s, 6H), 2.73-2.84 (m, 2H), 2.55-2.63 (m, 1H), 2.33-2.41 (m, 1H), 1.79 (d, *J* = 12.4 Hz, 2H), 1.54-1.64 (m, 2H), 1.49 (s, 9H), 0.88-0.94 (m, 2H), 0.65-0.70 (m, 2H).

### Step C: ((6- (6- cyclopropyl- 2- ( (4- (piperidin-4-yl) phenyl) amino)- 7H- pyrrolo [2,3-d] pyrimidin- 7-yl)pyridin-2-yl)imino)dimethyl-λ⁶-sulfanone

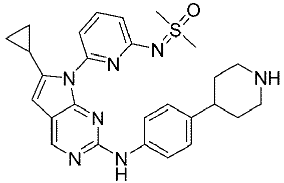

Acetyl chloride (2 mL) was dropwise added to methanol (4 mL) slowly, to the mixture was added tert-butyl 4- (4- ( (6- cyclopropyl- 7- (6- ( (dimethyl (oxo)- λ⁶- sulfanylidene) amino) pyridin- 2- yl)- 7*H*-pyrrolo [2,3-d] pyrimidin- 2- yl) amino) phenyl)- piperidin- 1-carboxylate (15 mg), and the mixture was stirred at room temperature for 1 hour. The solvent was evaporated, and the residue was purified through preparative TLC (10%MeOH/DCM) to obtain the target product (8 mg).

¹H NMR (400 MHz, CD₃OD) δ 8.50 (s, 1H), 7.82 (t, *J* = 8.0 Hz, 1H), 7.60 (d, *J* = 8.8 Hz, 2H), 7.19 (d, *J* = 7.6 Hz, 1H), 7.11 (d, *J* = 8.8 Hz, 2H), 6.82 (d, *J* = 8.0 Hz, 1H), 6.18 (s, 1H), 3.45-3.51 (m, 2H), 3.34 (s, 6H), 3.19-3.25 (brs, 1H), 3.11 (td, *J* = 12.8 Hz, 3.2Hz, 2H), 2.78-2.87 (m, 1H), 2.26-2.33 (m, 1H), 2.04 (d, *J* = 14.8 Hz, 2H), 1.81-1.93 (m, 2H), 0.84-0.88 (m, 2H), 0.66-0.70 (m, 2H).

### Example 71

### ((6-(6-cyclopropyl-5-fluoro-2-((4-(piperidin-4-yl)phenyl)amino)-7H-pyrrolo[2,3-d]pyrimidin -7-yl)pyridin-2-yl)imino)dimethyl-λ⁶-sulfanone

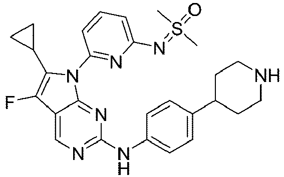

### Step A: tert-butyl 4- (4- ( (6- cyclopropyl- 7- (6- ((dimethyl (oxo)- λ⁶- sulfanylidene) amino) pyridin- 2- yl)-5-fluoro-7H-pyrrolo[2,3-d]pyrimidin-2-yl)amino)phenyl)-piperidin-1-carboxylate

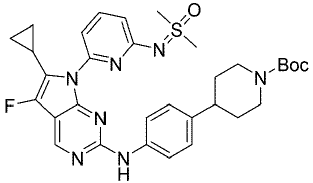

((6-(2-Chloro-5-fluoro-6-cyclopropyl-7*H*-pyrrolo[2,3-d]pyrimidin-7-yl)pyridin-2-yl)imino)di methyl-λ⁶-sulfanone (53 mg), tert-butyl 4-(4-aminophenyl) piperidin-1- carboxylate (20 mg), Pd₂(dba)₃ (13 mg), Dave-phos (17 mg) and sodium tert-butoxide (20 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (10 mg).

¹H NMR (400 MHz, CDCl₃) δ 8.59 (s, 1H), 7.74 (t, *J* = 8.0 Hz, 1H), 7.55 (d, *J* = 8.4 Hz, 2H), 7.35 (s, 1H), 7.23 (d, *J* = 7.6 Hz, 1H), 7.10 (d, *J* = 8.4 Hz, 2H), 6.82 (d, *J* = 8.0 Hz, 1H), 4.18-4.31 (m, 2H), 3.30 (s, 6H), 2.73-2.85 (m, 2H), 2.55-2.64 (m, 1H), 2.19-2.28 (m, 1H), 1.78-1.84 (m, 2H), 1.55-1.65 (m, 2H), 1.49 (s, 9H), 0.84-0.88 (m, 2H), 0.72-0.78 (m, 2H).

### Step B: ((6- (6- cyclopropyl- 5- fluoro- 2- ( (4- (piperidin- 4- yl) phenyl) amino)- 7H- pyrrolo [2,3-d] pyrimidin-7-yl)pyridin-2-yl)imino)dimethyl-λ⁶-sulfanone

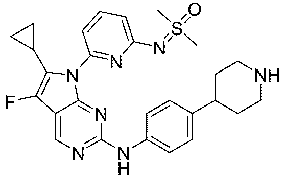

Acetyl chloride (2 mL) was dropwise added to methanol (4 mL) slowly, to the mixture was added tert-butyl 4- (4- ( (6- cyclopropyl- 7- (6- ( (dimethyl (oxo)- λ⁶- sulfanylidene) amino) pyridin- 2- yl)- 5- fluoro- 7*H*- pyrrolo [2,3-d] pyrimidin- 2- yl) amino) phenyl)- piperidin- 1-carboxylate (10 mg), and the mixture was stirred at room temperature for 1 hour. The solvent was evaporated, and the residue was purified through preparative TLC (10%MeOH/DCM) to obtain the target product (7 mg).

¹H NMR (400 MHz, CD₃OD) δ 8.57 (s, 1H), 7.80 (t, *J* = 8.0 Hz, 1H), 7.62 (d, *J* = 8.4 Hz, 2H), 7.21 (d, *J* = 8.0 Hz, 1H), 7.11 (d, *J* = 8.8 Hz, 2H), 6.80 (d, *J* = 8.0 Hz, 1H), 3.45-3.51 (m, 2H), 3.34 (s, 6H), 3.19-3.25 (brs, 1H), 3.11 (td, *J* = 13.2 Hz, 2.8Hz, 2H), 2.78-2.87 (m, 1H), 2.17-2.25 (m, 1H), 1.99-2.07 (m, 2H), 1.81-1.93 (m, 2H), 0.80-0.84 (m, 2H), 0.69-0.73 (m, 2H).

### Example 72

### ((6-(6-cyclopropyl-2-((4-(1-methyl-1,2,3,6-tetrahydropyridin-4-yl)phenyl)amino)-7H-pyrrolo [2,3-d]pyrimidin-7-yl)pyridin-2-yl)imino)dimethyl-λ⁶-sulfanone

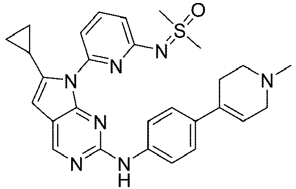

((6-(6-Cyclopropyl-2-((4-(1,2,3,6-tetrahydropyridin-4-yl)phenyl)amino)-7*H*-pyrrolo[2,3-d]py rimidin-7-yl)pyridin-2-yl)imino)dimethyl-λ⁶-sulfanone (10 mg), aqueous solution of 40% formaldehyde (0.05 mL) and glacial acetic acid (0.1 mL) were mixed in dichloromethane (5 mL), the mixture was stirred at room temperature for 20 minutes, then sodium triacetoxyborohydride (20 mg) was added, and the mixture was stirred for 3 hours. An aqueous solution of saturated sodium bicarbonate was used to adjust the pH to 7.0, the mixture was partitioned between water and dichloromethane, the solvent was removed from the organic phase, and the residue was purified through preparative TLC (10%MeOH/DCM) to obtain the product (10 mg).

¹H NMR (400 MHz, CDCl₃) δ 8.54 (s, 1H), 7.77 (t, *J* = 8.0 Hz, 1H), 7.62 (d, *J* = 8.8 Hz, 2H), 7.27 (d, *J* = 8.8 Hz, 2H), 7.23 (s, 1H), 7.21 (d, *J* = 7.6 Hz, 1H), 6.09 (s, 1H), 5.90-5.94 (m, 1H), 3.62-3.89 (m, 2H), 3.31 (s, 6H), 3.26-3.39 (m, 2H), 2.88 (s, 3H), 2.81-3.02 (m, 2H), 2.32-2.39 (m, 1H), 0.89-0.94 (m, 2H), 0.67-0.71 (m, 2H).

### Example 73

### ((6-(6-cyclopropyl-5-fluoro-2-((4-(1-methyl-1,2,3,6-tetrahydropyridin-4-yl)phenyl)amino)-7 H-pyrrolo[2,3-d]pyrimidin-7-yl)pyridin-2-yl)imino)dimethyl-λ⁶-sulfanone

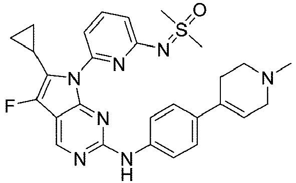

((6-(6-Cyclopropyl-5-fluoro-2-((4-(1,2,3,6-tetrahydropyridin-4-yl)phenyl)amino)-7*H*-pyrrolo [2,3-d]pyrimidin-7-yl)pyridin-2-yl)imino)dimethyl-λ⁶-sulfanone (8 mg), aqueous solution of 40% formaldehyde (0.05 mL) and glacial acetic acid (0.1 mL) were mixed in dichloromethane (5 mL), the mixture was stirred at room temperature for 20 minutes, then sodium triacetoxyborohydride (20 mg) was added, and the mixture was stirred for 3 hours. An aqueous solution of saturated sodium bicarbonate was used to adjust the pH to 7.0, the mixture was partitioned between water and dichloromethane, the solvent was removed from the organic phase, and the residue was purified through preparative TLC (10%MeOH/DCM) to obtain the product (8 mg).

¹H NMR (400 MHz, CDCl₃) δ 8.61 (s, 1H), 7.74 (t, *J* = 8.0 Hz, 1H), 7.59 (d, *J* = 8.8 Hz, 2H), 7.28 (d, *J* = 8.8 Hz, 2H), 7.23 (s, 1H), 7.22 (d, *J* = 8.0 Hz, 1H), 6.82 (d, *J* = 8.0 Hz, 1H), 5.95-5.99 (m, 1H), 3.38-3.41 (m, 2H), 3.30 (s, 6H), 2.94 (t, *J* = 6.0 Hz, 2H), 2.69-2.75 (m, 2H), 2.60 (s, 3H), 2.19-2.27 (m, 1H), 0.83-0.91 (m, 2H), 0.73-0.78 (m, 2H).

### Example 74

### ((6-(6-cyclopropyl-2-((4-(1-methylpiperidin-4-yl)phenyl)amino)-7H-pyrrolo[2,3-d]pyrimidin -7-yl)pyridin-2-yl)imino)dimethyl-λ⁶-sulfanone

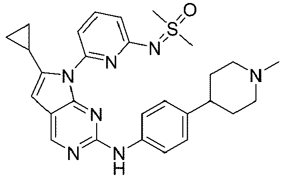

((6-(6-Cyclopropyl-2-((4-(piperidin-4-yl)phenyl)amino)-7*H*-pyrrolo[2,3-d]pyrimidin-7-yl)py ridin-2-yl)imino)dimethyl-λ⁶-sulfanone (8 mg), aqueous solution of 40% formaldehyde (0.05 mL) and glacial acetic acid (0.1 mL) were mixed in dichloromethane (5 mL), the mixture was stirred at room temperature for 20 minutes, then sodium triacetoxyborohydride (20 mg) was added, and the mixture was stirred for 3 hours. An aqueous solution of saturated sodium bicarbonate was used to adjust the pH to 7.0, the mixture was partitioned between water and dichloromethane, the solvent was removed from the organic phase, and the residue was purified through preparative TLC (10%MeOH/DCM) to obtain the product (5 mg).

¹H NMR (400 MHz, CDCl₃) δ 8.53 (s, 1H), 7.76 (t, *J* = 8.0 Hz, 1H), 7.56 (d, *J* = 8.4 Hz, 2H), 7.21 (d, *J* = 7.6 Hz, 1H), 7.11-7.14 (m, 3H), 6.84 (d, *J* = 8.0 Hz, 1H), 6.07 (s, 1H), 3.36-3.43 (m, 2H), 3.31 (s, 6H), 2.66 (s, 3H), 2.54-2.65 (m, 3H), 2.33-2.40 (m, 1H), 2.19-2.32 (m, 2H), 1.95 (d, *J* = 13.6 Hz, 2H), 0.88-0.93 (m, 2H), 0.66-0.70 (m, 2H).

### Example 75

### ((6-(6-cyclopropyl-5-fluoro-2-((4-(1-methylpiperidin-4-yl)phenyl)amino)-7H-pyrrolo[2,3-d]p yrimidin-7-yl)pyridin-2-yl)imino)dimethyl-λ⁶-sulfanone

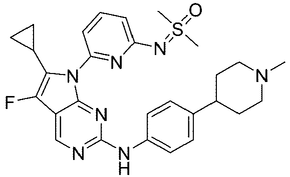

((6-(6-Cyclopropyl-5-fluoro-2-((4-(piperidin-4-yl)phenyl)amino)-7*H*-pyrrolo[2,3-d]pyrimidi n-7-yl)pyridin-2-yl)imino)dimethyl-λ⁶-sulfanone (7 mg), aqueous solution of 40% formaldehyde (0.05 mL) and glacial acetic acid (0.1 mL) were mixed in dichloromethane (5 mL), the mixture was stirred at room temperature for 20 minutes, then sodium triacetoxyborohydride (20 mg) was added, and the mixture was stirred for 3 hours. An aqueous solution of saturated sodium bicarbonate was used to adjust the pH to 7.0, the mixture was partitioned between water and dichloromethane, the solvent was removed from the organic phase, and the residue was purified through preparative TLC (10%MeOH/DCM) to obtain the product (5 mg).

¹H NMR (400 MHz, CDCl₃) δ 8.56 (s, 1H), 7.73 (t, *J* = 8.0 Hz, 1H), 7.56 (d, *J* = 8.4 Hz, 2H), 7.50 (s, 1H), 7.23 (d, *J* = 7.6 Hz, 1H), 7.12 (d, *J* = 8.4 Hz, 2H), 6.81 (d, *J* = 8.0 Hz, 1H), 3.30 (s, 6H), 3.12-3.19 (m, 2H), 2.45-2.52 (m, 1H), 2.42 (s, 3H), 2.19-2.29 (m, 3H), 1.81-1.96 (m, 4H), 0.83-0.89 (m, 2H), 0.71-0.77 (m, 2H).

### Example 76

### 2-(6-(2-((3-fluoro-4-(4-methylpiperazin-1-yl)phenyl)amino)-6-cyclopropyl-7H-pyrrolo[2,3-d] pyrimidin-7-yl)pyridin-2-yl)propan-2-ol

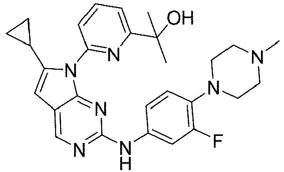

According to the method of step F of example 1, 2-(6-(2-chloro-6-cyclopropyl-7*H*-pyrrolo[2,3-d]pyrimidin-7-yl)pyridin-2-yl)propan-2-ol and 3-fluoro-4-(4-methylpiperazin-1-yl)aniline were used as the starting material to obtain the product (19 mg).

¹H NMR (400 MHz, CDCl₃) δ 8.57 (s, 1H), 8.01 (t, *J* = 8.0 Hz, 1H), 7.79-7.84 (m, 2H), 7.44 (d, *J* = 8.0 Hz, 1H), 7.05 (d, *J* = 8.0 Hz, 1H), 6.86-6.91 (m, 1H), 6.19 (s, 1H), 4.45-4.55 (brs, 1H), 3.19-3.30 (m, 4H), 2.80-3.01 (m, 4H), 2.59 (s, 3H), 2.18-2.20 (m, 1H), 1.60 (s, 6H), 0.88-0.93 (m, 2H), 0.73-0.77 (m, 2H).

### Example 77

### 2-(6-(2-((3-fluoro-4-(4-(4-methylpiperazin-1-yl)piperidin-1-yl)phenyl)amino)-6-cyclopropyl-7H-pyrrolo[2,3-d]pyrimidin-7-yl)pyridin-2-yl)propan-2-ol

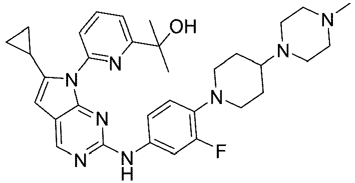

According to the method of step F of example 1, 2- (6- (2- chloro- 6- cyclopropyl- 7*H-*pyrrolo [2,3-d] pyrimidin- 7- yl) pyridin- 2- yl) propan-2-ol and 3- fluoro- 4- (4- (4- methyl piperazin- 1-yl)piperidin-1-yl)aniline were used as the starting material to obtain the product (17 mg).

¹H NMR (400 MHz, CDCl₃) δ 8.55 (s, 1H), 8.01 (t, *J* = 8.0 Hz, 1H), 7.87 (dd, *J* = 15.2 Hz, 2.4 Hz, 1H), 7.81 (d, *J* = 8.0 Hz, 1H), 7.45 (d, *J* = 8.0 Hz, 1H), 7.07 (s, 1H), 6.97 (d, *J* = 8.8 Hz, 1H), 6.86 (t, *J* = 8.8 Hz, 1H), 6.18 (s, 1H), 4.55-4.65 (brs, 1H), 3.43-3.46 (m, 2H), 2.80-3.31 (m, 8H), 2.5-2.8 (m, 6H), 2.18-2.25 (m, 1H), 1.70-2.15 (m, 4H), 1.60 (s, 6H), 0.89-0.92 (m, 2H), 0.74-0.77 (m, 2H).

### Example 78

### 2-(6-(2-((3-fluoro-4-(4-methylpiperazin-1-yl)phenyl)amino)-5-fluoro-6-cyclopropyl-7H-pyrr olo[2,3-d]pyrimidin-7-yl)pyridin-2-yl)propan-2-ol

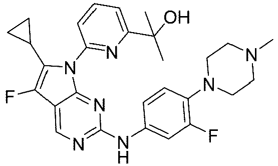

According to the method of step F of example 1, 2- (6- (2- chloro- 5- fluoro- 6- cyclopropyl-7H- pyrrolo [2,3-d] pyrimidin- 7- yl) pyridin- 2- yl) propan- 2-ol and 3- fluoro- 4- (4- methyl piperazin- 1-yl)aniline were used as the starting material to obtain the product (21 mg).

¹H NMR (400 MHz, CDCl₃) δ 8.64 (s, 1H), 8.02 (t, *J* = 8.0 Hz, 1H), 7.76-7.78 (m, 2H), 7.46 (d, *J* = 8.0 Hz, 1H), 7.31 (s, 1H), 7.10 (d, *J* = 8.0 Hz, 1H), 6.87-6.92 (m, 1H), 4.30-4.50 (brs, 1H), 3.00-3.60 (m, 8H), 2.82 (s, 3H), 2.01-2.10 (m, 1H), 1.60 (s, 6H), 0.85-0.95 (m, 2H), 0.73-0.80 (m, 2H).

### Example 79

### ((6-(2-((4-((1R,5S)-3,8-diazabicyclo[3.2.1]octan-3-yl)phenyl)amino)-6-cyclopropyl-7H-pyrr olo[2,3-d]pyrimidin-7-yl)pyridin-2-yl)imino)dimethyl-λ⁶-sulfanone

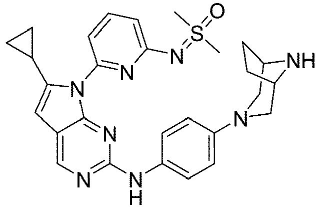

### Step A: ((6- (2- chloro- 6- cyclopropyl- 7H- pyrrolo [2,3-d] pyrimidin- 7- yl) pyridin- 2- yl) imino) dimethyl-λ6-sulfanone

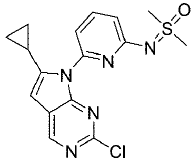

At room temperature, to a tube charged with DMF/TEA (20 mL/20 mL) were added ((6-((5-bromo-2-chloropyrimidin-4-yl)amino)pyridin-2-yl)imino)dimethyl-λ6-sulfanone (2.26 g), copper(I) iodide (120 mg) and Pd(dppf)Cl₂ (420 mg), the system was purged with nitrogen gas, cyclopropylacetylene (600 mg) was added, the mixture was stirred at 35 °C overnight, cooled to room temperature, filtered, and washed with dichloromethane, the solvent was removed from the filtrate, and the residue was purified through silica gel column chromatography (DCM/MeOH=30/1) to obtain the product (510 mg).

¹H NMR (400 MHz CDCl₃) δ 8.69 (s, 1H), 7.72 (t, *J* = 8.0 Hz, 1H), 7.06 (d, *J* = 7.6 Hz, 1H), 6.85 (d, *J* = 8.0 Hz, 1H), 6.18 (s, 1H), 3.43 (s, 6H), 2.12-2.18 (m, 1H), 0.99-1.05 (m, 2H), 0.78-0.83 (m, 2H).

### Step B: tert-butyl 3-(4-nitrophenyl)-3,8-diazabicyclo[3.2.1]octan-8-carboxylate

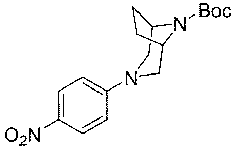

4-Fluoronitrobenzene (141 mg), tert-butyl (1R,5S)- 3,8- diazabicyclo [3.2.1] octan- 8-carboxylate (212 mg) and potassium carbonate (553 mg) were mixed in dimethyl sulfoxide (10 mL). Under nitrogen protection, the mixture was heated to 100 °C and stirred overnight. The mixture was cooled to room temperature and partitioned between water and dichloromethane, the solvent was removed from the organic phase, and the residue was purified through silica gel column chromatography (100%DCM) to obtain the product (215 mg).

¹H NMR (400 MHz, CDCl₃) δ 8.12 (d, *J* = 9.2 Hz, 2H), 6.77 (d, *J* = 9.2 Hz, 2H), 4.32-4.52 (m, 2H), 3.56 (dd, *J* = 12.0 Hz, 2.0Hz, 2H), 3.12-3.26 (m, 2H), 1.98-2.06 (m, 2H), 1.75-1.81 (m, 2H), 1.48 (s, 9H).

### Step C: tert-butyl (1R,5S)-3-(4-aminophenyl)-3,8-diazabicyclo[3.2.1]octan-8-carboxylate

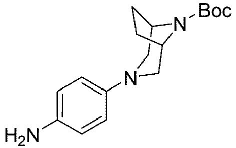

Tert-butyl 3-(4-nitrophenyl)-3,8-diazabicyclo[3.2.1]octan-8-carboxylate (215 mg) and Pd/C (30 mg) were mixed in methanol (10 mL), the system was sufficiently purged with hydrogen gas, and the mixture was stirred at room temperature for 3 hours. The reaction solution was filtered through celite, and the filtrate was concentrated and purified through silica gel column chromatography (10%MeOH/DCM) to obtain the product (120 mg).

¹H NMR (400 MHz, CDCl₃) δ 6.65-6.73 (m, 4H), 4.21-4.41 (m, 2H), 3.23 (d, *J* = 10.8 Hz, 2H), 2.41-3.01 (m, 4H), 1.83-1.96 (m, 4H), 1.47 (s, 9H).

### Step D: tert-butyl (1R,5S)- 3- (4- ( (6- cyclopropyl- 7- (6- ( (dimethyl (oxo)- λ⁶-sulfanylidene) amino) pyridin- 2- yl)- 7H- pyrrolo [2,3-d] pyrimidin- 2- yl) amino) phenyl)- 3,8-diazabicyclo [3.2.1]octan-8-carboxylate

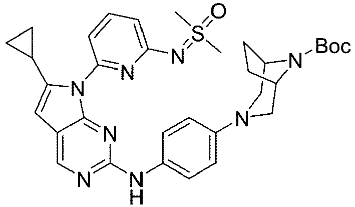

((6-(2-Chloro-6-cyclopropyl-7H-pyrrolo[2,3-d]pyrimidin-7-yl)pyridin-2-yl)imino)dimethyl-λ ⁶-sulfanone (72 mg), tert-butyl (1R,5S)- 3- (4- aminophenyl)- 3,8- diazabicyclo [3.2.1] octan- 8-carboxylate (30 mg), Pd₂(dba)₃ (19 mg), Dave-phos (24 mg) and sodium tert-butoxide (29 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (28 mg).

¹H NMR (400 MHz, CDCl₃) δ 8.52 (s, 1H), 7.73 (t, *J* = 8.0 Hz, 1H), 7.48 (d, *J* = 9.2 Hz, 2H), 7.20 (d, *J* = 8.0 Hz, 1H), 6.98 (s, 1H), 6.82 (d, *J* = 7.6 Hz, 1H), 6.74 (d, *J* = 9.2 Hz, 2H), 6.05 (s, 1H), 4.21-4.47 (m, 2H), 3.24-3.37 (m, 8H), 2.84-3.03 (m, 2H), 2.31-2.39 (m, 1H), 1.82-1.97 (m, 4H), 1.47 (s, 9H), 0.87-0.93 (m, 2H), 0.64-0.69 (m, 2H).

### Step E: ((6- (2- ( (4- ( (1R,5S)- 3,8- diazabicyclo [3.2.1] octan- 3- yl) phenyl) amino)- 6-cyclopropyl- 7H- pyrrolo[2,3-d]pyrimidin-7-yl)pyridin-2-yl)imino)dimethyl-λ⁶-sulfanone

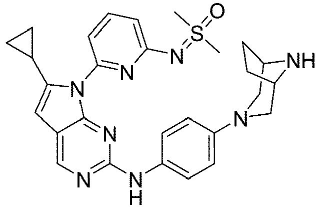

Acetyl chloride (2 mL) was dropwise added to methanol (4 mL) slowly, to the mixture was added tert-butyl (1R,5S)- 3- (4- ( (6- cyclopropyl- 7- (6- ( (dimethyl (oxo)- λ⁶- sulfanylidene) amino) pyridin- 2- yl)- 7*H*- pyrrolo [2,3-d] pyrimidin- 2- yl) amino) phenyl)- 3,8- diazabicyclo [3.2.1] octan- 8-carboxylate (28 mg), and the mixture was stirred at room temperature for 1 hour. The solvent was evaporated, and the residue was purified through preparative TLC (10%MeOH/DCM) to obtain the target product (12 mg).

¹H NMR (400 MHz, CD₃OD) δ 8.46 (s, 1H), 7.81 (t, *J* = 8.0 Hz, 1H), 7.45-7.56 (m, 2H), 7.17 (d, *J* = 7.6 Hz, 1H), 6.78-6.89 (m, 3H), 6.16 (s, 1H), 4.12-4.18 (m, 2H), 3.49-3.62 (m, 2H), 3.34 (s, 6H), 3.20-3.25 (brs, 1H), 2.98-3.18 (m, 2H), 2.21-2.32 (m, 1H), 2.06-2.18 (m, 4H), 0.83-0.88 (m, 2H), 0.66-0.70 (m, 2H).

### Example 80

### ((6-(2-((4-((1R,5S)-3,8-diazabicyclo[3.2.1]octan-3-yl)phenyl)amino)-6-cyclopropyl-5-fluoro-7H-pyrrolo[2,3-d]pyrimidin-7-yl)pyridin-2-yl)imino)dimethyl-λ⁶-sulfanone

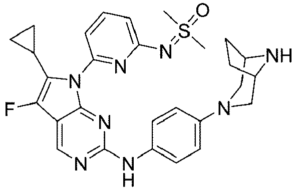

### Step A: ((6- (2- chloro- 6- cyclopropyl- 5- fluoro- 7H- pyrrolo [2,3-d] pyrimidin- 7- yl) pyridin- 2- yl)immo)dimethyl-λ6-sulfanone

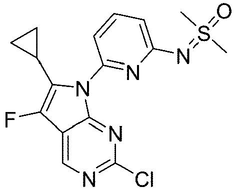

At room temperature, ((6- (2- chloro- 6- cyclopropyl- 7H- pyrrolo [2,3-d] pyrimidin- 7- yl) pyridin- 2-yl)imino)dimethyl-λ6-sulfanone (1.4 g) was added to acetonitrile (50 mL). In an ice-water bath and under nitrogen gas, Selectfluor^{®} (1.24 g) was added, the mixture was stirred for 1 hour, water was added to quench the reaction, the mixture was extracted with ethyl acetate, dried over anhydrous sodium sulfate, evaporated to remove the solvent, and purified through silica gel column chromatography (DCM/EA=1/1) to obtain the product (600 mg).

¹H NMR (400 MHz, CDCl₃) δ 8.78 (s, 1H), 7.72 (t, *J* = 8.0 Hz, 1H), 7.05 (d, *J* = 7.6 Hz, 1H), 6.85 (d, *J* = 8.0 Hz, 1H), 3.40 (s, 6H), 2.05-2.11 (m, 1H), 0.88-0.99 (m, 4H).

### Step B: tert-butyl (1R,5S)- 3- (4- ((6- cyclopropyl- 7- (6- ((dimethyl (oxo)- λ⁶- sulfanylidene) amino) pyridin- 2- yl)- 5- fluoro- 7H- pyrrolo [2,3-d] pyrimidin- 2- yl) amino) phenyl)- 3,8-diazabicyclo [3.2.1]octan-8-carboxylate

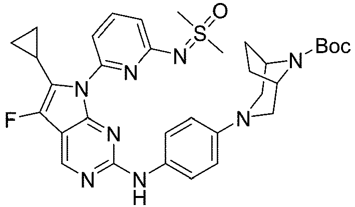

((6-(2-Chloro-5-fluoro-6-cyclopropyl-7*H*-pyrrolo[2,3-d]pyrimidin-7-yl)pyridin-2-yl)imino)di methyl-λ⁶-sulfanone (76 mg), tert-butyl (1R,5S)- 3- (4- aminophenyl)- 3,8- diazabicyclo [3.2.1] octan- 8- carboxylate (30 mg), Pd₂(dba)₃ (19 mg), Dave-phos (24 mg) and sodium tert-butoxide (29 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (30 mg).

¹H NMR (400 MHz, CDCl₃) δ 8.56 (s, 1H), 7.72 (t, *J* = 8.0 Hz, 1H), 7.47 (d, *J* = 8.8 Hz, 2H), 7.22 (d, *J* = 8.0 Hz, 1H), 6.99 (s, 1H), 6.79 (d, *J* = 8.0 Hz, 1H), 6.76 (d, *J* = 8.8 Hz, 2H), 4.24-4.44 (m, 2H), 3.31-3.36 (m, 2H), 3.30 (s, 6H), 2.86-3.02 (m, 2H), 2.19-2.27 (m, 1H), 1.82-1.98 (m, 4H), 1.47 (s, 9H), 0.82-0.88 (m, 2H), 0.73-0.76 (m, 2H).

### Step B: ( (6- (2- ( (4- ( (1R, 5S)- 3,8- diazabicyclo [3.2.1] octan- 3- yl) phenyl) amino)- 6-cyclopropyl- 5-fluoro-7H-pyrrolo[2,3-d]pyrimidin-7-yl)pyridin-2-yl)imino)dimethyl-λ⁶-sulfanone

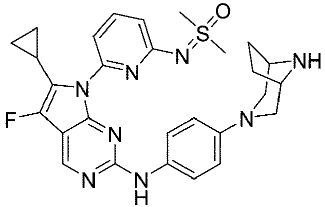

Acetyl chloride (2 mL) was dropwise added to methanol (4 mL) slowly, to the mixture was added tert-butyl (1R, 5S)- 3- (4- ( (6- cyclopropyl- 7-(6- ( (dimethyl (oxo)- λ⁶- sulfanylidene) amino) pyridin- 2- yl)- 5- fluoro- 7*H*- pyrrolo [2,3-d] pyrimidin- 2- yl) amino) phenyl)- 3,8-diazabicyclo [3.2.1]octan-8-carboxylate (30 mg), and the mixture was stirred at room temperature for 1 hour. The solvent was evaporated, and the residue was purified through preparative TLC (10%MeOH/DCM) to obtain the target product (17 mg).

¹H NMR (400 MHz, CD₃OD) δ 8.54 (s, 1H), 7.79 (t, *J* = 8.0 Hz, 1H), 7.53 (d, *J* = 9.2 Hz, 2H), 7.20 (d, *J* = 7.6 Hz, 1H), 6.84 (d, *J* = 9.2 Hz, 2H), 6.79 (d, *J* = 8.0 Hz, 1H), 4.13-4.18 (m, 2H), 3.55-3.61 (m, 2H), 3.34 (s, 6H), 3.19-3.25 (brs, 1H), 3.07 (d, *J* = 12.4 Hz, 2H), 2.07-2.24 (m, 5H), 0.78-0.82 (m, 2H), 0.68-0.72 (m, 2H).

### Example 81

### ((6-(6-cyclopropyl-2-((4-((1R,5S)-8-methyl-3,8-diazabicyclo[3.2.1]octan-3-yl)phenyl)amino) -7H-pyrrolo[2,3-d]pyrimidin-7-yl)pyridin-2-yl)imino)dimethyl-λ⁶-sulfanone

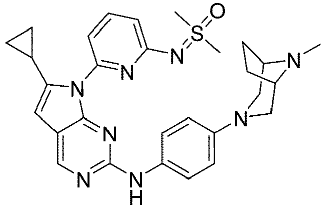

((6-(2-((4-((1R,5S)-3,8-diazabicyclo[3.2.1]octan-3-yl)phenyl)amino)-6-cyclopropyl-7*H*-pyrr olo[2,3-d]pyrimidin-7-yl)pyridin-2-yl)imino)dimethyl-λ⁶-sulfanone (10 mg), aqueous solution of 40% formaldehyde (0.05 mL) and glacial acetic acid (0.1 mL) were mixed in dichloromethane (5 mL), the mixture was stirred at room temperature for 20 minutes, then sodium triacetoxyborohydride (50 mg) was added, and the mixture was stirred for 3 hours. A solution of saturated sodium bicarbonate was used to adjust the pH to 7.0, the mixture was partitioned between water and dichloromethane, the solvent was removed from the organic phase, and the residue was purified through preparative TLC (10%MeOH/DCM) to obtain the product (5 mg).

¹H NMR (400 MHz, CDCl₃) δ 8.49 (s, 1H), 7.75 (t, *J* = 8.0 Hz, 1H), 7.51 (d, *J* = 8.8 Hz, 2H), 7.22-7.27 (brs, 1H), 7.19 (d, *J* = 7.6 Hz, 1H), 6.83 (d, *J* = 8.0 Hz, 1H), 6.77 (d, *J* = 8.8 Hz, 2H), 6.07 (s, 1H), 3.71-3.92 (m, 4H), 3.33-3.39 (m, 2H), 3.31 (s, 6H), 2.83 (s, 3H), 2.18-2.36 (m, 5H), 0.88-0.93 (m, 2H), 0.66-0.70 (m, 2H).

### Example 82

### ((6-(6-cyclopropyl-5-fluoro-2-((4-((1R,5S)-8-methyl-3,8-diazabicyclo[3.2.1]octan-3-yl)phen yl)amino)-7H-pyrrolo[2,3-d]pyrimidin-7-yl)pyridin-2-yl)imino)dimethyl-λ⁶-sulfanone

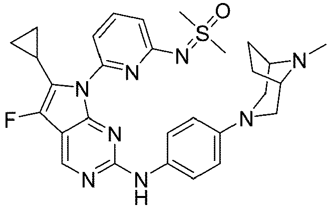

((6-(2-((4-((1R,5S)-3,8-diazabicyclo[3.2.1]octan-3-yl)phenyl)amino)-6-cyclopropyl-5-fluoro-7*H*-pyrrolo[2,3-d]pyrimidin-7-yl)pyridin-2-yl)imino)dimethyl-λ⁶-sulfanone (15 mg), aqueous solution of 40% formaldehyde (0.05 mL) and glacial acetic acid (0.1 mL) were mixed in dichloromethane (5 mL), the mixture was stirred at room temperature for 20 minutes, then sodium triacetoxyborohydride (50 mg) was added, and the mixture was stirred for 3 hours. A solution of saturated sodium bicarbonate was used to adjust the pH to 7.0, the mixture was partitioned between water and dichloromethane, the solvent was removed from the organic phase, and the residue was purified through preparative TLC (10%MeOH/DCM) to obtain the product (8 mg).

¹H NMR (400 MHz, CDCl₃) δ 8.56 (s, 1H), 7.73 (t, *J* = 8.0 Hz, 1H), 7.51 (d, *J* = 8.8 Hz, 2H), 7.21 (s, 1H), 7.19 (d, *J* = 8.0 Hz, 1H), 6.77-6.82 (m, 3H), 3.71-3.87 (m, 4H), 3.34-3.41 (m, 2H), 3.30 (s, 6H), 2.81 (s, 3H), 2.16-2.29 (m, 5H), 0.83-0.89 (m, 2H), 0.72-0.77 (m, 2H).

### Example 83

### ((6-(2-((4-(2,7-diazaspiro[3.5]nonan-7-yl)phenyl)amino)-6-cyclopropyl-7H-pyrrolo[2,3-d]py rimidin-7-yl)pyridin-2-yl)imino)dimethyl-λ⁶-sulfanone

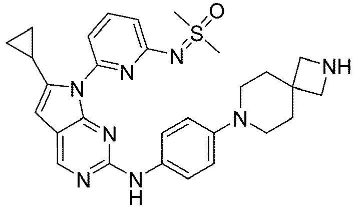

### Step A: tert-butyl 7-(4-nitrophenyl)-2,7-diazaspiro[3.5]nonan-2-carboxylate

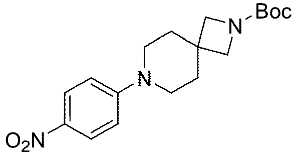

4-Fluoronitrobenzene (141 mg), tert-butyl 2,7-diazaspiro[3.5]nonan-2-carboxylate (226 mg) and potassium carbonate (553 mg) were mixed in dimethyl sulfoxide (10 mL). Under nitrogen protection, the mixture was heated to 100 °C and stirred overnight. The mixture was cooled to room temperature and partitioned between water and dichloromethane, the solvent was removed from the organic phase, and the residue was purified through silica gel column chromatography (100%DCM) to obtain the product (250 mg).

¹H NMR (400 MHz, CDCl₃) δ 8.11 (d, *J* = 9.6 Hz, 2H), 6.83 (d, *J* = 9.6 Hz, 2H), 3.71 (s, 4H), 3.38-3.41 (m, 4H), 1.85-1.88 (m, 4H), 1.45 (s, 9H).

### Step B: tert-butyl 7-(4-aminophenyl)-2,7-diazaspiro[3.5]nonan-2-carboxylate

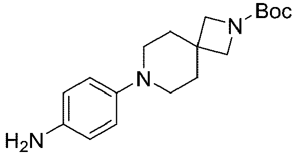

Tert-butyl 7-(4-nitrophenyl)-2,7-diazaspiro[3.5]nonan-2-carboxylate (250 mg) and Pd/C (25 mg) were mixed in methanol (10 mL), the system was sufficiently purged with hydrogen gas, and the mixture was stirred at room temperature for 3 hours. The reaction solution was filtered through celite, and the filtrate was concentrated and purified through silica gel column chromatography (10%MeOH/DCM) to obtain the product (205 mg).

¹H NMR (400 MHz, CDCl₃) δ 6.83 (d, *J* = 8.8 Hz, 2H), 6.64 (d, *J* = 8.8 Hz, 2H), 3.66 (s, 4H), 3.27-3.74 (brs, 2H), 2.92-2.99 (m, 4H), 1.86-1.94 (m, 4H), 1.45 (s, 9H).

### Step C: tert-butyl 7- (4- ( (6- cyclopropyl- 7- (6- ( (dimethyl (oxo)- λ⁶-sulfanylidene) amino) pyridin- 2- yl)- 7H- pyrrolo[2,3-d] pyrimidin-2-yl) amino) phenyl)- 2,7-diazaspiro[3.5]nonan- 2-carboxylate

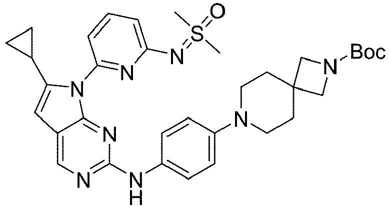

((6-(2-Chloro-6-cyclopropyl-7*H*-pyrrolo[2,3-d]pyrimidin-7-yl)pyridin-2-yl)imino)dimethyl-λ ⁶-sulfanone (72 mg), tert-butyl 7-(4-aminophenyl)-2,7-diazaspiro[3.5]nonan-2-carboxylate (32 mg), Pd₂(dba)₃ (19 mg), Dave-phos (24 mg) and sodium tert-butoxide (29 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (30 mg).

¹H NMR (400 MHz, CDCl₃) δ 8.52 (s, 1H), 7.72 (t, *J* = 8.0 Hz, 1H), 7.47-7.51 (m, 3H), 7.19 (d, *J* = 7.6 Hz, 1H), 6.80-6.86 (m, 3H), 6.04 (s, 1H), 3.67 (s, 4H), 3.27 (s, 6H), 3.01-3.03 (m, 4H), 2.28-2.37 (m, 1H), 1.87-1.90 (m, 4H), 1.46 (s, 9H), 0.87-0.92 (m, 2H), 0.65-0.69 (m, 2H).

### Step D: ((6-(2-((4-(2,7-diazaspiro[3.5]nonan-7-yl)phenyl)amino)-6-cyclopropyl- 7H- pyrrolo [2,3-d] pyrimidin-7-yl)pyridin-2-yl)imino)dimethyl-λ⁶-sulfanone

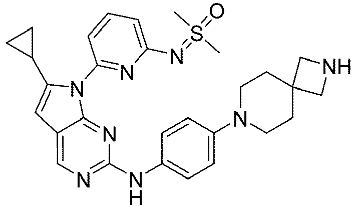

Acetyl chloride (2 mL) was dropwise added to methanol (4 mL) slowly, to the mixture was added tert-butyl 7-(4-((6-cyclopropyl-7-(6-((dimethyl(oxo)-λ⁶-sulfanylidene) amino) pyridin- 2-yl)-7*H*-pyrrolo[2,3-d]pyrimidin-2-yl)amino)phenyl)-2,7-diazaspiro[3.5]nonan-2-carboxylate (30 mg), and the mixture was stirred at room temperature for 1 hour. The solvent was evaporated, and the residue was purified through preparative TLC (10%MeOH/DCM) to obtain the target product (15 mg).

¹H NMR (400 MHz, DMSO) δ 9.05 (s, 1H), 8.56 (s, 1H), 7.83 (t, *J* = 8.0 Hz, 1H), 7.58 (d, *J* = 8.8 Hz, 2H), 7.15 (d, *J* = 8.0 Hz, 1H), 6.81 (d, *J* = 8.8 Hz, 2H), 6.74 (d, *J* = 8.0 Hz, 1H), 6.19 (s, 1H), 3.71-3.75 (m, 4H), 3.36 (s, 6H), 2.95-2.99 (m, 5H), 2.18-2.26 (m, 1H), 1.83-1.89 (m, 4H), 0.79-0.85 (m, 2H), 0.65-0.69 (m, 2H).

### Example 84

### ((6-(2-((4-(2,7-diazaspiro[3.5]nonan-7-yl)phenyl)amino)-6-cyclopropyl-5-fluoro-7H-pyrrolo[ 2,3-d]pyrimidin-7-yl)pyridin-2-yl)imino)dimethyl-λ⁶-sulfanone

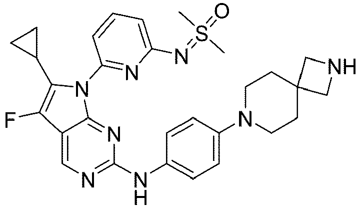

### Step A: tert-butyl 7-(4-((6-cyclopropyl-7-(6-((dimethyl (oxo)-λ⁶-sulfanylidene) amino) pyridin-2-yl)-5-fluoro-7H-pyrrolo[2,3-d]pyrimidin-2-yl)amino)phenyl)-2,7-diazaspiro[3.5]nonan-2-carboxylate

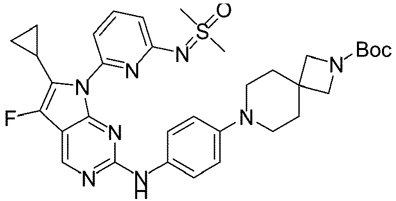

((6-(2-Chloro-5-fluoro-6-cyclopropyl-7*H*-pyrrolo[2,3-d]pyrimidin-7-yl)pyridin-2-yl)imino)di methyl-λ⁶-sulfanone (76 mg), tert-butyl 7-(4-aminophenyl)- 2,7- diazaspiro [3.5]nonan- 2-carboxylate (32 mg), Pd₂(dba)₃ (19 mg), Dave-phos (24 mg) and sodium tert-butoxide (29 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (29 mg).

¹H NMR (400 MHz, CDCl₃) δ 8.57 (s, 1H), 7.72 (t, *J* = 8.0 Hz, 1H), 7.48 (d, *J* = 9.2 Hz, 2H), 7.21 (d, *J* = 7.6 Hz, 1H), 6.85-6.89 (m, 3H), 6.79 (d, *J* = 8.0 Hz, 1H), 3.68 (s, 4H), 3.29 (s, 6H), 3.02-3.05 (m, 4H), 2.19-2.26 (m, 1H), 1.86-1.92 (m, 4H), 1.45 (s, 9H), 0.83-0.88 (m, 2H), 0.72-0.76 (m, 2H).

Step B: ((6-(2-((4-(2,7-diazaspiro[3.5]nonan-7-yl) phenyl) amino)- 6- cyclopropyl-5- fluoro-7*H*- pyrrolo [2,3-d] pyrimidin-7-yl)pyridin-2-yl)imino)dimethyl-λ⁶-sulfanone

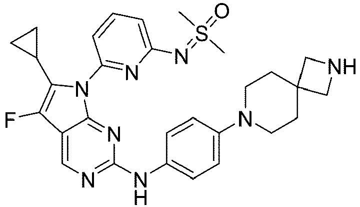

Acetyl chloride (2 mL) was dropwise added to methanol (4 mL) slowly, to the mixture was added tert-butyl 7-(4-((6-cyclopropyl-7-(6-((dimethyl(oxo)-λ⁶-sulfanylidene) amino) pyridin- 2-yl)-5-fluoro-7*H*-pyrrolo[2,3-d]pyrimidin-2-yl)amino)phenyl)-2,7-diazaspiro[3.5]nonan-2-carboxyl ate (29 mg), and the mixture was stirred at room temperature for 1 hour. The solvent was evaporated, and the residue was purified through preparative TLC (10%MeOH/DCM) to obtain the target product (15 mg).

¹H NMR (400 MHz, DMSO) δ 9.28 (s, 1H), 8.68 (s, 1H), 7.83 (t, *J* = 7.6 Hz, 1H), 7.57 (d, *J* = 8.8 Hz, 2H), 7.17 (d, *J* = 7.6 Hz, 1H), 6.82 (d, *J* = 8.8 Hz, 2H), 6.72 (d, *J* = 8.0 Hz, 1H), 3.73 (s, 4H), 3.34 (s, 6H), 2.97-3.02 (m, 5H), 2.12-2.19 (m, 1H), 1.82-1.89 (m, 4H), 0.76-0.81 (m, 2H), 0.64-0.68 (m, 2H).

### Example 85

### ((6-(6-cyclopropyl-2-((4-(2-methyl-2,7-diazaspiro[3.5]nonan-7-yl)phenyl)amino)-7H-pyrrolo [2,3-d]pyrimidin-7-yl)pyridin-2-yl)imino)dimethyl-λ⁶-sulfanone

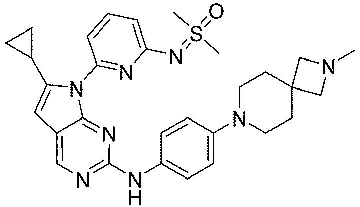

((6-(2-((4-(2,7-Diazaspiro[3.5]nonan-7-yl)phenyl)amino)-6-cyclopropyl-7*H*-pyrrolo[2,3-d]py rimidin-7-yl)pyridin-2-yl)imino)dimethyl-λ⁶-sulfanone (10 mg), aqueous solution of 40% formaldehyde (0.05 mL) and glacial acetic acid (0.1 mL) were mixed in dichloromethane (5 mL), the mixture was stirred at room temperature for 20 minutes, then sodium triacetoxyborohydride (20 mg) was added, and the mixture was stirred for 3 hours. A solution of saturated sodium bicarbonate was used to adjust the pH to 7.0, the mixture was partitioned between water and dichloromethane, the solvent was removed from the organic phase, and the residue was purified through preparative TLC (10%MeOH/DCM) to obtain the product (6 mg).

¹H NMR (400 MHz, CDCl₃) δ 8.49 (s, 1H), 7.74 (t, *J* = 8.0 Hz, 1H), 7.50 (d, *J* = 8.8 Hz, 2H), 7.20 (d, *J* = 8.0 Hz, 1H), 7.14 (s, 1H), 6.82-6.84 (m, 3H), 6.07 (s, 1H), 4.03-4.39 (m, 4H), 3.31 (s, 6H), 2.98-3.05 (m, 4H), 2.89 (s, 3H), 2.31-2.38 (m, 1H), 1.89-2.28 (m, 4H), 0.88-0.93 (m, 2H), 0.65-0.69 (m, 2H).

### Example 86

### ((6-(6-cyclopropyl-5-fluoro-2-((4-(2-methyl-2,7-diazaspiro[3.5]nonan-7-yl)phenyl)amino)-7 H-pyrrolo[2,3-d]pyrimidin-7-yl)pyridin-2-yl)imino)dimethyl-λ⁶-sulfanone

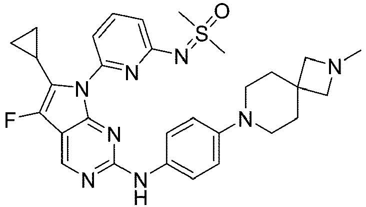

((6-(2-((4-(2,7-Diazaspiro[3.5]nonan-7-yl)phenyl)amino)-6-cyclopropyl-5-fluoro-7*H*-pyrrolo [2,3-d]pyrimidin-7-yl)pyridin-2-yl)imino)dimethyl-λ⁶-sulfanone (10 mg), aqueous solution of 40% formaldehyde (0.05 mL) and glacial acetic acid (0.1 mL) were mixed in dichloromethane (5 mL), the mixture was stirred at room temperature for 20 minutes, then sodium triacetoxyborohydride (20 mg) was added, and the mixture was stirred for 3 hours. A solution of saturated sodium bicarbonate was used to adjust the pH to 7.0, the mixture was partitioned between water and dichloromethane, the solvent was removed from the organic phase, and the residue was purified through preparative TLC (10%MeOH/DCM) to obtain the product (7 mg).

¹H NMR (400 MHz, CDCl₃) δ 8.57 (s, 1H), 7.73 (t, *J* = 8.0 Hz, 1H), 7.49 (d, *J* = 8.8 Hz, 2H), 7.21 (d, *J* = 8.0 Hz, 1H), 7.07 (s, 1H), 6.80-6.88 (m, 3H), 4.25-4.36 (m, 2H), 3.45-3.53 (m, 2H), 3.30 (s, 6H), 2.97-3.09 (m, 4H), 2.90 (s, 3H), 2.18-2.34 (m, 3H), 1.91-1.99 (m, 2H), 0.84-0.89 (m, 2H), 0.72-0.77 (m, 2H).

### Example 87

### ((6-(6-cyclopropyl-2-((3-methoxy-4-(4-(4-methylpiperazin-1-yl)piperidin-1-yl)phenyl)amino) -7H-pyrrolo[2,3-d]pyrimidin-7-yl)pyridin-2-yl)imino)dimethyl-λ⁶-sulfanone

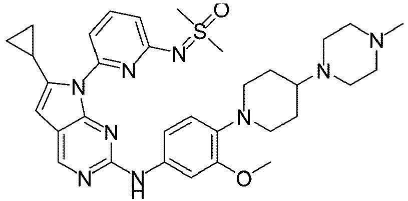

### Step A: 1-(1-(2-methoxy-4-nitrophenyl)piperidin-4-yl)-4-methylpiperazine

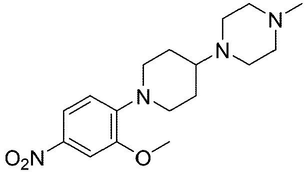

Under nitrogen gas, a solution of 1-methyl-4-(piperidin-4-yl)piperazine hydrochloride (8.4 g), 1-fluoro-2-methoxy-4-nitrobenzene (6.0 g) and potassium carbonate (19.4 g) in dimethyl sulfoxide (80 mL) was heated to 100 °C and stirred overnight. The mixture was cooled to room temperature and partitioned between water and dichloromethane, the solvent was removed from the organic phase, and the residue was purified through silica gel column chromatography (1%MeOH/DCM) to obtain the product (6 g).

¹H NMR (400 MHz, CDCl₃) δ 7.84 (dd, J = 8.8 Hz, 2.4Hz, 1H), 7.69 (d, J = 2.4 Hz, 1H), 6.88 (d, J = 8.8 Hz, 1H), 3.94 (s, 3H), 3.73-3.79 (m, 2H), 2.57-2.77 (m, 6H), 2.39-2.56 (m, 5H), 2.30 (s, 3H), 1.89-1.97 (m, 2H), 1.69-1.79 (m, 2H).

### Step B: 3-methoxy-4-(4-(4-methylpiperazin-1-yl)piperidin-1-yl)aniline

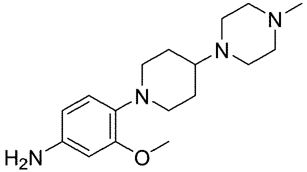

To a flask were sequentially added 1-(1-(2-methoxy-4-nitrophenyl) piperidin- 4- yl)- 4-methyl piperazine (4.0 g), 10% Pd/C (300 mg) and methanol (20 mL), the system was sufficiently purged with hydrogen gas, and the mixture was stirred at room temperature overnight. The reaction solution was filtered through celite, and the filtrate was concentrated and purified through silica gel column chromatography (10%MeOH/DCM) to obtain the product (2.5 g).

¹H NMR (400 MHz, CDCl₃) δ 7.85 (dd, *J* = 8.8 Hz, 2.8Hz, 1H), 7.69 (d, *J* = 2.4 Hz, 1H), 6.88 (d, *J* = 8.8 Hz, 1H), 3.94 (s, 3H), 3.72-3.79 (m, 2H), 2.59-2.78 (m, 6H), 2.39-2.54 (m, 5H), 2.30 (s, 3H), 1.89-1.98 (m, 2H), 1.68-1.81 (m, 2H), 1.59 (brs, 2H).

### Step C ((6-(6-cyclopropyl-2-((3-methoxy-4-(4-(4-methylpiperazin-1-yl) piperidin-1-yl) phenyl) amino)-7H-pyrrolo[2,3-d]pyrimidin-7-yl)pyridin-2-yl)imino)dimethyl-λ⁶-sulfanone

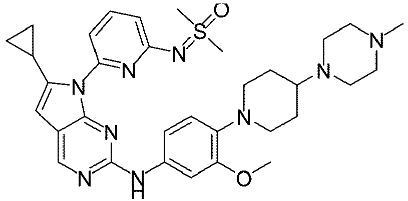

((6-(2-Chloro-6-cyclopropyl-7*H*-pyrrolo[2,3-d]pyrimidin-7-yl)pyridin-2-yl)imino)dimethyl-λ ⁶-sulfanone (72 mg), 3-methoxy-4-(4-(4-methylpiperazin-1-yl)piperidin-1-yl)aniline (31 mg), Pd₂(dba)₃ (19 mg), Dave-phos (24 mg) and sodium tert-butoxide (29 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (35 mg).

¹H NMR (400 MHz, CDCl₃) δ 8.52 (s, 1H), 7.69 (t, *J* = 8.0 Hz, 1H), 7.57 (d, *J* = 7.6 Hz, 1H), 7.12 (d, *J* = 7.6 Hz, 1H), 7.01 (s, 1H), 6.79-6.82 (m, 3H), 6.06 (s, 1H), 3.58 (s, 3H), 3.43-3.50 (m, 2H), 3.26 (s, 6H), 2.62-2.96 (m, 9H), 2.54 (t, *J* = 11.6 Hz, 2H), 2.45 (s, 3H), 2.12-2.19 (m, 1H), 1.91-2.02 (m, 2H), 1.78-1.89 (m, 2H), 0.86-0.91 (m, 2H), 0.67-0.71 (m, 2H).

### Example 88

### 2-(6-(6-cyclopropyl-2-((3-methyl-4-(4-methylpiperazin-1-yl)phenyl)amino)-7H-pyrrolo[2,3-d]pyrimidin-7-yl)pyridin-2-yl)propan-2-ol

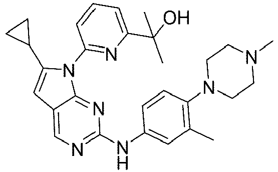

### Step A: 2-(6-((5-iodo-2-chloropyrimidin-4-yl)amino)pyridin-2-yl)propan-2-ol

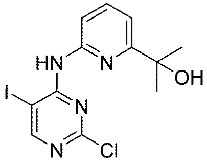

Under nitrogen gas, a mixture of 2-(6-aminopyridin-2-yl)propan-2-ol (29 g), 5-iodo-2,4-dichloropyrimidine (52.5 g), ethyldiisopropylamine (29.4 g) and isopropanol (200 mL) was heated to reflux and stirred overnight. The mixture was cooled to room temperature, and the solid precipitated was collected by filtration, washed with small amount of ethyl acetate, and dried to obtain the product (22.1 g).

¹H NMR (400 MHz, CDCl₃) δ 8.53 (s, 1H), 8.27 (d, J = 8.0 Hz, 1H), 7.91 (s, 1H), 7.82 (t, J = 8.0 Hz, 1H), 7.17 (d, J = 8.0 Hz, 1H), 4.51 (s, 1H), 1.56 (s, 6H).

### Step B: 2- (6- (2- chloro- 6- cyclopropyl- 7H- pyrrolo [2,3-d] pyrimidin- 7- yl) pyridin- 2-yl) propan-2-ol

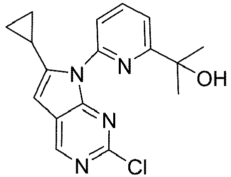

According to the method of step A of example 79, 2- (6- ( (5- iodo- 2- chloropyrimidin- 4- yl) amino) pyridin-2-yl)propan-2-ol and cyclopropylacetylene were used as the starting material to obtain the product (10.1 g).

¹H NMR (400 MHz, CDCl₃) δ 8.73 (s, 1H), 8.00 (t, J = 8.0 Hz, 1H), 7.67 (d, J = 8.0 Hz, 1H), 7.49 (d, J = 8.0 Hz, 1H), 6.29 (s, 1H), 4.31 (s, 1H), 2.05-2.14 (m, 1H), 1.58 (s, 6H), 0.94-0.97 (m, 2H), 0.80-0.83 (m, 2H).

### Step C: 1-methyl-4-(2-methyl-4-nitrophenyl)piperazine

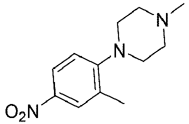

Under nitrogen gas, a suspension of 1-methylpiperazine (1.50 g), 3-methyl- 4- fluoronitro benzene (1.55 g) and potassium carbonate (5.50 g) in anhydrous DMSO (30 mL) was heated to 120 °C overnight. The mixture was cooled to room temperature, and the reaction solution was added into water (100 mL). The solid precipitated was filtered, washed with water, and dried to obtain the product (1.8 g).

¹H NMR (400 MHz CDCl₃) δ 8.02 (d, *J* = 8.4 Hz, 1H), 8.01 (s, 1H), 7.12 (d, *J* = 8.4 Hz, 1H), 3.00-3.02 (m, 4H), 2.49-2.51 (m, 4H), 2.33 (s, 3H), 2.24 (s, 3H).

### Step D: 3-methyl-4-(4-methylpiperazin-1-yl)aniline

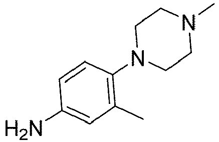

1-Methyl-4-(2-methyl-4-nitrophenyl)piperazine (400 mg) and Pd/C (10 mg) were mixed in methanol (10 mL), the system was sufficiently purged with hydrogen gas, and the mixture was stirred at room temperature overnight. The reaction solution was filtered through celite, and the filtrate was concentrated and purified through preparative TLC (10%MeOH/DCM) to obtain the target product (280 mg).

¹H NMR (400 MHz CDCl₃) δ 6.88 (s, 1H), 6.56 (d, *J* = 2.8 Hz, 1H), 6.52 (d, *J* = 2.8 Hz, 1H), 3.39-3.55 (brs, 2H), 2.85-2.87 (m, 4H), 2.49-2.59 (m, 4H), 2.35 (s, 3H), 2.23 (s, 3H).

### Step E: 2- (6- (6- cyclopropyl-2-( (3-methyl- 4- (4- methylpiperazin- 1- yl) phenyl) amino)-7H- pyrrolo [2,3-d]pyrimidin-7-yl)pyridin-2-yl)propan-2-ol

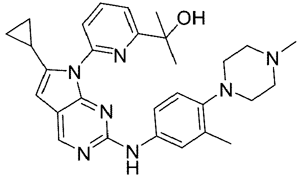

2-(6-(2-Chloro-6-cyclopropyl-7*H*-pyrrolo[2,3-d]pyrimidin-7-yl)pyridin-2-yl)propan-2-ol (33 mg), 3-methyl-4-(4-methylpiperazin-1-yl)aniline (15 mg), Pd₂(dba)₃ (9.2 mg), Dave-phos (12 mg) and sodium tert-butoxide (14 mg) were mixed in toluene (4 mL), the system was sufficiently purged with nitrogen gas, the tube was sealed, and the mixture was heated to 100 °C and stirred overnight. The mixture was cooled to room temperature, evaporated to remove the solvent, and purified through preparative TLC (10%MeOH/DCM) to obtain the target product (15 mg).

¹H NMR (400 MHz CDCl₃) δ 8.54 (s, 1H), 7.99 (t, *J* = 8.0 Hz, 1H), 7.81 (d, *J* = 8.0 Hz, 1H), 7.49 (dd, *J* = 8.0 Hz, 2.4 Hz, 1H), 7.45 (d, *J* = 8.0 Hz, 1H), 7.42(d, *J* = 2.4 Hz, 1H), 7.15-7.23 (brs, 1H), 7.00 (d, *J* = 8.0 Hz, 1H), 6.17 (s, 1H), 4.40-4.60 (brs, 1H), 3.30-3.65 (m, 4H), 2.90-3.20 (m, 4H), 2.86 (s, 3H), 2.24 (s, 3H), 2.16-2.18 (m, 1H), 1.61 (s, 6H), 0.87-0.91 (m, 2H), 0.73-0.76 (m, 2H).

### Example 89

### 2-(6-(6-cyclopropyl-5-fluoro-2-((3-methyl-4-(4-methylpiperazin-1-yl)phenyl)amino)-7H-pyrr olo[2,3-d]pyrimidin-7-yl)pyridin-2-yl)propan-2-ol

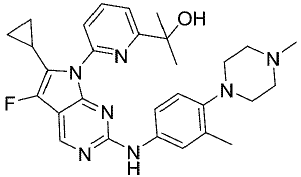

### Step A: 2-(6-(2-chloro-5-fluoro-6-cyclopropyl-7H-pyrrolo[2,3-d]pyrimidin-7-yl) pyridin-2-yl) propan-2-ol

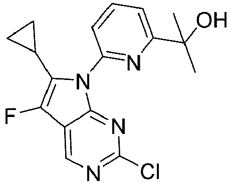

According to the method of step A of example 80, 2-(6-(2-chloro-6- cyclopropyl- 7H- pyrrolo [2,3-d]pyrimidin-7-yl)pyridin-2-yl)propan-2-ol and Selectfluor^{®} were used as the starting material to obtain the product (112 mg).

¹H NMR (400 MHz, CDCl₃) δ 8.82 (s, 1H), 7.98 (t, *J* = 8.0 Hz, 1H), 7.64 (dd, *J* = 8.0 Hz, 0.8Hz, 1H), 7.51 (dd, *J* = 8.0 Hz, 0.8Hz, 1H), 4.10-4.15 (m, 1H), 2.05 (s, 1H), 1.59 (s, 6H), 0.84-0.91 (m, 4H).

### Step B: 2-(6-(6-cyclopropyl-5-fluoro-2-((3-methyl-4-(4-methylpiperazin-1-yl) phenyl) amino)- 7H-pyrrolo[2,3-d]pyrimidin-7-yl)pyridin-2-yl)propan-2-ol

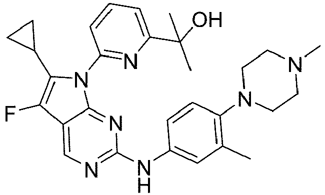

2-(6-(2-Chloro-5-fluoro-6-cyclopropyl-7*H*-pyrrolo[2,3-d]pyrimidin-7-yl)pyridin-2-yl)propan -2-ol (35 mg), 3-methyl-4-(4-methylpiperazin-1-yl)aniline (15 mg), Pd₂(dba)₃ (9.2 mg), Dave-phos (12 mg) and sodium tert-butoxide (14 mg) were mixed in toluene (4 mL), the system was sufficiently purged with nitrogen gas, the tube was sealed, and the mixture was heated to 100 °C and stirred overnight. The mixture was cooled to room temperature, evaporated to remove the solvent, and purified through preparative TLC (10%MeOH/DCM) to obtain the target product (25 mg).

¹H NMR (400 MHz CDCl₃) δ 8.62 (s, 1H), 7.97 (t, *J* = 8.0 Hz, 1H), 7.79 (d, *J* = 8.0 Hz, 1H), 7.47 (d, *J* = 2.4 Hz, 1H), 7.34-7.54 (m, 1H), 7.42(d, *J* = 2.4 Hz, 1H), 7.18-7.21(brs, 1H), 7.00 (d, *J* = 8.0 Hz, 1H), 4.30-4.60 (brs, 1H), 3.08-3.22 (m, 4H), 2.90-3.08 (m, 4H), 2.66 (s, 3H), 2.26 (s, 3H), 2.03-2.08 (m, 1H), 1.60 (s, 6H), 0.82-0.85 (m, 2H), 0.75-0.77 (m, 2H).

### Example 90

### ((6-(2-((3,5-difluoro-4-(4-methylpiperazin-1-yl)phenyl)amino)-6-cyclopropyl-7H-pyrrolo[2,3 -d]pyrimidin-7-yl)pyridin-2-yl)imino)dimethyl-λ⁶-sulfanone

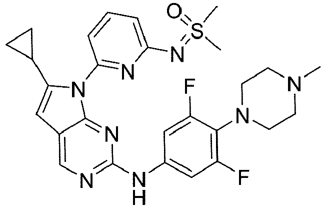

### Step A: 1-(2,6-difluoro-4-nitrophenyl)-4-methylpiperazine

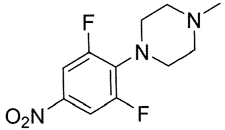

Under nitrogen gas, a suspension of 1-methylpiperazine (1.10 g), 1,2,3-trifluoro-5-nitro benzene (1.77 g) and potassium carbonate (5.52 g) in anhydrous DMSO (20 mL) was heated at 120 °C overnight. The mixture was cooled to room temperature, and the reaction solution was added into water (100 mL). The solid precipitated was filtered, washed with water, and dried to obtain the product (2.36 g).

¹H NMR (400 MHz CDCl₃) δ 7.76 (dd, *J* = 8.8 Hz, 1.2 Hz, 2H), 3.39-3.42 (m, 4H), 2.52-2.62 (m, 4H), 2.35 (s, 3H).

### Step B: 3,5-difluoro-4-(4-methylpiperazin-1-yl)aniline

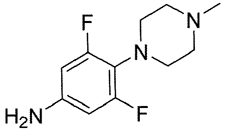

1-(2,6-Difluoro-4-nitrophenyl)-4-methylpiperazine (400 mg) and Pd/C (16 mg) were mixed in methanol (10 mL), the system was sufficiently purged with hydrogen gas, and the mixture was stirred at room temperature overnight. The reaction solution was filtered through celite, and the filtrate was concentrated and purified through preparative TLC (10%MeOH/DCM) to obtain the target product (290 mg).

¹H NMR (400 MHz CDCl₃) δ 6.14 (d, *J* = 10.8 Hz, 2H), 3.71-3.89 (brs, 2H), 3.05-3.08 (m, 4H), 2.45-2.58 (m, 4H), 2.33 (s, 3H).

### Step C: ((6-(2-((3,5-difluoro-4-(4-methylpiperazin-1-yl) phenyl) amino)- 6- cyclopropyl- 7H-pyrrolo [2,3-d]pyrimidin-7-yl)pyridin-2-yl)imino)dimethyl-λ⁶-sulfanone

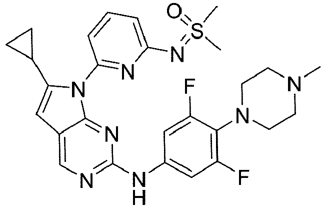

((6-(2-Chloro-6-cyclopropyl-7*H*-pyrrolo[2,3-d]pyrimidin-7-yl)pyridin-2-yl)imino)dimethyl-λ ⁶-sulfanone (36 mg), 3,5-difluoro-4-(4-methylpiperazin-1-yl)aniline (16 mg), Pd₂(dba)₃ (9.2 mg), Dave-phos (12 mg) and sodium tert-butoxide (14 mg) were mixed in toluene (4 mL), the system was sufficiently purged with nitrogen gas, the tube was sealed, and the mixture was heated to 100 °C and stirred overnight. The mixture was cooled to room temperature, evaporated to remove the solvent, and purified through preparative TLC (10%MeOH/DCM) to obtain the target product (28 mg).

¹H NMR (400 MHz CDCl₃) δ 8.54 (s, 1H), 7.79 (t, *J* = 8.0 Hz, 1H), 7.23-7.29 (m, 3H), 7.08-7.15 (brs, 1H), 6.85 (d, *J* = 8.0 Hz, 1H), 6.10 (s, 1H), 3.33 (s, 6H), 3.21-3.50 (m, 4H), 2.79-2.95 (m, 4H), 2.57 (s, 3H), 2.40-2.50 (m, 1H), 0.92-0.94 (m, 2H), 0.67-0.69 (m, 2H).

### Example 91

### ((6-(6-cyclopropyl-2-((2-fluoro-3-methyl-4-(4-methylpiperazin-1-yl)phenyl)amino)-7H-pyrro lo[2,3-d]pyrimidin-7-yl)pyridin-2-yl)imino)dimethyl-λ⁶-sulfanone

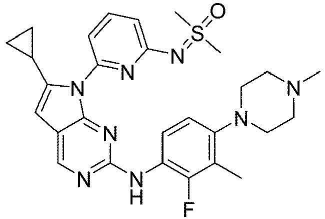

### Step A: 1-(3-fluoro-2-methyl-4-nitrophenyl)-4-methylpiperazine

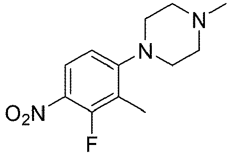

1,3-Difluoro-2-methyl-4-nitrobenzene (1 g), 1-methylpiperazine (578 mg) and potassium carbonate (3.2 g) were mixed in dimethyl sulfoxide (30 mL). Under nitrogen protection, the mixture was heated to 100 °C and stirred overnight. The mixture was cooled to room temperature and partitioned between water and dichloromethane, the solvent was removed from the organic phase, and the residue was purified through silica gel column chromatography (100%DCM) to obtain the product (1.1 g).

¹H NMR (400 MHz, CDCl₃) δ 7.91 (t, *J* = 8.8 Hz, 1H), 6.78 (dd, *J* = 8.8 Hz, 1.6Hz, 1H), 3.05-3.08 (m, 4H), 2.58-2.63 (m, 4H), 2.38 (s, 3H), 2.24 (d, *J* = 3.2 Hz, 3H).

### Step B: 2-fluoro-3-methyl-4-(4-methylpiperazin-1-yl)aniline

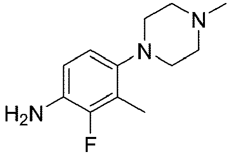

1-(3-Fluoro-2-methyl-4-nitrophenyl)-4-methylpiperazine (100 mg) and Pd/C (20 mg) were mixed in methanol (10 mL), the system was sufficiently purged with hydrogen gas, and the mixture was stirred at room temperature for 2 hours. The reaction solution was filtered through celite, and the filtrate was concentrated and purified through preparative TLC (10%MeOH/DCM) to obtain the target product (85 mg).

¹H NMR (400 MHz, CDCl₃) δ 6.68 (d, *J* = 8.8 Hz, 1H), 6.59 (t, *J* = 8.8 Hz, 1H), 3.52 (s, 2H), 2.84-2.87 (m, 4H), 2.48-2.64 (m, 4H), 2.36 (s, 3H), 2.19 (d, *J* = 2.8 Hz, 3H).

### Step C: ((6-(6-cyclopropyl-2-((2-fluoro-3-methyl-4-(4-methylpiperazin-1-yl) phenyl) amino)-7H- pyrrolo[2,3-d]pyrimidin-7-yl)pyridin-2-yl)imino)dimethyl-λ⁶-sulfanone

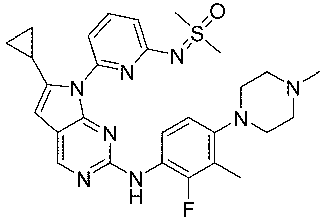

((6-(2-Chloro-6-cyclopropyl-7*H*-pyrrolo[2,3-d]pyrimidin-7-yl)pyridin-2-yl)imino)dimethyl-λ ⁶-sulfanone (72 mg), 2-fluoro-3-methyl-4-(4-methylpiperazin-1-yl)aniline (23 mg), Pd₂(dba)₃ (19 mg), Dave-phos (24 mg) and sodium tert-butoxide (29 mg) were mixed in toluene (5 mL), the mixture was sufficiently purged with nitrogen gas, the tube was sealed, and the mixture was heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (37 mg).

¹H NMR (400 MHz, CDCl₃) δ 8.56 (s, 1H), 8.32 (t, *J* = 9.2 Hz, 1H), 7.81 (t, *J* = 8.0 Hz, 1H), 7.23-7.34 (brs, 1H), 7.21 (d, *J* = 7.6 Hz, 1H), 6.87 (d, *J* = 8.4 Hz, 1H), 6.81 (d, *J* = 8.4 Hz, 1H), 6.09 (s, 1H), 2.91-3.68 (m, 14H), 2.87 (s, 3H), 2.36-2.44 (m, 1H), 2.19 (d, *J* = 2.4 Hz, 3H), 0.89-0.94 (m, 2H), 0.66-0.70 (m, 2H).

### Example 92

### 2-(6-(6-propyl-2-((3-methyl-4-(4-(4-methylpiperazin-1-yl)piperidin-1-yl)phenyl)amino)-7H-pyrrolo[2,3-d]pyrimidin-7-yl)pyridin-2-yl)propan-2-ol

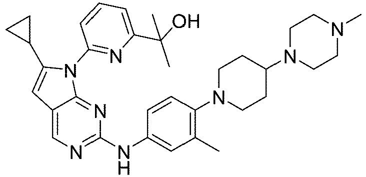

### Step A: 1-(1-(2-methyl-4-nitrophenyl)piperidin-4-yl)-4-methylpiperazine

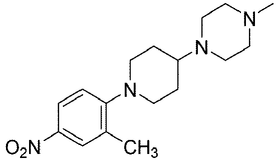

Under nitrogen gas, a solution of 1-methyl-4-(piperidin-4-yl)piperazine hydrochloride (1.6 g), 1-fluoro-2-methyl-4-nitrobenzene (1.0 g) and potassium carbonate (3.6 g) in dimethyl sulfoxide (20 mL) was heated to 100 °C and stirred overnight. The mixture was cooled to room temperature and partitioned between water and dichloromethane, and the solvent was removed from the organic phase to obtain the product, which was used directly in the next step.

### Step B: 3-methyl-4-(4-(4-methylpiperazin-1-yl)piperidin-1-yl)aniline

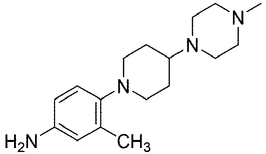

To a flask were sequentially added 1-(1-(2-methyl-4-nitrophenyl) piperidin-4-yl)- 4-methyl piperazine (200 mg), 10% Pd/C (60 mg) and methanol (10 mL), the system was sufficiently purged with hydrogen gas, and the mixture was stirred at room temperature overnight. The reaction solution was filtered through celite, and the filtrate was concentrated and purified through silica gel column chromatography (10%MeOH/DCM) to obtain the product (80 mg).

1H NMR (400 MHz, CDCl₃) δ 6.84 (d, J = 8.4 Hz, 1H), 6.55 (d, J = 2.8 Hz, 1H), 6.49 (dd, J = 8.0 Hz, 2.8 Hz, 1H), 3.38-3.52 (brs, 2H), 3.03-3.09 (m, 2H), 2.54-2.87 (m, 10H), 2.34-2.48 (m, 1H), 2.38 (s, 3H), 2.21 (s, 3H), 1.91-1.99 (m, 2H), 1.64-1.77 (m, 2H).

### Step C: 2-(6-(6-propyl-2-((3-methyl-4-(4-(4-methylpiperazin-1-yl) piperidin-1-yl) phenyl) amino)- 7H-pyrrolo[2,3-d]pyrimidin-7-yl)pyridin-2-yl)propan-2-ol

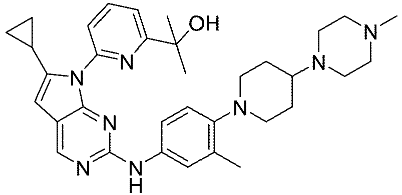

2-(6-(2-Chloro-6-cyclopropyl-7*H*-pyrrolo[2,3-d]pyrimidin-7-yl)pyridin-2-yl)propan-2-ol (33 mg), 3-methyl-4-(4-(4-methylpiperazin-1-yl)piperidin-1-yl)aniline (17 mg), Pd₂(dba)₃ (9 mg), Dave-phos (11 mg) and sodium tert-butoxide (14 mg) were mixed in toluene (5 mL), the system was sufficiently purged with nitrogen gas, and the tube was sealed and stirred at 106 °C for 5 hours. The reaction solution was concentrated to dryness and purified through preparative TLC (10%MeOH/DCM) to obtain the target product (30 mg).

¹H NMR (400 MHz, CDCl₃) δ 8.54 (s, 1H), 7.96 (t, *J* = 8.0 Hz, 1H), 7.82 (d, *J* = 8.0 Hz, 1H), 7.53 (d, *J* = 2.8 Hz, 1H), 7.42 (d, *J* = 7.6 Hz, 1H), 7.32 (dd, *J* = 8.8 Hz, 2.8 Hz, 1H), 6.99 (s, 1H), 6.91 (d, *J* = 8.8 Hz, 1H), 6.17 (s, 1H), 4.58 (s, 1H), 2.76-3.17 (m, 10H), 2.58-2.69 (m, 3H), 2.52 (s, 3H), 2.25 (s, 3H), 2.15-2.22 (m, 1H), 1.97-2.07 (m, 2H), 1.71-1.83 (m, 2H), 1.60 (s, 6H), 0.86-0.91 (m, 2H), 0.71-0.75 (m, 2H).

### Example 93

### 2-(6-(6-propyl-2-((4-(4-cyclopropylpiperazin-1-yl)phenyl)amino)-7H-pyrrolo[2,3-d]pyrimidi n-7-yl)pyridin-2-yl)propan-2-ol

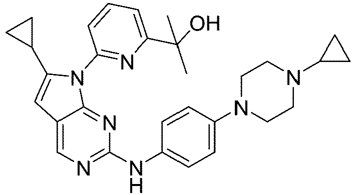

### Step A: 1-cyclopropyl-4-(4-nitrophenyl)piperazine

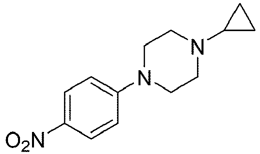

At room temperature, 1-(4-nitrophenyl)piperazine (1.04 g), (1-ethoxycyclopropoxy) trimethyl silane (3.48 g) and sodium cyanoborohydride (1.89 g) were added to methanol, the reaction solution was stirred at 60 °C overnight and evaporated to remove the solvent, and the residue was purified through preparative TLC (1%MeOH/DCM) to obtain the target product (820 mg).

¹H NMR (400 MHz, CDCl₃) δ 8.12 (d, *J* = 9.2 Hz, 2H), 6.82 (d, *J* = 9.2 Hz, 2H), 3.37-3.41 (m, 4H), 2.74-2.78 (m, 4H), 1.65-1.71 (m, 1H), 0.45-0.54 (m, 4H).

### Step B: 4-(4-cyclopropylpiperazin-1-yl)aniline

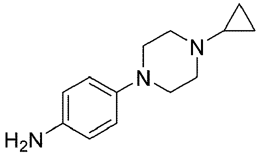

At room temperature, 1-cyclopropyl-4-(4-nitrophenyl)piperazine (200 mg) and Pd/C (20 mg) were added to methanol, and the reaction solution was stirred under an hydrogen atmosphere overnight and evaporated to remove the solvent to obtain the target product (150 mg)

¹ H NMR (400 MHz, CDCl₃) δ 6.81 (d, *J* = 8.8 Hz, 2H), 6.64 (d, *J* = 8.8 Hz, 2H), 3.27-3.56 (brs, 2H), 2.99-3.05 (m, 4H), 2.75-2.81 (m, 4H), 1.64-01.69 (m, 1H), 0.45-0.50 (m, 4H).

### Step C: 2-(6-(6- propyl- 2- ( (4- (4- cyclopropylpiperazin- 1- yl) phenyl) amino)- 7H- pyrrolo [2,3-d] pyrimidin-7-yl)pyridin-2-yl)propan-2-ol

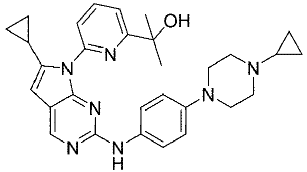

2-(6-(2-Chloro-6-cyclopropyl-7*H*-pyrrolo[2,3-d]pyrimidin-7-yl)pyridin-2-yl)propan-2-ol (40 mg), 4-(4-cyclopropylpiperazin-1-yl)aniline (17 mg), Pd₂(dba)₃ (9 mg), Dave-phos (16 mg) and sodium tert-butoxide (17 mg) were mixed in toluene (5 mL), the system was sufficiently purged with nitrogen gas, and the tube was sealed and stirred at 106 °C for 5 hours. The reaction solution was evaporated to remove the solvent, and the residue was purified through preparative TLC (10%MeOH/DCM) to obtain the target product (5 mg).

¹H NMR (400 MHz, CDCl₃) δ 8.53 (s, 1H), 7.95 (t, *J* = 8.0 Hz, 1H), 7.79 (d, *J* = 8.0 Hz, 1H), 7.51 (d, *J* = 8.8 Hz, 2H), 7.41 (d, *J* = 7.6 Hz, 1H), 6.98 (s, 1H), 6.88 (d, *J* = 9.2 Hz, 2H), 6.16 (s, 1H), 4.58 (s, 1H), 3.06-3.28 (m, 4H), 2.76-3.03 (m, 4H), 2.14-2.21 (m, 1H), 1.71-1.87 (m, 1H), 1.60 (s, 6H), 0.86-0.91 (m, 2H), 0.71-0.75 (m, 2H), 0.45-0.69 (m, 4H).

### Example 94

### ((6-(6-propyl-2-((4-(4-cyclopropylpiperazin-1-yl)phenyl)amino)-7H-pyrrolo[2,3-d]pyrimidin -7-yl)pyridin-2-yl)imino)dimethyl-λ⁶-sulfanone

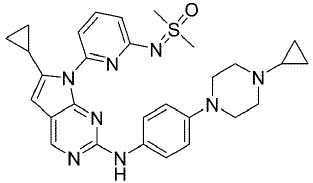

((6-(2-Chloro-6-cyclopropyl-7*H*-pyrrolo[2,3-d]pyrimidin-7-yl)pyridin-2-yl)imino)dimethyl-λ ⁶-sulfanone (40 mg), 4-(4-cyclopropylpiperazin-1-yl)aniline (14 mg), Pd₂(dba)₃ (9 mg), Dave-phos (12 mg) and sodium tert-butoxide (15 mg) were mixed in toluene (5 mL), the system was sufficiently purged with nitrogen gas, and the tube was sealed and stirred at 106 °C for 5 hours. The reaction solution was evaporated to remove the solvent, and the residue was purified through preparative TLC (10%MeOH/DCM) to obtain the target product (5 mg).

¹H NMR (400 MHz, CDCl₃) δ 8.49 (s, 1H), 7.74 (t, *J* = 8.0 Hz, 1H), 7.49 (d, *J* = 8.8 Hz, 2H), 7.20 (d, *J* = 8.0 Hz, 1H), 7.07 (s, 1H), 6.86 (d, *J* = 8.8 Hz, 2H), 6.82 (d, *J* = 8.0 Hz, 1H), 6.06 (s, 1H), 3.29 (s, 6H), 3.14-3.26 (m, 4H), 2.83-3.02 (m, 4H), 2.29-2.38 (m, 1H), 1.75-1.93 (m, 1H), 0.88-0.93 (m, 2H), 0.66-0.69 (m, 2H), 0.51-0.65 (m, 4H).

### Example 95

### 2-(6-(6-propyl-2-((3-fluoro-2-methyl-4-(4-methylpiperazin-1-yl)phenyl)amino)-7H-pyrrolo[2, 3-d]pyrimidin-7-yl)pyridin-2-yl)propan-2-ol

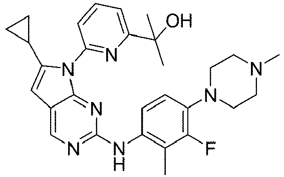

### Step A: 1-(2-fluoro-3-methyl-4-nitrophenyl)-4-methylpiperazine

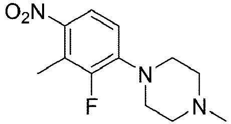

At room temperature, 1,2-difluoro-3-methyl-4-nitrobenzene (1.00 g), 1-methylpiperazine (580 mg) and potassium carbonate (2.4 g) were added to dimethyl sulfoxide (10 mL), the reaction solution was stirred at 120 °C overnight, water (50 mL) was added, and the mixture was filtered to obtain the target product as a solid (1.25 g).

¹H NMR (400 MHz, CDCl₃) δ 7.85 (dd, *J* = 8.8 Hz, 2.0 Hz, 1H), 6.80 (t, *J* = 8.8 Hz, 1H), 3.25-3.28 (m, 4H), 2.58-2.60 (m, 4H), 2.51 (d, *J* = 3.2 Hz, 3H), 2.36 (s, 3H).

### Step B: 3-fluoro-2-methyl-4-(4-methylpiperazin-1-yl)aniline

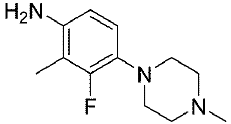

At room temperature, 1-(2-fluoro-3-methyl-4-nitrophenyl)-4-methylpiperazine (200 mg) and Pd/C (20 mg) were added to methanol, and the reaction solution was stirred under an hydrogen atmosphere overnight and evaporated to remove the solvent to obtain the target product (160 mg)

¹H NMR (400 MHz, CDCl₃) δ 6.71 (t, *J* = 8.8 Hz, 1H), 6.41 (dd, *J* = 8.4 Hz, 1.2 Hz, 1H), 3.48 (s, 2H), 2.98-3.04 (m, 4H), 2.56-2.63 (m, 4H), 2.35 (s, 3H), 2.07 (d, *J* = 2.0 Hz, 3H).

### Step C: 2-(6-(6-propyl-2-((3-fluoro-2- methyl- 4- (4- methylpiperazin- 1- yl) phenyl) amino)-7H- pyrrolo[2,3-d]pyrimidin-7-yl)pyridin-2-yl)propan-2-ol

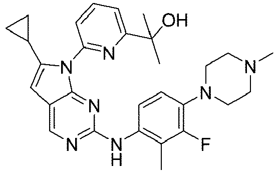

2-(6-(2-Chloro-6-cyclopropyl-7*H*-pyrrolo[2,3-d]pyrimidin-7-yl)pyridin-2-yl)propan-2-ol (50 mg), 3-fluoro-2-methyl-4-(4-methylpiperazin-1-yl)aniline (20 mg), Pd₂(dba)₃ (9 mg), Dave-phos (16 mg) and sodium tert-butoxide (21 mg) were mixed in toluene (5 mL), the system was sufficiently purged with nitrogen gas, and the tube was sealed and stirred at 106 °C for 5 hours. The reaction solution was evaporated to remove the solvent, and the residue was purified through preparative TLC (10%MeOH/DCM) to obtain the target product (10 mg).

¹H NMR (400 MHz, CDCl₃) δ 8.56 (s, 1H), 7.99 (t, *J* = 8.0 Hz, 1H), 7.88 (d, *J* = 8.4 Hz, 1H), 7.71 (d, *J* = 7.6 Hz, 1H), 7.46 (d, *J* = 7.6 Hz, 1H), 6.82-6.95 (brs, 1H), 6.80 (t, *J* = 9.2 Hz, 1H), 6.18 (s, 1H), 4.57 (s, 1H), 3.01-3.63 (m, 8H), 2.84 (s, 3H), 2.21 (d, *J* = 2.0 Hz, 3H), 2.12-2.19 (m, 1H), 1.60 (s, 6H), 0.87-0.92 (m, 2H), 0.72-0.76 (m, 2H).

### Example 96

### ((6-(6-propyl-2-((3-fluoro-2-methyl-4-(4-methylpiperazin-1-yl)phenyl)amino)-7H-pyrrolo[2, 3-d]pyrimidin-7-yl)pyridin-2-yl)imino)dimethyl-λ⁶-sulfanone

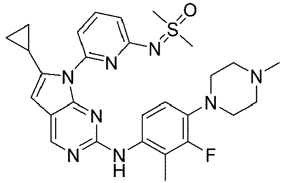

((6-(2-Chloro-6-cyclopropyl-7*H*-pyrrolo[2,3-d]pyrimidin-7-yl)pyridin-2-yl)imino)dimethyl-λ ⁶-sulfanone (50 mg), 3-fluoro-2-methyl-4-(4-methylpiperazin-1-yl)aniline (18 mg), Pd₂(dba)₃ (9 mg), Dave-phos (16 mg) and sodium tert-butoxide (19 mg) were mixed in toluene (5 mL), the system was sufficiently purged with nitrogen gas, and the tube was sealed and stirred at 106 °C for 5 hours. The reaction solution was evaporated to remove the solvent, and the residue was purified through preparative TLC (10%MeOH/DCM) to obtain the target product (8 mg).

¹H NMR (400 MHz, CDCl₃) δ 8.51 (s, 1H), 7.89 (d, *J* = 8.8 Hz, 1H), 7.76 (t, *J* = 8.0 Hz, 1H), 7.17 (d, *J* = 8.0 Hz, 1H), 6.76-6.86 (m, 3H), 6.08 (s, 1H), 2.94-3.72 (m, 14H), 2.82 (s, 3H), 2.33-2.40 (m, 1H), 2.20 (d, *J* = 2.0 Hz, 3H), 0.89-0.94 (m, 2H), 0.66-0.70 (m, 2H).

### Example 97

### 2-(6-(6-cyclopropyl-5-fluoro-2-((3-methyl-4-(4-(4-methylpiperazin-1-yl)piperidin-1-yl)phen yl)amino)-7H-pyrrolo[2,3-d]pyrimidin-7-yl)pyridin-2-yl)propan-2-ol

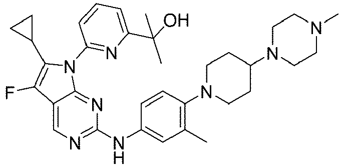

2-(6-(2-Chloro-6-cyclopropyl-5-fluoro-7*H*-pyrrolo[2,3-d]pyrimidin-7-yl)pyridin-2-yl)propan -2-ol (35 mg), 3-methyl-4-(4-(4-methylpiperazin-1-yl)piperidin-1-yl)aniline (17 mg), Pd₂(dba)₃ (9 mg), Dave-phos (11 mg) and sodium tert-butoxide (14 mg) were mixed in toluene (5 mL), the system was sufficiently purged with nitrogen gas, and the tube was sealed and stirred at 106 °C for 5 hours. The reaction solution was evaporated to remove the solvent, and the residue was purified through preparative TLC (10%MeOH/DCM) to obtain the target product (5 mg).

¹H NMR (400 MHz, CDCl₃) δ 8.61 (s, 1H), 7.94 (t, *J* = 8.0 Hz, 1H), 7.79 (d, *J* = 8.0 Hz, 1H), 7.53 (d, *J* = 2.0 Hz, 1H), 7.43 (d, *J* = 7.6 Hz, 1H), 7.31 (dd, *J* = 8.8 Hz, 2.0 Hz, 1H), 7.11 (s, 1H), 6.91 (d, *J* = 8.8 Hz, 1H), 4.39 (s, 1H), 2.91-3.45 (m, 10H), 2.59-2.72 (m, 4H), 2.25 (s, 3H), 2.02-2.13 (m, 3H), 1.76-1.89 (m, 4H), 1.60 (s, 6H), 0.81-0.88 (m, 2H), 0.73-0.77 (m, 2H).

### Example 98

### ((6-(6-cyclopropyl-2-((4-(4-(3-hydroxy-3-methylazetidin-1-yl)piperazin-1-yl)phenyl)amino)-7H-pyrrolo[2,3-d]pyrimidin-7-yl)pyridin-2-yl)imino)dimethyl-λ⁶-sulfanone

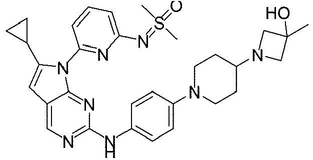

### Step A: 1-(4-nitrophenyl)piperidin-4-one

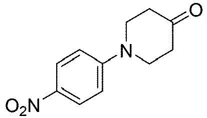

A suspension of 4-fluoronitrobenzene (3.54 g), 4-piperidone hydrochloride (3.50 g) and potassium carbonate (13.9 g) in dimethyl sulfoxide (50 mL) was heated to 120 °C and stirred for 3 hours. The reaction solution was cooled to room temperature and poured into 150 mL water to obtain a suspension, which was filtered and washed, and the solid thus produced was dried to obtain the product (2.60 g).

¹H NMR (400 MHz CDCl₃) δ 8.16 (d, *J* = 9.6 Hz 2H), 6.84 (d, *J* = 9.6 Hz, 2H), 3.81 (t, *J* = 7.6 Hz, 4H), 2.62 (t, J = 7.6 Hz, 4H).

### Step B: 3-methyl-1-(1-(4-nitrophenyl)piperidin-4-yl)azetidin-2-ol

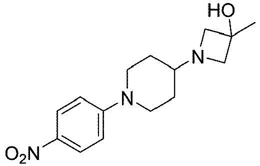

A solution of 1-(4-nitrophenyl)piperidin-4-one (200 mg), 3-methylazetidin-3-ol hydrochloride (112 mg) and 3 drops of acetic acid in dichloromethane (10 mL) was stirred at room temperature for 30 minutes, and to the reaction solution was portionwise added sodium triacetoxyborohydride (772 mg). The reaction solution was stirred at room temperature overnight, quenched with water, and washed with an aqueous solution of saturated sodium bicarbonate, the organic phase was evaporated to dryness to obtain the crude product, which was purified through column chromatography (100%AcOEt) to obtain the product (210 mg).

¹H NMR (400 MHz CDCl₃) δ 8.10 (d, *J* = 9.6 Hz, 2H), 6.79 (d, *J* = 9.6 Hz, 2H), 5.33-5.36 (brs, 1H), 3.80-3.85 (m, 2H), 3.31 (d, *J* = 8.0 Hz, 2H), 3.03-3.08 (m, 4H), 2.29-2.37 (m, 1H), 1.77-1.81 (m, 2H), 1.51 (s, 3H), 1.26-1.29 (m, 2H).

### Step C: 3-methyl-1-(1-(4-aminophenyl)piperidin-4-yl)azetidin-2-ol

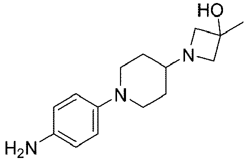

To a flask were sequentially added 3-methyl-1-(1-(4-nitrophenyl)piperidin-4-yl)azetidin-2-ol (210 mg), 10% Pd/C (10 mg) and methanol (10 mL), the system was sufficiently purged with hydrogen gas, and the mixture was stirred at room temperature overnight. The reaction solution was filtered through celite, and the filtrate was concentrated and purified through silica gel column chromatography (100%AcOEt) to obtain the product (150 mg).

¹H NMR (400 MHz, CDCl₃) δ 6.81 (d, *J* = 8.0 Hz 2H), 6.64 (d, *J* = 8.0 Hz, 2H), 5.35 (s, 1H), 3.71 (s, 2H), 3.29-3.42 (m, 4H), 3.06(d, *J* = 8.0 Hz, 2H), 2.60-2.66 (m, 2H), 2.20-2.24 (m, 1H), 1.50 (s, 3H), 1.40-1.46 (m, 2H), 1.23-1.26 (m, 2H).

### Step D: ((6-(6-propyl-2-((4-(4- (3- hydroxy- 3- methylazetidin- 1- yl) piperidin- 1- yl) phenyl) amino)- 7H-pyrrolo[2,3-d]pyrimidin-7-yl)pyridin-2-yl)imino)dimethyl-λ⁶-sulfanone

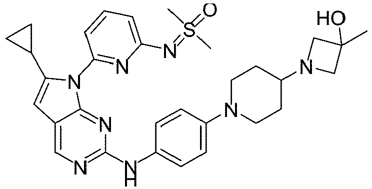

((6-(2-Chloro-6-cyclopropyl-7*H*-pyrrolo[2,3-d]pyrimidin-7-yl)pyridin-2-yl)imino)dimethyl-λ ⁶-sulfanone (258 mg), 3-methyl-1-(1-(4-aminophenyl)piperidin-4-yl)azetidin-2-ol (130 mg), Pd₂(dba)₃ (65 mg), Dave-phos (84 mg) and sodium tert-butoxide (102 mg) were mixed in toluene (5 mL), the system was sufficiently purged with nitrogen gas, and the tube was sealed, heated to 100 °C and stirred overnight. The mixture was cooled to room temperature and partitioned between water and dichloromethane, the solvent was removed from the organic phase, and the residue was purified through preparative TLC (10%MeOH/DCM) to obtain the product (25 mg).

¹H NMR (400 MHz, CDCl₃) δ 8.48 (s, 1H), 7.72 (t, *J* = 8.0 Hz, 1H), 7.44 (d, *J* = 8.0 Hz, 2H), 7.16 (d, *J* = 8.0 Hz, 2H), 7.16-7.19 (brs, 1H), 6.80 (t, *J* = 8.0 Hz, 2H), 6.76 (s, 1H), 4.10-4.13 (m, 2H), 3.90-3.92 (brs, 1H), 3.55-3.58 (m, 2H), 3.27 (s, 6H), 3.08-3.09 (brs, 1H), 2.61-2.67 (m, 4H), 2.25-2.29 (m, 1H), 1.85-1.96 (m, 4H), 1.58 (s, 3H), 0.88-0.89 (m, 2H), 0.66-0.67 (m, 2H).

### Example 99

### 1-(4-((6-cyclopropyl-7-(6-((dimethyl(oxo)-λ⁶-sulfanylidene)amino)pyridin-2-yl)-7H-pyrrolo[ 2,3-d]pyrimidin-2-yl)amino)phenyl)piperidin-4-carboxamide

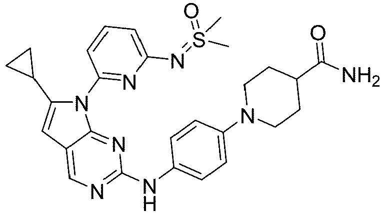

### Step A: 1-(4-nitrophenyl)piperidin-4-carboxamide

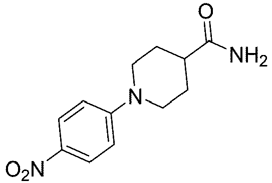

According to the method of step A of example 98, 4-fluoronitrobenzene and piperidin-4-carboxamide were used as the starting material and reacted to obtain the product (10 g).

¹H NMR (400 MHz CDCl₃) δ 8.11 (d, *J* = 8.0 Hz, 2H), 6.82 (d, *J* = 8.0 Hz, 2H), 5.29-5.33 (brs, 2H), 3.96-3.99 (m, 2H), 3.04 (t, *J* = 12.0 Hz, 2H), 2.41-2.47 (m, 1H), 1.99-2.02 (m, 2H), 1.83-1.90 (m, 2H).

### Step B: 1-(4-aminophenyl)piperidin-4-carboxamide

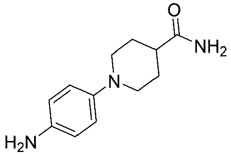

According to the method of step C of example 98, 1-(4-nitrophenyl)piperidin-4-carboxamide was used as the starting material to obtain the product (0.9 g).

¹H NMR (400 MHz CDCl₃) δ7.13-7.16 (brs, 2H), 6.79 (d, *J* = 8.0 Hz, 2H), 6.58 (d, *J* = 8.0 Hz, 2H), 5.29-5.33 (brs, 2H), 3.59-3.63 (m, 2H), 2.88-2.96 (m, 2H), 2.41-2.47 (m, 1H), 2.00-2.08 (m, 2H), 1.83-1.90 (m, 2H).

### Step C: 1-(4-((6-cyclopropyl-7-(6-((dimethyl(oxo)-λ⁶-sulfanylidene) amino) pyridin-2-yl)-7H-pyrrolo[2,3-d]pyrimidin-2-yl)amino)phenyl)piperidin-4-carboxamide

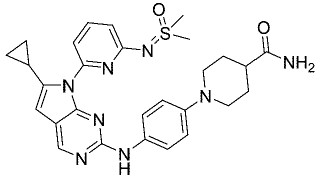

According to the method of step D of example 98, ((6-(2- chloro- 6- cyclopropyl- 7H-pyrrolo[2,3-d]pyrimidin-7-yl)pyridin- 2- yl) imino) dimethyl-λ⁶-sulfanone and 1- (4- aminophenyl) piperidin- 4-carboxamide were used as the main starting material to obtain the product (15 mg).

¹H NMR (400 MHz, CDCl₃) δ 8.47 (s, 1H), 7.73 (t, *J =* 8.0 Hz, 1H), 7.48 (d, *J =* 8.0 Hz, 2H), 7.19 (d, *J* = 8.0 Hz, 1H), 6.87(d, *J* = 8.0 Hz, 2H), 6.81(d, *J* = 8.0 Hz, 1H), 6.05 (s, 1H), 5.33-5.37 (brs, 2H), 3.59-3.62(d, *J* = 12.0 Hz, 2H), 3.28 (s, 6H), 2.66-2.72 (m, 2H), 2.19-2.32 (m, 2H), 1.92-2.00 (m, 1H), 1.77-1.90 (m, 4H), 0.85-0.92 (m, 2H), 0.65-0.67 (m, 2H).

### Example 100

### 1-(4-((6-cyclopropyl-7-(6-(2-hydroxypropan-2-yl)pyridin-2-yl)-7H-pyrrolo[2,3-d]pyrimidin-2-yl)amino)phenyl)piperidin-4-carboxamide

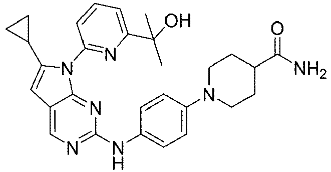

To a round-bottom flask charged with toluene (5 mL) were added 2- (6- (2- chloro- 6-cyclopropyl-7H-pyrrolo[2,3-d]pyrimidin-7-yl)pyridin-2-yl)propan-2-ol (30 mg), 1-(4-amino phenyl) piperidin-4-carboxamide (13 mg), Pd₂(dba)₃ (9 mg), Dave-phos (11 mg) and sodium tert-butoxide (14 mg). Under nitrogen gas protection, the mixture was reacted at 106 °C for 6 hours. The reaction solution was evaporated to remove the solvent, and the residue was purified through preparative TLC (10%MeOH/DCM) to obtain the target product (10 mg).

¹H NMR (400 MHz, CDCl₃), 8.48 (s, 1H), 7.97 (t, *J* = 8.0 Hz, 1H), 7.76 (d, *J* = 8.0 Hz, 1H), 7.50-7.56 (m, 3H), 7.45 (d, *J* = 8.0 Hz, 1H), 6.88-7.01 (m, 2H), 6.18 (s, 1H), 5.51-5.57 (brs, 1H), 5.30-5.37 (brs, 1H), 4.50 (s, 1H, ), 3.61-3.68 (m, 2H), 2.70-2.82 (m, 2H), 2.27-2.38 (m, 1H), 2.12-2.18 (m, 1H), 1.90-2.04 (m, 4H), 1.60 (s, 6H), 0.89-0.93 (m, 2H), 0.73-0.77 (m, 2H).

### Example 101

### ((6-(2-((4-(3,8-diazabicyclo[3.2.1]octan-3-yl)-3-fluorophenyl)amino)-6-cyclopropyl-7H-pyrr olo[2,3-d]pyrimidin-7-yl)pyridin-2-yl)imino)dimethyl-λ⁶-sulfanone

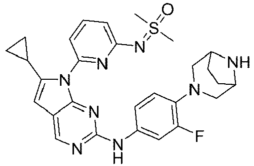

### Step A: tert-butyl 3-(2-fluoro-4-nitrophenyl)-3,8-diazabicyclo[3.2.1]octan-8-carboxylate

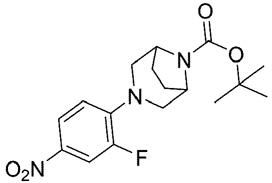

According to the method of step A of example 98, 1,2-difluoro-4-nitrobenzene and tert-butyl 3,8-diazabicyclo[3.2.1]octan-8-carboxylate were used as the main starting material to obtain the product (110 mg)

¹H NMR (400 MHz, CDCl₃) δ 7.96(dd, *J* = 9.2 Hz, 2.8 Hz, 1H), 7.89(dd, *J* = 13.2 Hz, 2.4 Hz, 1H), 6.84 (t, *J* = 8.8 Hz, 1H), 4.09-4.18 (m, 2H), 3.37-3.41 (m, 2H), 3.01-3.09 (m, 2H), 2.88-2.96 (m, 4H), 1.48 (s, 9H).

### Step B: tert-butyl 3-(2-fluoro-4-aminophenyl)-3,8-diazabicyclo[3.2.1]octan-8-carboxylate

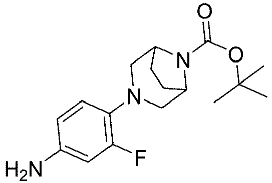

According to the method of step C of example 98, tert-butyl 3-(2- fluoro- 4- amino phenyl)-3,8-diazabicyclo[3.2.1]octan-8-carboxylate was used as the starting material to obtain the product (100 mg).

¹H NMR (400 MHz, CDCl₃) δ 7.44-7.58 (m, 2H), 6.64 (t, *J* = 8.8 Hz, 1H), 5.88 (s, 2H), 4.09-4.18 (m, 2H), 3.37-3.41 (m, 2H), 3.01-3.09 (m, 2H), 2.88-2.96 (m, 4H), 1.47 (s, 9H).

### Step C: tert-butyl 3- (4- ( (6-cyclopropyl- 7- (6- ( (dimethyl (oxo)-λ⁶-sulfanylidene) amino) pyridin-2-yl)-7H-pyrrolo[2,3-d]pyridin-2-yl)amino)-2-fluorophenyl)-3,8-diazabicyclo[3.2.1]octan-8-carboxylate

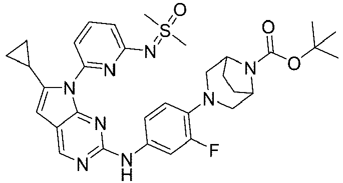

According to the method of step D of example 98, ((6-(2-chloro-6-cyclopropyl- 7*H*- pyrrolo [2,3-d]pyrimidin- 7- yl) pyridin- 2- yl) imino) dimethyl-λ⁶-sulfanone and tert-butyl 3-(2-fluoro-4-amino phenyl)-3,8-diazabicyclo[3.2.1]octan-8-carboxylate were used as the starting material and reacted to obtain the product (23 mg).

¹H NMR (400 MHz, CDCl₃) δ 8.48 (s, 1H), 7.72 (t, *J* = 8.0 Hz, 1H), 7.44-7.58 (m, 2H), 7.22(d, *J* = 8.0 Hz, 1H), 6.80 (m, 1H), 7.00 (s, 1H), 6.60 (t, *J* = 8.8 Hz, 1H), 6.10 (s, 1H), 4.09-4.18 (m, 2H), 3.37-3.41 (m, 2H), 3.25 (s, 6H), 3.01-3.09 (m, 2H), 2.88-2.96 (m, 4H), 2.34-2.37 (m, 1H), 1.47 (s, 9H), 0.87-0.92 (m, 2H), 0.65-0.69 (m, 2H).

### Step D: ( (6- (2- ( (4- (3,8- diazabicyclo [3.2.1] octan- 3- yl)- 3- fluorophenyl) amino)- 6-cyclopropyl- 7H-pyrrolo[2,3-d]pyrimidin-7-yl)pyridin-2-yl)imino)dimethyl-λ⁶-sulfanone

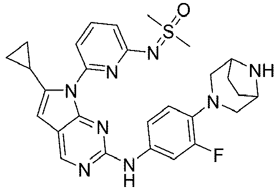

Tert-butyl 3-(4-((6-cyclopropyl-7- (6- ( (dimethyl (oxo)-λ⁶-sulfanylidene) amino) pyridin- 2-yl)-7*H*-pyrrolo[2,3-*d*]pyridin-2-yl)amino)-2-fluorophenyl)-3,8-diazabicyclo[3.2.1]octan-8-carboxy late (23 mg) was dissolved in a solution of 4 mol/L hydrochloric acid in 1,4-dioxane (1 mL), and the mixture was stirred at room temperature for 1 hour, evaporated to remove the solvent, and purified through preparative TLC (DCM/MeOH=10: 1) to obtain the product (10 mg).

¹H NMR (400 MHz, CDCl₃) δ 8.48 (s, 1H), 7.72 (t, *J* = 8.0 Hz, 1H), 7.40-7.55 (m, 2H), 7.20(d, *J* = 8.0 Hz, 1H), 6.81 (m, 2H), 6.58-6.62 (m, 1H), 6.07 (s, 1H), 4.05-4.18 (m, 2H), 3.33-3.38 (m, 2H), 3.21 (s, 6H), 2.99-3.11 (m, 2H), 2.70-2.85 (m, 4H), 2.31-2.36 (m, 1H), 0.87-0.92 (m, 2H), 0.65-0.69 (m, 2H).

### Example 102

### ((6-(2-((4-(8-methyl-3,8-diazabicyclo[3.2.1]octan-3-yl)-3-fluorophenyl)amino)-6-cyclopropy 1-7H-pyrrolo[2,3-d]pyrimidin-7-yl)pyridin-2-yl)imino)dimethyl-λ⁶-sulfanone

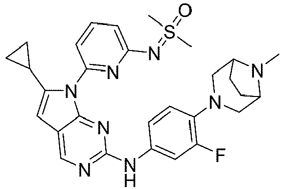

According to the method of step B of example 98, ((6-(2-((4-(3,8-diazabicyclo [3.2.1] octan-3-yl)-3-fluorophenyl)amino)-6-cyclopropyl-7*H*-pyrrolo[2,3-d]pyrimidin-7-yl)pyridin-2-yl)imino)d imethyl-λ⁶-sulfanone and aqueous solution of 30% formaldehyde were used as the main starting material to obtain the product (20 mg).

¹H NMR (400 MHz CDCl₃) δ 8.57 (s, 1H), 7.83 (dd, *J* = 15.2 Hz, 1.8 Hz, 1H), 7.77 (t, *J* = 8.0 Hz, 1H), 7.49-7.57 (brs, 1H), 7.22(d, t, *J* = 8.0 Hz, 1H), 7.02(dd, *J* = 8.8 Hz, 1.8 Hz, 1H), 6.85 (m, 2H), 6.08 (s, 1H), 3.76-3.80 (m, 4H), 3.32 (s, 6H), 3.14-3.16 (m, 2H), 2.80 (s, 3H), 2.35-2.38 (m, 1H), 2.19-2.33 (m, 4H), 0.88-0.93 (m, 2H), 0.65-0.69 (m, 2H).

### Example 103

### (S)-2-(4-(4-((6-cyclopropyl-7-(6-((dimethyl(oxo)-λ⁶-sulfanylidene)amino)pyridin-2-yl)-7H-p yrrolo[2,3-d]pyrimidin-2-yl)amino)phenyl)-1-methylpiperazine-2-yl) acetonitrile

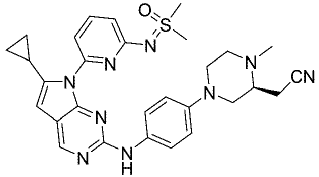

### Step A: (S)-2-(4-(4-nitrophenyl)piperazine-2-yl) acetonitrile

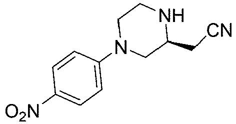

According to the method of step A of example 98, 4-fluoronitrobenzene and (S)-2-(piperazine-2-yl) acetonitrile were used as the main starting material to obtain the product (74 mg).

¹H NMR (400 MHz, CDCl₃) δ8.11 (d, *J* = 8.8 Hz, 2H), 6.79 (d, *J* = 8.8 Hz, 2H), 3.66-3.73 (m, 4H), 2.87-2.90 (dd, *J* = 8.4 Hz, 3.2 Hz, 2H), 2.45-2.55 (m, 3H).

### Step B: (S)-2-(1-methyl-4-(4-nitrophenyl)piperazine-2-yl) acetonitrile

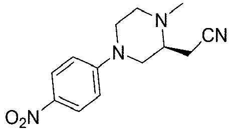

According to the method of step B of example 98, (S)-2-(4-(4-nitrophenyl)piperazine-2-yl) acetonitrile and aqueous solution of 30% formaldehyde were used as the main starting material to obtain the product (60 mg).

¹H NMR (400 MHz, CDCl₃) δ8.13 (d, *J* = 8.4 Hz, 2H), 6.80 (d, *J* = 8.4 Hz, 2H), 3.68-3.75 (m, 4H), 2.89-2.92 (dd, *J* = 8.4 Hz, 3.2 Hz, 2H), 2.48-2.56 (m, 3H), 2.36 (s, 3H).

### Step C: (S)-2-(1-methyl-4-(4-aminophenyl)piperazine-2-yl) acetonitrile

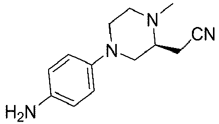

According to the method of step C of example 98, (S)- 2- (1- methyl- 4- (4- nitrophenyl) piperazine- 2-yl) acetonitrile was used as the starting material to obtain the product (50 mg).

¹H NMR (400 MHz, CDCl₃) δ7.54 (d, *J* = 8.8 Hz, 2H), 6.89 (d, *J* = 8.8 Hz, 2H), 5.06 (s, 2H), 3.28-3.30 (m, 4H), 2.85-2.87 (dd, *J* = 8.4 Hz, 3.2 Hz, 2H), 2.57-2.71 (m, 3H), 2.38 (s, 3H).

### Step D: (S)-2-(4-(4-((6-cyclopropyl-7-(6-((dimethyl(oxo)-λ⁶-sulfanylidene) amino) pyridin-2- yl)-7H-pyrrolo[2,3-d]pyrimidin-2-yl)amino)phenyl)-1-methylpiperazine-2-yl) acetonitrile

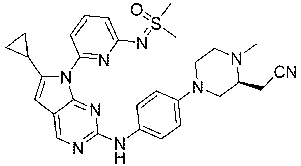

According to the method of step D of example 98, ((6-(2-chloro-6-cyclopropyl-7*H*-pyrrolo [2,3-d]pyrimidin-7-yl)pyridin-2-yl)imino)dimethyl-λ⁶-sulfanone and (S)-2- (1- methyl- 4- (4-nitrophenyl) piperazine-2-yl) acetonitrile were used as the starting material to obtain the product (10 mg).

¹H NMR (400 MHz, CDCl₃) δ 8.50 (s, 1H), 7.74 (t, *J* = 8.0 Hz, 1H), 7.51 (d, *J* = 8.8 Hz, 2H), 7.20 (d, *J* = 8.0 Hz, 1H), 7.00 (s, 1H), 6.85 (d, *J* = 8.8 Hz, 2H), 6.82 (d, *J* = 8.0 Hz, 1H), 6.06 (s, 1H), 3.29-3.37 (m, 2H), 3.25 (s, 6H), 3.20-3.24 (m, 2H), 2.82-2.85 (dd, *J* = 8.4 Hz, 3.2 Hz, 2H), 2.57-2.71 (m, 3H), 2.39 (s, 3H), 2.34-2.37 (m, 1H), 0.87-0.92 (m, 2H), 0.65-0.69 (m, 2H).

### Example 104

### 2-(6-(6-propyl-2-((4-(2-methyl-2,7-diazaspiro[3.5]nonan-7-yl)phenyl)amino)-7H-pyrrolo[2,3 -d]pyrimidin-7-yl)pyridin-2-yl)propan-2-ol

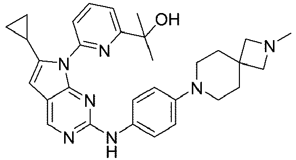

### Step A: tert-butyl 7-(4-nitrophenyl)-2,7-diazaspiro[3,4]nonan-2-carboxylate

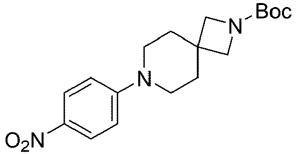

1-Fluoro-4-nitrobenzene (282 mg), tert-butyl 2,7-diazaspiro[3.5]nonan-2-carboxylate (452 mg) and potassium carbonate (552 mg) were added to dimethyl sulfoxide (10 mL), the mixture was heated to 120 °C, reacted for 3 hours, and cooled to room temperature, water (100 mL) was added, a solid was precipitated, and the mixture was filtered to obtain the product (600 mg).

¹H NMR (400 MHz, CDCl₃), 8.11 (d, *J* = 9.2 Hz, 2H), 6.82 (d, *J* = 9.2 Hz, 2H), 3.07 (s, 4H), 3.38-3.41 (m, 4H), 1.85-1.88 (m, 4H), 1.45 (s, 9H).

### Step B: 7-(4-nitrophenyl)-2,7-diazaspiro[3.5]nonane

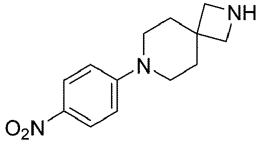

To a solution of HCl in methanol (3 M) was added tert-butyl 7- (4- nitrophenyl)- 2,7-diazaspiro [3,4]nonan-2-carboxylate (500 mg), and the mixture was stirred at room temperature overnight and evaporated to dryness to obtain the product as a hydrochloride salt (350 mg).

### Step C: 2-methyl-7-(4-nitrophenyl)-2,7-diazaspiro[3.5]nonane

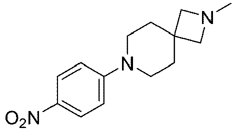

7-(4-Nitrophenyl)-2,7-diazaspiro[3.5]nonane (490 mg) and 37% formaldehyde aqueous solution (0.5 mL) were added to dichloromethane (10 mL), the mixture was stirred for 30 minutes,, sodium triacetoxyborohydride (1.6 g) was added, the mixture was stirred overnight, 1 N sodium hydroxide aqueous solution was used to adjust the pH to 10, and the mixture was extracted with dichloromethane, dried over anhydrous sodium sulfate, and evaporated to remove the solvent to obtain the product (450 mg).

### Step D: 4-(2-methyl-2,7-diazaspiro[3.5]nonan-7-yl)aniline

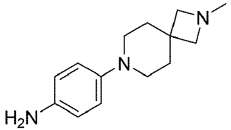

2-Methyl-7-(4-nitrophenyl)-2,7-diazaspiro[3.5]nonane (100 mg) and Pd/C (10 mg) were added to methanol (5 mL), the reaction solution was stirred under a hydrogen atmosphere overnight, filtered through celite and washed with dichloromethane, and the solvent was removed from the filtrate to obtain the product (50 mg).

¹H NMR (400 MHz, CDCl₃), 6.80 (d, *J* = 9.2 Hz, 2H), 6.63 (d, *J* = 9.2 Hz, 2H), 3.32-3.48 (brs, 2H), 3.08 (s, 4H), 2.91-2.94 (m, 4H), 2.37 (s, 3H), 1.86-1.89 (m, 4H).

### Step E: 2-(6-(6-propyl-2-((4-(2-methyl-2,7-diazaspiro[3.5]nonan-7-yl) phenyl) amino)- 7H-pyrrolo[2,3-d]pyrimidin-7-yl)pyridin-2-yl)propan-2-ol

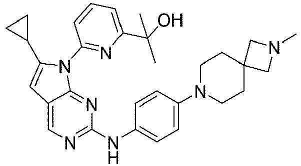

To a round-bottom flask charged with toluene (3 mL) were added 2-(6-(2-chloro- 6-cyclopropyl-7H-pyrrolo[2,3-d] pyrimidin-7-yl) pyridin-2-yl) propan-2-ol (50 mg), 4-(2- methyl-2,7- diazaspiro[3.5]nonan-7-yl)aniline (22 mg), Pd₂(dba)₃ (14 mg), Dave-phos (18 mg) and sodium tert-butoxide (22 mg), heated to 106 °C, and stirred for 6 hours. The reaction solution was evaporated to remove the solvent, and the residue was purified through preparative TLC (10%MeOH/DCM) to obtain the target product (20 mg).

¹H NMR (400 MHz, CDCl₃), 8.52 (s, 1H), 7.94 (t, *J =* 8.0 Hz, 1H), 7.77 (d, *J =* 8.0 Hz, 1H), 7.50 (d, *J* = 9.2 Hz, 2H), 7.42 (d, *J* = 8.0 Hz, 1H), 7.02 (s, 1H), 6.84 (d, *J* = 8.8 Hz, 2H), 6.15 (s, 1H), 4.63 (s, 1H), 3.70 (s, 4H), 2.99-3.02 (m, 4H), 2.77 (s, 3H), 2.12-2.18 (m, 1H, ), 2.04-2.07 (m, 4H), 1.59 (s, 6H), 0.85-0.90 (m, 2H), 0.70-0.74 (m, 2H).

### Example 105

### 4-(4-((6-cyclopropyl-7-(6-(2-hydroxypropan-2-yl)pyridin-2-yl)-7H-pyrrolo[2,3-d]pyrimidin-2-yl)amino)phenyl)-1-imino-1λ⁶-thiomorpholine 1-oxide

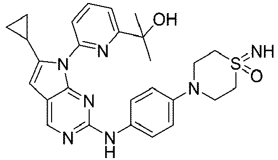

### Step A: 1-imino-4-(4-nitrophenyl)-1λ⁶-thiomorpholine 1-oxide

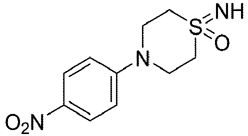

### 4-(4-Nitrophenyl)thiomorpholine (1.3 g), (diacetoxyiodo)benzene (11.3 g) and ammonium carbamate (3.6 g) were added to methanol (50 mL), the mixture was stirred for 5 hours, evaporated to remove the solvent, and purified through silica gel column chromatography (10%MeOH/DCM) to obtain the product (380 mg).

¹H NMR (400 MHz, CDCl₃), 8.18 (d, *J* = 8.0 Hz, 2H), 6.88 (d, *J* = 8.0 Hz, 2H), 3.97-4.09 (m, 4H), 3.13-3.15 (m, 4H), 2.65 (s, 1H).

### Step B: benzyl (4-(4-nitrophenyl)-1-oxido-1λ⁶-thiomorpholin-1-ylidene)carbamate

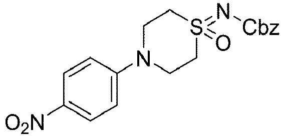

1-Imino-4-(4-nitrophenyl)-1λ⁶-thiomorpholine 1-oxide (310 mg), sodium bicarbonate (202 mg) and benzyl chloroformate (410 mg) were added to tetrahydrofuran (10 mL), and the mixture was stirred at room temperature overnight, filtered through celite, washed with dichloromethane, evaporated to remove the solvent, and purified through silica gel column chromatography (10%EA/DCM) to obtain the product (360 mg).

¹H NMR (400 MHz, CDCl₃), 8.19 (d, *J* = 8.0 Hz, 2H), 7.25-7.39 (m, 5H), 6.89 (d, *J* = 8.8 Hz, 2H), 5.13 (s, 2H), 4.10-4.14 (m, 2H), 3.88-3.94 (m, 2H), 3.69-3.74 (m, 2H), 3.31-3.37 (m, 2H).

### Step C: benzyl (4-(4-aminophenyl)-1-oxido-1λ⁶-thiomorpholin-1-ylidene)carbamate

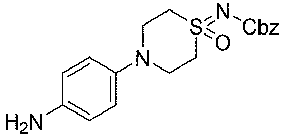

Benzyl (4-(4-nitrophenyl)-1-oxido-1λ⁶-thiomorpholin-1-ylidene)carbamate (360 mg) and Pd/C (40 mg) was added to methanol (5 mL), the reaction solution was stirred overnight, filtered through celite and washed with dichloromethane, and the solvent was removed from the filtrate to obtain the product (210 mg).

¹H NMR (400 MHz, CDCl₃), 7.28-7.40 (m, 5H), 6.79 (d, *J* = 8.8 Hz, 2H), 6.64 (d, *J* = 8.8 Hz, 2H), 5.14 (s, 2H), 3.63-3.70 (m, 4H), 3.51-3.58 (m, 2H), 3.36-3.43 (m, 2H).

### Step D: benzyl (4- (4- ( (6- cyclopropyl- 7- (6- (2- hydroxypropan- 2- yl) pyridin- 2- yl)- 7H-pyrrolo [2,3-d]pyrimidin-2-yl)amino)phenyl)-1-oxido-1λ⁶-thiomorpholin-1-ylidene)carbamate

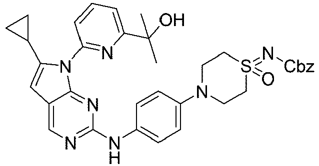

To a round-bottom flask charged with toluene (5 mL) were added 2- (6- (2- chloro- 6-cyclopropyl-7*H*-pyrrolo[2,3-d]pyrimidin-7-yl)pyridin-2-yl)propan-2-ol (66 mg), benzyl (4- (4-aminophenyl)-1-oxido-1λ⁶-thiomorpholin-1-ylidene)carbamate (43 mg), Pd₂(dba)₃ (18 mg), Dave-phos (23 mg) and sodium tert-butoxide (29 mg), and the mixture was heated to 106 °C and stirred for 6 hours. The reaction solution was evaporated to remove the solvent, and the residue was purified through preparative TLC (10%MeOH/DCM) to obtain the target product (30 mg).

¹H NMR (400 MHz, CDCl₃), 8.55 (s, 1H), 7.95 (t, *J* = 8.0 Hz, 1H), 7.76 (d, *J* = 7.6 Hz, 1H), 7.56 (d, *J* = 8.4 Hz, 2H), 7.30-7.44 (m, 6H), 6.97 (s, 1H), 6.87 (d, *J* = 8.4 Hz, 2H), 6.18 (s, 1H), 5.15 (s, 2H), 4.56 (s, 1H), 3.76-3.82 (m, 2H), 3.60-3.72 (m, 4H), 3.39-3.44 (m, 2H), 2.12-2.18 (m, 1H), 1.60 (s, 6H), 0.86-0.91 (m, 2H), 0.72-0.76 (m, 2H).

### Step E: 4-(4-((6-cyclopropyl-7-(6-(2-hydroxypropan-2- yl) pyridin- 2- yl)- 7H- pyrrolo [2,3-d] pyrimidin- 2-yl)amino)phenyl)-1-imino-1λ⁶-thiomorpholine 1-oxide

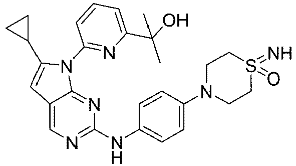

Benzyl (4- (4- ( (6- cyclopropyl- 7- (6- (2- hydroxypropan- 2- yl) pyridin- 2- yl)- 7H- pyrrolo [2,3-d] pyrimidin-2-yl)amino)phenyl)-1-oxido-1λ⁶-thiomorpholin-1-ylidene)carbamate (30 mg) was added to trifluoroacetic acid (5 mL), the mixture was stirred at room temperature overnight, 1 N sodium hydroxide aqueous solution was used to adjust the pH to 10, and the mixture was extracted with dichloromethane, dried over anhydrous sodium sulfate, and evaporated to remove the solvent to obtain the product (15 mg).

¹H NMR (400 MHz, CDCl₃), 8.54 (s, 1H), 7.95 (t, *J* = 8.0 Hz, 1H), 7.75 (t, *J* = 7.6 Hz, 1H), 7.54 (d, *J* = 9.2 Hz, 2H), 7.43 (d, *J* = 7.6 Hz, 1H), 7.25-7.29 (brs, 1H), 6.87 (d, *J* = 8.8 Hz, 2H), 6.17 (s, 1H), 4.42-4.62 (brs, 1H), 3.65-3.77 (m, 4H), 3.12-3.23 (m, 4H), 2.11-2.18 (m, 1H), 1.60 (s, 6H), 0.87-0.91 (m, 2H), 0.71-0.75 (m, 2H).

### Example 106

### ((6-(6-propyl-2-((4-(1-imino-1-oxido-1λ⁶-thiomorpholino)phenyl)amino)-7H-pyrrolo[2,3-d]p yrimidin-7-yl)pyridin-2-yl)imino)dimethyl-λ⁶-sulfanone

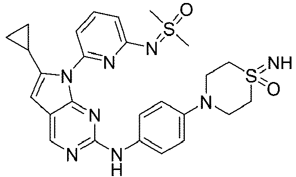

### Step A: benzyl (4-(4-((6-cyclopropyl-7-(6-((dimethyl(oxo)-λ⁶-sulfanylidene) amino) pyridin-2- yl)- 7H- pyrrolo [2,3-d] pyrimidin- pyridin- 2- yl) amino) phenyl)- 1- oxido- 1λ⁶-thiomorpholin-1-ylidene)carbamate

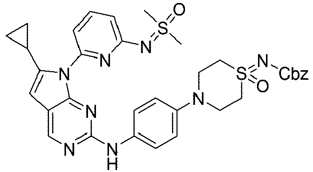

To a round-bottom flask charged with toluene (5 mL) were added ((6-(2-chloro-6-cyclopropyl-7*H*- pyrrolo[2,3-d]pyrimidin-7-yl)pyridin-2-yl)imino)dimethyl-λ⁶-sulfanone (74 mg), benzyl (4-(4-aminophenyl)-1-oxido-1λ⁶-thiomorpholin-1-ylidene)carbamate (43 mg), Pd₂(dba)₃ (18 mg), Dave-phos (23 mg) and sodium tert-butoxide (29 mg), and the mixture was heated to 106 °C and stirred for 6 hours. The reaction solution was evaporated to remove the solvent, and the residue was purified through preparative TLC (10%MeOH/DCM) to obtain the target product (29 mg).

¹H NMR (400 MHz, CDCl₃), 8.52 (s, 1H), 7.74 (t, *J* = 8.0 Hz, 1H), 7.55 (d, *J* = 8.8 Hz, 2H), 7.31-7.41 (m, 5H), 7.21 (d, *J* = 8.0 Hz, 1H), 6.97 (s, 1H), 6.82-6.86 (m, 3H), 6.07 (s, 1H), 5.15 (s, 2H), 3.76-3.81 (m, 2H), 3.59-3.71 (m, 4H), 3.37-3.43 (m, 2H), 3.31 (s, 6H), 2.33-2.39 (m, 1H), 0.87-0.93 (m, 2H), 0.65-0.69 (m, 2H).

### Step B: ((6-(6-propyl-2-((4-(1- imino-1-oxido-1λ⁶-thiomorpholino) phenyl) amino)- 7H-pyrrolo [2,3-d] pyrimidin-7-yl)pyridin-2-yl)imino)dimethyl-λ⁶-sulfanone

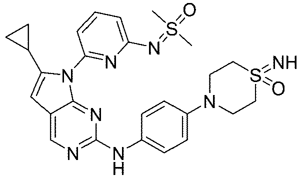

According to the method of step E of example 105, benzyl (4- (4- ( (6- cyclopropyl- 7- (6-((dimethyl(oxo)-λ⁶-sulfanylidene)amino)pyridin-2-yl)-7*H*-pyrrolo[2,3-d]pyrimidin-pyridin-2-yl)a mino)phenyl)-1-oxido-1λ⁶-thiomorpholin-1-ylidene)carbamate was used as the starting material to obtain the target product (20 mg).

¹H NMR (400 MHz, CDCl₃), 8.50 (s, 1H), 7.74 (t, *J =* 8.0 Hz, 1H), 7.54 (d, *J =* 7.2 Hz, 2H), 7.18-7.24 (m, 2H), 6.85 (d, *J* = 8.8 Hz, 2H), 6.83 (d, *J* = 8.0 Hz, 1H), 6.07 (s, 1H), 3.64-3.76 (m, 4H), 3.31 (s, 6H), 3.12-3.22 (m, 4H), 2.40-2.60 (brs, 1H), 2.32-2.38 (m, 1H), 0.88-0.93 (m, 2H), 0.65-0.69 (m, 2H).

### Example 107

### 2-(6-(6-propyl-2-((4-(8-methyl-3,8-diazabicyclo[3.2.1]octan-3-yl)phenyl)amino)-7H-pyrrolo[ 2,3-d]pyrimidin-7-yl)pyridin-2-yl)propan-2-ol

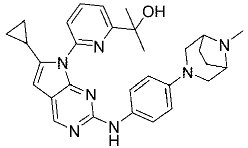

### Step A: 3-(4-nitrophenyl)-3,8-diazabicyclo[3.2.1]octane

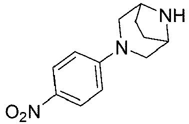

At room temperature, tert-butyl 3-(4-nitrophenyl)-3,8-diazabicyclo[3.2.1]octan-8-carboxylate (0.5 g) was dissolved in a solution of 4 mol/L hydrochloric acid in 1,4-dioxane (4 mL), and the mixture was stirred at room temperature for 1 hour and concentrated to obtain the product (0.4 g).

¹H NMR (400 MHz, CDCl₃) δ 8.10(d, *J =* 8.0 Hz, 2H), 6.68(d, *J =* 8.0 Hz, 2H), 5.66 (s, 1H), 3.67-3.78 (m, 2H), 3.44-3.60 (m, 2H), 3.22-3.30 (m, 2H), 2.32-2.39 (m, 4H).

### Step B: 8-methyl-3-(4-nitrophenyl)-3,8-diazabicyclo[3.2.1]octane

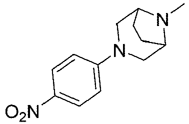

According to the method of step B of example 98, 3-(4-nitrophenyl)- 3,8- diazabicyclo [3.2.1] octane and aqueous solution of 30% formaldehyde were used as the main starting material to obtain the product (0.3 g).

¹H NMR (400 MHz, CDCl₃) δ 8.14(d, *J* = 8.0 Hz, 2H), 6.81(d, *J* = 8.0 Hz, 2H), 3.82-3.93 (m, 2H), 3.53-3.61 (m, 2H), 3.09-3.13 (m, 2H), 2.76 (s, 3H), 2.10-2.16 (m, 4H).

### Step C: 4-(8-methyl-3,8-diazabicyclo[3.2.1]octan-3-yl)aniline

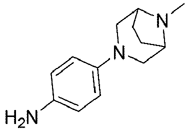

According to the method of step C of example 98, 8- methyl- 3- (4- nitro phenyl)- 3,8-diazabicyclo [3.2.1]octane was used as the starting material to obtain the product (180 mg).

¹H NMR (400 MHz, CDCl₃) δ 7.17-7.21 (brs, 2H), 6.81 (d, *J* = 8.0 Hz, 2H), 6.62 (d, *J* = 8.0 Hz, 2H), 3.95-4.03 (m, 2H), 3.67-3.71 (m, 2H), 3.08-3.14 (m, 2H), 2.74 (s, 3H), 2.10-2.16 (m, 4H).

### Step D: 2-(6- (6- propyl- 2- ( (4- (8- methyl- 3,8- diazabicyclo[3.2.1] octan- 3- yl) phenyl) amino)- 7H- pyrrolo[2,3-d]pyrimidin-7-yl)pyridin-2-yl)propan-2-ol

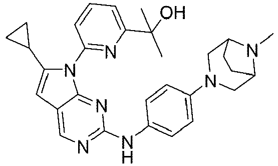

According to the method of step D of example 98, 4-(8-methyl-3,8-diazabicyclo [3.2.1]octan-3-yl)aniline and 2-(6-(2- chloro- 6-cyclopropyl- 7*H*- pyrrolo [2,3-d] pyrimidin- 7-yl) pyridin- 2-yl)propan-2-ol were used as the starting material to obtain the product (15 mg).

¹H NMR (400 MHz, CDCl₃) δ 9.12 (s, 1H), 8.57 (s, 1H), 8.07 (t, *J* = 8.0 Hz, 1H), 7.51 (d, *J* = 8.8 Hz, 2H), 7.72(d, *J =* 8.0 Hz, 1H), 7.65(d, *J =* 8.0 Hz, 1H), 7.59(d, *J* = 9.2 Hz, 2H), 6.78(d, *J =* 9.2 Hz, 2H), 6.24 (s, 1H), 5.32 (s, 1H), 3.95-4.03 (m, 2H), 3.50-3.61 (m, 2H), 3.03-3.11 (m, 2H), 2.74 (s, 3H), 2.13-2.22 (m, 2H), 1.96-2.01 (m, 1H), 1.44 (s, 6H), 0.73-0.76 (m, 2H), 0.63-0.65 (m, 2H).

### Example 108

### ((6-(6-propyl-2-((4-(4-(2-(dimethylamino)ethyl)piperazin-1-yl)phenyl)amino)-7H-pyrrolo[2, 3-d]pyrimidin-7-yl)pyridin-2-yl)imino)dimethyl-λ⁶-sulfanone

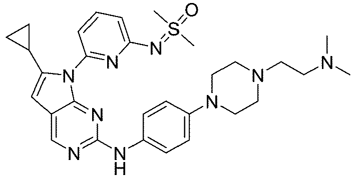

### Step A: N,N-dimethyl-2-(4-(4-nitrophenyl)piperazin-1-yl)ethan-1-amine

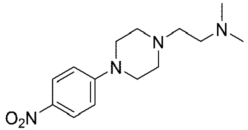

1-fluoro-4-nitrobenzene (141 mg), N,N-dimethyl-2-(piperazin-1-yl)ethan-1-amine (235 mg) and *N*-ethyl-*N*-isopropyl propan-2-amine (387 mg) were added to acetonitrile (10 mL), the tube was sealed, the mixture was heated to 80 °C, reacted for 3 hours, and cooled to room temperature, water (100 mL) was added to precipitate the solid, and the mixture was filtered to obtain the product (300 mg).

¹H NMR (400 MHz, CDCl₃), 8.12 (d, *J* = 9.6 Hz, 2H), 6.81 (d, *J* = 9.6 Hz, 2H), 3.42-3.45 (m, 4H), 2.61-2.64 (m, 4H), 2.53-2.56 (m, 2H), 2.44-2.48 (m, 2H), 2.27 (s, 6H).

### Step B: 4-(4-(2-(dimethylamino)ethyl)piperazin-1-yl)aniline

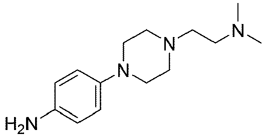

According to the method of step D of example 104, *N,N-* dimethyl- 2- (4- (4- nitrophenyl) piperazin- 1-yl)ethan-1-amine was used as the starting material to obtain the target product (90 mg).

¹H NMR (400 MHz, CDCl₃), 6.80 (d, *J* = 8.8 Hz, 2H), 6.64 (d, *J* = 8.8 Hz, 2H), 3.06-3.09 (m, 4H), 2.71-2.78 (m, 4H), 2.66-2.69 (m, 4H), 2.51 (s, 6H), 2.18-2.48 (brs, 2H).

### Step C: ((6-(6-propyl-2-((4- (4- (2- (dimethylamino) ethyl) piperazin- 1- yl) phenyl) amino)-7H- pyrrolo[2,3-d]pyrimidin-7-yl)pyridin-2-yl)imino)dimethyl-λ⁶-sulfanone

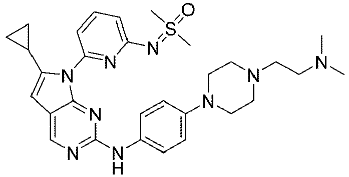

According to the method of step E of example 104, ((6- (2- chloro- 6- cyclopropyl- 7H-pyrrolo[2,3-d]pyrimidin-7-yl)pyridin-2- yl) imino) dimethyl- λ⁶-sulfanone and 4- (4- (2- (dimethyl amino) ethyl)piperazin-1-yl)aniline were used as the starting material to obtain the target product (10 mg).

¹H NMR (400 MHz, CDCl₃), 8.48 (s, 1H), 7.74 (t, *J* = 7.6 Hz, 1H), 7.49 (d, *J* = 8.8 Hz, 2H), 7.22 (s, 1H), 7.19 (d, *J* = 7.6 Hz, 1H), 6.81-6.85 (m, 3H), 6.06 (s, 1H), 3.29 (s, 6H), 3.11-3.17 (m, 6H), 2.96 (t, *J* = 6.0 Hz, 2H), 2.85 (s, 6H), 2.72-2.75 (m, 4H), 2.29-2.35 (m, 1H), 0.87-0.92 (m, 2H), 0.66-0.69 (m, 2H).

### Example 109

### 2-(6-(6-propyl-2-((4-(4-(2-(dimethylamino)ethyl)piperazin-1-yl)phenyl)amino)-7H-pyrrolo[2, 3-d]pyrimidin-7-yl)pyridin-2-yl)propan-2-ol

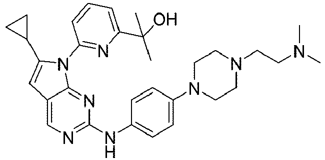

According to the method of step E of example 104, 2-(6- (2-chloro-6- cyclopropyl-7*H*-pyrrolo [2,3-d] pyrimidin-7-yl)pyridin-2-yl)propan-2-ol and 4-(4-(2- (dimethylamino) ethyl) piperazin-1-yl)aniline were used as the starting material to obtain the target product (20 mg).

¹H NMR (400 MHz, CDCl₃), 8.52 (s, 1H), 7.94 (t, *J* = 7.6 Hz, 1H), 7.79 (d, *J* = 8.0 Hz, 1H), 7.49 (d, *J* = 8.8 Hz, 2H), 7.40 (d, *J* = 8.0 Hz, 1H), 6.93 (s, 1H), 6.87 (d, J = 8.8 Hz, 2H), 6.15 (s, 1H), 4.54-4.68 (brs, 1H), 3.12-3.17 (m, 4H), 2.58-2.68 (m, 8H), 2.39 (s, 6H), 2.13-2.19 (m, 1H), 1.59 (s, 6H), 0.85-0.90 (m, 2H), 0.70-0.74 (m, 2H).

### Example 110

### ((6-(6-propyl-2-((5-(4-methylpiperazin-1-yl)pyridin-2-yl)amino)-7H-pyrrolo[2,3-d]pyrimidin -7-yl)pyridin-2-yl)imino)dimethyl-λ⁶-sulfanone

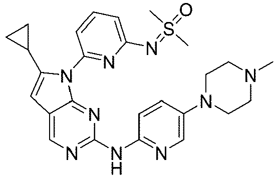

### Step A: 1-methyl-4-(6-nitro-pyridin-3-yl)piperazine

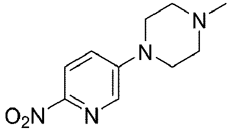

According to the method of step A of example 104, 5-bromo-2-nitropyridine and 1-methylpiperazine were used as the starting material to obtain the product (3 g).

¹H NMR (400 MHz, CDCl₃), 8.15 (d, *J* = 9.2 Hz, 1H), 8.13 (d, *J* = 3.2 Hz, 1H), 7.19 (dd, *J* = 9.2 Hz, 3.2 Hz, 1H), 3.45-3.48 (m, 4H), 2.56-2.59 (m, 4H), 2.36 (s, 3H).

### Step B: 5-(4-methylpiperazin-1-yl)pyridin-2-amine

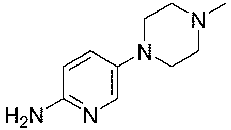

According to the method of step D of example 104, 1-methyl- 4- (6- nitro- pyridin- 3- yl) piperazine was used as the starting material to obtain the product (1 g).

¹H NMR (400 MHz, CDCl₃), 7.79 (d, *J =* 2.8 Hz, 1H), 6.99 (dd, *J* =8.8 Hz, 3.2 Hz, 1H), 6.58 (d, *J* = 8.8 Hz, 1H), 3.41-3.44 (m, 4H), 3.22-3.34 (brs, 2H), 2.57-2.60 (m, 4H), 2.37 (s, 3H).

### Step C: ((6-(6-propyl-2-((5-(4-methylpiperazin-1-yl) pyridin-2-yl) amino)- 7H- pyrrolo[2,3-d] pyrimidin- 7-yl)pyridin-2-yl) imino) dimethyl-λ⁶-sulfanone

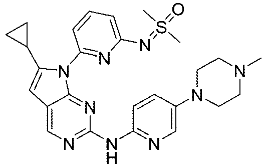

According to the method of step E of example 104, ( (6- (2- chloro- 6- cyclopropyl- 7H-pyrrolo[2,3-d] pyrimidin- 7- yl) pyridin- 2- yl) imino) dimethyl- λ⁶- sulfanone and 5- (4- methyl piperazin- 1-yl)pyridin-2-amine were used as the starting material to obtain the product (15 mg).

¹H NMR (400 MHz, CDCl₃), 8.49 (s, 1H), 8.27 (d, *J* = 2.8 Hz, 1H), 7.98 (dd, *J* = 8.8 Hz, 2.8 Hz, 1H), 7.71 (t, *J* = 7.6 Hz, 1H), 7.14 (d, *J* = 7.2 Hz, 1H), 7.09 (s, 1H), 6.81 (d, *J* = 8.4 Hz, 1H), 6.61 (d, *J* = 9.2 Hz, 1H), 6.05 (s, 1H), 3.71-3.74 (m, 4H), 3.29 (s, 6H), 2.90-2.93 (m, 4H), 2.60 (s, 3H), 2.59-2.33 (m, 1H), 0.87-0.91 (m, 2H), 0.64-0.68 (m, 2H).

### Example 111

### 2-(6-(6-propyl-2-((5-(4-methylpiperazin-1-yl)pyridin-2-yl)amino)-7H-pyrrolo[2,3-d]pyrimidi n-7-yl)pyridin-2-yl)propan-2-ol

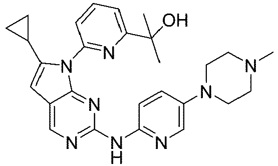

According to the method of step E of example 104, 2- (6- (2- chloro- 6- cyclopropyl- 7H-pyrrolo [2,3-d] pyrimidin- 7- yl) pyridin- 2- yl) propan- 2- ol and 5- (4- methyl piperazin- 1- yl) pyridin- 2-amine were used as the starting material to obtain the product (20 mg).

¹H NMR (400 MHz, CDCl₃), 8.52 (s, 1H), 8.32 (d, *J* = 2.4 Hz, 1H), 7.90-7.95 (m, 2H), 7.72 (d, *J* = 7.6 Hz, 1H), 7.41 (d, *J* = 7.6 Hz, 1H), 6.99 (s, 1H), 6.62 (d, *J* = 9.2 Hz, 1H), 6.15 (s, 1H), 4.46-4.72 (brs, 1H), 3.50-3.59 (m, 4H), 2.58-2.68 (m, 4H), 2.42 (s, 3H), 2.10-2.17 (m, 1H), 1.59 (s, 6H), 0.85-0.90 (m, 2H), 0.70-0.74 (m, 2H).

### Example 112

### ((6-(6-cyclopropyl-5-fluoro-2-((5-(4-methylpiperazin-1-yl)pyridin-2-yl)amino)-7H-pyrrolo[2, 3-d]pyrimidin-7-yl)pyridin-2-yl)imino)dimethyl-λ⁶-sulfanone

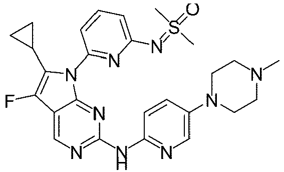

According to the method of step E of example CT-5132, ((6-(2-chloro-6- cyclopropyl- 5-fluoro- 7*H*-pyrrolo[2,3-d] pyrimidin- 7-yl) pyridin- 2- yl) imino) dimethyl- λ⁶- sulfanone and 5-(4- methyl piperazin-1-yl)pyridin-2-amine were used as the starting material to obtain the product (20 mg).

¹H NMR (400 MHz, CDCl₃), 8.65 (s, 1H), 8.23 (d, *J* = 9.2 Hz, 1H), 7.95-7.98 (m, 2H), 7.73 (t, *J* = 8.0 Hz, 1H), 7.18-7.23 (m, 2H), 7.81 (d, *J* = 8.0 Hz, 1H), 3.31 (s, 6H), 3.17-3.20 (m, 4H), 2.66-2.69 (m, 4H), 2.41 (s, 3H), 2.17-2.24 (m, 1H), 0.83-0.88 (m, 2H), 0.73-0.77 (m, 2H).

### Example 113

### 2-(6-(6-propyl-2-((6-(4-methylpiperazin-1-yl)pyridin-3-yl)amino)-7H-pyrrolo[2,3-d]pyrimidi n-7-yl)pyridin-2-yl)propan-2-ol

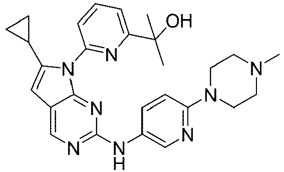

### Step A: 1-methyl-4-(5-nitropyridin-2-yl)piperazine

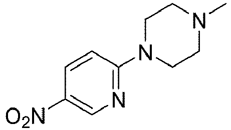

According to the method of step A of example 108, 2-chloro-5-nitropyridine and 1-methyl piperazine were used as the main starting material to obtain the product(2 g).

¹H NMR (400 MHz, CDCl₃), 9.03 (s, 1H), 8.20 (d, *J* = 9.2 Hz, 1H), 6.56 (d, *J* = 9.2 Hz, 1H), 3.76-3.82 (m, 4H), 2.49-2.54 (m, 4H), 2.35 (s, 3H).

### Step B: 6-(4-methylpiperazin-1-yl)pyridin-3-amine

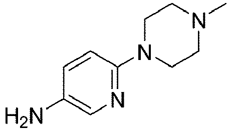

According to the method of step D of 104, 1-methyl-4-(5-nitropyridin-2-yl)piperazime was used as the starting material to obtain the product (1 g).

### Step C: 2- (6- (6- propyl- 2- ( (6- (4- methylpiperazin- 1- yl) pyridin- 3- yl) amino)- 7H-pyrrolo [2,3-d] pyrimidin-7-yl)pyridin-2-yl)propan-2-ol

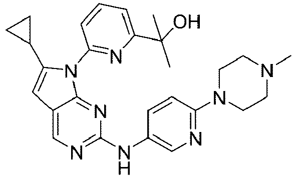

According to the method of step E of example 104, 2- (6- (2- chloro-6- cyclopropyl- 7H-pyrrolo[2,3-d] pyrimidin- 7- yl) pyridin- 2- yl) propan-2-ol and 6- (4- methyl piperazin- 1- yl) pyridin- 3-amine were used as the starting material to obtain the product (16 mg).

¹H NMR (400 MHz, CDCl₃), 8.50 (1H, s), 8.38 (d, *J* = 2.8 Hz, 1H), 7.94-7.98 (m, 2H), 7.67 (d, *J* = 8.0 Hz, 1H), 7.46 (d, *J* = 7.6 Hz, 1H), 7.39-7.44 (brs, 1H), 6.64 (d, *J* = 8.8 Hz, 1H), 6.18 (s, 1H), 4.40-4.62 (brs, 1H), 3.70-4.20 (m, 4H), 2.94-3.44 (m, 4H), 2.80 (s, 3H), 2.07-2.14 (m, 1H), 1.60 (s, 6H), 0.88-0.92 (m, 2H), 0.72-0.76 (m, 2H).

### Example 114

### 2-(6-(6-cyclopropyl-5-fluoro-2-((6-(4-methylpiperazin-1-yl)pyridin-3-yl)amino)-7H-pyrrolo[ 2,3-d]pyrimidin-7-yl)pyridin-2-yl)propan-2-ol

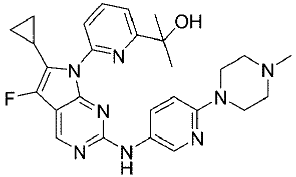

According to the method of step E of example 104, 2-(6-(2-chloro-6- cyclopropyl-5-fluoro-7H- pyrrolo[2,3-d] pyrimidin- 7- yl) pyridin- 2- yl) propan- 2- ol and 6-(4- methylpiperazin- 1-yl) pyridin- 3-amine were used as the starting material to obtain the product (16 mg).

¹H NMR (400 MHz, CDCl₃), 8.58 (s, 1H), 8.30 (d, *J* = 2.8 Hz, 1H), 7.85-7.92 (m, 2H), 7.69 (d, *J* = 8.0 Hz, 1H), 7.40 (d, *J* = 7.6 Hz, 1H), 7.08-7.12 (brs, 1H), 6.61 (d, *J* = 8.8 Hz, 1H), 4.36-4.54 (brs, 1H), 3.52-3.58 (m, 4H), 2.58-2.68 (m, 4H), 2.41 (s, 3H), 1.99-2.07 (m, 1H), 1.57 (s, 6H), 0.79-0.84 (m, 2H), 0.71-0.75 (m, 2H).

### Example 115

### 2-(6-(6-propyl-2-((6-(4-(4-methylpiperazin-1-yl)piperidin-1-yl)pyridin-3-yl)amino)-7H-pyrro lo[2,3-d]pyrimidin-7-yl)pyridin-2-yl)propan-2-ol

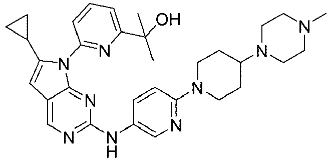

### Step A: 1-methyl-4-(1-(5-nitropyridin-2-yl)piperidin-4-yl)piperazine

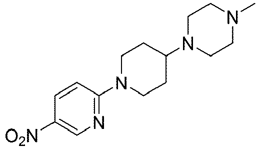

According to the method of step A of example 104, 2-chloro-5-nitropyridine and 1-methyl-4-(piperidin-4-yl)piperazine were used as the starting material to obtain the product (1 g).

¹H NMR (400 MHz, CDCl₃), 9.02 (d, *J* = 2.8 Hz, 1H), 8.17 (dd, *J* = 9.6 Hz, 2.8 Hz, 1H), 6.57 (d, *J* = 9.6 Hz, 1H), 4.52-4.55 (m, 2H), 2.99-3.07 (m, 2H), 2.38-2.68 (m, 9H), 2.29 (s, 3H), 1.96-2.01 (m, 2H), 1.48-1.58 (m, 2H).

### Step B: 6-(4-(4-methylpiperazin-1-yl)piperidin-1-yl)pyridin-3 -amine

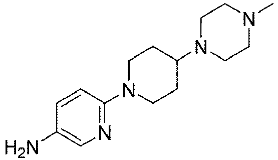

According to the method of step D of example 104, 1-methyl- 4- (1- (5- nitropyridin- 2- yl) piperidin- 4-yl)piperazine was used as the starting material to obtain the product (500 mg).

¹H NMR (400 MHz, CDCl₃), 7.76 (d, *J* = 2.8 Hz, 1H), 6.95 (dd, *J* = 8.8 Hz, 2.8 Hz, 1H), 6.57 (d, *J* = 8.8 Hz, 1H), 4.06-4.11 (m, 2H), 3.12-3.36 (brs, 2H), 2.71 (td, J = 12.4 Hz, 2.4 Hz, 2H), 2.34-2.66 (m, 9H), 2.27 (s, 3H), 1.88-1.94 (m, 2H), 1.52-1.62 (m, 2H).

### Step C: 2-(6-(6-propyl-2- ( (6- (4- (4- methylpiperazin- 1-yl) piperidin- 1-yl) pyridin- 3- yl) amino)- 7H-pyrrolo[2,3-d]pyrimidin-7-yl)pyridin-2-yl)propan-2-ol

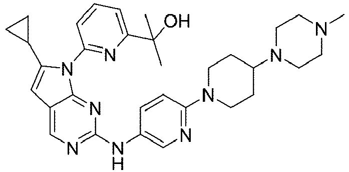

According to the method of step E of example 104, 2-(6-(2-chloro- 6- cyclopropyl- 7H-pyrrolo[2,3-d] pyrimidin- 7- yl) pyridin- 2- yl) propan- 2- ol and 6- (4- (4- methylpiperazin- 1- yl) piperidin- 1-yl)pyridin-3-amine were used as the starting material to obtain the product (18 mg).

¹H NMR (400 MHz, CDCl₃), 8.50 (s, 1H), 8.29 (d, *J* = 2.4 Hz, 1H), 7.94 (t, *J =* 8.0 Hz, 1H), 7.89 (dd, *J =* 8.8 Hz, 2.4 Hz, 1H), 7.70 (d, *J =* 8.0 Hz, 1H), 7.43 (d, *J* = 7.6 Hz, 1H), 6.98 (s, 1H), 6.62 (d, *J* = 9.2 Hz, 1H), 6.14 (s, 1H), 4.52-4.72 (brs, 1H), 4.22-4.29 (m, 2H), 2.89-3.18 (m, 7H), 2.68-2.82 (m, 4H), 2.61 (s, 3H), 2.09-2.16 (m, 1H), 1.96-2.04 (m, 2H), 1.52-1.68 (m, 8H), 0.84-0.89 (m, 2H), 0.69-0.73 (m, 2H).

### Example 116

### 2-(6-(6-cyclopropyl-5-fluoro-2-((6-(4-(4-methylpiperazin-1-yl)piperidin-1-yl)pyridin-3-yl)a mino)-7H-pyrrolo[2,3-d]pyrimidin-7-yl)pyridin-2-yl)propan-2-ol

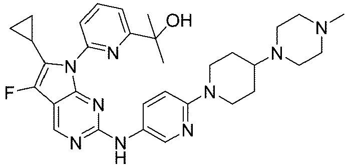

According to the method of step E of example 104, 2- (6- (2- chloro- 6- cyclopropyl- 5-fluoro-7*H*- pyrrolo [2,3-d] pyrimidin- 7- yl) pyridin- 2- yl) propan- 2- ol and 6- (4- (4- methyl piperazin- 1- yl)piperidin-1-yl)pyridin-3-amine were used as the starting material to obtain the product (25 mg).

¹H NMR (400 MHz, CDCl₃), 8.58 (s, 1H), 8.29 (d, *J* = 2.8 Hz, 1H), 7.91 (t, *J* = 8.0 Hz, 1H), 7.85 (dd, *J* = 9.2 Hz, 2.8 Hz, 1H), 7.69 (d, *J* = 7.6 Hz, 1H), 7.42 (d, *J* = 7.6 Hz, 1H), 6.96 (s, 1H), 6.63 (d, *J* = 9.2 Hz, 1H), 4.30-4.58 (brs, 1H), 4.24-4.28 (m, 2H), 2.62-3.02 (m, 11H), 2.54 (s, 3H), 1.96-2.06 (m, 3H), 1.55-1.66 (m, 8H), 0.79-0.83 (m, 2H), 0.71-0.75 (m, 2H).

### Example 117

### ((6-(6-propyl-2-((6-(4-(4-methylpiperazin-1-yl)piperidin-1-yl)pyridin-3-yl)amino)-7H-pyrrol o[2,3-d]pyrimidin-7-yl)pyridin-2-yl)imino)dimethyl-λ⁶-sulfanone

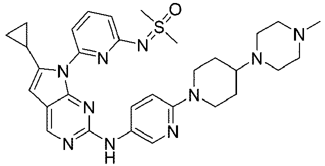

According to the method of step E of example 104, ((6- (2- chloro- 6- cyclopropyl- 7*H-*pyrrolo[2,3-d] pyrimidin- 7- yl) pyridin- 2- yl) imino) dimethyl- λ⁶- sulfanone and 6- (4- (4-methyl piperazin- 1-yl)piperidin-1-yl)pyridin-3-amine were used as the starting material to obtain the product (25 mg).

¹H NMR (400 MHz, CDCl₃), 8.47 (s, 1H), 8.21 (d, *J* = 2.4 Hz, 1H), 7.92 (dd, *J* = 9.2 Hz, 2.4 Hz, 1H), 7.70 (t, *J* = 8.0 Hz, 1H), 7.14 (d, *J* = 8.0 Hz, 1H), 6.99 (s, 1H), 6.79 (d, *J* = 7.6 Hz, 1H), 6.60 (d, *J* = 9.2 Hz, 1H), 6.03 (s, 1H), 4.20-4.23 (m, 2H), 3.28 (s, 6H), 2.69-2.99 (m, 10H), 2.58-2.68 (m, 1H), 2.50 (s, 3H), 2.25-2.31 (m, 1H), 1.94-1.97 (m, 2H), 1.53-1.63 (m, 2H), 0.84-0.89 (m, 2H), 0.63-0.67 (m, 2H).

### Example 118

### ((6-(6-cyclopropyl-5-fluoro-2-((6-(4-(4-methylpiperazin-1-yl)piperidin-1-yl)pyridin-3-yl)ami no)-7H-pyrrolo[2,3-d]pyrimidin-7-yl)pyridin -2-yl)imino)dimethyl-λ⁶-sulfanone

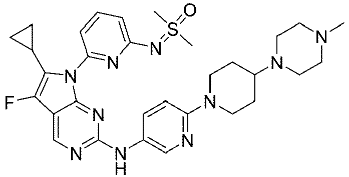

According to the method of step E of example 104, ((6-(2- chloro- 6- cyclopropyl- 5- fluoro-7H- pyrrolo[2,3-d] pyrimidin- 7- yl) pyridin- 2- yl) imino) dimethyl-λ⁶- sulfanone and 6- (4-(4-methyl piperazin-1-yl)piperidin-1-yl)pyridin-3-amine were used as the starting material to obtain the product (30 mg).

¹H NMR (400 MHz, CDCl₃), 8.56 (s, 1H), 8.25 (d, *J* = 2.8 Hz, 1H), 7.93 (dd, *J* = 8.8 Hz, 2.8 Hz, 1H), 7.72 (t, *J =* 8.0 Hz, 1H), 7.15 (d, *J* = 7.6 Hz, 1H), 6.89 (s, 1H), 6.80 (d, *J* = 8.0 Hz, 1H), 6.64 (d, *J* = 8.8 Hz, 1H), 4.24-4.31 (m, 2H), 3.30 (s, 6H), 2.89-3.13 (m, 8H), 2.78-2.84 (m, 2H), 2.59-2.72 (m, 4H), 2.16-2.22 (m, 1H), 1.96-2.04 (m, 2H), 1.56-1.66 (m, 2H), 0.83-0.88 (m, 2H), 0.72-0.76 (m, 2H).

### Example 119

### 2-(6-(6-propyl-2-((5-(4-(4-methylpiperazin-1-yl)piperidin-1-yl)pyridin-2-yl)amino)-7H-pyrro lo[2,3-d]pyrimidin-7-yl)pyridin-2-yl)propan-2-ol

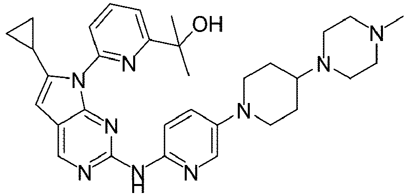

### Step A: 1-methyl-4-(1-(6-nitro-pyridin-3-yl)piperidin-4-yl)piperazine

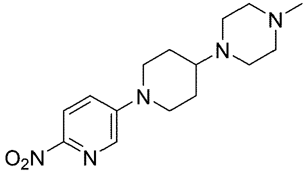

According to the method of step A of example 104, 5-bromo-2-nitropyridine and 1-methyl-4-(piperidin-4-yl)piperazine were used as the starting material to obtain the product (1.1 g).¹H NMR (400 MHz, CDCl₃), 8.13 (d, *J* = 9.2 Hz, 1H), 8.11 (d, *J* = 2.4 Hz, 1H), 7.17 (dd, *J =* 9.2 Hz, 2.4 Hz, 1H), 3.94-4.01 (m, 2H), 2.99-3.06 (m, 2H), 2.40-2.64 (m, 9H), 2.29 (s, 3H), 1.98-2.02 (m, 2H), 1.58-1.68 (m, 2H).

### Step B: 5-(4-(4-methylpiperazin-1-yl)piperidin-1-yl)pyridin-2-amine

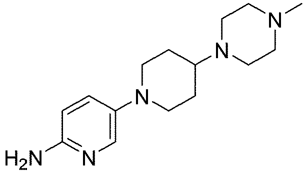

According to the method of step D of example 104, 1-methyl- 4- (1-(6- nitro- pyridin- 3- yl) piperidin- 4- yl)piperazine was used as the starting material to obtain the product (500 mg).

¹H NMR (400 MHz, CDCl₃), 7.76 (d, *J =* 2.8 Hz, 1H), 7.17 (dd, *J* = 9.2 Hz, 2.8 Hz, 1H), 6.46 (d, J = 8.4 Hz, 1H), 4.14 (s, 2H), 3.42-3.48 (m, 2H), 2.47-2.70 (m, 10H), 2.34-2.40 (m, 1H), 2.32 (s, 3H), 1.90-1.96 (m, 2H), 1.64-1.74 (m, 2H).

### Step C: 2-(6-(6-propyl- 2- ( (5- (4- (4-methylpiperazin- 1- yl) piperidin-1-yl) pyridin-2-yl) amino)- 7H- pyrrolo[2,3-d] pyrimidin-7-yl)pyridin-2-yl)propan-2-ol

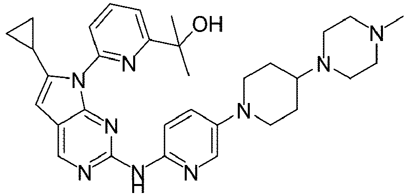

According to the method of step E of example 104, 2- (6- (2- chloro- 6- cyclopropyl- 7H-pyrrolo[2,3-d] pyrimidin- 7- yl) pyridin- 2- yl) propan- 2- ol and 5- (4- (4- methylpiperazin- 1- yl) piperidin- 1-yl)pyridin-2-amine were used as the starting material to obtain the product (30 mg).

¹H NMR (400 MHz, CD₃OD), 8.58 (s, 1H), 8.05 (t, *J* = 8.0 Hz, 1H), 8.00 (d, *J* = 7.6 Hz, 1H), 7.86 (d, *J* = 3.2 Hz, 1H), 7.77 (d, *J* = 7.6 Hz, 1H), 7.62 (d, *J =* 8.4 Hz, 1H), 7.35 (dd, *J* = 9.2 Hz, 3.2 Hz, 1H), 6.26 (s, 1H), 3.57-3.63 (m, 2H), 2.74-2.90 (m, 8H), 2.62-2.70 (m, 2H), 2.46-2.54 (m, 4H), 2.14-2.21 (m, 1H), 1.92-2.01 (m, 2H), 1.58-1.68 (m, 2H), 1.57 (s, 6H), 0.79-0.84 (m, 2H), 0.67-0.71 (m, 2H).

### Example 120

### ((6-(6-cyclopropyl-5-fluoro-2-((5-(4-(4-methylpiperazin-1-yl)piperidin-1-yl)pyridin-2-yl)ami no)-7H-pyrrolo[2,3-d]pyrimidin-7-yl)pyridin-2-yl)imino)dimethyl-λ⁶-sulfanone

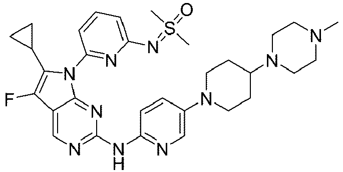

According to the method of step E of example 104, ((6-(2-chloro-6-cyclopropyl-5- fluoro-7*H*- pyrrolo[2,3-d] pyrimidin- 7- yl) pyridin- 2- yl) imino) dimethyl- λ⁶- sulfanone and 5- (4- (4-methyl piperazin- 1-yl)piperidin-1-yl)pyridin-2-amine were used as the starting material to obtain the product (30 mg).

¹H NMR (400 MHz, CDCl₃), 8.64 (s, 1H), 8.20 (d, *J* = 9.2 Hz, 1H), 8.05 (s, 1H), 7.96 (d, *J* = 2.8 Hz, 1H), 7.72 (t, *J =* 8.0 Hz, 1H), 7.21 (dd, *J* = 9.2 Hz, 2.8 Hz, 1H), 7.19 (d, *J* = 7.6 Hz, 1H), 6.80 (d, *J* = 8.0 Hz, 1H), 3.56-3.60 (m, 2H), 3.31 (s, 6H), 2.60-2.82 (m, 10H), 2.42-2.49 (m, 1H), 2.41 (s, 3H), 2.16-2.22 (m, 1H), 1.92-1.99 (m, 2H), 1.65-1.75 (m, 2H), 0.83-0.88 (m, 2H), 0.73-0.77 (m, 2H).

### Example 121

### 2-(6-(6-cyclopropyl-5-fluoro-2-((5-(4-(4-methylpiperazin-1-yl)piperidin-1-yl)pyridin-2-yl)a mino)-7H-pyrrolo[2,3-d]pyrimidin-7-yl)pyridin-2-yl)propan-2-ol

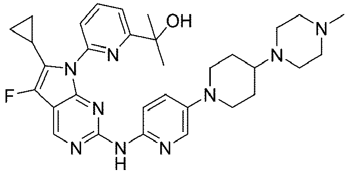

According to the method of step E of example 104, 2-(6- (2- chloro- 6- cyclopropyl- 5-fluoro- 7*H*- pyrrolo[2,3-d] pyrimidin- 7- yl) pyridin- 2- yl) propan- 2- ol and 5- (4- (4- methyl piperazin- 1- yl) piperidin-1-yl)pyridin-2-amine were used as the starting material to obtain the product (17 mg).

¹H NMR (400 MHz, CDCl₃), 8.67 (s, 1H), 8.18 (d, *J* = 8.8 Hz, 1H), 8.04-8.12 (brs, 1H), 7.96 (d, J = 2.8 Hz, 1H), 7.94 (t, *J* = 7.6 Hz, 1H), 7.73 (d, *J =* 7.6 Hz, 1H), 7.44 (d, *J =* 8.0 Hz, 1H), 7.21 (dd, J = 9.2 Hz, 2.8 Hz, 1H), 4.24-4.50 (brs, 1H), 3.57-3.62 (m, 2H), 2.80-3.01 (m, 8H), 2.70 (t, J = 11.6 Hz, 2H), 2.51-2.61 (m, 4H), 1.97-2.06 (m, 3H), 1.68-1.76 (m, 2H), 1.59 (s, 6H), 0.79-0.86 (m, 2H), 0.72-0.77 (m, 2H).

### Example 122

### ((6-(6-propyl-2-((5-(4-(4-methylpiperazin-1-yl)piperidin-1-yl)pyridin-2-yl)amino)-7H-pyrrol o[2,3-d]pyrimidin-7-yl)pyridin-2-yl)imino)dimethyl-λ⁶-sulfanone

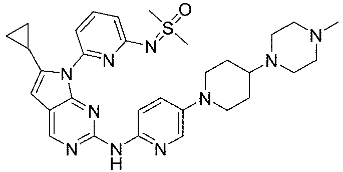

According to the method of step E of example 104, ((6-(2-chloro- 6- cyclopropyl- 7H-pyrrolo[2,3-d]pyrimidin- 7- yl) pyridin- 2- yl) imino) dimethyl- 10⁶- sulfanone and 5- (4- (4- methyl piperazin- 1-yl)piperidin-1-yl)pyridin-2-amine were used as the starting material to obtain the target product (10 mg).

¹H NMR (400 MHz, CDCl₃), 8.56 (s, 1H), 8.20-8.25 (brs, 1H), 7.88-7.96 (m, 2H), 7.73 (t, *J* = 8.0 Hz, 1H), 7.20 (dd, *J* = 9.2 Hz, 2.8 Hz, 1H), 7.17 (d, *J =* 8.0 Hz, 1H), 6.82 (d, *J* = 8.4 Hz, 1H), 6.07 (s, 1H), 3.54-3.58 (m, 2H), 3.31 (s, 6H), 2.78-2.98 (m, 8H), 2.64-2.70 (m, 2H), 2.49-2.57 (m, 4H), 2.26-2.34 (m, 1H), 1.93-2.01 (m, 2H), 1.66-1.76 (m, 2H), 0.85-0.91 (m, 2H), 0.64-0.69 (m, 2H).

### Example 123

### ((6-(6-propyl-2-((4-(9-methyl-3,9-diazaspiro[5.5]undecan-3-yl)phenyl)amino)-7H-pyrrolo[2, 3-d]pyrimidin-7-yl)pyridin-2-yl)imino)dimethyl-λ⁶-sulfanone

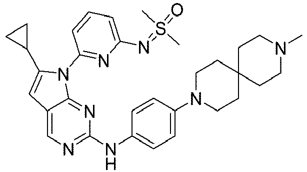

### Step A: tert-butyl 9-(4-nitrophenyl)-3,9-diazaspiro[5.5]undecan-3-carboxylate

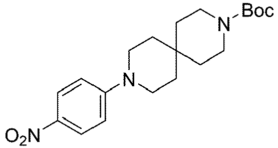

According to the method of step A of example 104, 1-fluoro-4-nitrobenzene and tert-butyl 3,9-diazaspiro[5.5]undecan-3-carboxylate were used as the starting material to obtain the target product (500 mg).

¹H NMR (400 MHz, CDCl₃), 8.10 (d, J = 9.2 Hz, 2H), 6.79 (d, J = 9.2 Hz, 2H), 3.40-3.44 (m, 8H), 1.63-1.66 (m, 4H), 1.48-1.51 (m, 4H), 1.46 (s, 9H).

### Step B: 3-(4-nitrophenyl)-3,9-diazaspiro[5.5]undecane

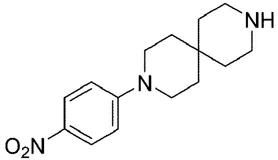

According to the method of step B of example 104, tert-butyl 9- (4- nitro phenyl)- 3,9-diazaspiro [5.5]undecan-3-carboxylate was used as the starting material to obtain the target product (200 mg).

### Step C: 3-methyl-9-(4-nitrophenyl)-3,9-diazaspiro[5.5]undecane

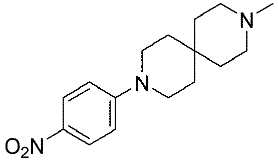

According to the method of step C of example 104, 3-(4-nitrophenyl)- 3,9- diazaspiro [5.5] undecane was used as the starting material to obtain the target product (200 mg).

¹H NMR (400 MHz, CDCl₃), 8.09 (d, *J* = 9.2 Hz, 2H), 6.78 (d, *J* = 9.2 Hz, 2H), 3.38-3.41 (m, 4H), 2.38-2.42 (m, 4H), 2.30 (s, 3H), 1.58-1.63 (m, 8H).

### Step D: 4-(-methyl-3,9-diazaspiro[5.5]undecan-3-yl)aniline

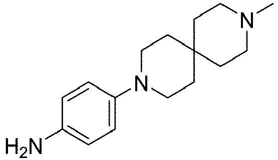

According to the method of step D of example 104, 3- methyl- 9- (4- nitrophenyl)- 3,9-diazaspiro [5.5]undecane was used as the starting material to obtain the target product (100 mg).

¹H NMR (400 MHz, CDCl₃), 6.82 (d, *J* = 9.2 Hz, 2H), 6.64 (d, *J* = 9.2 Hz, 2H), 3.30-3.51 (brs, 2H), 2.96-2.99 (m, 4H), 2.36-2.41 (m, 4H), 2.28 (s, 3H), 1.61-1.64 (m, 4H), 1.55-1.58 (m, 4H).

### Step E: ((6-(6-propyl-2- ( (4- (9- methyl- 3,9-diazaspiro [5.5]undecan- 3- yl) phenyl) amino)-7H- pyrrolo[2,3-d]pyrimidin-7-yl)pyridin-2-yl)imino)dimethyl-λ⁶-sulfanone

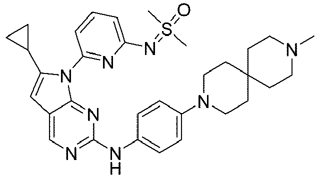

According to the method of step E of example 104, ((6-(2-chloro-6- cyclopropyl- 7H- pyrrolo [2,3-d]pyrimidin-7-yl)pyridin-2-yl)imino)dimethyl-λ⁶-sulfanone and 4- (-methyl- 3,9- diazaspiro [5.5] undecan-3-yl)aniline were used as the starting material to obtain the target product (10 mg).

¹H NMR (400 MHz, CDCl₃), 8.48 (s, 1H), 7.71 (t, *J* = 8.0 Hz, 1H), 7.47 (d, *J* = 8.8 Hz, 2H), 7.18 (d, J = 7.6 Hz, 1H), 7.05 (s, 1H), 6.79-6.84 (m, 3H), 6.04 (s, 1H), 3.27 (s, 6H), 2.84-3.18 (m, 8H), 2.73 (s, 3H), 2.27-2.33 (m, 1H), 1.88-2.02 (m, 4H), 1.66-1.72 (m, 4H), 0.84-0.90 (m, 2H), 0.64-0.68 (m, 2H).

### Example 124

### 2-(6-(6-propyl-2-((4-(9-methyl-3,9-diazaspiro[5.5]undecan-3-yl)phenyl)amino)-7H-pyrrolo[2, 3-d]pyrimidin-7-yl)pyridin-2-yl)propan-2-ol

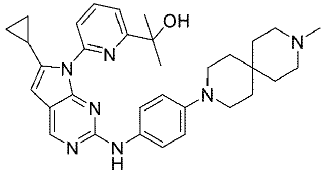

According to the method of step E of example 104, 2- (6- (2- chloro- 6- cyclopropyl- 7H-pyrrolo [2,3-d] pyrimidin- 7- yl) pyridin- 2- yl) propan- 2- ol and 4- (-methyl- 3,9- diazaspiro[5.5] undecan- 3-yl)aniline were used as the starting material to obtain the target product (10 mg).

¹H NMR (400 MHz, CDCl₃), 8.51 (s, 1H), 7.93 (t, *J* = 7.6 Hz, 1H), 7.78 (d, *J* = 8.0 Hz, 1H), 7.49 (d, *J* = 8.8 Hz, 2H), 7.40 (d, *J* = 8.0 Hz, 1H), 7.03 (s, 1H), 6.87 (d, *J* = 8.8 Hz, 2H), 6.14 (s, 1H), 4.50-4.71 (brs, 1H), 3.05-3.08 (m, 4H), 2.61-2.68 (m, 4H), 2.45 (s, 3H), 2.12-2.19 (m, 1H), 1.70-1.73 (m, 4H), 1.65-1.68 (m, 4H), 1.58 (s, 6H), 0.84-0.88 (m, 2H), 0.69-0.73 (m, 2H).

### Example 125

### 2-(6-(6-cyclopropyl-5-fluoro-2-((4-(9-methyl-3,9-diazaspiro[5.5]undecan-3-yl)phenyl)amino) -7H-pyrrolo[2,3-d]pyrimidin-7-yl)pyridin-2-yl)propan-2-ol

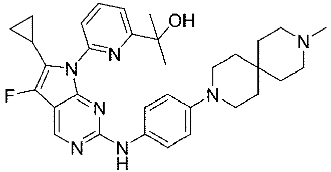

According to the method of step E of CT-5132, 2-(6- (2- chloro- 6- cyclopropyl- 5- fluoro-7H- pyrrolo[2,3-d]pyrimidin-7-yl)pyridin- 2- yl) propan- 2- ol and 4- (-methyl- 3,9- diazaspiro[5.5] undecan- 3-yl)aniline were used as the starting material to obtain the product (16 mg).

¹H NMR (400 MHz, CDCl₃), 8.58 (s, 1H), 7.91 (t, *J* = 8.0 Hz, 1H), 7.76 (d, *J* = 8.0 Hz, 1H), 7.47 (d, *J* = 8.8 Hz, 2H), 7.39 (d, *J* = 8.0 Hz, 1H), 7.13 (s, 1H), 6.87 (d, *J* = 8.8 Hz, 2H), 4.26-4.62 (brs, 1H), 3.05-3.08 (m, 4H), 2.58-2.64 (m, 4H), 2.43 (s, 3H)2.02-2.09 (m, 1H), 1.69-1.72 (m, 4H), 1.64-1.67 (m, 4H), 1.58 (s, 6H), 0.79-0.84 (m, 2H), 0.71-0.75 (m, 2H).

### Example 126

### ((6-(6-cyclopropyl-5-fluoro-2-((4-(9-methyl-3,9-diazaspiro[5.5]undecan-3-yl)phenyl)amino)-7H-pyrrolo[2,3-d]pyrimidin-7-yl)pyridin-2-yl)imino)dimethyl-λ⁶-sulfanone

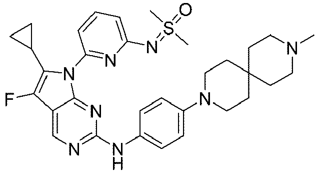

According to the method of step E of example 104, ((6-(2-chloro-6-cyclopropyl-5-fluoro-7*H*-pyrrolo[2,3-d]pyrimidin-7-yl)pyridin-2-yl)imino)dimethyl-λ⁶-sulfanone and 4-(-methyl- 3,9- diazaspiro[5.5]undecan-3-yl)aniline were used as the starting material to obtain the product (20 mg).

¹H NMR (400 MHz, CDCl₃), 8.55 (s, 1H), 7.70 (t, *J =* 8.0 Hz, 1H), 7.46 (d, *J =* 8.8 Hz, 2H), 7.19 (d, *J* = 7.6 Hz, 1H), 7.05 (s, 1H), 6.85 (d, *J* = 8.8 Hz, 2H), 6.78 (d, *J* = 8.4 Hz, 1H), 3.28 (s, 6H), 3.04-3.07 (m, 4H), 2.76-2.82 (m, 4H), 2.55 (s, 3H), 2.16-2.22 (m, 1H), 1.79-1.82 (m, 4H), 1.66-1.69 (m, 4H), 0.81-0.86 (m, 2H), 0.71-0.75 (m, 2H).

### Example 127

### methyl 2-(4-(4-((6-cyclopropyl-7-(6-((dimethyl(oxo)-X⁶- sulfanylidene)amino)pyridin-2- yl)-7H- pyrrolo[2,3-d]pyrimidin-2-yl)amino)phenyl)piperazin-1-yl)acetate

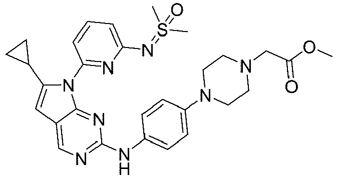

### Step A: methyl 2-(4-(4-nitrophenyl)piperazin-1-yl)acetate

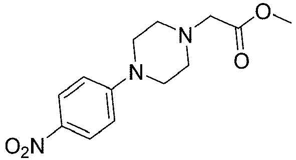

To a flask were added 1-(4-nitrophenyl)piperazine (1.0 g), methyl 2-chloroacetate (0.49 g), triethylamine (1.24 g) and N,N-dimethylformamide. Under nitrogen gas, the mixture was heated to 40 °C and stirred overnight. After the temperature restored to room temperature, the mixture was diluted with ethyl acetate and washed with water, the organic phase was dried and concentrated, and the residue was purified through silica gel column chromatography (100% ethyl acetate ) to obtain the product (1.1 g).

¹H NMR (400 MHz, CDCl₃) δ8.10 (d, J = 8.0 Hz, 2H), 6.81 (d, J = 8.0 Hz, 2H), 3.78 (s, 3H), 3.31 (s, 2H), 3.18-3.22 (m, 4H), 2.71-2.73 (m, 4H).

### Step B: methyl 2-(4-(4-aminophenyl)piperazin-1-yl)acetate

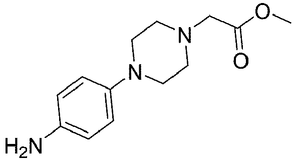

According to the method of step C of example 98, methyl 2- (4- (4- nitro phenyl) piperazin-1-yl)acetate was used as the starting material to obtain the product (80 mg).

¹H NMR (400 MHz, CDCl₃) δ 8.00 (s, 2H), 6.80(dd, *J* = 6.4 Hz, 2.4 Hz, 2H), 6.64(dd, *J* = 6.4 Hz, 2.4 Hz, 2H), 3.73 (s, 3H), 3.27 (s, 2H), 3.08-3.11 (m, 4H), 2.71-2.74 (m, 4H).

### Step C: methyl 2-(4-(4-((6-cyclopropyl-7-(6-((dimethyl(oxo)-λ⁶-sulfanylidene)amino) pyridin-2-yl)-7H-pyrrolo[2,3-d]pyrimidin-2-yl)amino)phenyl)piperazin-1-yl)acetate

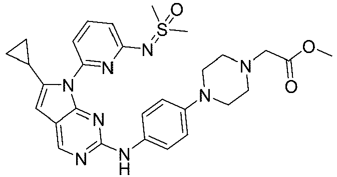

According to the method of step D of example 98, methyl ((6-(2-chloro-6-cyclopropyl-7*H*-pyrrolo[2,3-d]pyrimidin-7-yl)pyridin-2-yl)imino)dimethyl-λ⁶-sulfanone and 2-(4-(4-amino phenyl)piperazin-1-yl)acetate were used as the starting material to obtain the product (20 mg).

¹H NMR (400 MHz, CDCl₃) δ 8.49 (s, 1H), 7.72 (t, *J* = 8.0 Hz, 1H), 7.55 (d, *J* = 9.2 Hz, 2H), 7.19 (d, *J* = 8.0 Hz, 1H), 7.00 (s, 1H), 6.79-6.89 (m, 3H), 6.04 (s, 1H), 3.74 (s, 3H), 3.28 (s, 6H), 3.26 (s, 2H), 3.15-3.18 (m, 4H), 2.72-2.75 (m, 4H), 2.31-2.35 (m, 1H), 0.86-0.90 (m, 2H), 0.64-0.68 (m, 2H).

### Example 128

### 2-(4-(4-((6-cyclopropyl-7-(6-((dimethyl(oxo)-λ⁶-sulfanylidene)amino)pyridin-2-yl)-7H-pyrro lo[2,3-d]pyrimidin-2-yl)amino)phenyl)piperazin-1-yl) acetic acid

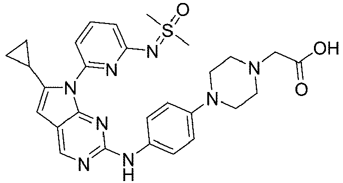

According to the method of step D of example 98, ((6-(2-chloro-6-cyclopropyl-7*H-*pyrrolo[2,3-d]pyrimidin-7-yl)pyridin-2-yl)imino)dimethyl-λ⁶-sulfanone and methyl 2-(4-(4-amino phenyl)piperazin-1-yl)acetate were used as the starting material to obtain the product (20mg).

¹H NMR (400MHz, CD₃OD), 8.45 (s, 1H), 7.80 (t, *J* = 8.0 Hz, 1H), 7.52 (d, *J* = 8.4 Hz, 2H), 7.16 (d, *J* = 8.0 Hz, 1H), 6.89 (d, *J* = 9.2 Hz, 2H), 6.82 (d, *J* = 8.4 Hz, 1H), 6.14 (s, 1H), 3.69 (s, 2H), 3.43-3.51 (m, 4H), 3.34-3.39 (m, 4H), 3.33 (s, 6H), 2.22-2.29 (m, 1H), 0.82-0.87 (m, 2H), 0.64-0.68 (m, 2H).

### Example 129

### 2-(4-(4-((6-cyclopropyl-7-(6-(2-hydroxypropan-2-yl)pyridin-2-yl)-7H-pyrrolo[2,3-d]pyrimidi n-2-yl)amino)phenyl)piperazin-1-yl)-N-hydroxy acetamide

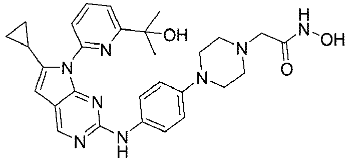

### Step A: 2-(4-(4-nitrophenyl)piperazin-1-yl)acetic acid

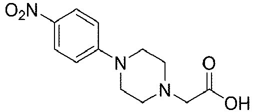

Methyl 2-(4-(4-nitrophenyl)piperazin-1-yl)acetate (1.968 g) and an aqueous solution of 1 mol/L LiOH (0.150 g) were added into a flask, the mixture was stirred at room temperature for 3 hours and filtered, and the solvent was removed from the filtrate to obtain the product (1.852 g).

### Step B: 2-(4-(4-nitrophenyl)piperazin-1-yl)-N-((tetrahydro-2H-pyran-2-yl)oxy)acetamide

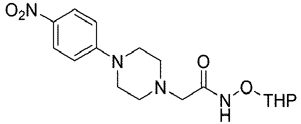

A mixture of 1-(3-dimethylaminopropyl)-3-ethyl-carbodiimide hydrochloride (1.510 g), 1-hydroxybenzotriazole (1.060 g), O-(tetrahydro-2H-pyran-2-yl)hydroxylamine (0.920 g) and DIEA (2.03 g) were stirred at room temperature for 4 hours. The mixture was let stay for a day to generate a precipitate, the mixture was filtered, the filtrate was partitioned between water and dichloromethane, and the organic phase was evaporated to dryness to obtain the product (1.223 g).

¹H NMR (400MHz, CDCl₃), 9.39 (s, 1H), 8.12 (d, J = 9.2 Hz, 2H), 6.82 (d, J = 9.2 Hz, 2H), 4.97-5.01 (m, 1H), 3.94-4.01 (m, 1H), 3.62-3.68 (m, 1H), 3.41-3.44 (m, 4H), 3.17 (s, 2H), 2.68-2.71 (m, 4H), 1.78-1.89 (m, 3H), 1.55-1.65 (m, 3H).

### Step C: 2-(4-(4-((6-cyclopropyl-7-(6-(2-hydroxypropyl)pyridin-2-yl)-7H-pyrrolo[2,3-d] pyrimidin-2-yl)amino)phenyl)piperazin-1-yl)-N-((tetrahydro-2H-pyran-2-yl)oxy)acetamide

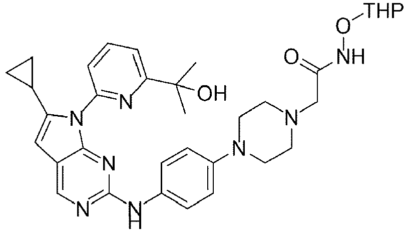

According to the method of step A of SY-5266, 2-(6-(2-chloro-6-cyclopropyl-7*H*-pyrrolo [2,3-d]pyrimidin-7-yl)pyridin-2-yl)propan-2-ol and 2-(4-(4-nitrophenyl)piperazin-1-yl)-*N-*((tetrahydro-2*H*-pyran-2-yl)oxy)acetamide were used as the starting material to obtain the product (30 mg).

¹H NMR (400MHz, CDCl₃), 9.42-9.55 (brs, 1H), 8.51 (s, 1H), 7.95 (t, *J* = 8.0 Hz, 1H), 7.78 (d, *J* = 8.0 Hz, 1H), 7.52 (d, *J* = 9.2 Hz, 2H), 7.41 (d, *J* = 7.6 Hz, 1H), 7.12 (s, 1H), 6.86 (d, *J* = 9.2 Hz, 2H), 6.16 (s, 1H), 4.98-5.01 (m, 1H), 4.46-4.72 (brs, 1H), 3.95-4.01 (m, 1H), 3.62-3.67 (m, 1H), 3.48 (s, 6H), 3.17 (s, 2H), 3.11-3.14 (m, 4H), 2.69-2.72 (m, 4H), 2.12-2.19 (m, 1H), 1.62-1.90 (m, 6H), 0.86-0.90 (m, 2H), 0.70-0.74 (m, 2H).

### Step D: 2-(4-(4-((6-cyclopropyl-7-(6-(2-hydroxypropan-2-yl)pyridin-2-yl)-7H-pyrrolo[2,3-d] pyrimidin-2-yl)amino)phenyl)piperazin-1-yl)-N-hydroxy acetamide

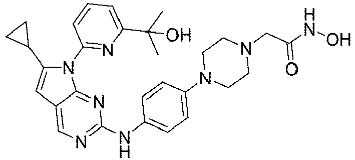

Under nitrogen gas, *2-(4-(4-((6-cyclopropyl-7-(6-(2-hydroxypropyl)pyridin-2-yl)-7H-pyrrolo [2,3-d]pyrimidin*-2-yl)amino)phenyl)piperazin-1-yl)-*N*-((tetrahydro-2*H*-pyran-2-yl)oxy)acetamide (0.010 g) and p-toluenesulfonic acid (0.006 g) were mixed. The mixture was stirred at room temperature overnight, the solvent was removed from the filtrate, and the residue was purified through silica gel column chromatography (50%EtOAc/PE-100%EtOAc) to obtain the product (0.001 g).

¹H NMR (400MHz, CD₃OD), 8.50 (s, 1H), 8.05 (t, *J* = 8.0 Hz, 1H), 7.76 (d, *J* = 8.0 Hz, 1H), 7.65 (d, *J* = 8.0 Hz, 1H), 7.53 (d, *J* = 8.4 Hz, 2H), 6.94 (d, *J* = 8.4 Hz, 2H), 6.27 (s, 1H), 3.57 (s, 2H), 3.12-3.33 (m, 8H), 2.18-2.22 (m, 1H), 1.57 (s, 6H), 0.79-0.84 (m, 2H), 0.68-0.72 (m, 2H).

### Example 130

### 1-(6-(6-propyl-2-((4-(4-methylpiperazin-1-yl)phenyl)amino)-7H-pyrrolo[2,3-d]pyrimidin-7-y l)pyridin-2-yl)-3-methylazetidin-3-ol

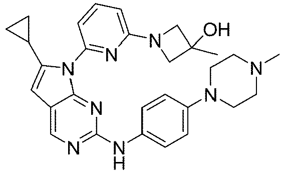

### Step A: 1-(6-bromopyridin-2-yl)-3-methylazetidin-3-ol

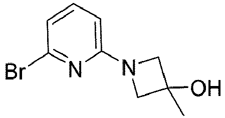

Under nitrogen gas, a suspension of 2,6-di-bromopyridine (7.68 g), 3-methylazetidin-3-ol hydrochloride (2.0 g), caesium carbonate (26.4 g), L-proline (0.93 g) and copper(I) iodide (1.54 g) in 1,4-dioxane (100 mL) was heated to 90 °C and stirred overnight. The reaction solution was cooled to room temperature, filtered through celite, the filtrate was partitioned between ethyl acetate and brine, and the organic phase was dried and concentrated to obtain the crude product, which was purified through silica gel column chromatography (ethyl acetate /petroleum ether =10%-50%) to obtain the product (3.5 g).

¹H NMR (400 MHz CDCl₃) δ7.25 (t, *J* = 8.0 Hz, 1H), 6.74 (d, *J* = 8.0 Hz, 1H), 6.17 (d, *J* = 8.0 Hz, 1H), 3.88-3.97 (m, 4H), 1.58 (s, 3H).

### Step B: 1-(6-aminopyridin-2-yl)-3-methylazetidin-3-ol

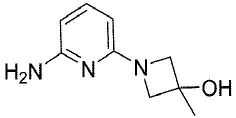

A mixture of 1-(6-bromopyridin-2-yl)-3-methylazetidin-3-ol (3.5 g), copper (1) oxide (0.206 g), N,N-Dimethyl-1,2-ethanediamine (0.127 g), anhydrous potassium carbonate (4.00 g), ammonia (10 mL) and ethylene glycol (10 mL) was heated to 60 °C and stirred overnight. The mixture was cooled to room temperature, filtered through celite, washed with dichloromethane, and partitioned, the aqueous phase was extracted with dichloromethane (100 mL x 4), and the organic phases were combined and evaporated to dryness to obtain the product (1.6 g).

¹H NMR (400 MHz, CDCl₃) δ 7.21 (t, *J* = 8.0 Hz, 1H), 5.82 (d, *J* = 8.0 Hz, 1H), 5.64 (d, *J* = 8.0 Hz, 1H), 4.26-4.29(brs, 2H), 3.80-3.87 (m, 4H), 1.56 (s, 3H).

### Step C: 1-(6-((2-chloro-5-iodopyrimidin-4-yl)amino)pyridin-2-yl)-3-methylazetidin-3-ol

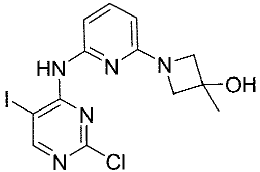

Under nitrogen gas protection, a mixture of 1-(6-aminopyridin-2-yl)-3-methylazetidin-3-ol (1.6 g), 5-iodo-2,4-dichloropyrimidine (2.46 g), ethyldiisopropylamine (1.4 g) and isopropanol (20 mL) was heated and refluxed and stirred for 48 hours. The mixture was cooled to room temperature, and the solid precipitated was collected by filtration, washed with small amount of ethyl acetate, and dried to obtain the product (1.36 g).

¹H NMR (400 MHz CDCl₃) δ 8.47 (s, 1H), 7.66 (d, *J* = 8.0 Hz, 1H), 7.54 (t, *J* = 8.0 Hz, 1H), 6.09 (d, 1H), 3.96 (d, *J* = 8.8Hz, 2H), 3.90 (d, *J* = 8.8Hz, 2H), 1.98 (s, 1H), 1.60 (s, 3H).

### Step D: 1-(6-(2-chloro-6-cyclopropyl-7H-pyrrolo[2,3-d]pyrimidin-7-yl)pyridin-2-yl)-3-methylazetidin-3-ol

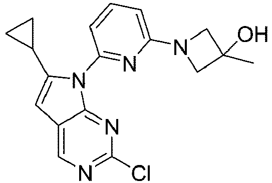

Under nitrogen gas protection, to a solution of 1-(6-((2-chloro-5-iodopyrimidin-4-yl)amino) pyridin-2-yl)-3-methylazetidin-3-ol (1.36 g), copper(I) iodide (0.31 g), and Pd(dppf)Cl₂ (0.234 g) in DMF (10 mL) and triethylamine (10 mL) was slowly added cyclopropylacetylene (0.215 g), the reaction solution was stirred at room temperature overnight, heated to 50 °C and stirred overnight, filtered through celite and washed with dichloromethane, the solvent was removed from the filtrate, and the residue was purified through silica gel column chromatography (50%AcOEt/PE-100%AcOEt) to obtain the product (0.75 g).

¹H NMR (400 MHz, CDCl₃) δ 8.49 (s, 1H), 7.66 (t, *J* = 8.0 Hz, 1H), 7.55 (d, *J* = 8.8 Hz, 2H), 7.02 (t, *J* = 8.0 Hz, 1H), 6.99 (s, 1H), 6.92(d, *J* = 8.0 Hz, 2H), 6.34(d, *J* = 8.8 Hz, 1H), 6.07 (s, 1H), 4.73 (s, 1H), 3.94-4.00 (m, 4H), 3.18-3.21 (m, 4H), 2.60-2.80 (m, 4H), 2.44 (s, 3H), 2.19-2.21 (m, 1H), 1.56 (s, 3H), 0.83-0.88 (m, 2H), 0.71-0.72 (m, 2H).

### Step E: 1-(6-(6-propyl-2-((4-(4-methylpiperazin-1-yl)phenyl)amino)-7H-pyrrolo[2,3-d] pyrimidin-7-yl)pyridin-2-yl)-3-methylazetidin-3-ol

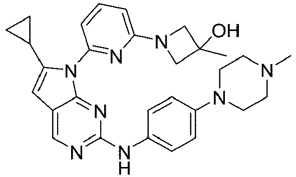

According to the method of step D of example 98, 1-(6-(2-chloro-6-cyclopropyl-7*H-*pyrrolo [2,3 -d]pyrimidin-7-yl)pyridin-2-yl)-3 -methylazetidin-3 -ol and 4-(4-methylpiperazin-1-yl) aniline were used as the main starting material to obtain the product (15 mg).

¹H NMR (400 MHz, CDCl₃) δ 8.49 (s, 1H), 7.66 (t, *J* = 8.0 Hz, 1H), 7.55 (d, *J* = 8.8 Hz, 2H), 7.02 (t, *J* = 8.0 Hz, 1H), 6.99 (s, 1H), 6.92(d, *J* = 8.0 Hz, 2H), 6.34(d, *J* = 8.8 Hz, 1H), 6.07 (s, 1H), 4.73 (s, 1H), 3.94-4.00 (m, 4H), 3.18-3.21 (m, 4H), 2.60-2.80 (m, 4H), 2.44 (s, 3H), 2.19-2.21 (m, 1H), 1.56 (s, 3H), 0.83-0.88 (m, 2H), 0.71-0.72 (m, 2H).

### Example 131

### 1-((6-(6-propyl-2-((4-(4-methylpiperazin-1-yl)phenyl)amino)-7H-pyrrolo[2,3-d]pyrimidin-7-yl)pyridin-2-yl)oxy)-2-methylpropan-2-ol

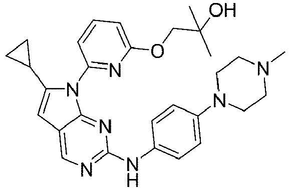

### Step A: 1-((6-bromopyridin-2-yl)oxy)-2-methylpropan-2-ol

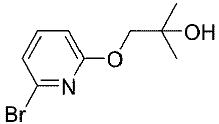

2-Methylpropan-1,2-diol (0.95 g) was dissolved in N,N-dimethylformamide (10 mL), the mixture was cooled to 0 °C, 60% NaH (208 mg) was portionwise added to the the reaction solution, the reaction solution was stirred at this temperature for 30 minutes, and 2,6-di-bromopyridine was added. The reaction solution was let restore to room temperature and stirred for 1 hour, water was added to quench the reaction, the mixture was extracted with ethyl acetate and washed with brine, the organic phase was dried, concentrated, and purified through silica gel column chromatography (petroleum ether: ethyl acetate =2: 1) to obtain the product (0.7

g). ¹H NMR (400 MHz CDCl₃) δ 7.44 (t, *J* = 8.0 Hz, 1H), 7.08(d, *J =* 8.0 Hz, 1H), 6.74 (d, *J* = 8.0 Hz, 1H), 4.18 (s, 2H), 2.02 (s, 1H), 1.32 (s, 6H).

### Step B: 1-((6-aminopyridin-2-yl)oxy)-2-methylpropan-2-ol

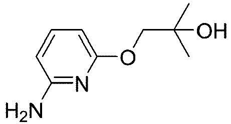

According to the method of step B of example 131, 1-((6-bromopyridin-2-yl)oxy)-2-methyl propan-2-ol and ammonia were used as the starting material to obtain the product (190 mg).

¹H NMR (400 MHz CDCl₃) δ 7.33 (t, *J* = 8.0 Hz, 1H), 6.04-6.08 (m, 2H), 4.70 (s, 3H), 4.04 (s, 2H), 1.26 (s, 6H).

### Step C: 1-((6-((2-chloro-5-iodopyrimidin-4-yl)amino)pyridin-2-yl)oxy)-2-methylpropan-2-ol

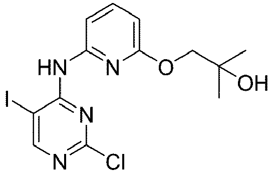

According to the method of step C of example 131, 1-((6-aminopyridin-2-yl)oxy)-2-methyl propan-2-ol and 5-iodo-2,4-dichloropyrimidine were used as the main starting material to obtain the product (116 mg).

¹H NMR (400 MHz CDCl₃) δ 8.75 (s, 1H), 7.95 (d, *J* = 8.0 Hz, 1H), 7.73 (s, 1H), 7.70 (t, *J* = 8.0 Hz, 1H), 6.58 (d, *J* = 8.0 Hz, 1H), 4.15 (s, 2H), 2.38 (s, 1H), 1.35 (s, 6H).

### Step D: 1-((6-(2-chloro-6-cyclopropyl-7H-pyrrolo[2,3-d]pyrimidin-7-yl)pyridin-2-yl)oxy)-2-methylpropan-2-ol

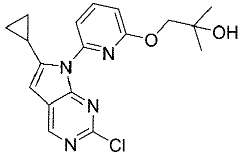

According to the method of step D of example 131, 1-((6-((2-chloro-5-iodopyrimidin-4-yl) amino)pyridin-2-yl)oxy)-2-methylpropan-2-ol and cyclopropylacetylene were used as the main starting material to obtain the product (70 mg).

¹H NMR (400 MHz CDCl₃) δ 8.70 (s, 1H), 7.83 (t, *J* = 8.0 Hz, 1H), 7.22(d, *J* = 8.0 Hz, 1H), 6.90 (d, *J* = 8.0 Hz, 1H), 6.23 (s, 1H), 4.18 (s, 2H), 2.06-2.08 (m, 1H), 1.31 (s, 6H), 0.91-0.96 (m, 2H), 0.77-0.80 (m, 2H).

### Step E: 1-((6-(6-propyl-2-((4-(4-methylpiperazin-1-yl)phenyl)amino)-7H-pyrrolo[2,3-d] pyrimidin-7-yl)pyridin-2-yl)oxy)-2-methylpropan-2-ol

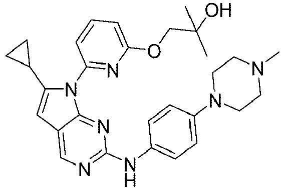

According to the method of step D of example 98, 1-((6-(2-chloro-6-cyclopropyl-7H-pyrrolo[2,3-d]pyrimidin-7-yl)pyridin-2-yl)oxy)-2-methylpropan-2-ol and 4-(4-methylpiperazin-1-yl)aniline were used as the main starting material to obtain the product (25 mg).

¹H NMR (400 MHz CDCl₃) δ 8.50 (s, 1H), 7.80 (t, *J* = 8.0 Hz, 1H), 7.53(d, *J* = 8.8 Hz, 2H), 7.48 (s, 1H), 7.27(d, *J* = 8.0 Hz, 1H), 6.84-6.88 (m, 3H), 6.10 (s, 1H), 4.26 (s, 2H), 3.47 (s, 1H), 3.28-3.31 (m, 4H), 2.85-2.90 (m, 4H), 2.56 (s, 3H), 2.06-2.08 (m, 1H), 1.32 (s, 6H), 0.86-0.90 (m, 2H), 0.72-0.76 (m, 2H).

### Example 132

### 6-cyclopropyl-7-(6-methoxypyridin-2-yl)-N-(6-(4-methylpiperazin-1-yl)pyridin-3-yl)-7H-pyr rolo[2,3-d]pyrimidin-2-amine

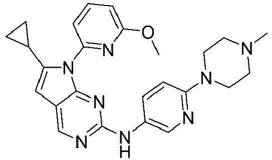

### Step A: 2-chloro-5-iodo-N-(6-methoxypyridin-2-yl)pyrimidin-4-amine

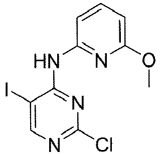

According to the method of step C of example 131, 2-amino-6-methoxypyridine and 5-iodo-2,4-dichloropyrimidine were used as the main starting material to obtain the product (0.51g).

¹H NMR (400 MHz CDCl₃) δ 8.50 (s, 1H), 7.92 (d, *J* = 8.0 Hz, 1H), 7.73 (s, 1H), 7.66 (t, *J* = 8.0 Hz, 1H), 6.52 (d, *J* = 8.0 Hz, 1H), 3.91 (s, 3H).

### Step B: 2-chloro-6-cyclopropyl-7-(6-methoxypyridin-2-yl)-7H-pyrrolo[2,3-d]pyrimidine

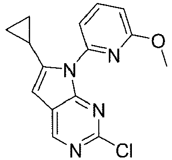

According to the method of step C of example 131, 2-chloro-5-iodo-N-(6-methoxy pyridin-2-yl)pyrimidin-4-amine and cyclopropylacetylene were used as the main starting material to obtain the product (110 mg).

¹H NMR (400 MHz, CDCl₃) δ 8.69 (s, 1H), 7.81 (t, *J* = 8.0 Hz, 1H), 7.24 (d, *J* = 8.0 Hz, 1H), 6.84 (d, *J* = 8.0 Hz, 1H), 6.23 (s, 1H), 3.92 (s, 3H), 2.15-2.19 (m, 1H), 0.94-0.95 (m, 2H), 0.76-0.81 (m, 2H).

### Step C: 6-cyclopropyl-7-(6-methoxypyridin-2-yl)-N-(6-(4-methylpiperazin-1-yl)pyridin-3-yl)-7H-pyrrolo[2,3-d]pyrimidin-2-amine

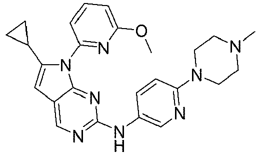

According to the method of step D of example 98, 2-chloro-6-cyclopropyl-7-(6-methoxy pyridin-2-yl)-7*H*-pyrrolo[2,3-*d*]pyrimidine and 6-(4-methylpiperazin-1-yl)pyridin-3-amine were used as the main starting material to obtain the product (8 mg).

¹H NMR (400 MHz CDCl₃) δ 8.50 (s, 1H), 8.31 (s, 1H), 8.02(d, *J =* 8.0 Hz, 1H), 7.79 (t, *J* = 8.0 Hz, 1H), 7.29 (d, *J* = 8.0 Hz, 1H), 7.08 (d, *J* = 8.0 Hz, 1H), 6.79 (d, *J* = 8.0 Hz, 1H), 6.61 (d, *J* = 8.8 Hz, 1H), 6.12 (s, 1H), 3.93 (s, 3H), 3.65-3.80 (m, 4H), 2.85-2.96 (m, 4H), 2.61 (s, 3H), 2.20-2.25 (m, 1H), 0.84-0.88 (m, 2H), 0.71-0.73 (m, 2H).

### Example 133

### N-(2-(6-(6-cyclopropyl-5-fluoro-2-((4-(9-methyl-3,9-diazaspiro[5.5]undecan-3-yl)phenyl)am ino)-7H-pyrrolo[2,3-d]pyrimidin-7-yl)pyridin-2-yl)prop-2-yl)acetamide

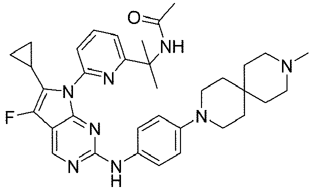

To a flask were charged with 2-(6-(6-cyclopropyl-5-fluoro-2-((4-(9-methyl- 3,9-diazaspiro [5.5]undecan-3-yl)phenyl)amino)-7*H*-pyrrolo[2,3-d]pyrimidin-7-yl)pyridin-2-yl)propan-2-ol (20 mg), acetonitrile (3 mL) and 98% boron trifluoride diethyl etherate (0.1 mL). Under nitrogen gas protection, the mixture was heated at 70 °C overnight. The mixture was cooled to room temperature, water was added to quench the reaction, the mixture was extracted with dichloromethane, the organic phase was dried and concentrated, and the residue was purified through preparative TLC (methanol : dichloromethane =20:1) to obtain the target product (20 mg).

¹H NMR (400 MHz CDCl₃) δ 8.60 (s, 1H), 7.90 (t, *J* = 8.0 Hz, 1H), 7.69 (d, *J* = 8.0 Hz, 1H), 7.48(d, *J =* 8.8 Hz, 2H), 7.42(d, *J =* 8.0 Hz, 1H), 7.18 (s, 1H), 7.02 (s, 1H), 6.86 (d, *J* = 8.8 Hz, 2H), 3.18-3.21 (m, 4H), 3.07-3.10 (m, 4H), 2.77 (s, 3H), 2.18-2.20 (m, 1H), 2.02 (s, 3H), 1.89-2.01 (m, 4H), 1.67-1.73 (m, 10H), 0.83-0.87 (m, 2H), 0.75-0.76 (m, 2H).

### Example 134

### ((6-(5-chloro-6-propyl-2-((4-(4-methylpiperazin-1-yl)phenyl)amino)-7H-pyrrolo[2,3-d]pyrim idin-7-yl)pyridin-2-yl)imino)dimethyl-λ⁶-sulfanone

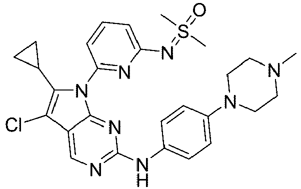

### Step A: ((6-(2,5-dichloro-6-cyclopropyl-7H-pyrrolo[2,3-d]pyrimidin-7-yl)pyridin-2-yl)imino) dimethyl-λ⁶-sulfanone

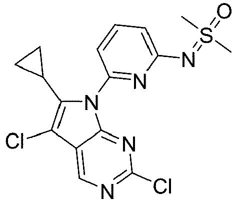

At room temperature, ((6-(2-Chloro-6-cyclopropyl-7*H*-pyrrolo[2,3-d]pyrimidin-7-yl) pyridin-2-yl)imino)dimethyl-λ⁶-sulfanone (100 mg) was dissolved in dichloromethane (5 mL), to the solution was added N-chlorosuccinimide (41 mg), the mixture was stirred for 48 hours, water was added to quench the reaction, the mixture was extracted with dichloromethane, the organic phase was dried, evaporated to remove the solvent, and purified through preparative TLC (100% ethyl acetate ) to obtain the product (63 mg).

¹H NMR (400 MHz CDCl₃) δ 8.76 (s, 1H), 7.71 (t, *J* = 8.0 Hz, 1H), 7.00 (d, *J* = 7.6 Hz, 1H), 6.86 (d, *J* = 8.0 Hz, 1H), 3.38 (s, 6H), 2.07-2.11 (m, 1H), 1.01-1.07 (m, 2H), 0.80-0.85 (m, 2H).

### Step B: ((6-(5-chloro-6-propyl-2-((4-(4-methylpiperazin-1-yl)phenyl)amino)-7H-pyrrolo [2,3-d]pyrimidin-7-yl)pyridin-2-yl)imino)dimethyl-λ⁶-sulfanone

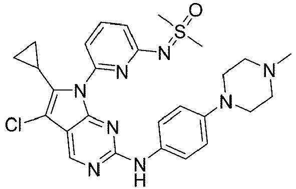

According to the method of step D of example 98, ((6-(2,5-dichloro-6-cyclopropyl-7*H-*pyrrolo[2,3-d]pyrimidin-7-yl)pyridin-2-yl)imino)dimethyl-λ⁶-sulfanone and 4-(4-methyl piperazin-1-yl)aniline were used as the main starting material to obtain the product (10 mg).

¹H NMR (400 MHz CDCl₃) δ 8.54 (s, 1H), 7.74 (t, *J =* 8.0 Hz, 1H), 7.54(d, *J =* 8.0 Hz, 2H), 7.15 (d, *J* = 8.0 Hz, 1H), 6.81-6.86 (m, 4H), 3.25-3.40 (m, 4H), 3.19 (s, 6H), 2.67-2.80 (m, 4H), 2.45(s, 3H), 2.19(m, 1H), 0.82-0.89 (m, 2H), 0.68-0.72 (m, 2H).

### Example 135

### dimethyl((6-(2-((4-(4-methylpiperazin-1-yl)phenyl)amino)-6-(prop-1-en-2-yl)-7H-pyrrolo[2, 3-d]pyrimidin-7-yl)pyridin-2-yl)imino)-λ⁶-sulfanone

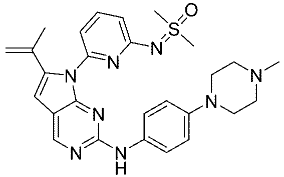

### Step A: ((6-(2-chloro-6-(2-hydroxyprop-2-yl)-7H-pyrrolo[2,3-d]pyrimidin-7-yl)pyridin-2-yl) imino)dimethyl-λ⁶-sulfanone

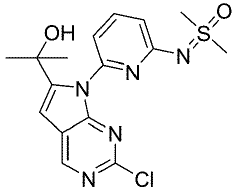

At room temperature, to a tube charged with DMF/TEA(2 ml/2 ml) were added ((6-((5-iodo-2-chloropyrimidin-4-yl)amino)pyridin-2-yl)imino)dimethyl-λ⁶-sulfanone (226 mg), copper(I) iodide (12 mg) and Pd(dppf)Cl₂ (42 mg), the system was purged with nitrogen gas, 2-methylbut-3-yn-2-ol (45 mg) was added, the mixture was cooled to 0 °C, stirred for 7 hours, heated to 50 °C, stirred overnight, cooled to room temperature, filtered, and washed with dichloromethane, the solvent was removed from the filtrate, and the residue was purified through silica gel column chromatography (DCM/MeOH=30/1) to obtain the product (70 mg).

¹H NMR (400 MHz, CDCl₃), 8.80 (s, 1H), 7.81 (t, *J* = 8.0 Hz, 1H), 7.12 (d, *J* = 8.0 Hz, 1H), 6.91 (d, *J* = 8.0 Hz, 1H), 6.63 (s, 1H), 5.77 (s, 1H), 3.25 (s, 6H), 1.48 (s, 6H).

### Step B: ((6-(2-chloro-6-(prop-1-en-2-yl)-7H-pyrrolo[2,3-d]pyrimidin-7-yl)pyridin-2-yl)imino) dimethyl-λ⁶-sulfanone

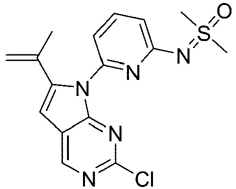

To a round-bottom flask charged with toluene (10 mL) were added ((6-(2-chloro-6-(2-hydroxyprop-2-yl)-7*H*-pyrrolo[2,3-d]pyrimidin-7-yl)pyridin-2-yl)imino)dimethyl-λ⁶-sulfanone (150 mg) and p-toluenesulfonic acid (34 mg), the mixture was heated to 90 °C and stirred for 3 hours, an aqueous solution of saturated sodium bicarbonate was used to adjust the pH to 10, the mixture was extracted with dichloromethane (100 mL x 4), and the organic phases were combined, evaporated to dryness, and purified through silica gel column chromatography (DCM/EA=4/1) to obtain the product (60 mg).

¹H NMR (400 MHz, CDCl₃), 8.78 (s, 1H), 7.66 (t, *J* = 8.0 Hz, 1H), 6.84-6.88 (m, 2H), 6.59 (s, 1H), 5.21-5.23 (m, 1H), 5.12-5.14 (m, 1H), 3.42 (s, 6H), 1.90 (s, 3H).

### Step C: dimethyl((6-(2-((4-(4-methylpiperazin-1-yl)phenyl)amino)-6-(prop-1-en-2-yl)-7H-pyrrolo[2,3-d]pyrimidin-7-yl)pyridin-2-yl)imino)-λ⁶-sulfanone

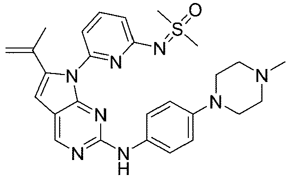

According to the method of step E of example 104, ((6-(2-chloro-6-(prop-1-en-2-yl)-7*H*-pyrrolo[2,3-d]pyrimidin-7-yl)pyridin-2-yl)imino)dimethyl-λ⁶-sulfanone and 4-(4-methyl piperazin-1-yl)aniline were used as the starting material to obtain the product (30 mg).

¹H NMR (400 MHz, CDCl₃), 8.60 (s, 1H), 7.73 (t, *J* = 8.0 Hz, 1H), 7.50 (d, *J* = 8.8 Hz, 2H), 7.10 (d, *J* = 7.6 Hz, 1H), 7.03-7.08 (brs, 1H), 6.82-6.86 (m, 3H), 6.48 (s, 1H), 5.05 (s, 1H), 4.95 (s, 1H), 3.39-3.42 (m, 4H), 3.25 (s, 6H), 3.02-3.13 (m, 4H), 2.69 (s, 3H), 1.92 (s, 3H).

### Example 136

### dimethyl((6-(2-((4-(4-(4-methylpiperazin-1-yl)piperidin-1-yl)phenyl)amino)-6-propyl-7H-py rrolo[2,3-d]pyrimidin-7-yl)pyridin-2-yl)imino)-λ⁶-sulfanone

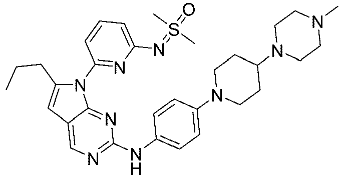

Under nitrogen gas, ((6-(2-chloro-6-propyl-7*H*-pyrrolo[2,3-d]pyrimidin-7-yl)pyridin-2-yl) imino)dimethyl-λ⁶-sulfanone (0.075 g), 4-(4-(4-methylpiperazin-1-yl)piperidin-1-yl)aniline (0.032 g), Pd₂(dba)₃ (0.019 g), Dave-phos (0.025 g) and sodium tert-butoxide (0.03 g) were mixed in toluene (10 mL), the mixture was heated to 106 °C and stirred overnight, the reaction solution was extracted through water and dichloromethane, the solvent was removed from the solution, and the residue was purified through preparative TLC (100%EtOAc) to obtain the product (40 mg).

¹H NMR (400MHz, CDCl₃), 8.52 (s, 1H), 7.72 (t, *J =* 8.0 Hz, 1H), 7.48 (d, *J =* 8.8 Hz, 2H), 7.16 (d, *J* = 8.0 Hz, 1H), 6.99 (s, 1H), 6.84 (d, *J* = 8.8 Hz, 2H), 6.80 (d, *J* = 8.0 Hz, 1H), 6.22 (s, 1H), 3.58-3.64 (m, 2H), 3.28 (s, 6H), 2.81-3.05 (m, 10H), 2.58-2.69 (m, 3H), 2.55 (s, 3H), 1.95-2.04 (m, 2H), 1.68-1.79 (m, 2H), 1.49-1.58 (m, 2H), 0.91 (t, *J* = 7.2 Hz, 3H).

### Example 137

### 2-(6-(2-((4-(4-(4-methylpiperazin-1-yl)piperidin-1-yl)phenyl)amino)-6-propyl-7H-pyrrolo[2, 3-d]pyrimidin-7-yl)pyridin-2-yl)propan-2-ol

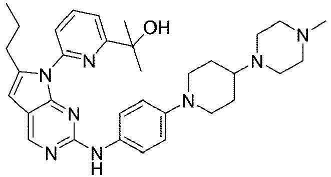

According to the method of step E of example 104, 2-(6-(2-chloro-6-propyl-7*H*-pyrrolo [2,3-d]pyrimidin-7-yl)pyridin-2-yl)propan-2-ol and 1-methyl-4-(1-(4-aminophenyl) piperidin-4-yl) piperazine were used as the starting material to obtain the product (20 mg).

¹H NMR (400 MHz, CDCl₃), 8.54 (s, 1H), 7.94 (t, *J =* 8.0 Hz, 1H), 7.73 (d, *J =* 7.6 Hz, 1H), 7.49 (d, *J* = 8.8 Hz, 2H), 7.45 (d, *J* = 7.6 Hz, 1H), 6.98 (s, 1H), 6.85 (d, *J* = 9.2 Hz, 2H), 6.29 (s, 1H), 4.02-4.22 (brs, 1H), 3.60-3.64 (m, 2H), 2.92-3.12 (m, 8H), 2.90 (t, *J* = 7.6 Hz, 2H), 2.61-2.70 (m, 3H), 2.59 (s, 3H), 1.98-2.04 (m, 2H), 1.70-1.80 (m, 2H), 1.60 (s, 6H), 1.48-1.58 (m, 2H), 0.91 (t, *J* = 7.2 Hz, 3H).

### Example 138

### ((6-(6-isopropyl-2-((4-(4-(4-methylpiperazin-1-yl)piperidin-1-yl)phenyl)amino)-7H-pyrrolo[ 2,3-d]pyrimidin-7-yl)pyridin-2-yl)imino)dimethyl-λ⁶-sulfanone

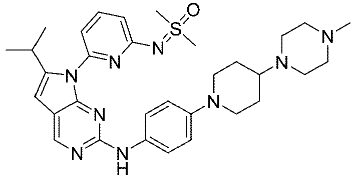

According to the method of step E of example 104, ((6-(2-chloro-6-isopropyl-7*H*-pyrrolo [2,3-d]pyrimidin-7-yl)pyridin-2-yl)imino)dimethyl-λ⁶-sulfanone and 1-methyl-4-(1-(4-amino phenyl)piperidin-4-yl)piperazine were used as the starting material to obtain the product (30 mg).

¹H NMR (400 MHz, CDCl₃), 8.53 (s, 1H), 7.74 (t, *J* = 8.0 Hz, 1H), 7.47 (d, *J* = 8.8 Hz, 2H), 7.15 (d, *J* = 7.2 Hz, 1H), 6.82-6.86 (m, 4H), 6.25 (s, 1H), 3.59-3.69 (m, 3H), 3.27 (s, 6H), 2.46-2.78 (m, 10H), 2.38-2.42 (m, 1H), 2.33 (s, 3H), 1.92-1.95 (m, 2H), 1.64-1.74 (m, 2H), 1.15 (d, *J* = 7.2 Hz, 6H).

### Example 139

### ((6-(6-methyl-2-((4-(9-methyl-3,9-diazaspiro[5.5]undecan-3-yl)phenyl)amino)-7H-pyrrolo[2, 3-d]pyrimidin-7-yl)pyridin-2-yl)imino)dimethyl-λ⁶-sulfanone

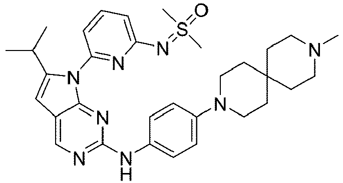

According to the method of step E of example 104, ((6-(2-chloro-6-isopropyl-7*H*-pyrrolo [2,3-d]pyrimidin-7-yl)pyridin-2-yl)imino)dimethyl-λ⁶-sulfanone and 4-(9-methyl-3,9-diazaspiro [5.5]undecan-3-yl)aniline were used as the starting material to obtain the product (10 mg).

¹H NMR (400MHz, CDCl₃), 8.53 (s, 1H), 7.73 (t, *J* = 8.0 Hz, 1H), 7.47 (d, *J* = 9.2 Hz, 2H), 7.14 (d, *J* = 7.2 Hz, 1H), 6.90 (s, 1H), 6.85 (d, *J* = 9.2 Hz, 2H), 6.83 (d, *J* = 8.0 Hz, 1H), 6.25 (s, 1H), 3.62-3.69 (m, 1H), 3.27 (s, 6H), 3.04-3.07 (m, 4H), 2.51-2.59 (m, 4H), 2.40 (s, 3H), 1.64-1.68 (m, 8H), 1.15 (d, *J* = 7.2 Hz, 6H).

### Example 140

### 2-(6-(6-isopropyl-2-((4-(4-(4-methylpiperazin-1-yl)piperidin-1-yl)phenyl)amino)-7H-pyrrolo [2,3-d]pyrimidin-7-yl)pyridin-2-yl)propan-2-ol

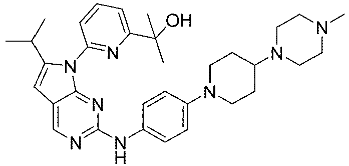

### Step A: 2-(6-(2-chloro-6-isopropyl-7H-pyrrolo[2,3-d]pyrimidin-7-yl)pyridin-2-yl) propan-2-ol

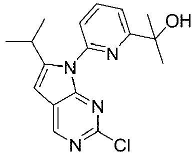

According to the method of step A of example 138, 2-(6-((2-chloro-5-iodopyrimidin-4-yl) amino)pyridin-2-yl)propan-2-ol and 3-methyl-1- butyne were used as the starting material to obtain the product (280 mg).

¹H NMR (400 MHz, CDCl₃), 8.75 (s, 1H), 7.98 (t, *J* = 8.0 Hz, 1H), 7.59 (d, *J* = 8.0 Hz, 1H), 7.53 (d, *J* = 7.6 Hz, 1H), 6.49 (s, 1H), 3.88 (s, 1H), 3.48-3.55 (m, 1H), 1.59 (s, 6H), 1.20 (d, *J* = 6.8 Hz, 6H).

### Step B: 2-(6-(6-isopropyl-2-((4-(4-(4-methylpiperazin-1-yl)piperidin-1-yl)phenyl) amino)-7H-pyrrolo[2,3-d]pyrimidin-7-yl)pyridin-2-yl)propan-2-ol

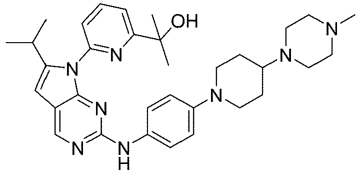

According to the method of step E of example 104, 2-(6-(2-chloro-6-isopropyl-7*H*-pyrrolo [2,3-d]pyrimidin-7-yl)pyridin-2-yl)propan-2-ol and 1-methyl-4-(1-(4-aminophenyl)piperidin-4-yl) piperazine were used as the starting material to obtain the product (20 mg).

¹H NMR (400 MHz, CDCl₃), 8.55 (s, 1H), 7.94 (t, *J* = 8.0 Hz, 1H), 7.69 (d, *J* = 8.0 Hz, 1H), 7.45-7.49 (m, 3H), 7.04 (s, 1H), 6.84 (d, *J* = 8.8 Hz, 2H), 6.31 (s, 1H), 4.02-4.28 (brs, 1H), 3.53-3.62 (m, 3H), 2.84-3.02 (m, 8H), 2.58-2.68 (m, 3H), 2.55 (s, 3H), 1.97-2.01 (m, 2H), 1.69-1.79 (m, 2H), 1.59 (s, 6H), 1.16 (d, *J* = 7.2 Hz, 6H).

### Example 141

### 2-(6-(6-isopropyl-2-((4-(4-methylpiperazin-1-yl)phenyl)amino)-7H-pyrrolo[2,3-d]pyrimidin-7-yl)pyridin-2-yl)propan-2-ol

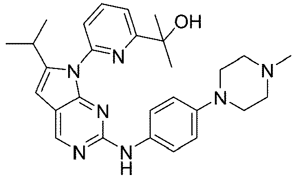

According to the method of step E of example 104, 2-(6-(2-chloro-6-isopropyl-7*H*-pyrrolo [2,3-d]pyrimidin-7-yl)pyridin-2-yl)propan-2-ol and 4-(4-methylpiperazin-1-yl)aniline were used as the starting material to obtain the product (20 mg).

¹H NMR (400 MHz, CDCl₃), 8.56 (s, 1H), 7.95 (t, *J* = 8.0 Hz, 1H), 7.71 (d, *J* = 8.4 Hz, 1H), 7.50 (d, *J* = 8.8 Hz, 2H), 7.46 (d, *J* = 7.6 Hz, 1H), 6394 (s, 1H), 6.86 (d, *J* = 8.8 Hz, 2H), 6.33 (s, 1H), 4.02-418 (brs, 1H), 3.54-3.61 (m, 1H), 3.22-3.25 (m, 4H), 2.74-2.82 (m, 4H), 2.48 (s, 3H), 1.60 (s, 6H), 1.18 (d, *J* = 6.4 Hz, 6H).

### Example 142

### 2-(6-(6-isopropyl-2-((4-(9-methyl-3,9-diazaspiro[5.5]undecan-3-yl)phenyl)amino)-7H-pyrrol o[2,3-d]pyrimidin-7-yl)pyridin-2-yl)propan-2-ol

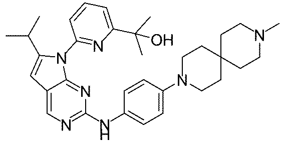

According to the method of step E of example 104, 2-(6-(2-chloro-6-isopropyl-7*H*-pyrrolo [2,3-d]pyrimidin-7-yl)pyridin-2-yl)propan-2-ol and 4-(9-methyl-3,9-diazaspiro[5.5]undecan-3-yl) were used as the starting material to obtain the product (39 mg).

¹H NMR (400MHz, CDCl₃), 8.55 (s, 1H), 7.95 (t, *J* = 7.6 Hz, 1H), 7.69 (d, *J* = 7.6 Hz, 1H), 7.49 (d, *J* = 9.2 Hz, 2H), 7.46 (d, *J* = 7.6 Hz, 1H), 7.06-7.12 (brs, 1H), 6.85 (d, *J* = 9.2 Hz, 2H), 6.33 (s, 1H), 4.06-4.16 (brs, 1H), 3.52-3.59 (m, 1H), 3.24-3.48 (m, 2H), 3.06-3.08 (m, 4H), 2.80-2.97 (m, 2H), 2.77 (s, 3H), 1.66-2.22 (m, 8H), 1.60 (s, 6H), 1.17 (d, *J* = 6.8 Hz, 6H).

### Example 143

### 2-(6-(2-((4-(4-(2-(dimethylamino)ethyl)piperazin-1-yl)phenyl)amino)-6-isopropyl-7H-pyrrol o[2,3-d]pyrimidin-7-yl)pyridin-2-yl)propan-2-ol

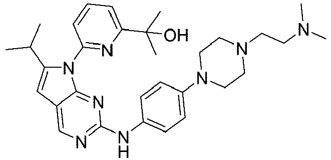

According to the method of step E of example 104, 2-(6-(2-chloro-6-isopropyl-7*H*-pyrrolo [2,3-d]pyrimidin-7-yl)pyridin-2-yl)propan-2-ol and 4-(4-(2-(dimethylamino)ethyl) piperazin-1-yl) aniline were used as the starting material to obtain the product (5 mg).

¹H NMR (400MHz, CDCl₃), 8.54 (s, 1H), 7.96 (t, *J* = 8.0 Hz, 1H), 7.68 (d, *J* = 7.6 Hz, 1H), 7.46-7.51 (m, 3H), 7.14-7.22 (brs, 1H), 6.85 (d, *J* = 8.4 Hz, 2H), 6.32 (s, 1H), 3.52-3.60 (m, 1H), 3.12-3.22 (m, 6H), 2.94-3.01 (m, 2H), 2.86 (s, 6H), 2.72-2.80 (m, 4H), 1.60 (s, 6H), 1.16 (d, *J* = 6.8 Hz, 6H).

### Example 144

### ((6-(2-((4-(4-(2-(dimethylamino)ethyl)piperazin-1-yl)phenyl)amino)-6-isopropyl-7H-pyrrolo[ 2,3-d]pyrimidin-7-yl)pyridin-2-yl)imino)dimethyl-λ⁶-sulfanone

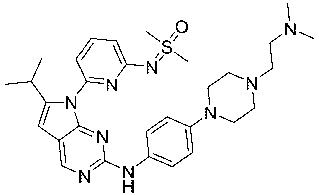

According to the method of step E of example 104, ((6-(2-chloro-6-isopropyl-7*H*-pyrrolo [2,3-d]pyrimidin-7-yl)pyridin-2-yl)imino)dimethyl-λ⁶-sulfanone and 4-(4-(2-(dimethylamino)ethyl) piperazin-1-yl)aniline were used as the starting material to obtain the product (6 mg).

¹H NMR (400MHz, CDCl₃), 8.44 (s, 1H), 7.88-8.01 (brs, 1H), 7.75 (t, *J* = 7.6 Hz, 1H), 7.49 (d, *J* = 8.8 Hz, 2H), 7.10 (d, *J* = 7.6 Hz, 1H), 6.84-6.86 (m, 3H), 6.29 (s, 1H), 3.59-3.64 (m, 1H), 3.25 (s, 6H), 3.16-3.21 (m, 4H), 3.14 (t, *J* = 6.0 Hz, 2H), 2.97 (t, *J* = 6.0 Hz, 2H), 2.85 (s, 6H), 2.74-2.78 (m, 4H), 1.15 (d, *J* = 6.8 Hz, 6H).

### Example 145

### 2-(6-(6-methyl-2-((4-(4-methylpiperazin-1-yl)phenyl)amino)-7H-pyrrolo[2,3-d]pyrimidin-7-yl)pyridin-2-yl)propan-2-ol

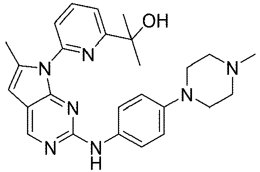

### Step A: 2-(6-(2-chloro-6-methyl-7H-pyrrolo[2,3-d]pyrimidin-7-yl)pyridin-2-yl)propan-2-ol

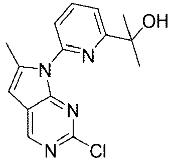

At room temperature, to a round-bottom flask charged with DMF/TEA (5 mL/5 mL) were added 2-(6-((2-chloro-5-iodopyrimidin-4-yl)amino)pyridin-2-yl)propan-2-ol (1.18 g), copper(I) iodide (285 mg), Pd(dppf)Cl₂ (215 mg), potassium fluoride dihydrate (250 mg), and trimethyl(prop-1-yn-1-yl)silane (336 mg), the mixture was heated to 80 °C, stirred overnight, cooled to room temperature, filtered, and washed with dichloromethane, the solvent was removed from the filtrate, and the residue was purified through silica gel column chromatography (DCM/EA=10/1) to obtain the product (170 mg).

¹H NMR (400 MHz, CDCl₃), 8.74 (s, 1H), 7.97 (t, *J* = 8.0 Hz, 1H), 7.66 (d, *J* = 8.0 Hz, 1H), 7.51 (d, *J* = 8.0 Hz, 1H), 6.45 (s, 1H), 3.90 (s, 1H), 2.55 (s, 3H), 1.59 (s, 6H).

### Step B: 2-(6-(6-methyl-2-((4-(4-methylpiperazin-1-yl)phenyl)amino)-7H-pyrrolo[2,3-d] pyrimidin-7-yl)pyridin-2-yl)propan-2-ol

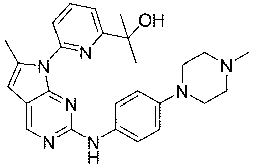

According to the method of step E of example 104, 2-(6-(2-chloro-6-methyl-7*H*-pyrrolo [2,3-d]pyrimidin-7-yl)pyridin-2-yl)propan-2-ol and 4-(4-methylpiperazin-1-yl)aniline were used as the starting material to obtain the product (20 mg).

¹H NMR (400 MHz, CD₃OD), 8.49 (s, 1H), 8.01 (t, *J* = 8.0 Hz, 1H), 7.70-7.72 (m, 2H), 7.55 (d, *J* = 9.2 Hz, 2H), 6.90 (d, *J* = 8.8 Hz, 2H), 6.30 (s, 1H), 3.18-3.22 (m, 4H), 2.91-2.96 (m, 4H), 2.58 (s, 3H), 2.47 (s, 3H), 1.58 (s, 6H).

### Example 146

### 2-(6-(2-((4-(4-(2-(dimethylamino)ethyl)piperazin-1-yl)phenyl)amino)-6-methyl-7H-pyrrolo[2, 3-d]pyrimidin-7-yl)pyridin-2-yl)propan-2-ol

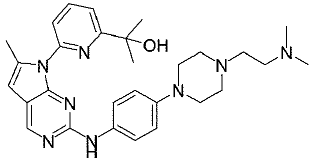

According to the method of step E of example 104, 2-(6-(2-chloro-6-methyl-7*H*-pyrrolo [2,3-d]pyrimidin-7-yl)pyridin-2-yl)propan-2-ol and 4-(4-(2-(dimethylamino)ethyl) piperazin-1-yl) aniline were used as the starting material to obtain the product (10 mg).

¹H NMR (400MHz, CDCl₃), 8.53 (s, 1H), 7.95 (t, *J* = 8.0 Hz, 1H), 7.79 (d, *J* = 8.0 Hz, 1H), 7.51 (d, *J* = 9.2 Hz, 2H), 7.44 (d, *J* = 8.0 Hz, 1H), 7.12-7.19 (brs, 1H), 6.88 (d, *J* = 8.4 Hz, 2H), 6.29 (s, 1H), 3.16-3.20 (m, 4H), 3.13 (t, *J* = 6.0 Hz, 2H), 2.97 (t, *J* = 6.0 Hz, 2H), 2.86 (s, 6H), 2.74-2.88 (m, 4H), 2.52 (s, 3H), 1.60 (s, 6H).

### Example 147

### 2-(6-(6-methyl-2-((4-(9-methyl-3,9-diazaspiro[5.5]undecan-3-yl)phenyl)amino)-7H-pyrrolo[ 2,3-d]pyrimidin-7-yl)pyridin-2-yl)propan-2-ol

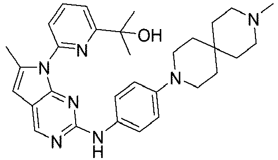

According to the method of step E of example 104, 2-(6-(2-chloro-6-methyl-7*H*-pyrrolo [2,3-d]pyrimidin-7-yl)pyridin-2-yl)propan-2-ol and 4-(9-methyl-3,9-diazaspiro[5.5]undecan-3-yl) were used as the starting material to obtain the product (40 mg).

¹H NMR (400MHz, CDCl₃), 8.54 (s, 1H), 7.93 (t, *J* = 8.0 Hz, 1H), 7.79 (d, *J* = 8.0 Hz, 1H), 7.51 (d, *J* = 9.2 Hz, 2H), 7.43 (d, *J* = 8.0 Hz, 1H), 6.96 (s, 1H), 6.87 (d, *J* = 8.8 Hz, 2H), 6.28 (s, 1H), 4.15 (s, 1H), 2.80-3.42 (m, 8H), 2.75 (s, 3H), 2.52 (s, 3H), 1.82-2.24 (m, 4H), 1.70-1.78 (m, 4H), 1.60 (s, 6H).

### Example 148

### 2-(6-(6-methyl-2-((4-(4-(4-methylpiperazin-1-yl)piperidin-1-yl)phenyl)amino)-7H-pyrrolo[2, 3-d]pyrimidin-7-yl)pyridin-2-yl)propan-2-ol

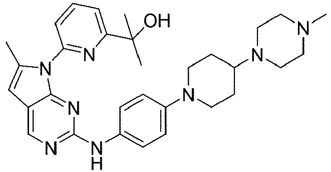

According to the method of step E of example 104, 2-(6-(2-chloro-6-methyl-7*H*-pyrrolo [2,3-d]pyrimidin-7-yl)pyridin-2-yl)propan-2-ol and 1-methyl-4-(1-(4-aminophenyl) piperidin-4-yl) piperazine were used as the starting material to obtain the product (20 mg).

¹H NMR (400 MHz, CDCl₃), 8.54 (s, 1H), 7.93 (t, *J* = 8.0 Hz, 1H), 7.79 (d, *J* = 8.4 Hz, 1H), 7.50 (d, *J* = 8.8 Hz, 2H), 7.44 (d, *J* = 8.0 Hz, 1H), 7.01 (s, 1H), 6.86 (d, *J* = 9.2 Hz, 2H), 6.28 (s, 1H), 4.06-4.22 (brs, 1H), 3.61-3.64 (m, 2H), 2.86-3.16 (m, 8H), 2.57-2.71 (m, 6H), 2.52 (s, 3H), 1.98-2.06 (m, 2H), 1.70-1.82 (m, 2H), 1.60 (s, 6H).

### Example 149

dimethyl((6-(6-methyl-2-((4-(9-methyl-3,9-diazaspiro[5.5]undecan-3-yl)phenyl)amino)-7*H*-p yrrolo[2,3-d]pyrimidin-7-yl)pyridin-2-yl)imino)-λ⁶-sulfanone

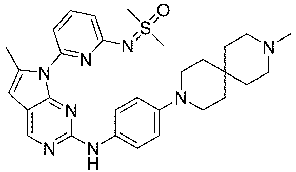

According to the method of step E of example 104, ((6-(2-chloro-6-methyl-7*H*-pyrrolo[2,3-d] pyrimidin-7-yl)pyridin-2-yl)imino)dimethyl-λ⁶-sulfanone and 4-(9-methyl-3,9-diazaspiro[5.5] undecan-3-yl)aniline were used as the starting material to obtain the product (35 mg).

¹H NMR (400MHz, CDCl₃), 8.51 (s, 1H), 7.72 (t, *J =* 8.0 Hz, 1H), 7.49 (d, *J =* 8.8 Hz, 2H), 7.21 (d, *J* = 8.8 Hz, 1H), 6.87 (d, *J* = 8.8 Hz, 2H), 6.86 (s, 1H), 6.79 (d, *J* = 8.0 Hz, 1H), 6.21 (s, 1H), 3.29 (s, 6H), 3.05-3.07 (m, 4H), 2.51 (s, 3H), 2.39-2.46 (m, 4H), 2.31 (s, 3H), 1.58-1.66 (m, 8H).

### Example 150

### ((6-(6-methyl-2-((4-(4-(4-methylpiperazin-1-yl)piperidin-1-yl)phenyl)amino)-7H-pyrrolo[2,3 -d]pyrimidin-7-yl)pyridin-2-yl)imino)-λ⁶-sulfanone

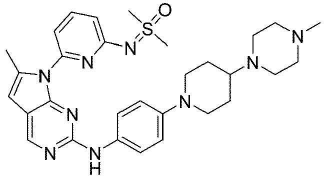

According to the method of step E of example CT-5132, ((6-(2-chloro-6-methyl-7*H*-pyrrolo [2,3-d]pyrimidin-7-yl)pyridin-2-yl)imino)dimethyl-λ⁶-sulfanone and 1-methyl-4-(1-(4-amino phenyl) piperidin-4-yl)piperazine were used as the starting material to obtain the product (30 mg).

¹H NMR (400 MHz, CDCl₃), 8.50 (s, 1H), 7.72 (t, *J =* 8.0 Hz, 1H), 7.49 (d, *J =* 8.8 Hz, 2H), 7.19 (d, *J* = 7.6 Hz, 1H), 7.01 (s, 1H), 6.84 (d, *J* = 8.8 Hz, 2H), 6.79 (d, *J* = 7.6 Hz, 1H), 6.20 (s, 1H), 3.58-3.62 (m, 2H), 3.28 (s, 6H), 2.91-3.04 (m, 8H), 2.59-2.68 (m, 3H), 2.57 (s, 3H), 2.50 (s, 3H), 1.98-2.02 (m, 2H), 1.69-1.78 (m, 2H).

### Example 151

### ((6-(2-((4-(4-(2-(dimethylamino)ethyl)piperazin-1-yl)phenyl)amino)-6-methyl-7H-pyrrolo[2, 3-d]pyrimidin-7-yl)pyridin-2-yl)imino)dimethyl-λ⁶-sulfanone

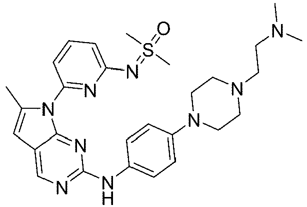

According to the method of step E of example 104, ((6-(2-chloro-6-methyl-7*H*-pyrrolo[2,3-d] pyrimidin-7-yl)pyridin-2-yl)imino)dimethyl-λ⁶-sulfanone and 4-(4-(2-(dimethylamino)ethyl) piperazin-1-yl)aniline were used as the starting material to obtain the product (20 mg).

¹H NMR (400MHz, CDCl₃), 8.50 (s, 1H), 7.71 (t, *J =* 8.0 Hz, 1H), 7.49 (d, *J =* 8.8 Hz, 2H), 7.19 (d, *J* = 7.6 Hz, 1H), 6.93 (s, 1H), 6.84 (d, *J* = 8.8 Hz, 2H), 6.78 (d, *J* = 8.0 Hz, 1H), 6.20 (s, 1H), 3.28 (s, 6H), 3.11-3.14 (m, 4H), 2.74-2.84 (m, 4H), 2.67-2.70 (m, 4H), 2.58 (s, 6H), 2.50 (s, 3H).

### Example 152

### ((6-(6-ethyl-2-((4-(4-methylpiperazin-1-yl)phenyl)amino)-7H-pyrrolo[2,3-d]pyrimidin-7-yl)p yridin-2-yl)imino)dimethyl-λ⁶-sulfanone

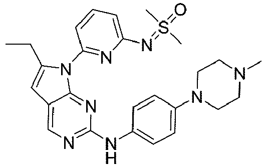

### Step A: ((6-(2-chloro-6-ethyl-7H-pyrrolo[2,3-d]pyrimidin-7-yl)pyridin-2-yl)imino)dimethyl-λ⁶-sulfanone

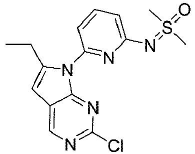

A solution of ((6-((5-iodo-2-chloropyrimidin-4-yl)amino)pyridin-1-yl)imino) dimethyl-λ⁶-sulfanone (400 mg), 1-trimethylsilyl-1-butyne (120 mg), copper(I) iodide (92 mg), Pd(dppf)Cl₂ (67 mg), potassium fluoride trihydrate (97 mg) and triethylamine (5 mL) in N,N-dimethylformamide (10 mL) was sufficiently purged with nitrogen gas, heated to 80 °C, and reacted for 36 hours. The reaction solution was cooled to room temperature, diluted by addition of dichloromethane, and washed with water, the solvent was evaporated, and the residue was purified through silica gel column chromatography (5%MeOH/DCM) to obtain the target product (230 mg).

¹H NMR (400 MHz, CDCl₃) δ 8.67 (s, 1H), 7.95 (s, 1H), 7.67 (t, *J =* 8.0 Hz, 1H), 6.92(d, *J* = 8.0 Hz, 1H), 6.79(d, *J* = 8.0 Hz, 1H), 3.32 (s, 6H), 2.88(q, *J* = 7.2 Hz, 2H), 1.20 (t, *J =* 7.2 Hz, 3H).

### Step B: ((6-(6-ethyl-2-((4-(4-methylpiperazin-1-yl)phenyl)amino)-7H-pyrrolo[2,3-d] pyrimidin-7-yl)pyridin-2-yl)imino)dimethyl-λ⁶-sulfanone

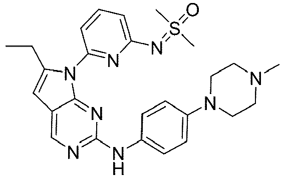

According to the method of step D of example 98, ((6-(2-chloro-6-ethyl-7*H*-pyrrolo[2,3-d] pyrimidin-7-yl)pyridin-2-yl)imino)dimethyl-λ⁶-sulfanone and 4-(4-methylpiperazin-1-yl)aniline were used as the main starting material to obtain the product (20 mg).

¹H NMR (400 MHz, CDCl₃) δ 8.52 (s, 1H), 7.75 (t, *J* = 8.0 Hz, 1H), 7.55 (d, *J* = 8.8 Hz, 2H), 7.45 (s, 1H), 7.16 (d, *J* = 8.0 Hz, 1H), 6.82-6.87 (m, 3H), 6.26 (s, 1H), 3.40-3.48 (m, 4H), 3.33 (s, 6H), 3.09-3.15 (m, 4H), 2.95(q, *J* = 7.2 Hz, 2H), 2.79 (s, 3H), 1.20 (t, *J =* 7.2 Hz, 3H).

### Example 153

### ((6-(6-ethyl-2-((4-(9-methyl-3,9-diazaspiro[5.5]undecan-3-yl)phenyl)amino)-7H-pyrrolo[2,3-d]pyrimidin-7-yl)pyridin-2-yl)imino)dimethyl-λ⁶-sulfanone

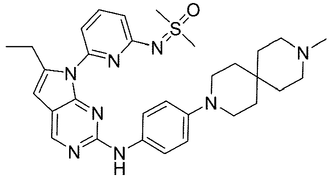

According to the method of step D of example 98, ((6-(2-chloro-6-ethyl-7*H*-pyrrolo[2,3-d] pyrimidin-7-yl)pyridin-2-yl)imino)dimethyl-λ⁶-sulfanone and 4-(9-methyl-3,9-diazaspiro[5.5] undecan-3-yl)aniline were used as the main starting material to obtain the product (10 mg).

¹H NMR (400 MHz, CDCl₃) δ 8.52 (s, 1H), 7.72 (t, *J* = 8.0 Hz, 1H), 7.50 (d, *J* = 8.8 Hz, 2H), 7.17 (d, *J* = 8.0 Hz, 1H), 6.79-6.1 (m, 4H), 6.23 (s, 1H), 3.28 (s, 6H), 3.04-3.08 (m, 4H), 2.95(q, *J* = 7.2 Hz, 2H), 2.55-2.65 (m, 4H), 2.41 (s, 3H), 1.80-2.00 (m, 4H), 1.64-1.69 (m, 4H), 1.18 (t, *J* = 7.2 Hz, 3H).

### Example 154

### ((6-(6-ethyl-2-((4-(4-(4-methylpiperazin-1-yl)piperidin-1-yl)phenyl)amino)-7H-pyrrolo[2,3-d] pyrimidin-7-yl)pyridin-2-yl)imino)dimethyl-λ⁶-sulfanone

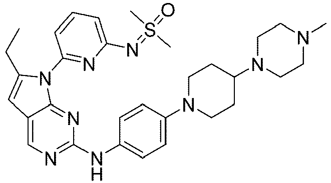

According to the method of step E of example 104, ((6-(2-chloro-6-ethyl-7*H*-pyrrolo[2,3-d] pyrimidin-7-yl)pyridin-2-yl)imino)dimethyl-λ⁶-sulfanone and 1-methyl-4-(1-(4-aminophenyl) piperidin-4-yl) piperazine were used as the starting material to obtain the product (18 mg).

¹H NMR (400 MHz, CDCl₃), 8.53 (s, 1H), 7.72 (t, *J =* 8.0 Hz, 1H), 7.48 (d, *J =* 8.8 Hz, 2H), 7.17 (d, *J* = 8.0 Hz, 1H), 6.84-6.87 (m, 3H), 6.80 (d, *J* = 8.0 Hz, 1H), 6.23 (s, 1H), 3.60-3.63 (m, 2H), 3.28 (s, 6H), 2.95 (q, *J* = 7.6 Hz, 2H), 2.72-2.92 (m, 9H), 2.62-2.69 (m, 2H), 2.47 (s, 3H), 1.95-2.01 (m, 2H), 1.68-1.78 (m, 2H), 1.19 (t, *J* = 7.6 Hz, 3H).

### Example 155

### ((6-(2-((4-(4-(2-(dimethylamino)ethyl)piperazin-1-yl)phenyl)amino)-6-ethyl-7H-pyrrolo[2,3-d]pyrimidin-7-yl)pyridin-2-yl)imino)dimethyl-λ⁶-sulfanone

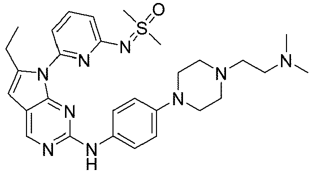

According to the method of step E of example 104, ((6-(2-chloro-6-ethyl-7*H*-pyrrolo[2,3-d] pyrimidin-7-yl)pyridin-2-yl)imino)dimethyl-λ⁶-sulfanone and 4-(4-(2-(dimethylamino)ethyl) piperazin- 1- yl) aniline were used as the starting material to obtain the product (18 mg).

¹H NMR (400 MHz, CD₃OD), 8.49 (s, 1H), 7.80 (t, *J* = 8.0 Hz, 1H), 7.47-7.53 (m, 2H), 7.14 (d, *J* = 7.6 Hz, 1H), 6.88-6.96 (m, 2H), 6.82 (d, *J* = 8.4 Hz, 1H), 6.35 (s, 1H), 3.31-3.35 (m, 8H), 3.12-3.24 (m, 4H), 2.86-2.96 (m, 8H), 2.71-2.82 (m, 6H), 1.16 (t, *J* = 7.6 Hz, 3H).

### Example 156

### 2-(6-(6-ethyl-2-((4-(4-(4-methylpiperazin-1-yl)piperidin-1-yl)phenyl)amino)-7H-pyrrolo[2,3-d]pyrimidin-7-yl)pyridin-2-yl)propan-2-ol

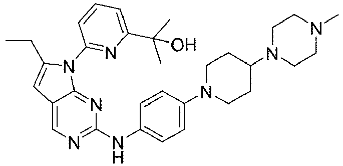

### Step A: 2-(6-(2-chloro-6-ethyl-7H-pyrrolo[2,3-d]pyrimidin-7-yl)pyridin-2-yl)propan-2-ol

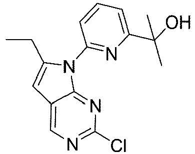

A solution of 2-(6-((2-chloro-5-iodopyrimidin-4-yl)amino)pyridin-2-yl)propan-2-ol (1.5 g), 1-trimethylsilyl-1- butyne (480 mg), copper(I) iodide (372 mg), Pd(dppf)Cl₂ (272 mg), potassium fluoride trihydrate (393 mg) and triethylamine (10 mL) in N,N-dimethylformamide (20 mL) was sufficiently purged with nitrogen gas, heated to 80 °C, and reacted for 36 hours. The reaction solution was cooled to room temperature, diluted by addition of dichloromethane, and washed with water, the solvent was evaporated, and the residue was purified through silica gel column chromatography (100% ethyl acetate ) to obtain the target product (0.52 g).

¹H NMR (400 MHz, CDCl₃) δ 8.64 (s, 1H), 7.88 (t, *J* = 8.0 Hz, 1H), 7.49-7.54 (m, 2H), 6.38 (s, 1H), 4.15 (s, 1H), 2.85(q, *J =* 7.2 Hz, 2H), 1.51 (s, 6H), 1.14 (t, *J* = 7.2 Hz, 3H).

### Step B: 2-(6-(6-ethyl-2-((4-(4-(4-methylpiperazin-1-yl)piperidin-1-yl)phenyl)amino)-7H-pyrrolo[2,3-d]pyrimidin-7-yl)pyridin-2-yl)propan-2-ol

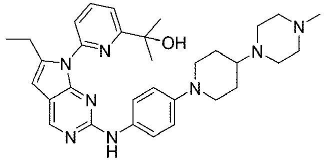

According to the method of step D of example 98, 2-(6-(2-chloro-6-ethyl-7*H*-pyrrolo[2,3-d] pyrimidin-7-yl)pyridin-2-yl)propan-2-ol and 1-methyl-4-(1-(4-aminophenyl)piperidin-4-yl) piperazine were used as the main starting material to obtain the product (11 mg).

¹H NMR (400 MHz, CDCl₃) δ 8.54 (s, 1H), 7.92 (t, *J =* 8.0 Hz, 1H), 7.74(d, *J =* 8.0 Hz, 1H), 7.42-7.49 (m, 3H), 7.07 (s, 1H), 6.85(d, *J* = 8.8 Hz, 2H), 6.29 (s, 1H), 4.15 (s, 1H), 3.46-3.62 (m, 2H), 2.70-2.96 (m, 10H), 2.65(q, *J* = 7.2 Hz, 2H), 2.40-2.60 (m, 4H), 1.90-1.99 (m, 2H), 1.70-1.76 (m, 2H), 1.59 (s, 6H), 1.17 (t, *J* = 7.2 Hz, 3H).

### Example 157

### 2-(6-(6-ethyl-2-((4-(9-methyl-3,9-diazaspiro[5.5]undecan-3-yl)phenyl)amino)-7H-pyrrolo[2, 3-d]pyrimidin-7-yl)pyridin-2-yl)propan-2-ol

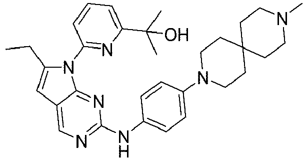

According to the method of step D of example 98, 2-(6-(2-chloro-6-ethyl-7*H*-pyrrolo[2,3-d] pyrimidin-7-yl)pyridin-2-yl)propan-2-ol and 4-(9-methyl-3,9-diazaspiro[5.5]undecan-3-yl)aniline were used as the main starting material to obtain the product (5 mg).

¹H NMR (400 MHz, CDCl₃) δ 8.53 (s, 1H), 7.92 (t, *J =* 8.0 Hz, 1H), 7.72(d, *J =* 8.0 Hz, 1H), 7.43-7.51 (m, 3H), 6.84(d, *J* = 8.4 Hz, 2H), 6.29 (s, 1H), 4.15 (s, 1H), 2.98-3.13 (m, 6H), 2.89-2.93 (m, 4H), 2.78(q, *J* = 7.2 Hz, 2H), 2.60-2.72 (m, 2H), 1.81-2.01 (m, 4H), 1.60-1.68 (m, 4H), 1.59 (s, 6H), 1.17 (t, *J* = 7.2 Hz, 3H).

### Example 158

### 2-(6-(2-((4-(4-(2-(dimethylamino)ethyl)piperazin-1-yl)phenyl)amino)-6-ethyl-7H-pyrrolo[2,3 -d]pyrimidin-7-yl)pyridin-2-yl)propan-2-ol

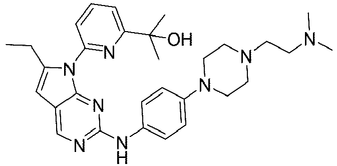

According to the method of step D of example 98, 2-(6-(2-chloro-6-ethyl-7*H*-pyrrolo[2,3-d] pyrimidin-7-yl)pyridin-2-yl)propan-2-ol and 4-(4-(2-(dimethylamino)ethyl)piperazin-1-yl)aniline were used as the main starting material to obtain the product (12 mg).

¹H NMR (400 MHz, CDCl₃) δ 8.55 (s, 1H), 7.93 (t, *J* = 8.0 Hz, 1H), 7.75(d, *J* = 8.0 Hz, 1H), 7.43-7.51 (m, 3H), 7.05 (s, 1H), 6.85(d, *J* = 9.2 Hz, 2H), 6.30 (s, 1H), 3.42 (s, 1H), 3.14 (s, 6H), 2.76-2.97 (m, 8H), 2.69(q, *J* = 7.2 Hz, 2H), 2.55-2.620 (m, 4H), 1.60 (s, 6H), 1.17 (t, *J =* 7.2 Hz, 3H).

### Example 159

### 2-(6-(6-ethyl-2-((4-(4-methylpiperazin-1-yl)phenyl)amino)-7H-pyrrolo[2,3-d]pyrimidin-7-yl) pyridin-2-yl)propan-2-ol

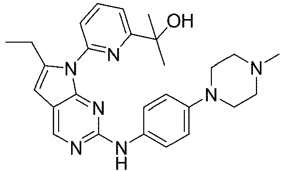

According to the method of step E of example 104, 2-(6-(2-chloro-6-ethyl-7*H*-pyrrolo[2,3-d] pyrimidin-7-yl)pyridin-2-yl)propan-2-ol and 4-(4-methylpiperazin-1-yl)aniline were used as the starting material to obtain the product (18 mg).

¹H NMR (400 MHz, CDCl₃), 8.56 (s, 1H), 7.94 (t, *J =* 8.0 Hz, 1H), 7.75 (d, *J =* 8.4 Hz, 1H), 7.52 (d, *J* = 9.2 Hz, 2H), 7.43 (d, *J* = 8.0 Hz, 1H), 6.97 (s, 1H), 6.87 (d, *J* = 8.8 Hz, 2H), 6.31 (s, 1H), 4.12-4.22 (brs, 1H), 3.22-3.25 (m, 4H), 2.94 (q, *J* = 6.8 Hz, 2H), 2.74-2.80 (m, 4H), 2.48 (s, 3H), 1.60 (s, 6H), 1.20 (t, *J* = 7.2 Hz, 3H).

### Example 160

### 2-(6-(6-(tert-butyl)-2-((4-(4-methylpiperazin-1-yl)phenyl)amino)-7H-pyrrolo[2,3-d]pyrimidi n-7-yl)pyridin-2-yl)propan-2-ol

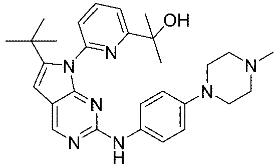

### Step A: 2-(6-(6-(tert-butyl)-2-chloro-7H-pyrrolo[2,3-d]pyrimidin-7-yl)pyridin-2-yl) propan-2-ol

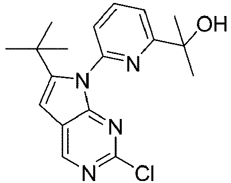

According to the method of step A of example CT-5274, 2-(6-((2-chloro-5-iodopyrimidin-4-yl)amino)pyridin-2-yl)propan-2-ol and 3,3-dimethyl-1- butyne were used as the starting material to obtain the product (180 mg).

¹H NMR (400 MHz, CDCl₃), 8.72 (s, 1H), 7.94 (t, *J* = 8.0 Hz, 1H), 7.61 (d, *J* = 7.6 Hz, 1H), 7.29 (d, *J* = 7.6 Hz, 1H), 6.49 (s, 1H), 3.94 (s, 1H), 1.58 (s, 6H), 1.24 (s, 9H).

### Step B: 2-(6-(6-ethyl-2-((4-(4-methylpiperazin-1-yl)phenyl)amino)-7H-pyrrolo[2,3-d] pyrimidin-7-yl)pyridin-2-yl)propan-2-ol

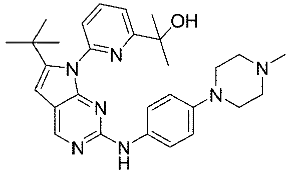

According to the method of step E of example 104, 2-(6-(6-(tert-butyl)-2-chloro-7*H*-pyrrolo [2,3-d]pyrimidin-7-yl)pyridin-2-yl)propan-2-ol and 4-(4-methylpiperazin-1-yl)aniline were used as the starting material to obtain the product (15 mg)

¹H NMR (400 MHz, CDCl₃), 8.54 (s, 1H), 7.94 (t, *J* = 8.0 Hz, 1H), 7.60 (d, *J* = 8.0 Hz, 1H), 7.32-7.35 (m, 3H), 7.08 (s, 1H), 6.73 (d, *J* = 8.8 Hz, 2H), 6.32 (s, 1H), 3.90-4.30 (brs, 1H), 3.26-3.31 (m, 4H), 2.92-2.99 (m, 4H), 2.61 (s, 3H), 1.60 (s, 6H), 1.24 (s, 9H).

### Example 161

### 2-(6-(6-(tert-butyl)-2-((4-(4-(4-methylpiperazin-1-yl)piperidin-1-yl)phenyl)amino)-7H-pyrrol o[2,3-d]pyrimidin-7-yl)pyridin-2-yl)propan-2-ol

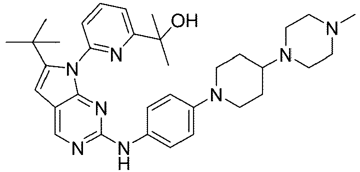

According to the method of step E of example 104, 2-(6-(6-(tert-butyl)-2-chloro-7*H-*pyrrolo[2,3-d]pyrimidin-7-yl)pyridin-2-yl)propan-2-ol and 1-methyl-4-(1-(4-aminophenyl) piperidin-4-yl)piperazine were used as the starting material to obtain the product (25 mg)

¹H NMR (400 MHz, CDCl₃), 8.53 (s, 1H), 7.92 (t, *J* = 8.0 Hz, 1H), 7.58 (d, *J* = 7.6 Hz, 1H), 7.30-7.33 (m, 3H), 6.96-7.04 (brs, 1H), 6.73 (d, *J* = 8.4 Hz, 2H), 6.31 (s, 1H), 3.96-4.36 (brs, 1H), 3.53-3.56 (m, 2H), 2.82-3.02 (m, 8H), 2.48-2.64 (m, 6H), 1.94-1.99 (m, 2H), 1.66-1.76 (m, 2H), 1.60 (s, 6H), 1.24 (s, 9H).

### Example 162

### ((6-(6-(2-tert-butyl)-2-((4-(4-methylpiperazin-1-yl)phenyl)amino)-7H-pyrrolo[2,3-d]pyrimidi n-7-yl)pyridin-2-yl)imino)dimethyl-λ⁶-sulfanone

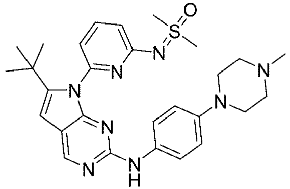

### Step A: ((6-((2-chloro-5-(3,3 -dimethylbut-1-yn-1-yl)pyrimidin-4-yl)amino)pyridin-2-yl) imino) dimethyl-λ⁶sulfanone

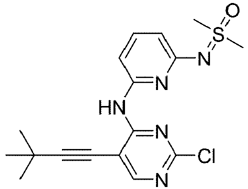

Under nitrogen gas, ((6-((2-chloro-5-iodopyrimidin-4-yl)amino)pyridin-2-yl)imino)dimethyl-λ⁶-sulfanone (1.000 g), 3,3-dimethylbut-1-yne (0.180 g), Pd(dppf)Cl₂ (0.169 g), and copper(I) iodide (0.225 g) were mixed and dissolved in DMF (15 mL) and TEA (15 mL), the mixture was stirred in an ice bath overnight and partitioned between water and dichloromethane, and the organic phase was concentrated to remove the solvent and purified through silica gel column chromatography (100%EtOAc) to obtain the product (0.355 g).

### Step B: ((6-(2-chloro-6-(2-tert-butyl)-7H-pyrrolo[2,3-d]pyrimidin-7-yl)pyridin-2-yl)imino) dimethyl-λ⁶-sulfanone

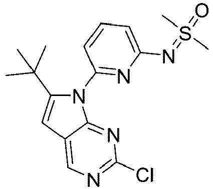

Under nitrogen gas, ((6-((2-chloro-5-(3,3-dimethylbut-1-yn-1-yl)pyrimidin-4-yl)amino) pyridin-2-yl)imino)dimethyl-λ⁶sulfanone (0.335 g) and copper(I) iodide (0.190 g) were mixed, DMF (10 mL) was used as the solvent, the mixture was heated to 153 °C, refluxed for 1 hour, partitioned between water and dichloromethane, and the organic phase was concentrated to remove the solvent and purified through silica gel column chromatography (100%EtOAc) to obtain the product (0.340 g).

¹H NMR (400 MHz, CDCl₃), 8.69 (s, 1H), 7.68 (t, *J* = 8.0 Hz, 1H), 6.93 (d, *J* = 8.0 Hz, 1H), 6.86 (d, *J* = 8.0 Hz, 1H), 6.42 (s, 1H), 3.25 (s, 6H), 1.27 (s, 9H).

### Step C: ((6-(6-(2-tert-butyl)-2-((4-(4-methylpiperazin-1-yl)phenyl)amino)-7H-pyrrolo[2,3-d] pyrimidin-7-yl)pyiridin-2-yl)imino)dimethyl-λ⁶-sulfanone

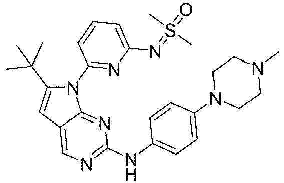

According to the method of step E of example 104, ((6-(2-chloro-6-(2-tert-butyl)-7*H-*pyrrolo[2,3-d]pyrimidin-7-yl)pyridin-2-yl)imino)dimethyl-λ⁶-sulfanone and 4-(4-methyl piperazin- 1-yl)aniline were used as the starting material to obtain the product (45 mg).

¹H NMR (400 MHz, CDCl₃), 8.51 (s, 1H), 7.70 (t, *J* = 8.0 Hz, 1H), 7.35 (d, *J* = 8.8 Hz, 2H), 6.94 (d, *J* = 8.0 Hz, 1H), 6.90 (d, *J* = 7.6 Hz, 1H), 6.88 (s, 1H), 6.77 (d, *J* = 8.8 Hz, 2H), 6.25 (s, 1H), 3.19 (s, 6H), 3.08-3.11 (m, 4H), 2.56-2.59 (m, 4H), 2.35 (s, 3H), 1.28 (s, 9H).

### Example 163

### ((6-(6-(2-tert-butyl)-2-((4-(4-(2-(dimethylamino)ethyl)piperazin-1-yl)phenyl)amino)-7H-pyrr olo[2,3-d]pyrimidin-7-yl)pyridin-2-yl)imino)dimethyl-λ⁶-sulfanone

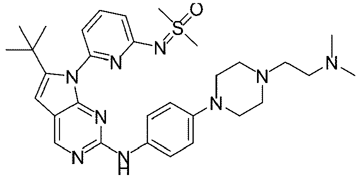

According to the method of step E of example 104, ((6-(2-chloro-6-(2-tert-butyl)-7*H*-pyrrolo[2,3-d]pyrimidin-7-yl)pyridin-2-yl)imino)dimethyl-λ⁶-sulfanone and 4-(4-(2-(dimethyl amino)ethyl)piperazin-1-yl)aniline were used as the starting material to obtain the product (45 mg). ¹H NMR (400 MHz, CDCl₃), 8.51 (s, 1H), 7.70 (t, *J* = 8.0 Hz, 1H), 7.35 (d, *J* = 8.8 Hz, 2H), 6.94 (d, *J* = 8.0 Hz, 1H), 6.90 (d, *J* = 7.6 Hz, 1H), 6.87 (s, 1H), 6.75 (d, *J* = 8.8 Hz, 2H), 6.25 (s, 1H), 3.18 (s, 6H), 3.06-3.12 (m, 4H), 2.50-2.66 (m, 8H), 2.31 (s, 6H), 1.27 (s, 9H).

### Example 164

### ((6-(6-(tert-butyl)-2-((4-(4-(4-methylpiperazin-1-yl)piperidin-1-yl)phenyl)amino)-7H-pyrrolo [2,3-d]pyrimidin-7-yl)pyridin-2-yl)imino)dimethyl-1-λ⁶-sulfanone

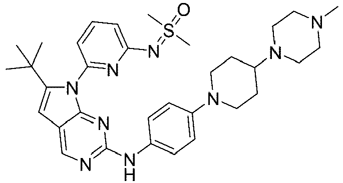

According to the method of step E of example 104, ((6-(6-(tert-butyl)-2-chloro-7*H*-pyrrolo [2,3-d]pyrimidin-7-yl)pyridin-2-yl)imino)dimethyl-λ⁶-sulfanone and 4-(4-(4-methylpiperazin-1-yl) piperidin-1-yl)aniline were used as the starting material to obtain the product (9 mg).

¹H NMR (400 MHz, CDCl₃), 8.50 (s, 1H), 7.69 (t, *J* = 8.0 Hz, 1H), 7.33 (d, *J* = 8.8 Hz, 2H), 6.98 (s, 1H), 6.93 (d, J = 8.4 Hz, 1H), 6.88 (d, J = 7.6 Hz, 1H), 6.75 (d, J = 8.8 Hz, 2H), 6.24 (s, 1H), 3.54-3.58 (m, 2H), 3.17 (s, 6H), 2.50-2.74 (m, 10H), 2.34-2.41 (m, 1H), 2.33 (s, 3H), 1.88-1.94 (m, 2H), 1.62-1.72 (m, 2H), 1.26 (s, 9H).

### Example 165

### ((6-(6-(tert-butyl)-2-((4-(9-methyl-3,9-diazaspiro[5.5]undecan-3-yl)phenyl)amino)-7H-pyrrol o[2,3-d]pyrimidin-7-yl)pyridin-2-yl)imino)dimethyl-1-λ⁶-sulfanone

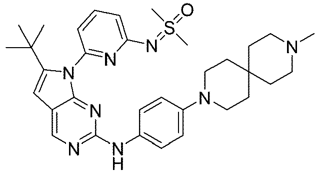

According to the method of step E of example 104, ((6-(6-(tert-butyl)-2-chloro-7*H*-pyrrolo [2,3-d]pyrimidin-7-yl)pyridin-2-yl)imino)dimethyl-λ⁶-sulfanone and 4-(9-methyl-3,9-diazaspiro [5.5]undecan-3-yl)aniline were used as the starting material to obtain the product (11 mg).

¹H NMR (400 MHz, CDCl₃), 8.51 (s, 1H), 7.71 (t, *J =* 8.0 Hz, 1H), 7.34 (d, *J =* 8.8 Hz, 2H), 6.95 (d, J = 8.0 Hz, 1H), 6.90 (d, J = 7.6 Hz, 1H), 6.82 (s, 1H), 6.77 (d, J = 8.8 Hz, 2H), 6.25 (s, 1H), 3.18 (s, 6H), 3.01-3.04 (m, 4H), 2.68-2.80 (m, 4H), 2.52 (s, 3H), 1.75-1.82 (m, 4H), 1.65-1.68 (m, 4H), 1.28 (s, 9H).

### Assay for bioactivity of compounds

### 1. Assay for WEE1 kinase inhibitory activity (IC₅₀) on compounds

The WEE1 kinase binding assay was established and optimized with LanthaScreen EU for the determination of the activity of compounds. 10 mM compounds were subjected to ten-fold gradient dilution in 100% DMSO (6 concentrations in total), 5 µL of diluent from each concentration was added to 45 µL of reaction buffer (50 mM HEPES (4-hydroxyethylpiperazine ethanesulfonic acid), pH 7.5, 10 mM MgCl₂ (magnesium chloride), 1 mM EGTA (ethylene glycol diethyl ether diamine tetraacetic acid), 0.01% Brij-35 (polyoxyethylene lauryl ether)) for blending. On this basis, 2 µL diluent from each concentration was added to 48 µL of reaction buffer for blending as a 4 ^{∗} compound (final concentrations of 1000, 100, 10, 1, 0.1, 0 nM) for later use. Reaction buffer was used to formulate 4 ^{∗} WEE1 kinase (purchased from Carna bioscience, a final concentration of 20 nM), 4^{∗}LanthaScreen EU-anti-GST Antibody (purchased from Invitrogen, PV5594, a final concentration of 2 nM), and 4 ^{∗} Kinase Tracer 178 (purchased from Invitrogen, PV5593, a final concentration of 100 nM). 2.5 µL of 4 ^{∗} compound was added to a 384-well plate (OptiPlate-384, purchased from PerkinElmer), followed by adding 2.5 µL of 4 ^{∗} WEE 1 kinase, standing for 5 min at room temperature. Then 2.5 µL of 4^{∗}LanthaScreen EU-anti-GST Antibody was added followed by 2.5 µL of 4 ^{∗} Kinase Tracer 178. The reaction was started by centrifugation (a total reaction volume of 10 µL). The 384-well plate was wrapped with tin foil paper and placed in an incubator at 23°C for 60 min. Fluorescence values (excitation at 320 nm, detecting emitted light at 665 nm and 615 nm, and a ratio of the two as enzyme activity) were read on Envision (purchased from PerkinElmer). The enzyme activity of each compound was measured at 6 concentrations, and the IC₅₀ value of the compound was calculated using GraFit6.0 software (Erithacus Software).

The aforementioned "^{∗}" refers to multiplying, representing multiples.

| Compound | WEE1 IC₅₀ (nM) | Compound | WEE1 IC₅₀ (nM) |
|---|---|---|---|
| Example 1 | 0.57 | Example 35 | 0.61 |
| Example 2 | 0.88 | Example 37 | 1.08 |
| Example 4 | 0.76 | Example 38 | 0.30 |
| Example 6 | 0.96 | Example 40 | 0.51 |
| Example 7 | 0.92 | Example 47 | 0.97 |
| Example 13 | 0.51 | Example 48 | 0.80 |
| Example 14 | 1.00 | Example 52 | 0.63 |
| Examples 27 | 0.57 | Example 54 | 1.04 |
| Example 28 | 1.30 | Example 55 | 0.98 |
| Example 32 | 0.69 | Example 63 | 0.85 |
| Compound | WEE1 IC₅₀ (nM) | Compound | WEE1 IC₅₀ (nM) |
| Example 80 | 1.18 | Example 127 | 0.66 |
| Example 81 | 0.33 | Example 135 | 0.68 |
| Example 82 | 0.58 | Example 136 | 0.90 |
| Example 85 | 0.85 | Example 139 | 0.69 |
| Example 86 | 0.54 | Example 141 | 0.84 |
| Example 87 | 0.98 | Example 145 | 0.60 |
| Example 98 | 0.79 | Example 147 | 0.91 |
| Example 101 | 0.70 | Example 150 | 0.22 |
| Example 108 | 0.61 | Example 151 | 0.60 |
| Example 109 | 1.12 | Example 152 | 0.20 |
| Example 110 | 0.69 | Example 154 | 0.53 |
| Example 123 | 0.30 | Example 155 | 1.02 |
| Example 124 | 1.03 | Example 159 | 0.59 |
| Example 126 | 0.48 | | |

### 2. Determination of cell proliferation activity of compounds:

NCI-H1299 cell proliferation inhibition screening method was established using CellTiter-Glo detection reagent from Promega.

Human non-small cell lung cancer cells NCI-H1299 were cultured in 25 cm² or 75 cm² plastic tissue culture flasks (Corning) filled with DMEM (Gibco) medium supplemented with 10% fetal bovine serum (Hyclone) under 37°C, 95% air, and 5% CO₂, and subcultured 2-3 times a week.

Cells were seeded at 1000 cells/well in a 96-well cell culture plate (Corning), 195 (µL/well. and cultured under 37°C, 95% air, and 5% CO₂. The next day test compound was added: 10 mM compounds (dissolved in DMSO) were subjected to 3-fold gradient dilution in DMSO, 2 µL of diluent from each concentration was added to 48 µL of serum-free medium, and finally 5 µL of the diluted compound was added to the plate seeded with cells. The final concentration of DMSO in the cell culture was 0.1% and the final concentration of the test compound was 0.3 nM-10 µM. The cells were cultured at 37°C for 3 days.

After 3 days, the cell viability assay was performed by CellTiter-Glo (Promega) kit and finally, the data was processed by software Prism to obtain the inhibitory concentration 50% of the compound on cell proliferation, i.e. the IC₅₀ value.

| Compound | NCI-H1975 IC₅₀ (nM) | Compound | NCI-H1975 IC₅₀ (nM) |
|---|---|---|---|
| Example 1 | 43 | Example 32 | 31 |
| Example 4 | 33 | Example 40 | 42 |
| Example 7 | 42 | Example 47 | 63 |
| Example 13 | 59 | Example 48 | 42 |
| Examples 27 | 60 | Example 52 | 56 |
| Example 28 | 40 | | |

| Compound | NCI-H1975 IC₅₀ (nM) | Compound | NCI-H1975 IC₅₀ (nM) |
|---|---|---|---|
| Example 81 | 38 | Example 135 | 74 |
| Example 82 | 64 | Example 136 | 87 |
| Example 85 | 79 | Example 139 | 64 |
| Example 86 | 60 | Example 145 | 67 |
| Example 87 | 90 | Example 147 | 85 |
| Example 98 | 84 | Example 150 | 32 |
| Example 101 | 66 | Example 151 | 66 |
| Example 108 | 70 | Example 152 | 23 |
| Example 110 | 88 | Example 154 | 69 |
| Example 123 | 44 | Example 155 | 89 |
| Example 126 | 53 | Example 159 | 54 |
| Example 127 | 70 | | |

### 3. Animal pharmacokinetic studies of compounds:

Animal pharmacokinetic experiments were conducted with 3 healthy adult male rats from Beijing Vital River Laboratory Animal Technology Co., Ltd. The compound was suspended in a 20% solution of sulfobutyl ether-β-cyclodextrin (W/W/V) at a concentration of 1 mg/mL, The rats were subjected to 5 mg/kg suspension of the compound by single intragastric administration with a dosing volume of 5 mL/kg. Animals were fasted overnight prior to the experiment from 10 h before dosing to 4 h after dosing. Blood was collected 0.25, 0.5, 1, 2, 4, 6, 8 and 24 h after administration. Animals were lightly anesthetized with isoflurane, and approximately 0.4 mL of whole blood was collected from the orbital venous plexus with a glass blood collection tube, placed in a heparin anticoagulant tube, centrifuged at 4200 rpm for 5 min at 4°C, plasma transferred to the centrifuge tube, and stored at -80°C until analysis. Test compounds extracted from rat plasma using acetonitrile protein precipitation and internal standard (Warfarin or Propranolol) were used for Plasma sample analysis, and the extracts were analyzed by LC/MS/MS. The measured plasma concentration-time data for individual animals were analyzed using non-compartmental model of WinNonlin (version 5.2.1; Pharsight) software to obtain the following pharmacokinetic parameters: maximum (peak) plasma drug concentration Cₘₐₓ; a peak time Tₘₐₓ; half-life T_{1/2} and the area under the plasma concentration-time curve from time 0 extrapolated to infinite time AUC_{0-inf}.

| Compound | AUC0-inf (hr^{∗}ng/mL) | T1/2 (hr) | Tmax (hr) | Cmax (ng/mL) |
|---|---|---|---|---|
| Example 56 | 1424 | 7.6 | 4.0 | 116 |
| Example 57 | 1084 | 11.2 | 2.3 | 68 |
| Example 58 | 1751 | 10.9 | 6.0 | 83 |
| Example 59 | 4581 | 33.4 | 6.7 | 117 |
| Example 77 | 2342 | 13.6 | 5.33 | 98 |
| Compound | AUC_{0-inf} (hr^{∗}ng/mL) | T_{1/2} (hr) | Tₘₐₓ (hr) | Cₘₐₓ (ng/mL) |
| Example 124 | 4066 | 19.2 | 4.8 | 163 |
| Example 148 | 1102 | 9.2 | 5.4 | 64 |

### Industrial Applicability

The present invention provides a WEE1 inhibitor and preparation and use thereof. The WEE1 inhibitor of the present invention is a compound of Formula 1 or Formula 11, or a pharmaceutically acceptable salt, solvate, polymorph, or tautomer thereof. The present invention also provides use of the WEE1 inhibitor in the preparation of a drug for treating diseases related to WEE1 activity. The WEE1 inhibitor provided by the present invention can be used for effectively treating diseases related to WEE1 activity, and has good economic value and application prospects.

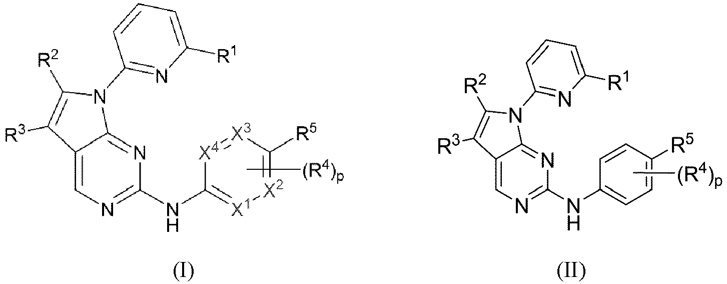

## Claims

1. A compound of Formula 1, or a pharmaceutically acceptable salt, solvate, polymorph or tautomer thereof, 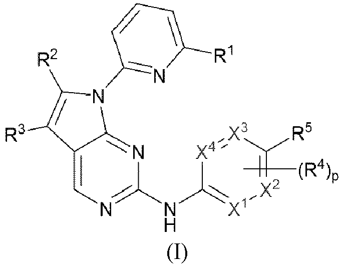 wherein
X¹, X², X³ and X⁴ are independently selected from N and C-R⁴;
R¹ is selected from C₁₋₆alkyl, -O-C₁₋₆alkyl, 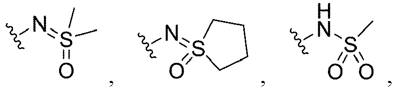 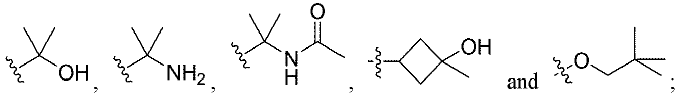
R² is selected from H, C₁₋₆alkyl, C₃₋₈cycloalkyl, C₂₋₆alkenyl, allyl, -(CH₂)ₙ-OH and -(CH₂)ₙ-O-C₁₋₆alkyl;
R³ is selected from H, halogen and C₁₋₆alkyl;
R⁴ is selected from H, halogen, CF₃, C₁₋₆alkyl and -O-C₁₋₆alkyl;
R⁵ is selected from -(CH₂)ₘ-NR^{a}R^{b}, -(CH₂)-(CO)-N(CH₃)₂ and 3-10 membered heterocycle, said 3-10 membered heterocycle can be optionally substituted with 1-3 R⁶; or
R⁴ and R⁵ joins together to form a 3-8 membered heterocycle, said 3-8 membered heterocycle can be optionally substituted with C₁₋₆alkyl;
R⁶ is selected from C₁₋₆alkyl, C₃₋₈cycloalkyl, -(CO)-NH₂, -CH₂(CO)OH, -CH₂(CO)OCH₃, -CH₂CN, -CH2(CO)NHOH, -(CH₂)₂-NR^{c}R^{d}, -NR^{c}R^{d}, -(CH₂)ₙ-OH, -(CO)-O-C₁₋₆alkyl, and 3-8 membered heterocycle, said 3-8 membered heterocycle can be optionally substituted with C₁₋₆alkyl or C₃₋₈cycloalkyl;
R^{a} and R^{b} are independently selected from H and C₁₋₆alkyl, said alkyl can be optionally substituted with -NR^{c}R^{d}_{;}
R^{c} and R^{d} are independently selected from H and C₁₋₆alkyl;
m is 0, 1, 2,3, or 4;
n is independently 1, 2,3, or 4;
p is 1 or 2.

2. The compound according to claim 1, wherein R¹ is selected from 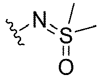 and 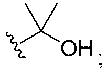
Preferably, R² is selected from C₁₋₆alkyl and C₃₋₈cycloalkyl;
Preferably, R³ is selected from H and halogen ;
Preferably, R⁴ is selected from H and halogen ;
Preferably, R⁵ is selected from 3-10 membered heterocycle, said 3-10 membered heterocycle can be optionally substituted with 1-3 R⁶, and
R⁶ is selected from 3-8 membered heterocycle, and R⁶ can be optionally substituted with C₁₋₆alkyl.

3. The compound according to claim 1, wherein said compound is selected from the following: 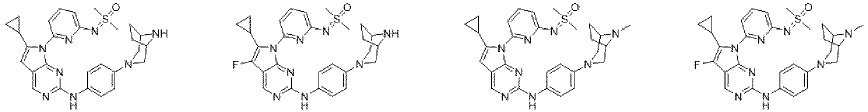 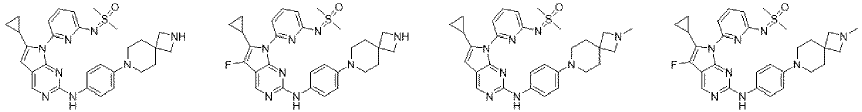 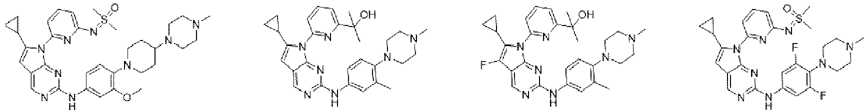 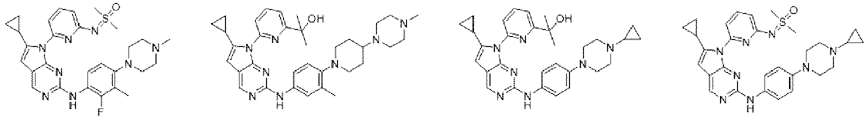 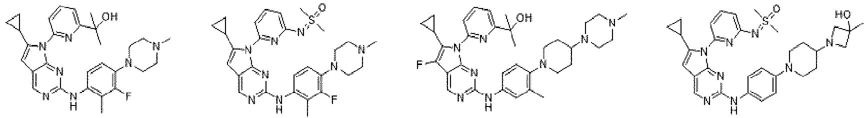 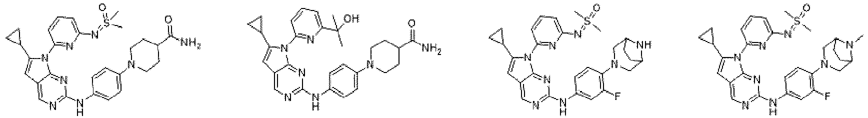 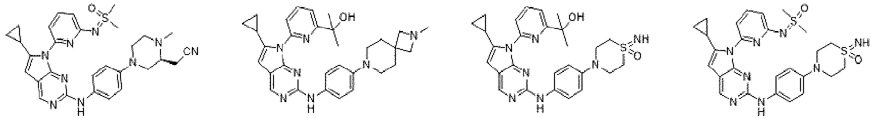 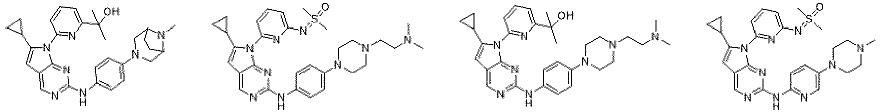 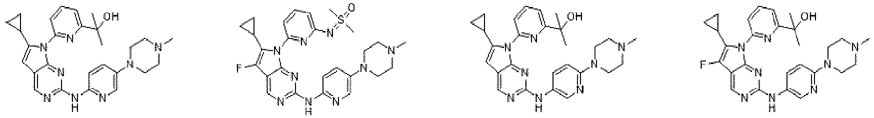 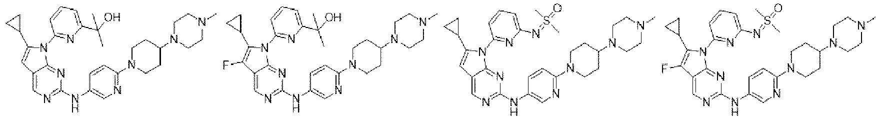 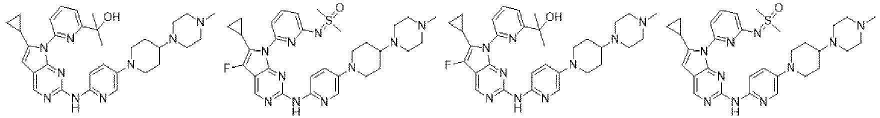 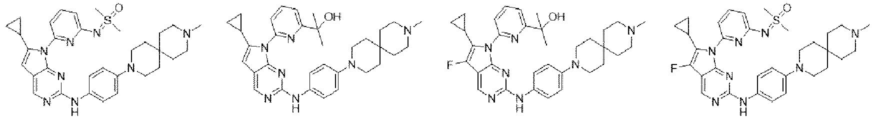 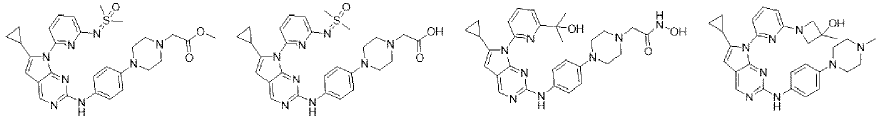 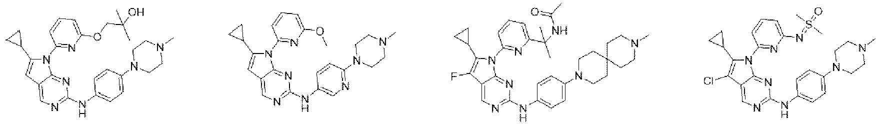 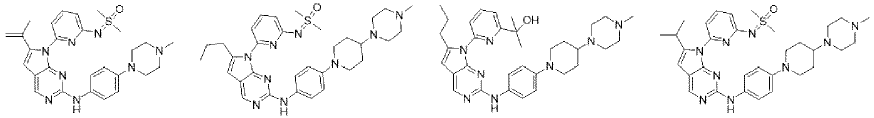 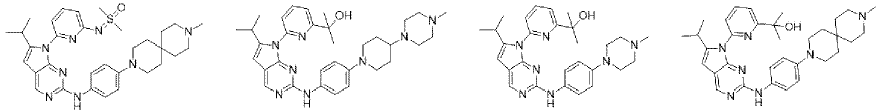 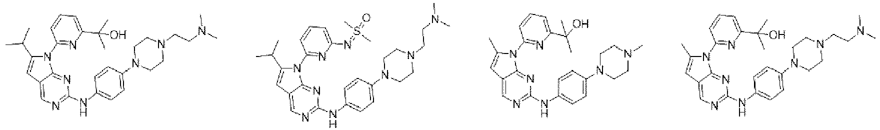 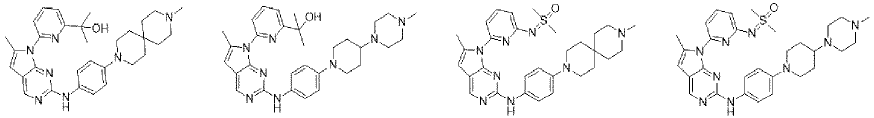 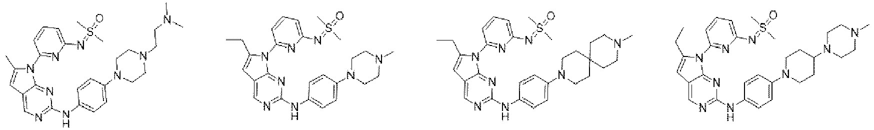 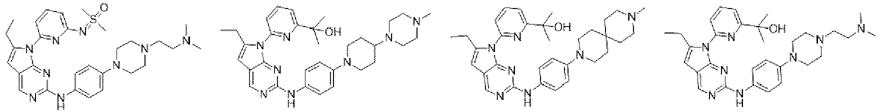 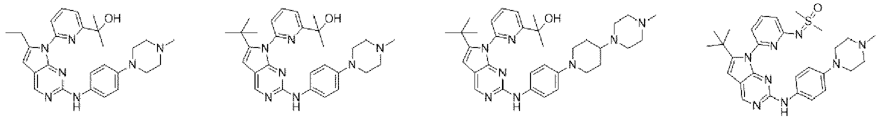 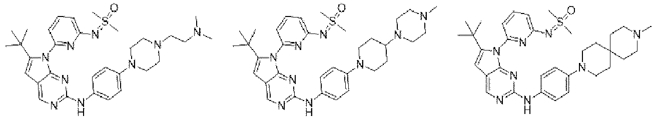 or a pharmaceutically acceptable salt, solvate, polymorph or tautomer thereof.

4. A compound of Formula 11, or a pharmaceutically acceptable salt, solvate, polymorph or tautomer thereof, 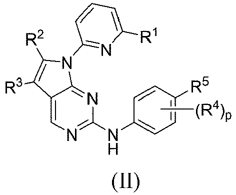 wherein
R¹ is selected from C₁₋₆alkyl, 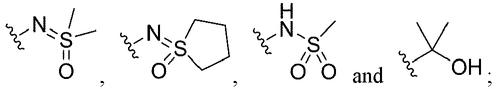
R² is selected from H, C₁₋₆alkyl, C₃₋₈cycloalkyl, allyl, -(CH₂)ₙ-OH and -(CH₂)ₙ-O-C₁₋₆alkyl;
R³ is selected from H, halogen and C₁₋₆alkyl;
R⁴ is selected from H, halogen, CF₃, C₁₋₆alkyl and -O-C₁₋₆alkyl;
R⁵ is selected from -(CH₂)ₘ-NR^{a}R^{b}, -(CH₂)-(CO)-N(CH₃)₂ and 3-8 membered heterocycle, said 3-8 membered heterocycle can be optionally substituted with 1-3 R⁶; or
R⁴ and R⁵ joins together to form a 3-8 membered heterocycle, said 3-8 membered heterocycle can be optionally substituted with C₁₋₆alkyl;
R⁶ is selected from C₁₋₆alkyl, C₃₋₈cycloalkyl, -NR^{c}R^{d}, -(CH₂)ₙ-OH, -(CO)-O-C₁₋₆alkyl, and 3-8 membered heterocycle, said 3-8 membered heterocycle can be optionally substituted with C₁₋₆alkyl or C₃₋₈cycloalkyl;
R^{a} and R^{b} are independently selected from H and C₁₋₆alkyl, said alkyl can be optionally substituted with -NR^{c}R^{d}_{;}
R^{c} and R^{d} are independently selected from H and C₁₋₆alkyl;
m is 0, 1, 2,3, or 4;
n is independently 1, 2,3, or 4;
p is 1 or 2.

5. The compound according to claim 4, wherein R¹ is selected from 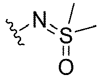 and 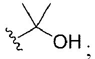
Preferably, R² is selected from C₁₋₆alkyl and C₃₋₈cycloalkyl;
Preferably, R³ is selected from H and halogen ;
Preferably, R⁴ is selected from H and halogen ;
Preferably, R⁵ is selected from 3-8 membered heterocycle, said 3-8 membered heterocycle can be optionally substituted with 1-3 R⁶, and
R⁶ is selected from 3-8 membered heterocycle, and R⁶ can be optionally substituted with C₁₋₆alkyl.

6. The compound according to claim 4, wherein said compound is selected from the following: 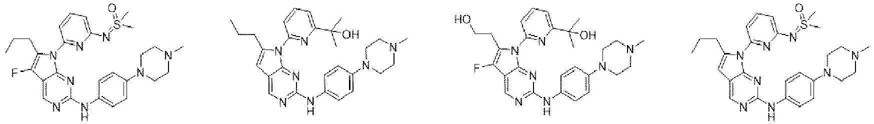 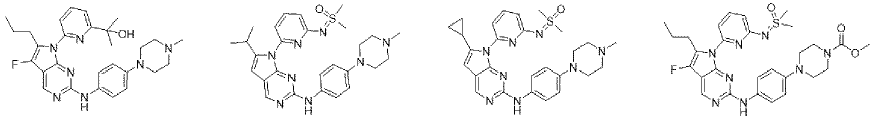 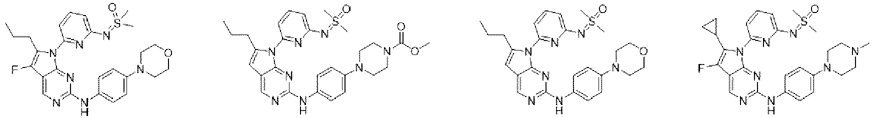 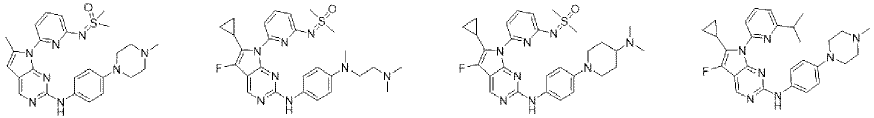 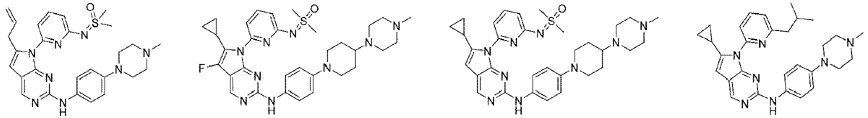 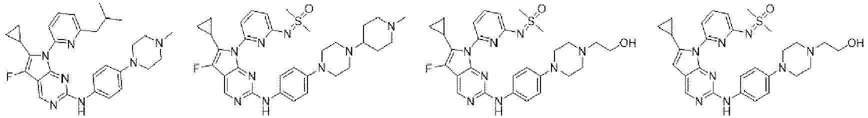 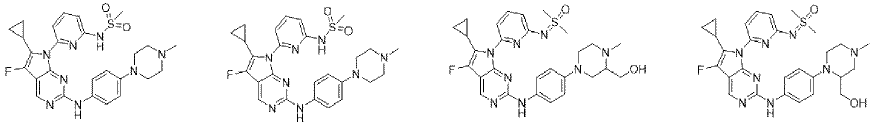 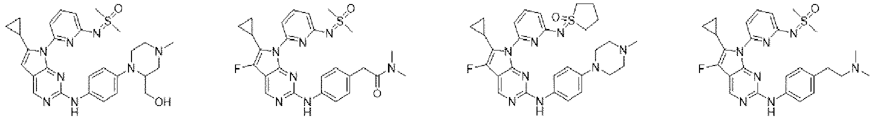 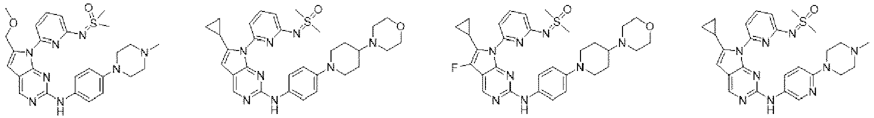 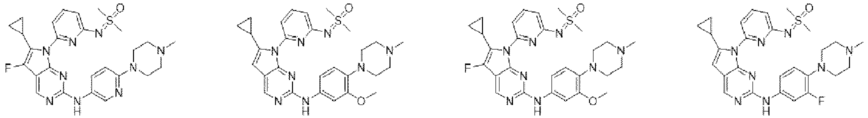 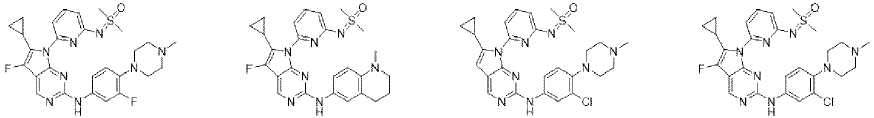 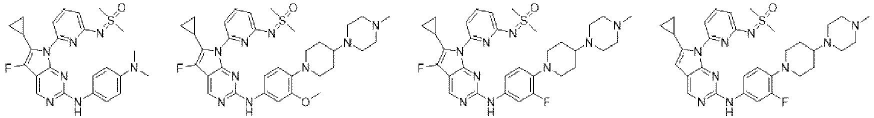 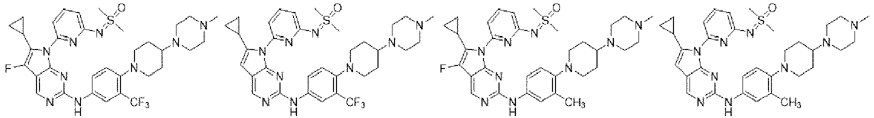 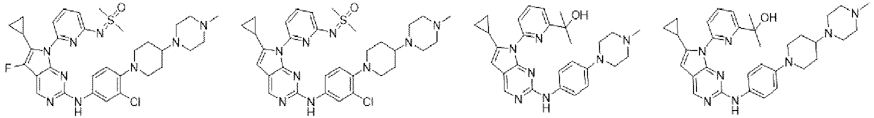 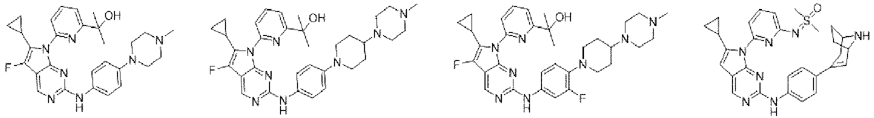 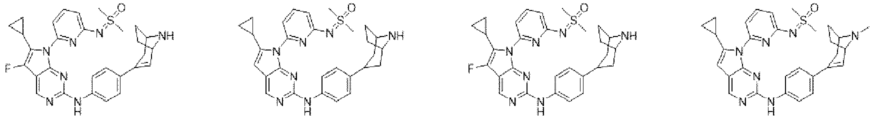 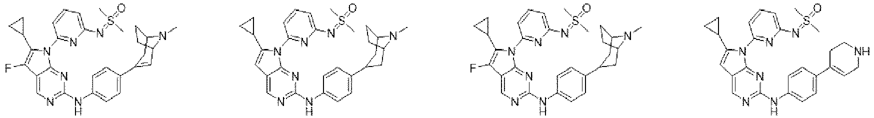 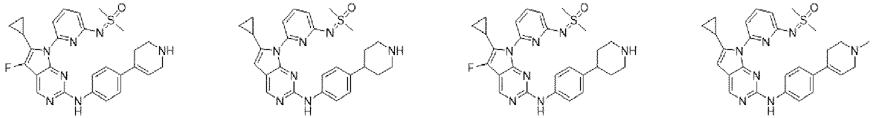 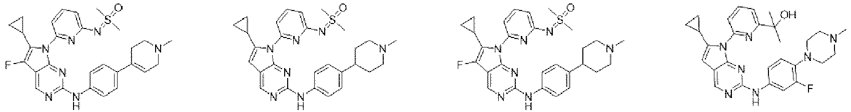 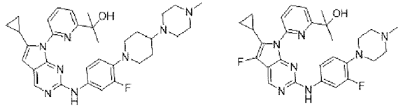 or a pharmaceutically acceptable salt, solvate, polymorph or tautomer thereof.

7. A pharmaceutical composition comprising the compound according to anyone of claims 1-6, or a pharmaceutically acceptable salt, solvate, polymorph or tautomer thereof, and a pharmaceutically acceptable carrier.

8. Use of the compound according to anyone of claims 1-6 or a pharmaceutically acceptable salt, solvate, polymorph or tautomer thereof and the composition according to claim 7 in the manufacture of a medicament for the treatment of a disease associated with WEE1 activity.

9. The use according to claim 8, wherein the disease associated with WEE1 activity is liver cancer, breast cancer, glioblastoma, melanoma, adult brain tumor, pediatric brain tumor, ovarian cancer, colon cancer, cervical cancer, osteosarcoma, lung cancer, gastric cancer, head and neck cancer, or leukemia.
